# EUROPEAN PATENT APPLICATION

(11) **EP 3 584 325 A1**
(43) Date of publication of application: **25.12.2019**
(21) Application number: 19174921.7
(22) Date of filing: 03.10.2008
(51) Int. Cl.: C12Q 1/68, C12N 5/04, A01H 1/00, C12N 9/88, C12N 15/82

(54) **MUTATED ACETOHYDROXYACID SYNTHASE GENES IN BRASSICA**

(30) Priority: 05.10.2007 US 97794407 P
(62) Divisional of application: 13175393.1
(71) Applicant: Cibus Europe B.V., 4421 AD Kapelle (NL)
(72) Inventor: SCHOPKE, Christian, San Diego, CA 92126 (US); GOCAL, Gregory F.W., San Diego, CA 92129 (US); WALKER, Keith A., San Diego, CA 92130 (US); BEETHAM, Peter R., Carlsbad, CA 92011 (US)
(74) Representative: Zimmermann & Partner Patentanwälte mbB

(57) **Abstract**

The invention relates to mutated acetohydroxyacid synthase (AHAS) nucleic acids, expression vectors comprising the mutated nucleic acids, and proteins encoded by the mutated nucleic acids. The invention further relates to plants and seeds, respectively, which comprise an acetohydroxyacid synthase (AHAS) gene, wherein said gene encodes a mutated AHAS protein. Further provided is a method for producing an herbicide-resistant plant, a method for increasing the herbicide-resistance of a plant, and a method of controlling weeds in a field.

## Description

### CROSS-REFERENCE TO RELATED PATENT APPLICATIONS

This application claims the benefit of U.S. Prov. App. No. 60/977,944, filed October 5, 2007, which is incorporated herein by reference in its entirety including specification, figures, and tables, and for all purposes.

### FIELD OF THE INVENTION

This invention relates to the field of herbicide resistant plants and seeds and more specifically to mutations in the acetohydroxyacid synthase (AHAS) gene and protein.

### BACKGROUND OF THE INVENTION

The following description is provided simply as an aid in understanding the invention and is not admitted to describe or constitute prior art to the invention.

Benefits of herbicide-tolerant plants are known. For example, herbicide-tolerant plants may reduce the need for tillage to control weeds thereby effectively reducing soil erosion.

The introduction of exogenous mutant genes into plants is well documented. For example, U.S. Pat. No. 4,545,060 relates to increasing a plant's resistance to glyphosate by introducing into the plant's genome a gene coding for an EPSPS variant having at least one mutation that renders the enzyme more resistant to its competitive inhibitor, i.e., glyphosate.

Examples of some of the mutations in AHAS genes are known. *See e.g.* U.S. Patent No. 7,094,606.

Through chemical mutagenesis, a mutation was found in the lower expressed AHAS I gene. This mutation is known as PM-1 (a mutation at an equivalent position known as 653 based on the acetolactate synthase (ALS) amino acid sequence of *Arabidopsis,* a serine to asparagine amino acid change, respectively encoded as follows - A**G**T to A**A**T). Another mutation was found in the higher expressed gene AHAS III, known as PM-2 (a mutation at an equivalent position known as 574, a tryptophan to leucine amino acid change, respectively encoded as follows - TGG to TTG). These two mutations, PM-1 and PM-2, are combined in a commercial variety of Canola known as Clearfield Canola (Tan *et al.,* 2005).

### SUMMARY OF THE INVENTION

The invention relates in part to mutated acetohydroxyacid synthase (AHAS) nucleic acids and the proteins encoded by the mutated nucleic acids. The invention also relates in part to canola plants, cells, and seeds comprising these mutated nucleic acids and proteins.

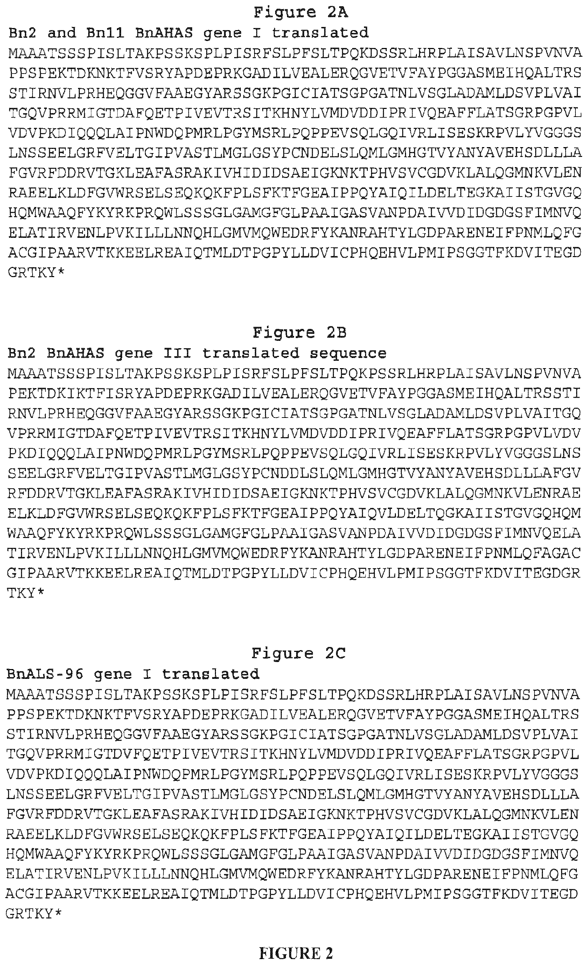

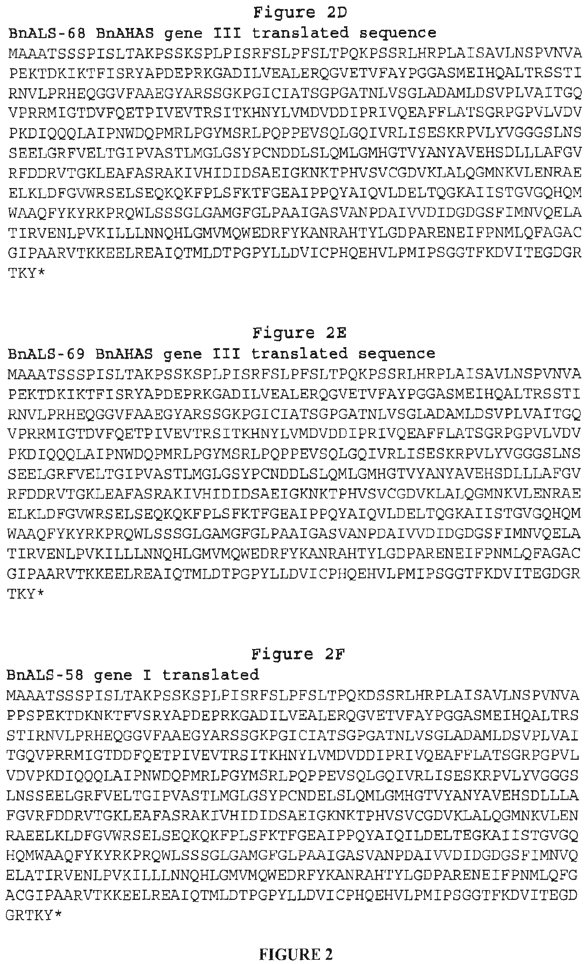

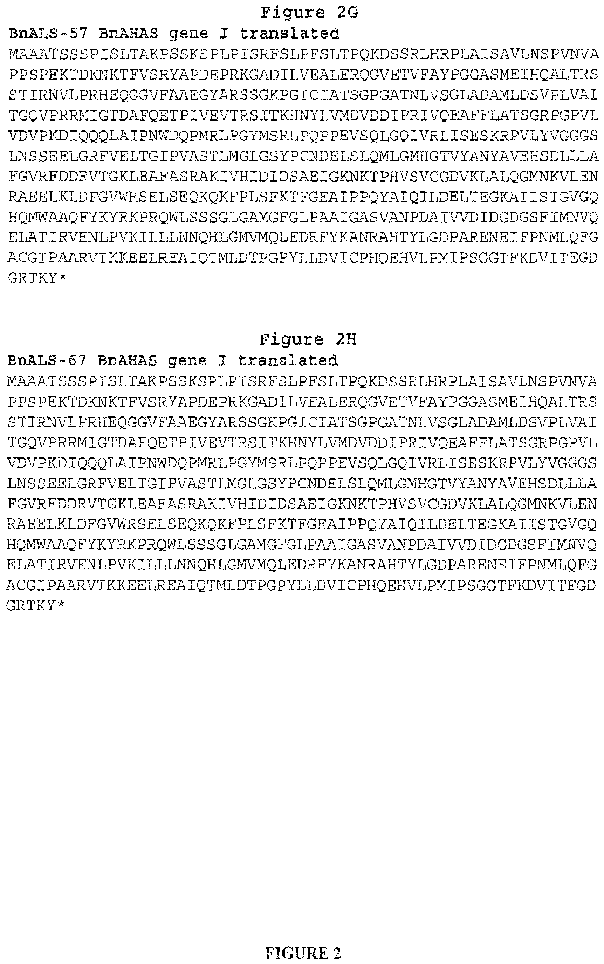

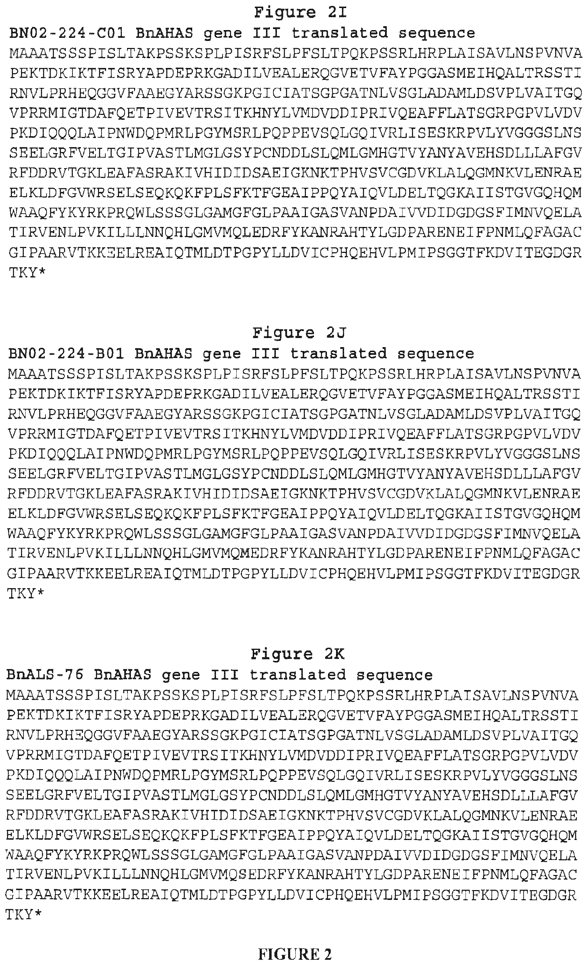

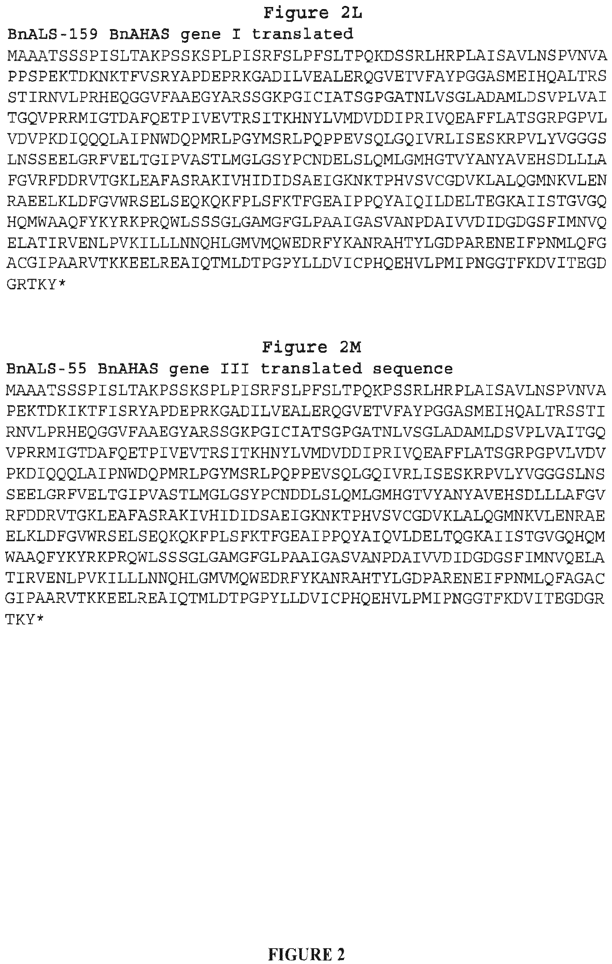

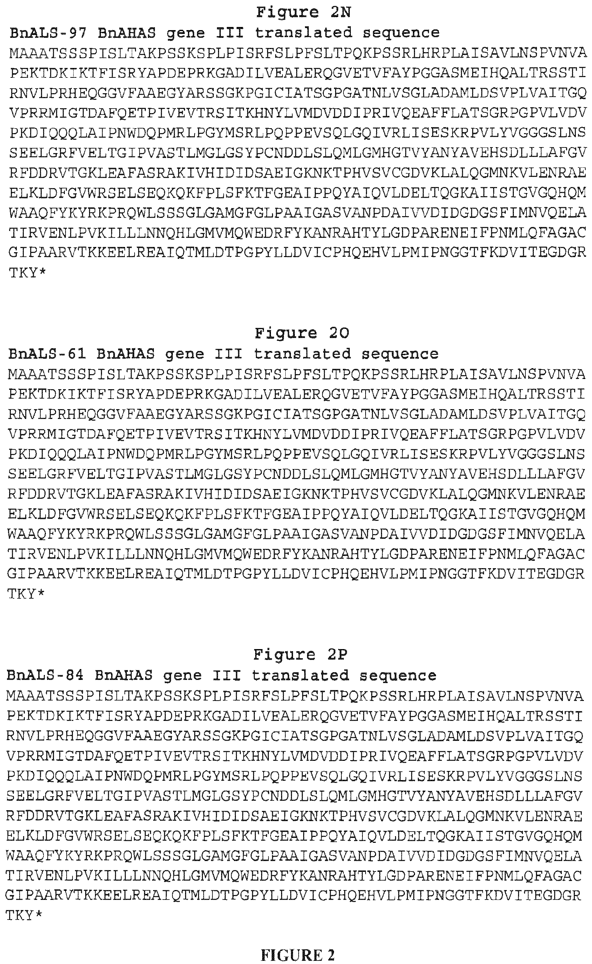

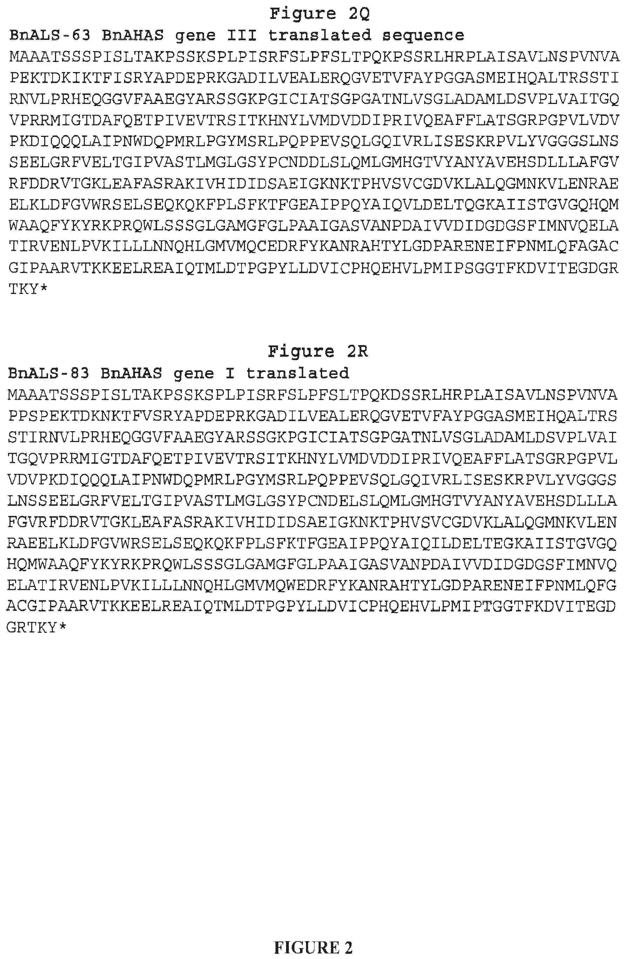

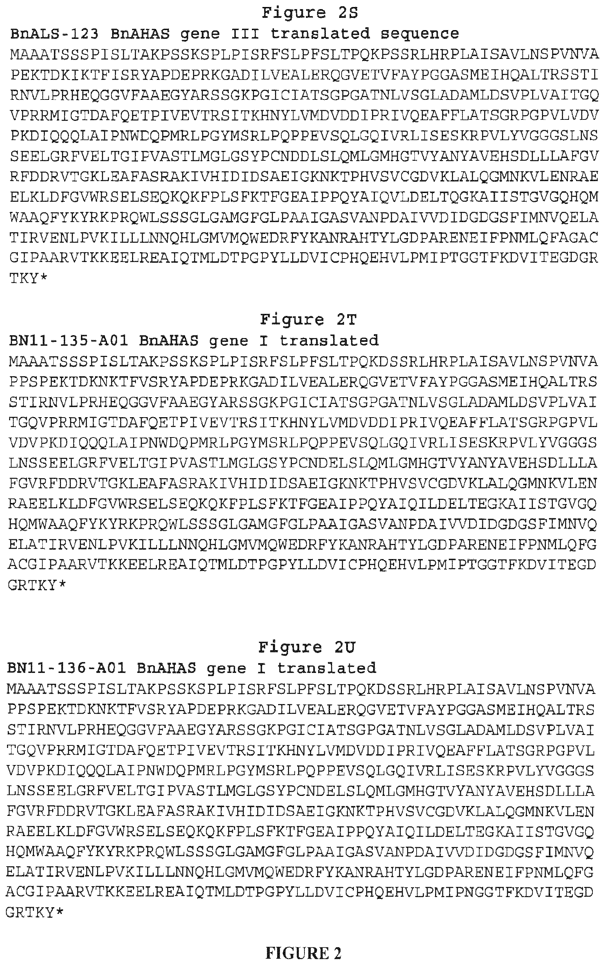

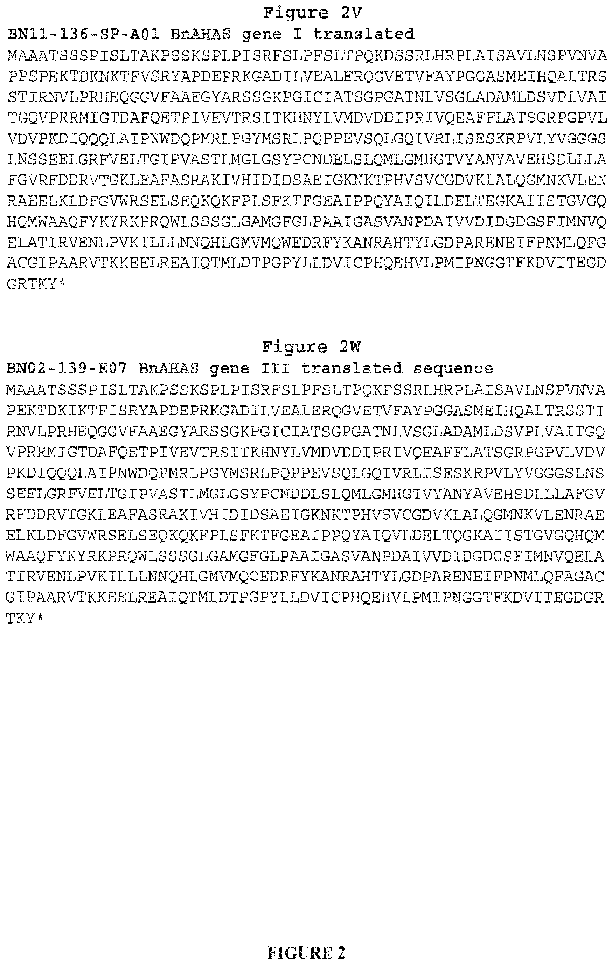

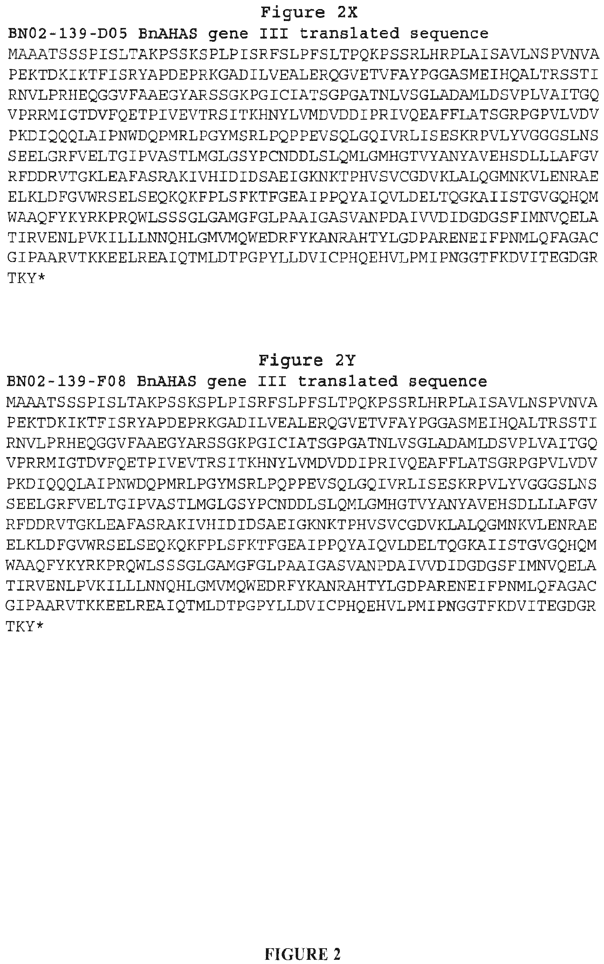

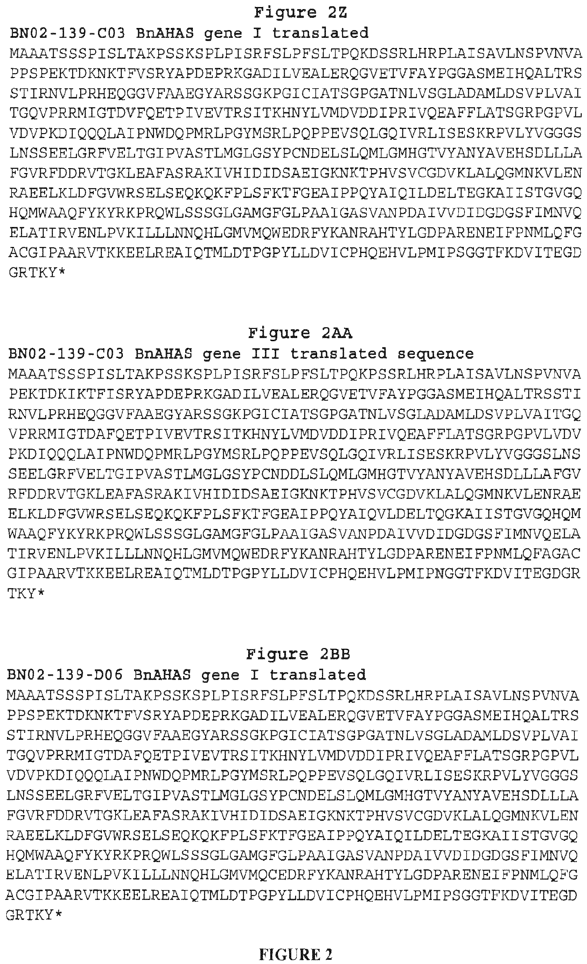

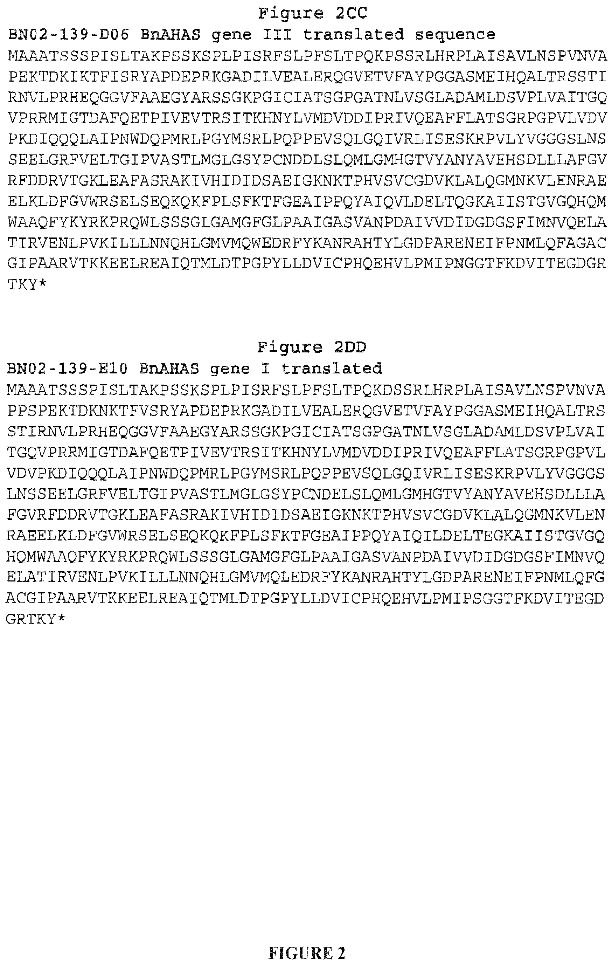

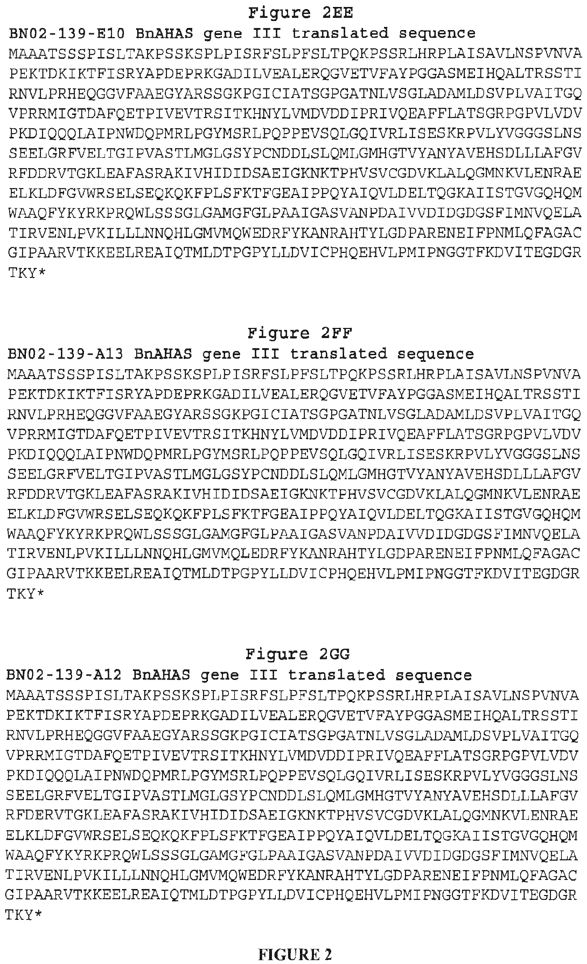

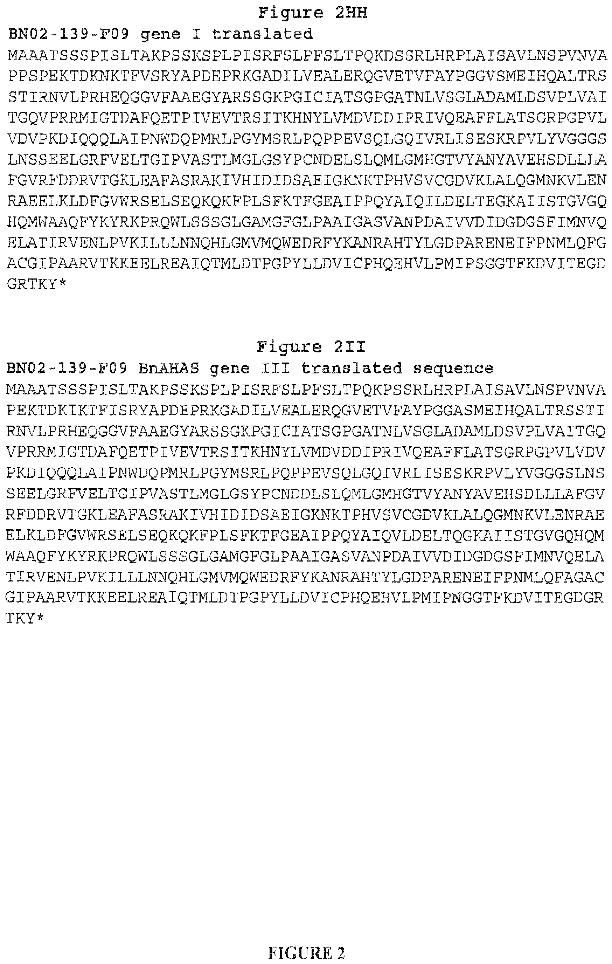

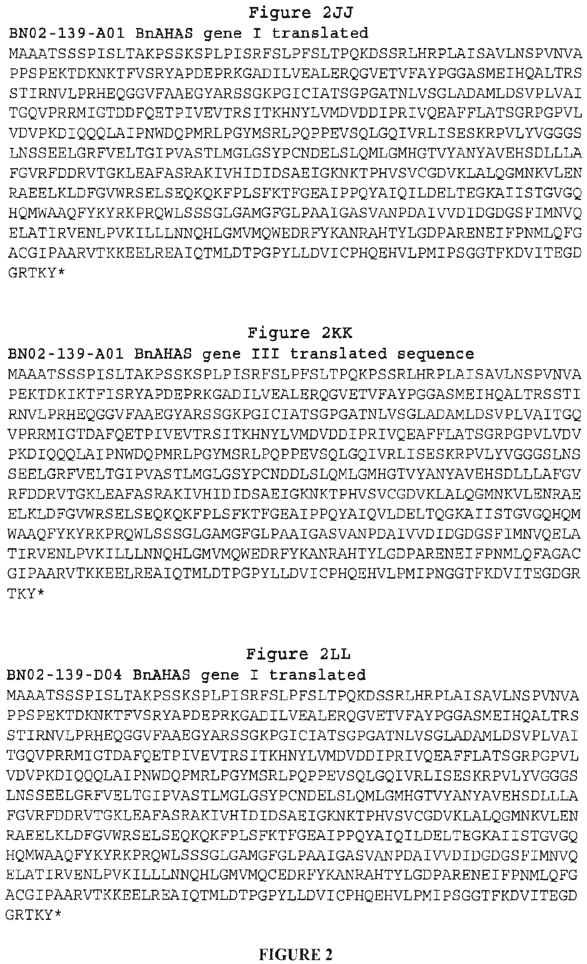

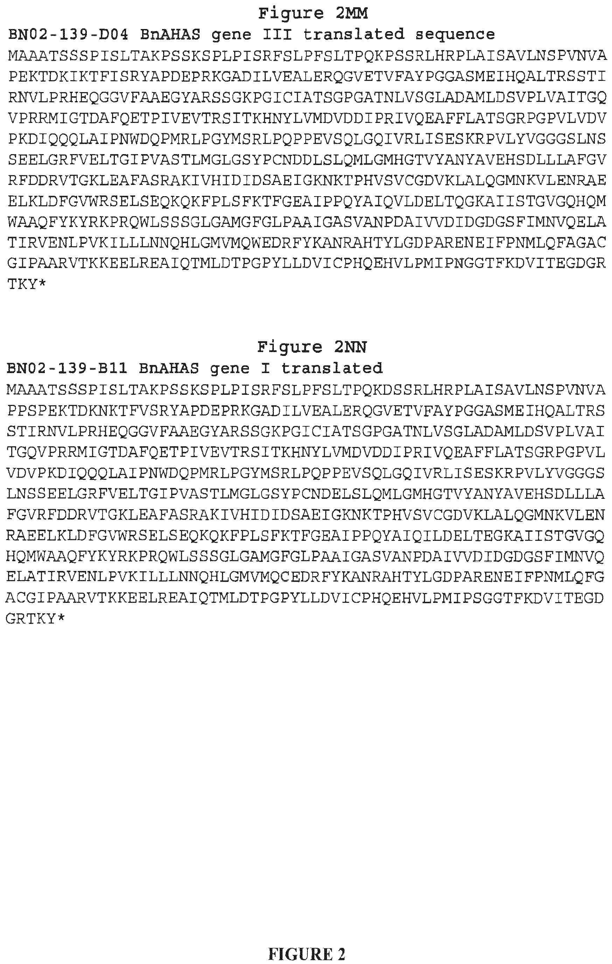

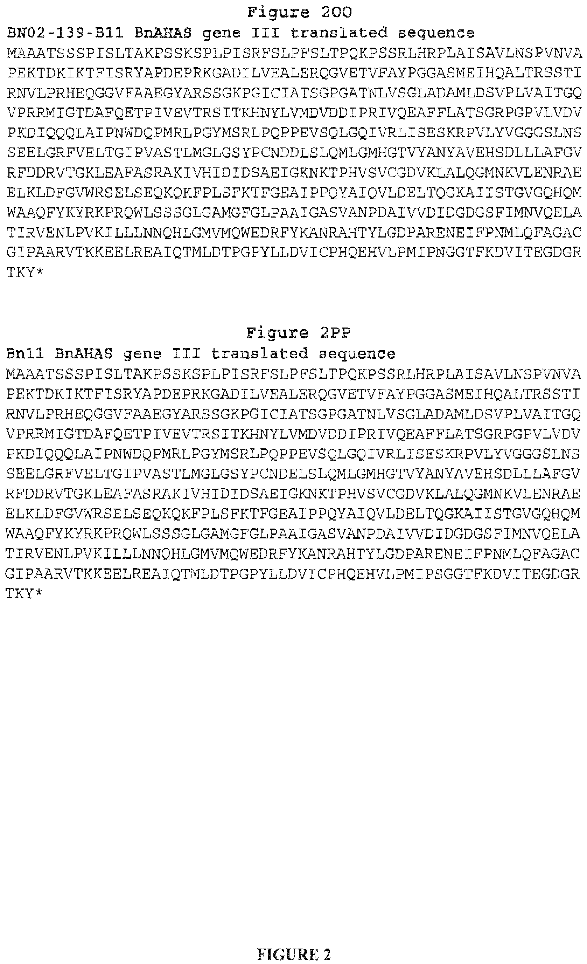

In one aspect, there is provided an isolated nucleic acid encoding a *Brassica* acetohydroxyacid synthase protein having a mutation at one or more amino acid positions corresponding to a position selected from the group consisting of: A205, D376, W574, R577, and S653 of SEQ ID NO: 1. In some embodiments, the isolated nucleic acid encodes a protein having one or more mutations selected from the group consisting of: an alanine to valine substitution at a position corresponding to position 205 of SEQ ID NO: 1, an alanine to aspartic acid substitution at a position corresponding to position 205 of SEQ ID NO: 1, an aspartic acid to glutamic acid substitution at a position corresponding to position 376 of SEQ ID NO: 1, a tryptophan to cysteine substitution at a position corresponding to position 574 of SEQ ID NO: 1, a tryptophan to leucine substitution at a position corresponding to position 574 of SEQ ID NO: 1, a tryptophan to methionine substitution at a position corresponding to position 574 of SEQ ID NO: 1, a tryptophan to serine substitution at a position corresponding to position 574 of SEQ ID NO: 1, an arginine to tryptophan substitution at a position corresponding to position 577 of SEQ ID NO: 1, a serine to asparagine at a position corresponding to position 653 of SEQ ID NO: 1, and a serine to threonine at a position corresponding to position 653 of SEQ ID NO: 1. In some embodiments, the mutation is a serine to asparagine substitution at a position corresponding to position 653 of SEQ ID NO: 1 in which the mutation is not S653N in *Brassica* AHAS I gene. 4. In some embodiments, the mutation is a serine to threonine substitution at a position corresponding to position 653 of SEQ ID NO: 1. In some embodiments, the mutation is a tryptophan to leucine substitution at a position corresponding to position 574 of SEQ ID NO: 1, in which the mutation is not W574L in *Brassica* AHAS III gene. In some embodiments, the mutation is an alanine to valine substitution at a position corresponding to position 205 of SEQ ID NO: 1. In other embodiments the mutation is an alanine to aspartic acid substitution at a position corresponding to position 205 of SEQ ID NO: 1. In other embodiments, the isolated nucleic acid encodes a protein having a mutation selected from the mutations shown in Table 2. In certain embodiments, the isolated nucleic acid encodes a protein having two or more mutations. In some embodiments, the two or more mutations are selected from Table 2. In some embodiments, the isolated nucleic acid encodes a protein having a mutation at a position corresponding to S653 of SEQ ID NO: 1 and a mutation at one or more amino acid positions corresponding to a position selected from the group consisting of: A205, D376, W574, and R577 of SEQ ID NO: 1. In other embodiments, the isolated nucleic acid encodes a protein having a mutation at a position corresponding to W574 of SEQ ID NO: 1 and a mutation at a position corresponding to R577 of SEQ ID NO: 1. In some embodiments, the isolated nucleic acid encodes an acetohydroxyacid synthase (AHAS) protein that is resistant to inhibition by an AHAS-inhibiting herbicide. In some embodiments, the AHAS-inhibiting herbicide is selected from the group consisting of herbicides of: imidazolinone, sulfonylurea, triazolopyrimidine, pyrimidinylthiobenzoate, sulfonylamino-carbonyltriazolinone, and mixtures thereof. In some embodiments, the herbicide is an imidazolinone herbicide. In some embodiments, the herbicide is a sulfonylurea herbicide. In some embodiments the the isolated nucleic acid encodes an AHAS protein comprising 70% or more identity to one or more of the amino acid sequences in Figure 2. In some embodiments, the isolated nucleic acid encodes a *Brassica napus* AHAS protein. In other embodiments, the isolated nucleic acid encodes a *Brassica napus* AHAS I protein. In certain embodiments, the isolated nucleic acid encodes a *Brassica napus* AHAS III protein.

In another aspect, there is provided an expression vector containing an isolated nucleic acid encoding a *Brassica* acetohydroxyacid synthase protein having a mutation at one or more amino acid positions corresponding to a position selected from the group consisting of: A205, D376, W574, R577, and S653 of SEQ ID NO: 1. In some embodiments, the expression vector contains an isolated nucleic acid encoding a protein having one or more mutations selected from the group consisting of: an alanine to valine substitution at a position corresponding to position 205 of SEQ ID NO: 1, an alanine to aspartic acid substitution at a position corresponding to position 205 of SEQ ID NO: 1, an aspartic acid to glutamic acid substitution at a position corresponding to position 376 of SEQ ID NO: 1, a tryptophan to cysteine substitution at a position corresponding to position 574 of SEQ ID NO: 1, a tryptophan to leucine substitution at a position corresponding to position 574 of SEQ ID NO: 1, a tryptophan to methionine substitution at a position corresponding to position 574 of SEQ ID NO: 1, a tryptophan to serine substitution at a position corresponding to position 574 of SEQ ID NO: 1, an arginine to tryptophan substitution at a position corresponding to position 577 of SEQ ID NO: 1, a serine to asparagine at a position corresponding to position 653 of SEQ ID NO: 1, and a serine to threonine at a position corresponding to position 653 of SEQ ID NO: 1. In some embodiments, the mutation is a serine to asparagine substitution at a position corresponding to position 653 of SEQ ID NO: 1 in which the mutation is not S653N in *Brassica* AHAS I gene. 4. In some embodiments, the mutation is a serine to threonine substitution at a position corresponding to position 653 of SEQ ID NO: 1. In some embodiments, the mutation is a tryptophan to leucine substitution at a position corresponding to position 574 of SEQ ID NO: 1, in which the mutation is not W574L in *Brassica* AHAS III gene. In some embodiments, the mutation is an alanine to valine substitution at a position corresponding to position 205 of SEQ ID NO: 1. In other embodiments the mutation is an alanine to aspartic acid substitution at a position corresponding to position 205 of SEQ ID NO: 1.

In another aspect, there is provided a plant having a *Brassica* acetohydroxyacid synthase (AHAS) gene, in which the gene encodes a protein having a mutation at one or more amino acid positions corresponding to a position selected from the group consisting of: A205, D376, W574, R577, and S653 of SEQ ID NO: 1. In another aspect, there is provided a plant having a *Brassica* acetohydroxyacid synthase (AHAS) gene, in which the plant is resistant to an AHAS-inhibiting herbicide, in which the gene encodes a protein having a mutation at one or more amino acid positions corresponding to a position selected from the group consisting of:
A205, D376, W574, R577, and S653 of SEQ ID NO: 1. In some embodiments of the above aspects, the plant has an AHAS gene which encodes a protein having one or more mutations selected from the group consisting of: an alanine to valine substitution at a position corresponding to position 205 of SEQ ID NO: 1, an alanine to aspartic acid substitution at a position corresponding to position 205 of SEQ ID NO: 1, an aspartic acid to glutamic acid substitution at a position corresponding to position 376 of SEQ ID NO: 1, a tryptophan to cysteine substitution at a position corresponding to position 574 of SEQ ID NO: 1, a tryptophan to leucine substitution at a position corresponding to position 574 of SEQ ID NO: 1, a tryptophan to methionine substitution at a position corresponding to position 574 of SEQ ID NO: 1, a tryptophan to serine substitution at a position corresponding to position 574 of SEQ ID NO: 1, an arginine to tryptophan substitution at a position corresponding to position 577 of SEQ ID NO: 1, a serine to asparagine at a position corresponding to position 653 of SEQ ID NO: 1, and a serine to threonine at a position corresponding to position 653 of SEQ ID NO: 1. In some embodiments, the mutation is a serine to asparagine substitution at a position corresponding to position 653 of SEQ ID NO: 1 in which the mutation is not S653N in *Brassica* AHAS I gene. 4. In some embodiments, the mutation is a serine to threonine substitution at a position corresponding to position 653 of SEQ ID NO: 1. In some embodiments, the mutation is a tryptophan to leucine substitution at a position corresponding to position 574 of SEQ ID NO: 1, in which the mutation is not W574L in *Brassica* AHAS III gene. In some embodiments, the mutation is an alanine to valine substitution at a position corresponding to position 205 of SEQ ID NO: 1. In other embodiments the mutation is an alanine to aspartic acid substitution at a position corresponding to position 205 of SEQ ID NO: 1. In other embodiments, the mutation selected from the mutations shown in Table 2. In certain embodiments, the plant has an AHAS gene which encodes a protein having two or more mutations. In some embodiments, the two or more mutations are selected from Table 2. In some embodiments, the plant has an AHAS gene which encodes a protein having a mutation at a position corresponding to S653 of SEQ ID NO: 1 and a mutation at one or more amino acid positions corresponding to a position selected from the group consisting of: A205, D376, W574, and R577 of SEQ ID NO: 1. In other embodiments, the plant has an AHAS gene which encodes a protein having a mutation at a position corresponding to W574 of SEQ ID NO: 1 and a mutation at a position corresponding to R577 of SEQ ID NO: 1. In some embodiments, the plant has an AHAS gene which encodes a protein that is resistant to inhibition by an AHAS-inhibiting herbicide. In some embodiments the plant has an AHAS gene which encodes a protein comprising 70% or more identity to one or more of the amino acid sequences in Figure 2. In some embodiments, the plant is resistant to the application of at least one AHAS-inhibiting herbicide. In some embodiments, the AHAS-inhibiting herbicide is selected from the group consisting of herbicides of: imidazolinone, sulfonylurea, triazolopyrimidine, pyrimidinylthiobenzoate, sulfonylamino-carbonyltriazolinone, and mixtures thereof. In some embodiments, the herbicide is an imidazolinone herbicide. In some embodiments, the herbicide is a sulfonylurea herbicide. In some embodiments, the plant is a *Brassica* plant produced by growing a seed of a line selected from the lines listed in Table 2. In some embodiments, the plant is a *Brassica* species. In other embodiments, the plant is *Brassica napus.* In some embodiments, the plant is selected from Spring Oilseed Rape and Winter Oilseed Rape. In some embodiments, the plant has an AHAS gene which encodes a *Brassica napus* AHAS protein. In other embodiments, the plant has an AHAS gene which encodes a *Brassica napus* AHAS I protein. In certain embodiments, the plant has an AHAS gene which encodes a *Brassica napus* AHAS III protein. In some embodiments, the plant is non-transgenic.

In one aspect there is provided a seed having a *Brassica* acetohydroxyacid synthase (AHAS) gene encoding a protein having a mutation at one or more amino acid positions corresponding to a position selected from the group consisting of: A205, D376, W574, R577, and S653 of SEQ ID NO: 1. In some embodiments, the seed has an AHAS gene which encodes a protein having one or more mutations selected from the group consisting of: an alanine to valine substitution at a position corresponding to position 205 of SEQ ID NO: 1, an alanine to aspartic acid substitution at a position corresponding to position 205 of SEQ ID NO: 1, an aspartic acid to glutamic acid substitution at a position corresponding to position 376 of SEQ ID NO: 1, a tryptophan to cysteine substitution at a position corresponding to position 574 of SEQ ID NO: 1, a tryptophan to leucine substitution at a position corresponding to position 574 of SEQ ID NO: 1, a tryptophan to methionine substitution at a position corresponding to position 574 of SEQ ID NO: 1, a tryptophan to serine substitution at a position corresponding to position 574 of SEQ ID NO: 1, an arginine to tryptophan substitution at a position corresponding to position 577 of SEQ ID NO: 1, a serine to asparagine at a position corresponding to position 653 of SEQ ID NO: 1, and a serine to threonine at a position corresponding to position 653 of SEQ ID NO: 1. In some embodiments, the mutation is a serine to asparagine substitution at a position corresponding to position 653 of SEQ ID NO: 1 in which the mutation is not S653N in *Brassica* AHAS I gene. 4. In some embodiments, the mutation is a serine to threonine substitution at a position corresponding to position 653 of SEQ ID NO: 1. In some embodiments, the mutation is a tryptophan to leucine substitution at a position corresponding to position 574 of SEQ ID NO: 1, in which the mutation is not W574L in *Brassica* AHAS III gene. In some embodiments, the mutation is an alanine to valine substitution at a position corresponding to position 205 of SEQ ID NO: 1. In other embodiments the mutation is an alanine to aspartic acid substitution at a position corresponding to position 205 of SEQ ID NO: 1. In other embodiments, the mutation selected from the mutations shown in Table 2. In certain embodiments, the seed has an AHAS gene which encodes a protein having two or more mutations. In some embodiments, the two or more mutations are selected from Table 2. In some embodiments, the seed has an AHAS gene which encodes a protein having a mutation at a position corresponding to S653 of SEQ ID NO: 1 and a mutation at one or more amino acid positions corresponding to a position selected from the group consisting of: A205, D376, W574, and R577 of SEQ ID NO: 1. In other embodiments, the seed has an AHAS gene which encodes a protein having a mutation at a position corresponding to W574 of SEQ ID NO: 1 and a mutation at a position corresponding to R577 of SEQ ID NO: 1. In some embodiments, the seed has an AHAS gene which encodes a protein that is resistant to inhibition by an AHAS-inhibiting herbicide. In some embodiments the seed has an AHAS gene which encodes a protein comprising 70% or more identity to one or more of the amino acid sequences in Figure 2. In some embodiments, the seed is resistant to the application of at least one AHAS-inhibiting herbicide. In some embodiments, the AHAS-inhibiting herbicide is selected from the group consisting of herbicides of: imidazolinone, sulfonylurea, triazolopyrimidine, pyrimidinylthiobenzoate, sulfonylamino-carbonyltriazolinone, and mixtures thereof. In some embodiments, the herbicide is an imidazolinone herbicide. In some embodiments, the herbicide is a sulfonylurea herbicide. In some embodiments, the seed is a *Brassica* seed. In some embodiments, the seed has an AHAS gene which encodes a *Brassica napus* AHAS protein. In other embodiments, the seed has an AHAS gene which encodes a *Brassica napus* AHAS I protein. In certain embodiments, the seed has an AHAS gene which encodes a *Brassica napus* AHAS III protein. In some embodiments, the seed is a seed of a *Brassica* plant line in which the line is selected from the lines listed in Table 2. In some embodiments, the seed is non-transgenic. In some embodiments, there is provided a seed produced by a plant of the methods disclosed herein. In other embodiments, the seed is a canola seed.

In another aspect, there is provided a method for producing an herbicide-resistant plant by introducing into a plant cell a gene repair oligonucleobase (GRON) with a targeted mutation in an acetohydroxyacid synthase (AHAS) gene to produce a plant cell with an AHAS gene that expresses an AHAS protein having a mutation at one or more amino acid positions corresponding to a position selected from the group consisting of: A205, D376, W574, and S653 of SEQ ID NO: 1; and identifying a plant cell having substantially normal growth and catalytic activity as compared to a corresponding wild-type plant cell in the presence of an AHAS-inhibiting herbicide; and regenerating a non-transgenic herbicide-resistant plant having a mutated AHAS gene from said plant cell. In another aspect, there is provided a method for increasing the herbicide-resistance of a plant by: (a) crossing a first *Brassica* plant to a second *Brassica* plant, in which the first plant comprises a *Brassica* acetohydroxyacid synthase (AHAS) gene, in which the gene encodes a protein having a mutation at one or more amino acid positions corresponding to a position selected from the group consisting of: A205, D376, W574, R577, and S653 of SEQ ID NO: 1; (b) screening a population resulting from the cross for increased AHAS herbicide-resistance; (c) selecting a member resulting from the cross having increased AHAS herbicide-resistance; and (d) producing seeds resulting from the cross. In some embodiments, a hybrid seed is produced by any of the above methods. In some embodiments, plants are grown from seeds produced by any of the above methods. In another aspect, there is provided a method of controlling weeds in a field containing plants by applying an effective amount of at least one AHAS-inhibiting herbicide to a field containing said weeds and plants, the plant having a *Brassica* acetohydroxyacid synthase (AHAS) gene, in which the gene encodes a protein having a mutation at one or more amino acid positions corresponding to a position selected from the group consisting of: A205, D376, W574, R577, and S653 of SEQ ID NO: 1. 134. In some embodiments, the AHAS-inhibiting herbicide is selected from the group consisting of herbicides of: imidazolinone, sulfonylurea, triazolopyrimidine, pyrimidinylthiobenzoate, sulfonylamino-carbonyltriazolinone, and mixtures thereof. In other embodiments, the AHAS-inhibiting herbicide is an imidazolinone herbicide. In other embodiments, the AHAS-inhibiting herbicide is a sulfonylurea herbicide.

The term "nucleic acid" or "nucleic acid sequence" refers to an oligonucleotide, nucleotide or polynucleotide, and fragments or portions thereof, which may be single or double stranded, and represent the sense or antisense strand. A nucleic acid may include DNA or RNA, and may be of natural or synthetic origin. For example, a nucleic acid may include mRNA or cDNA. Nucleic acid may include nucleic acid that has been amplified (e.g., using polymerase chain reaction). The convention "NTwt###NTmut" is used to indicate a mutation that results in the wild-type nucleotide NTwt at position ### in the nucleic acid being replaced with mutant NTmut. The single letter code for nucleotides is as described in the U.S. Patent Office Manual of Patent Examining Procedure, section 2422, table 1. In this regard, the nucleotide designation "R" means purine such as guanine or adenine, "Y" means pyrimidine such as cytosine or thymine (uracil if RNA); "M" means adenine or cytosine; "K" means guanine or thymine; and "W" means adenine or thymine.

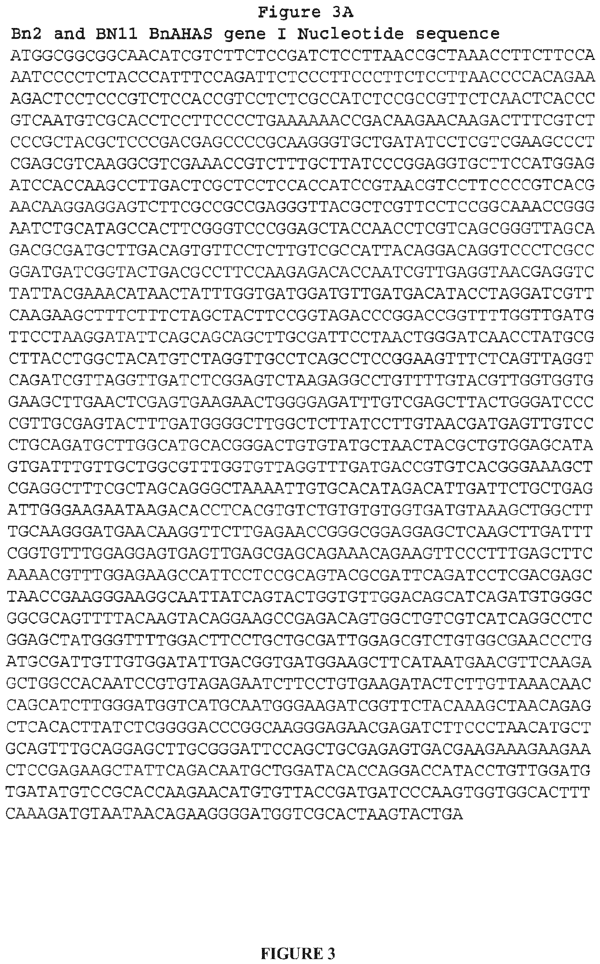

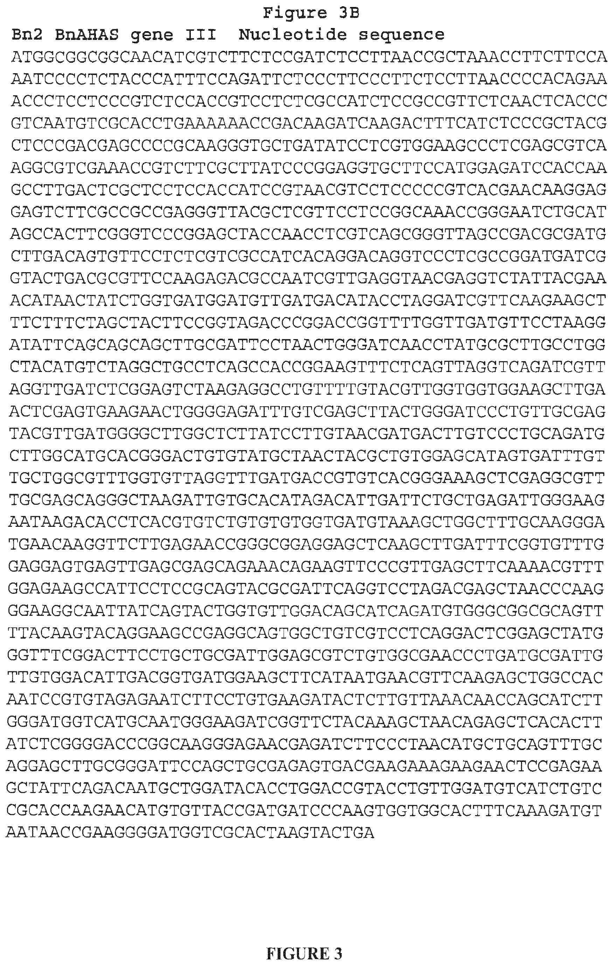

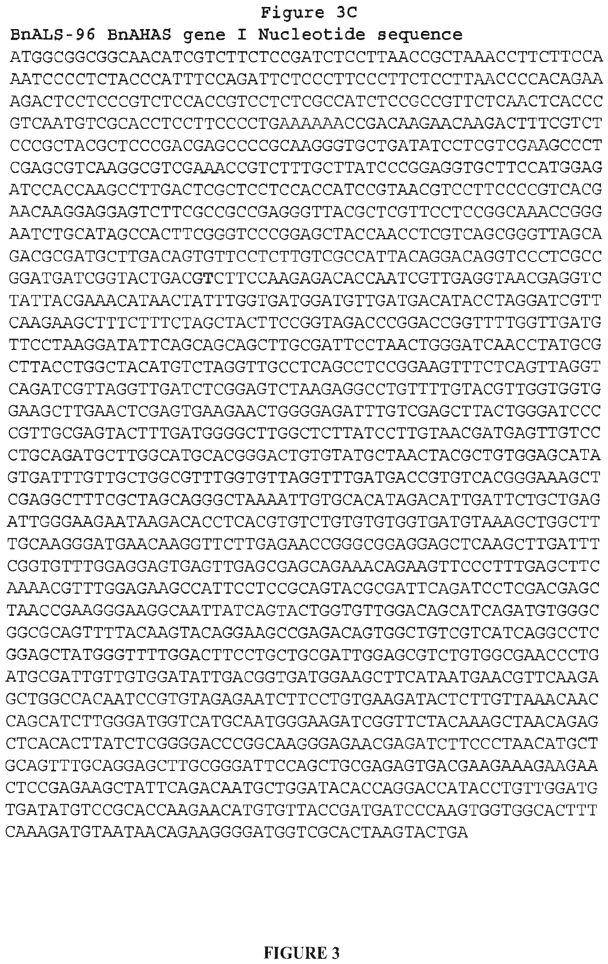

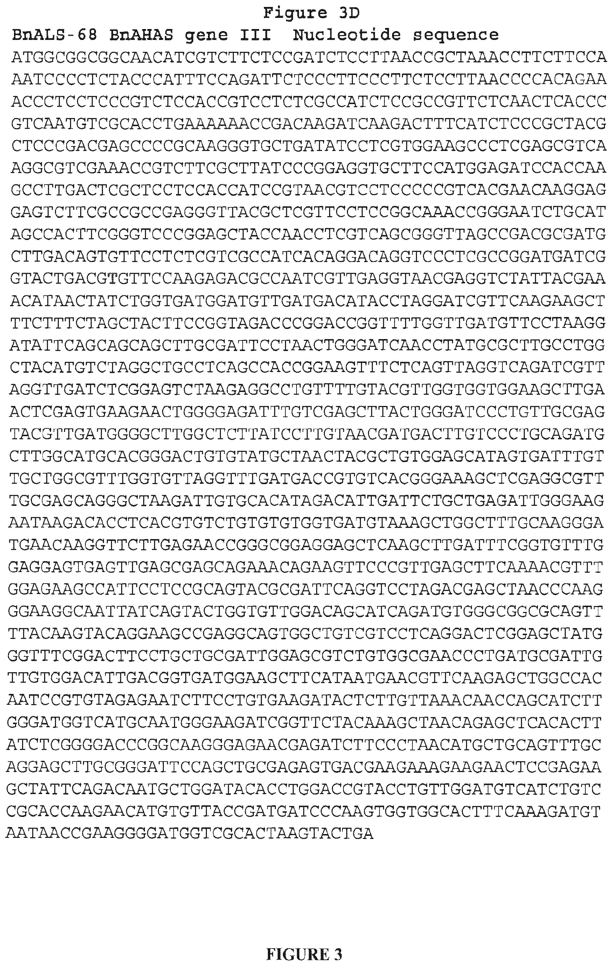

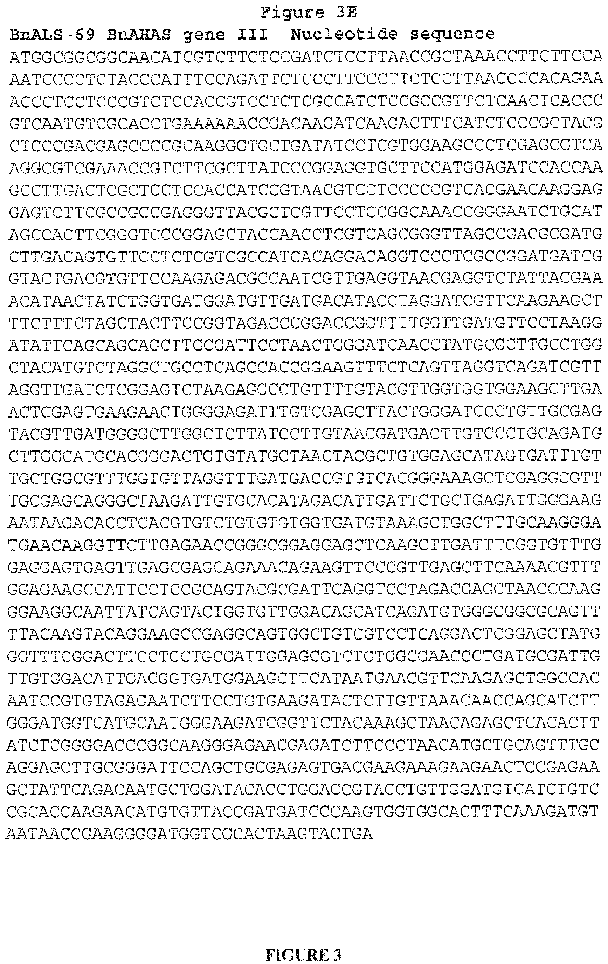

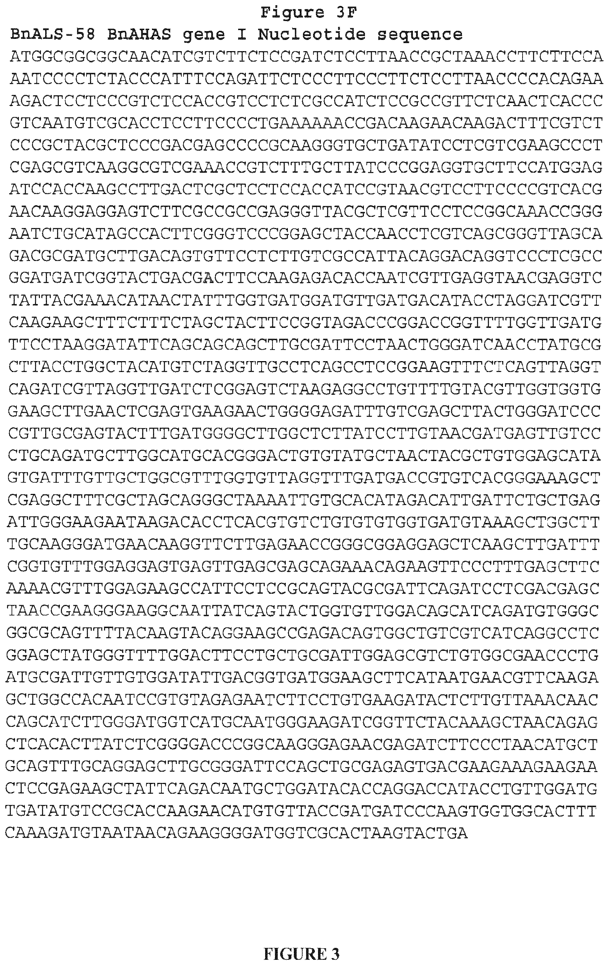

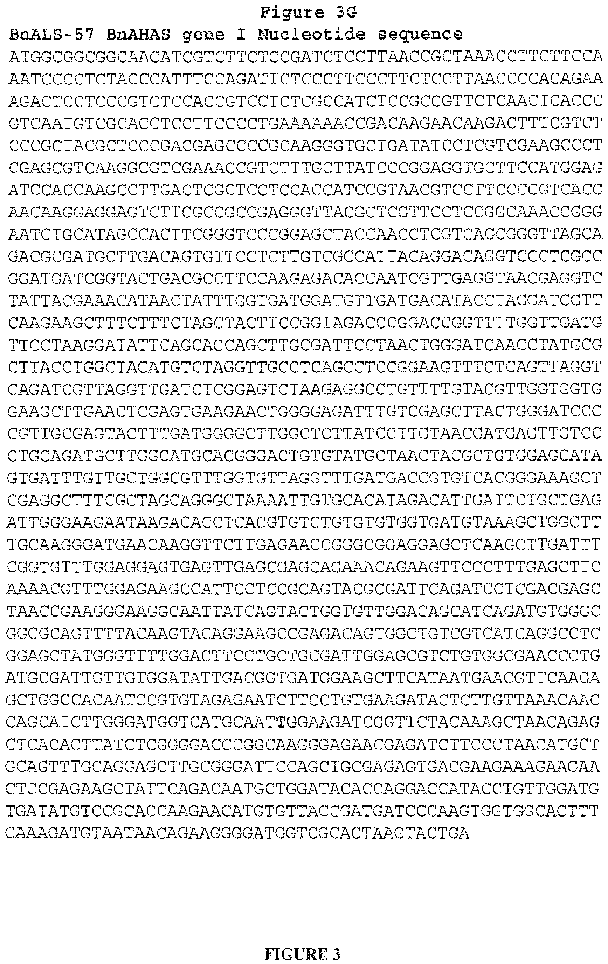

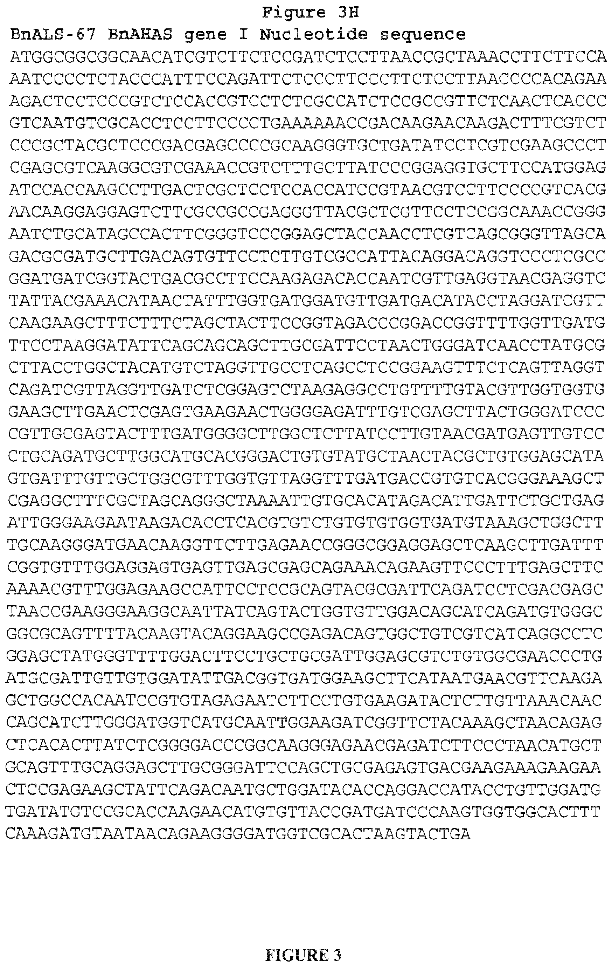

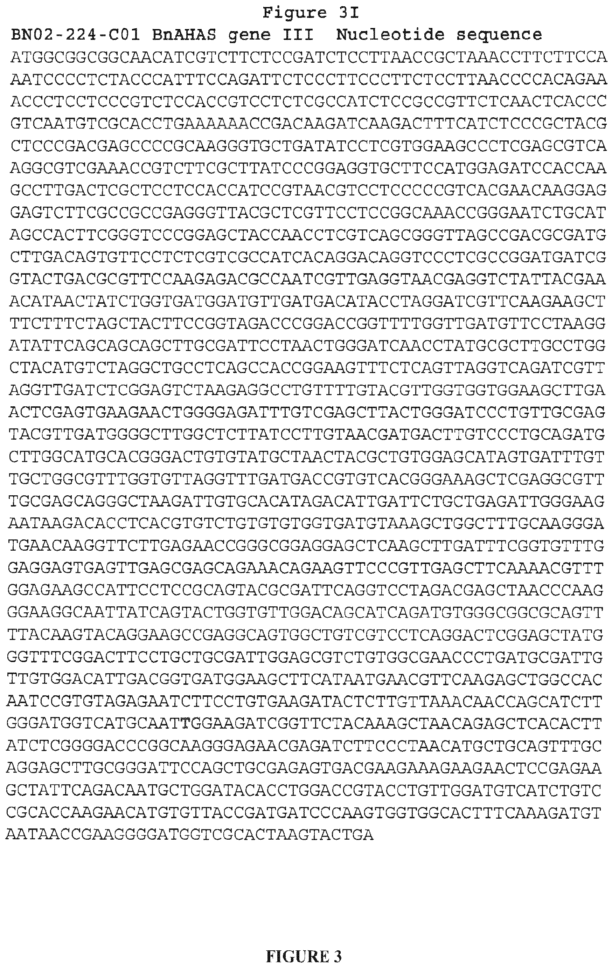

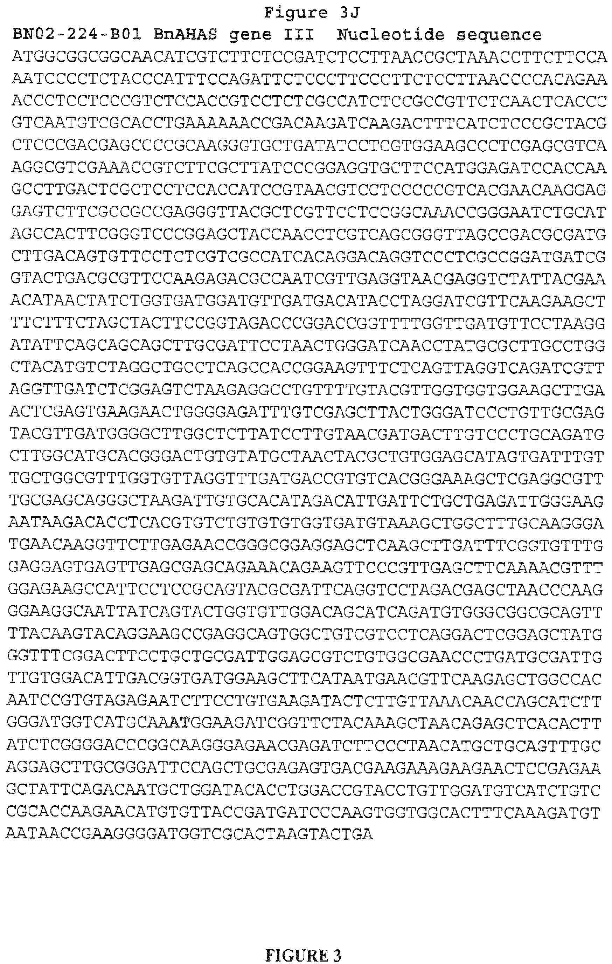

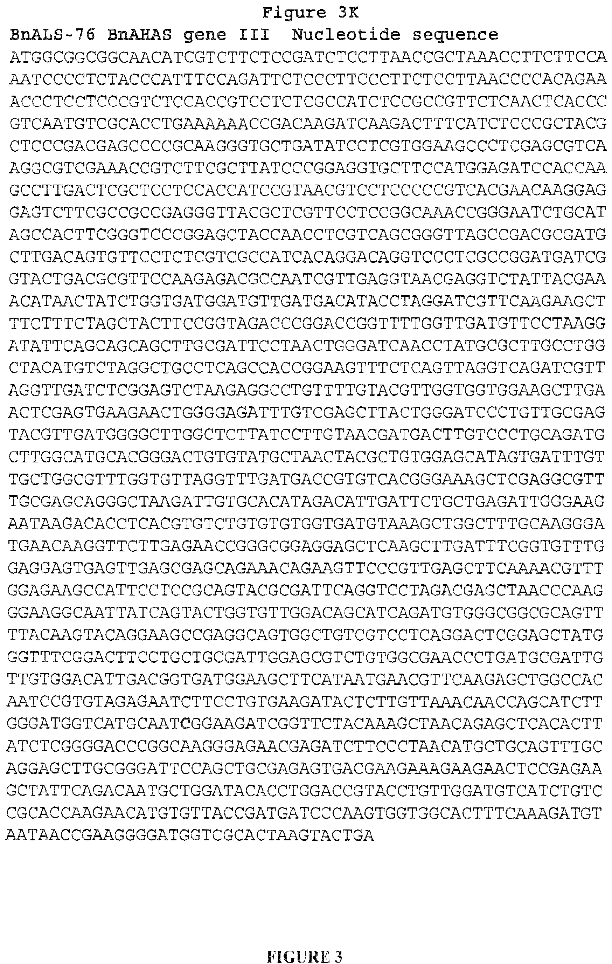

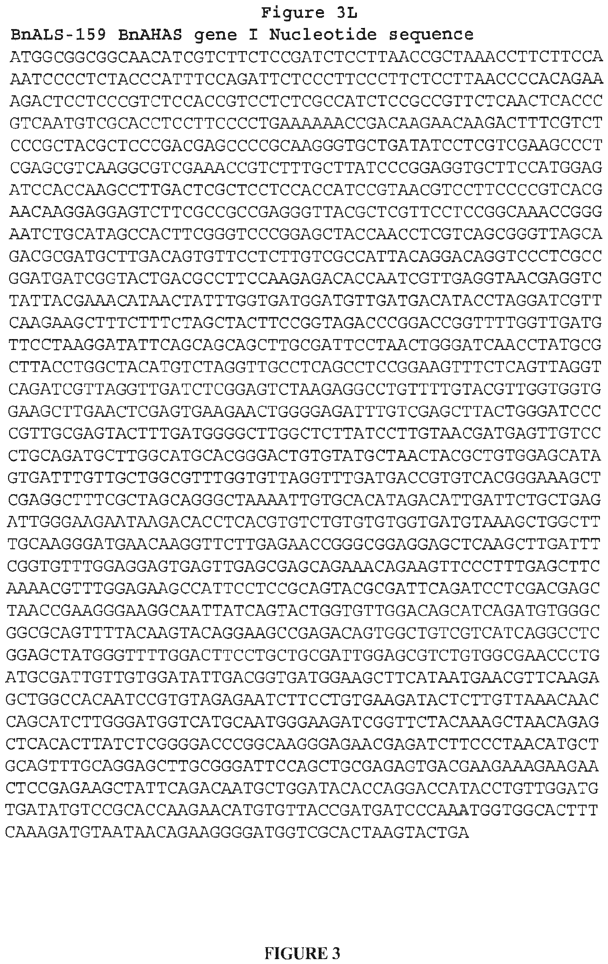

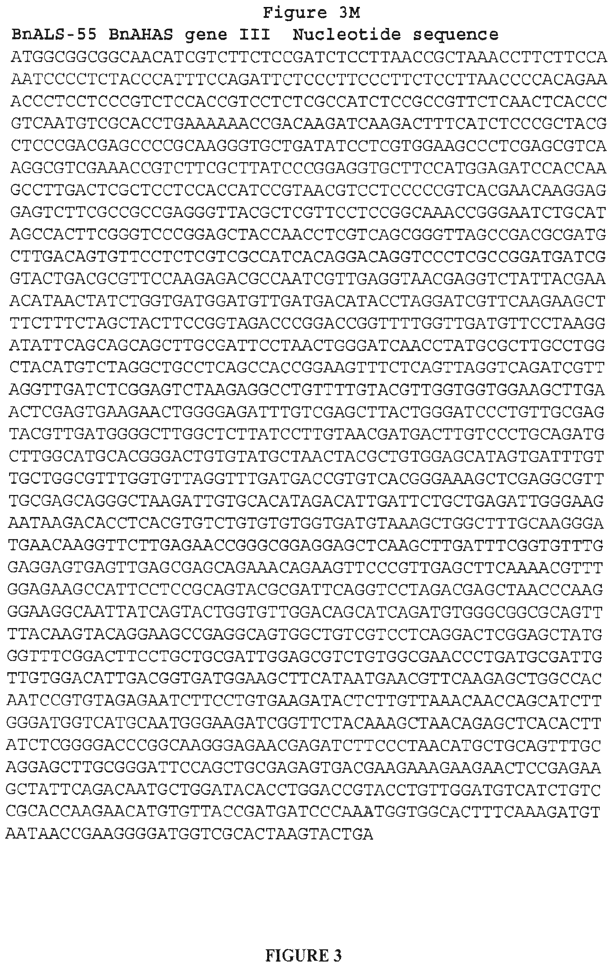

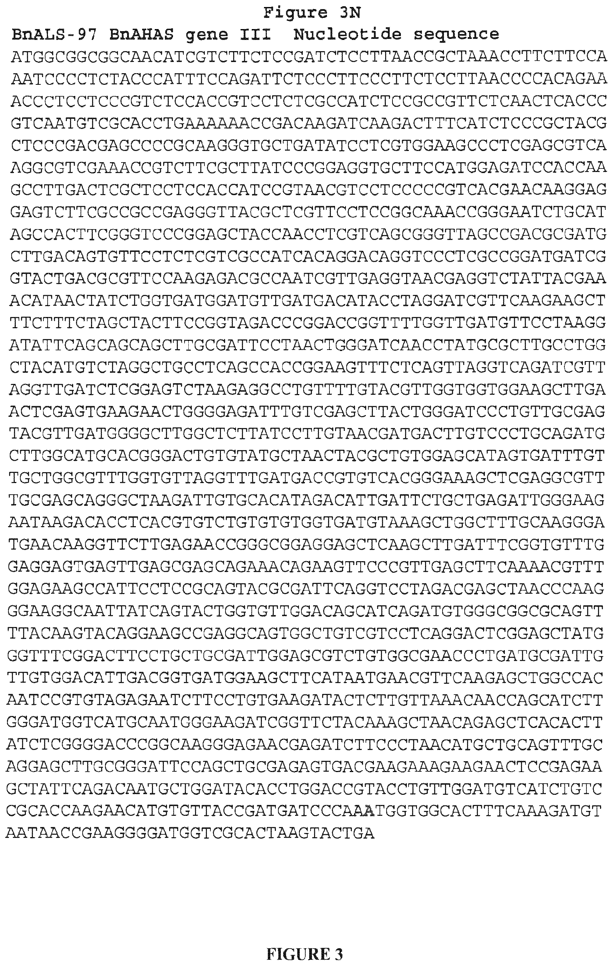

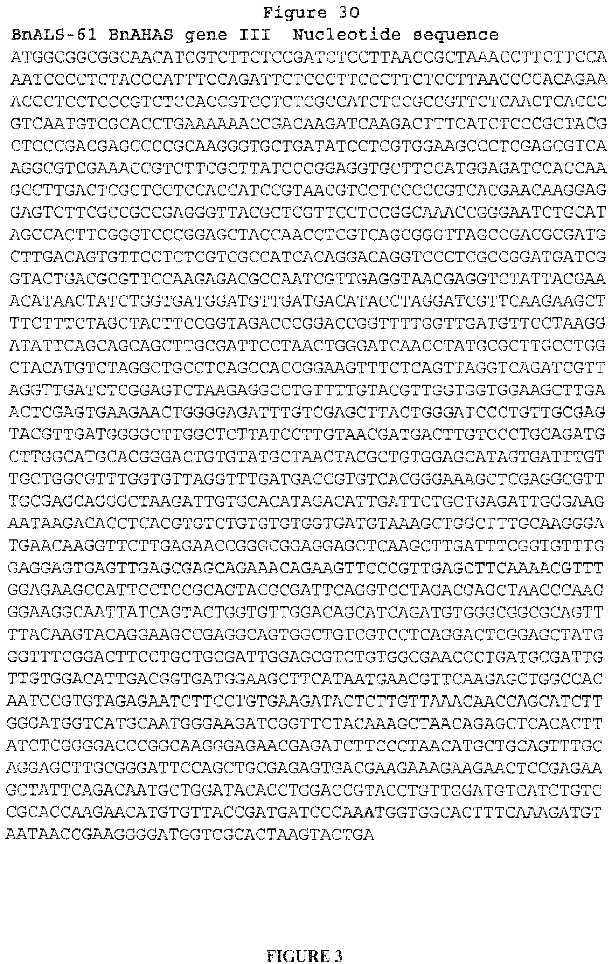

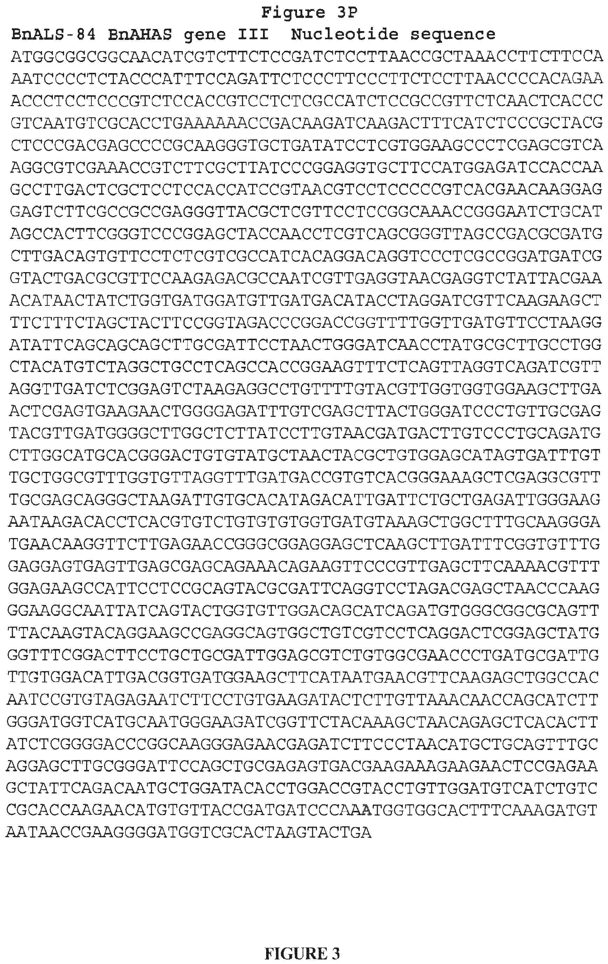

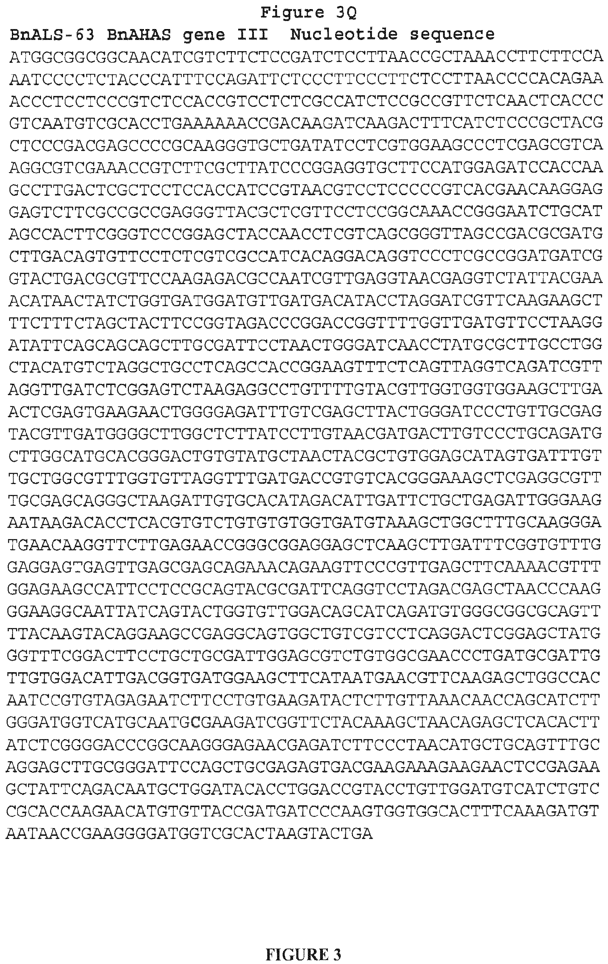

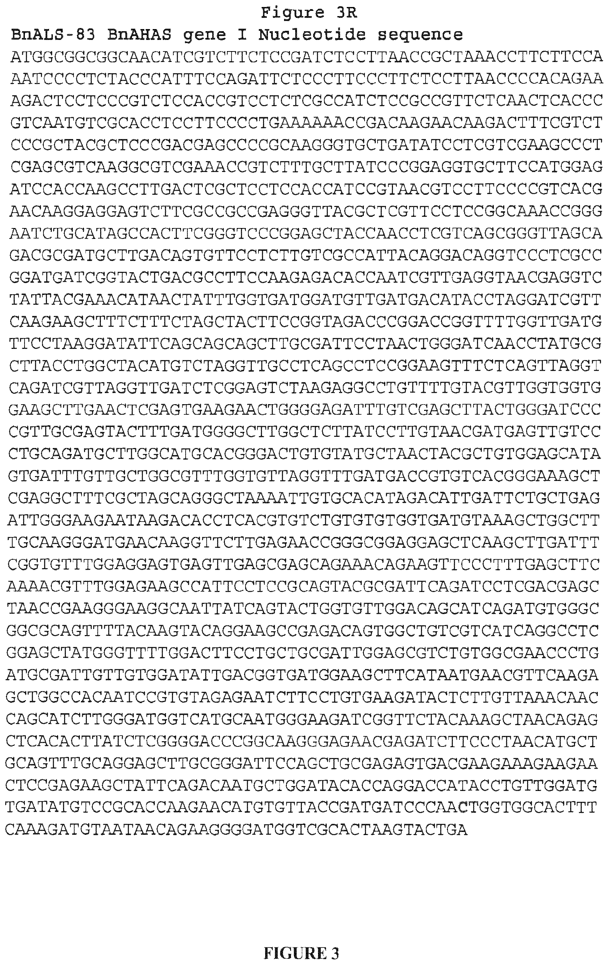

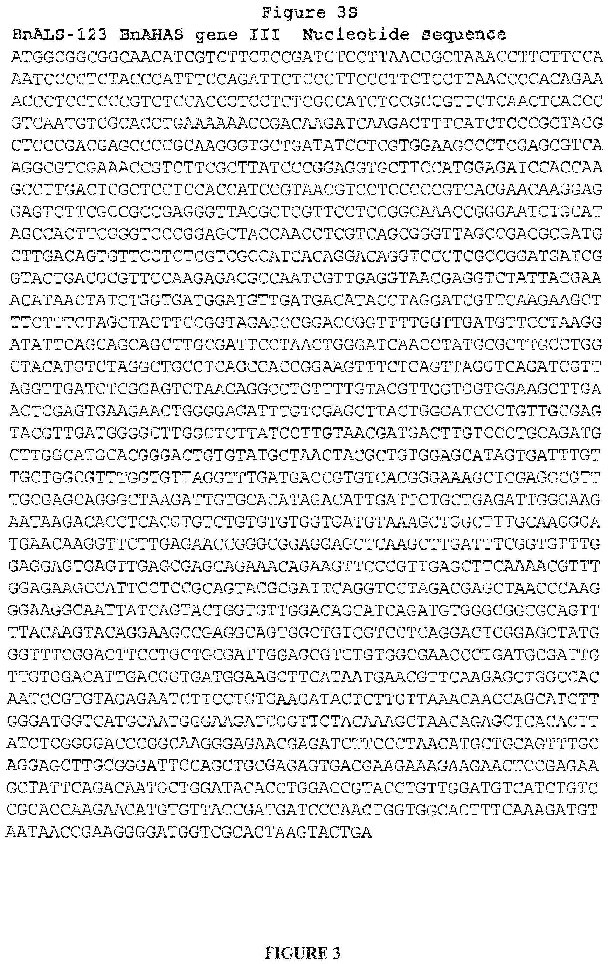

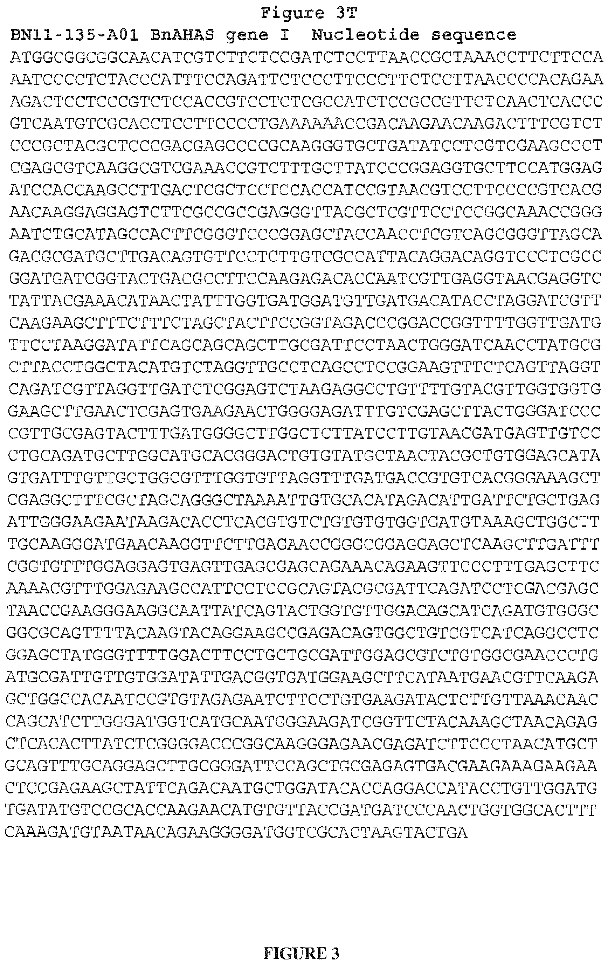

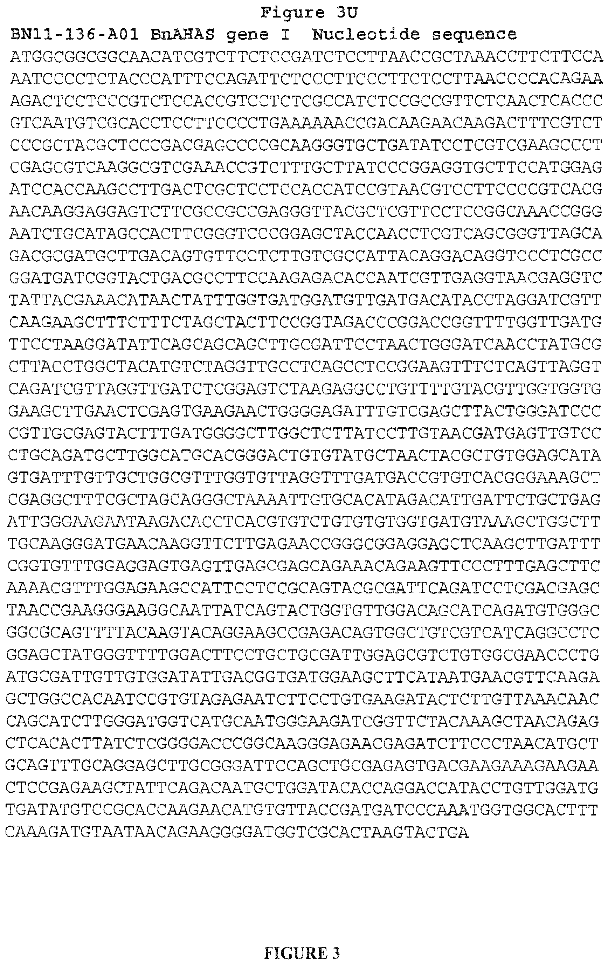

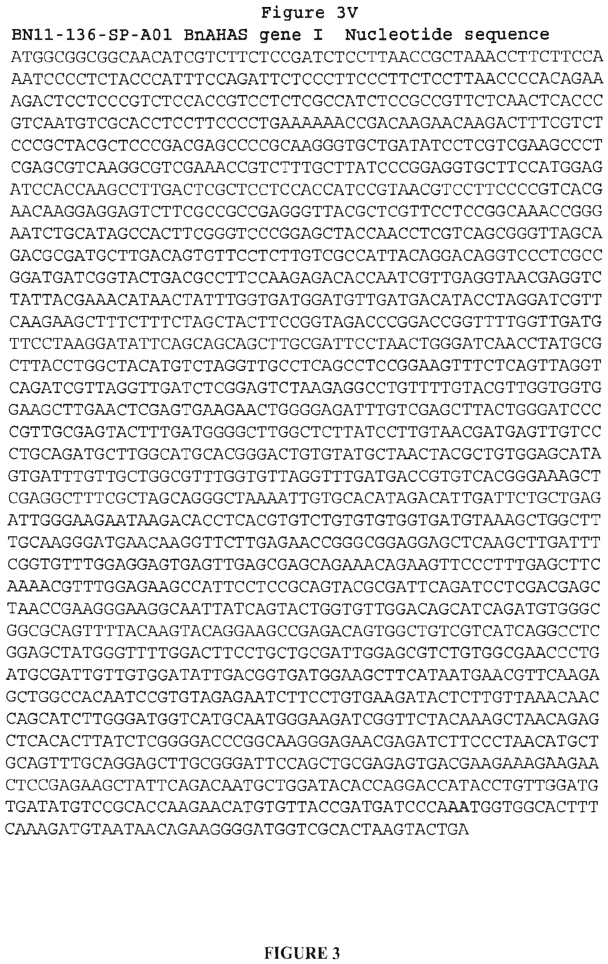

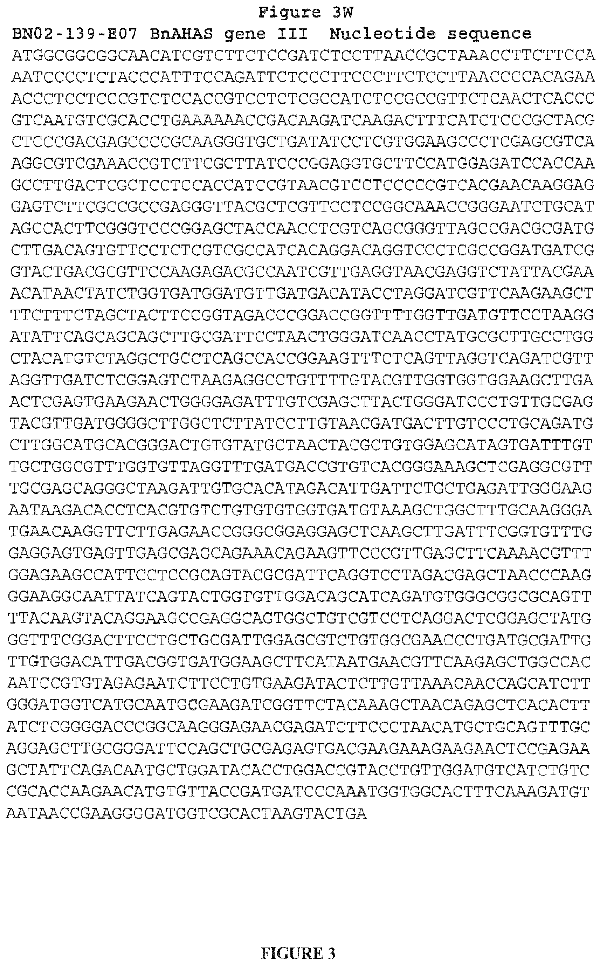

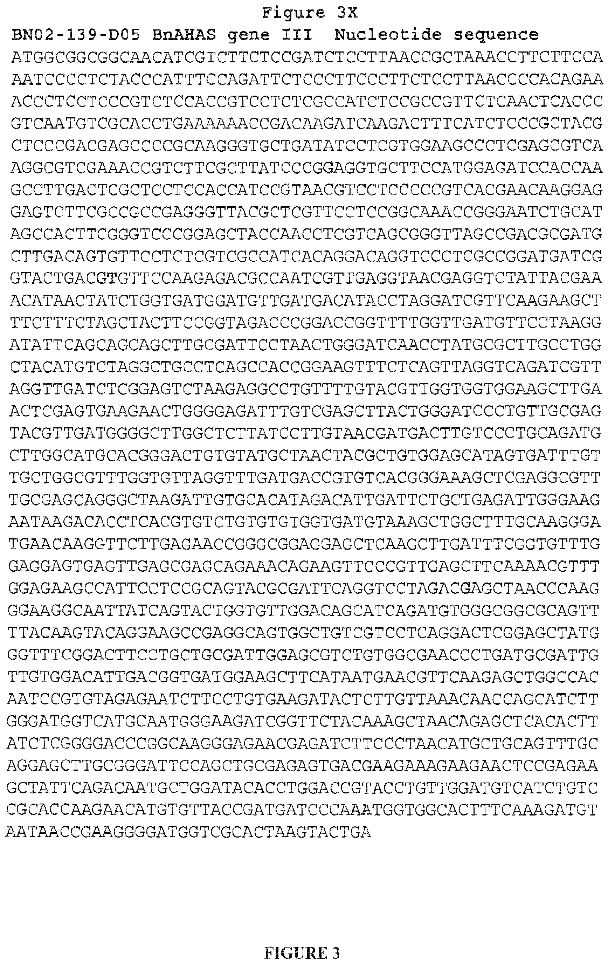

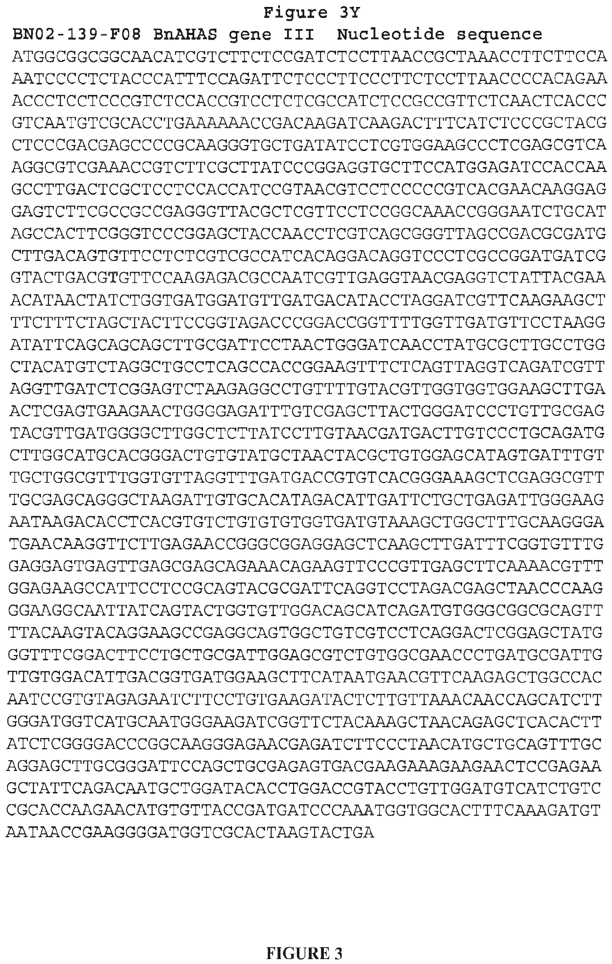

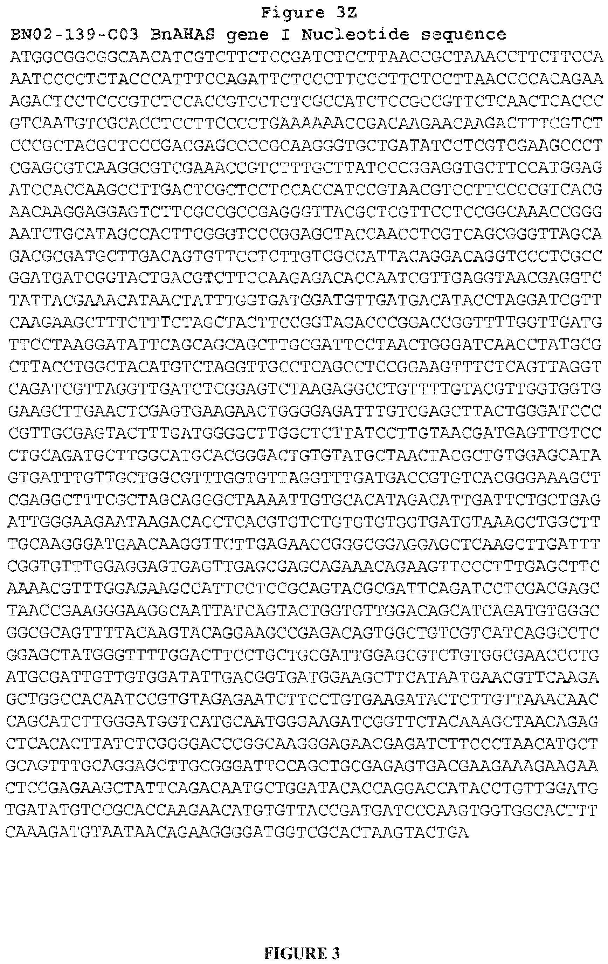

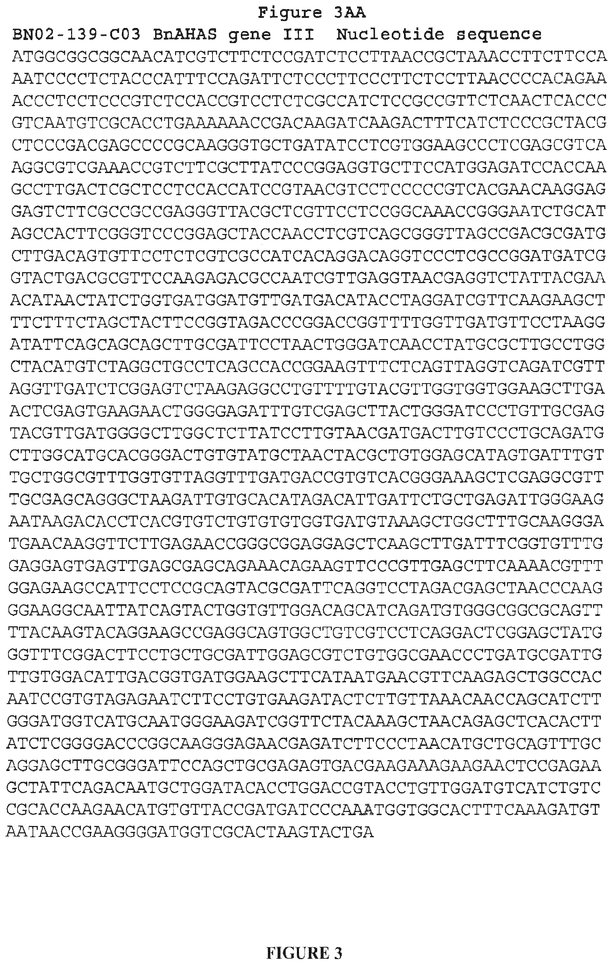

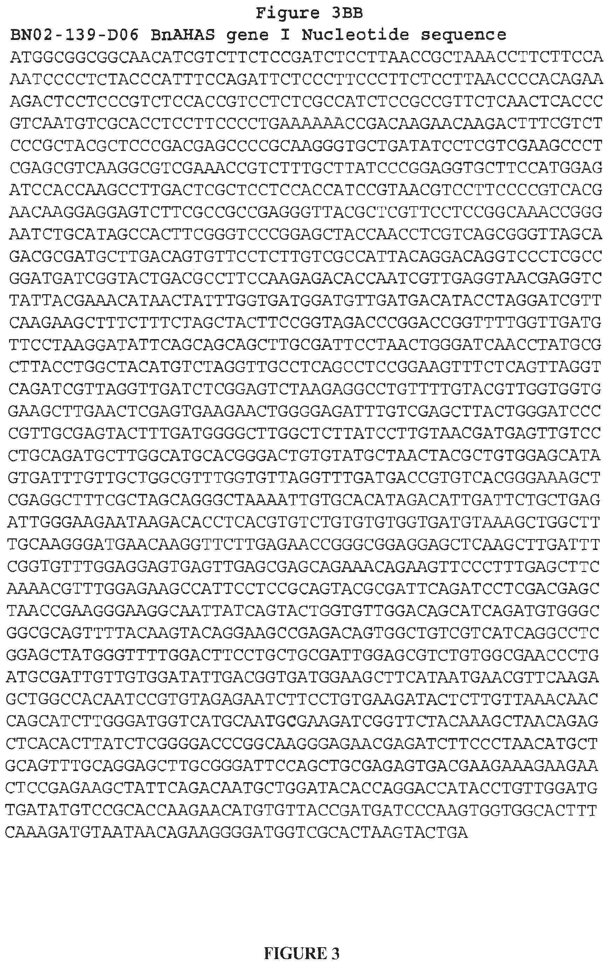

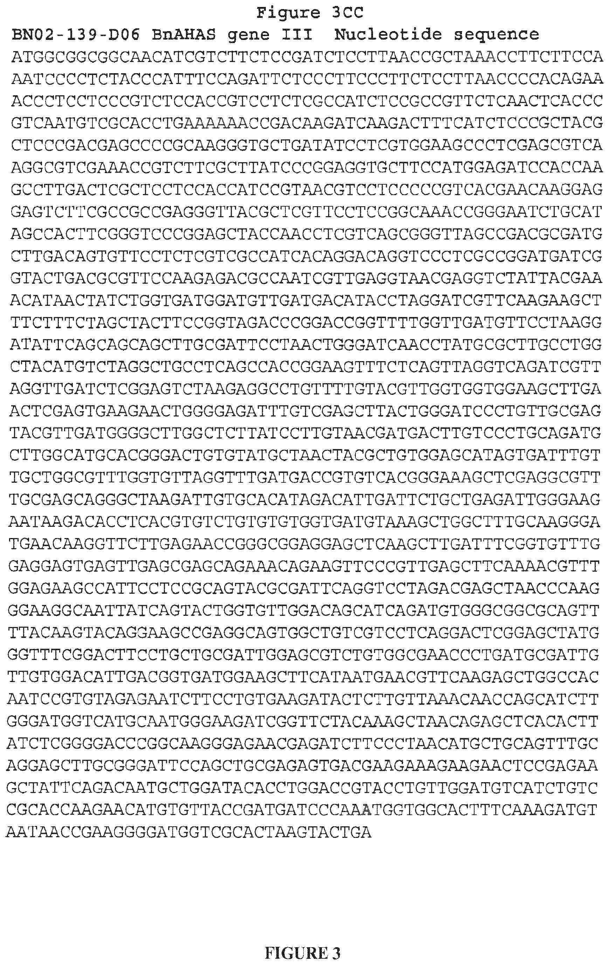

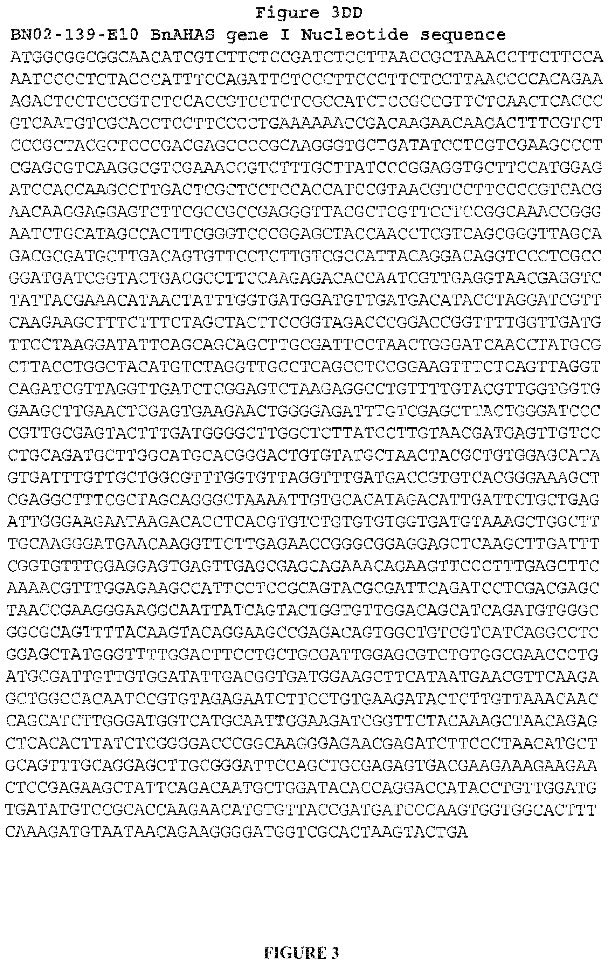

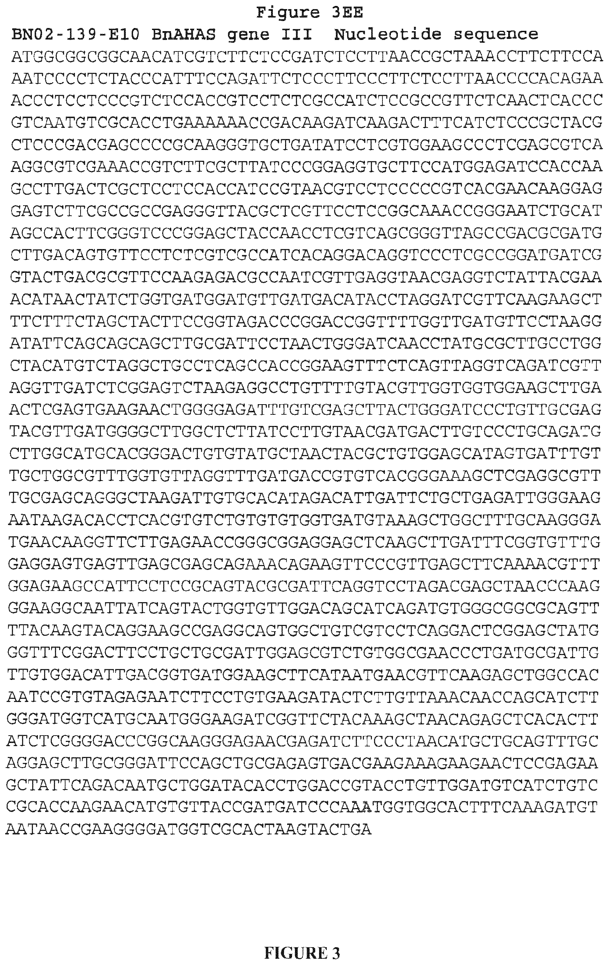

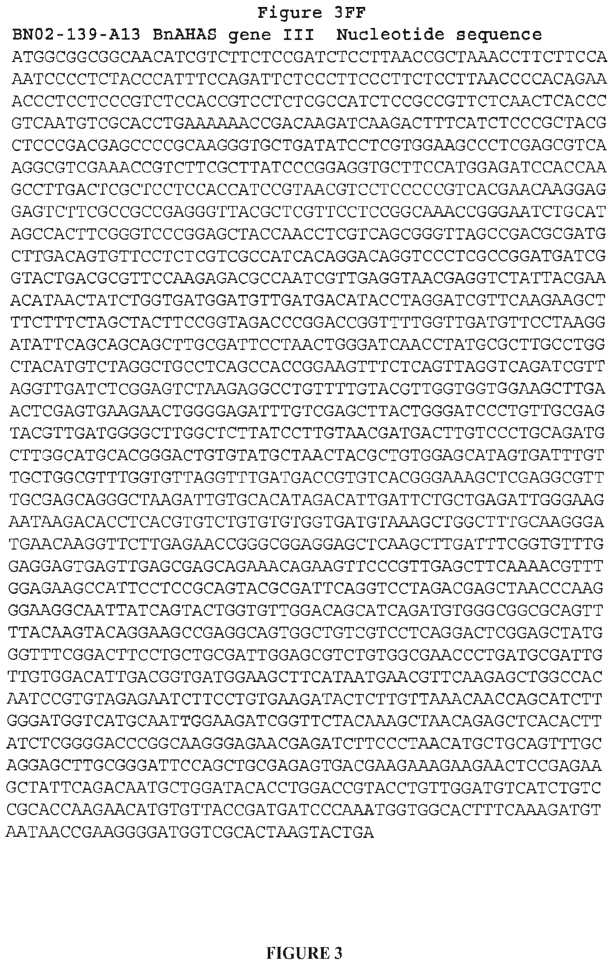

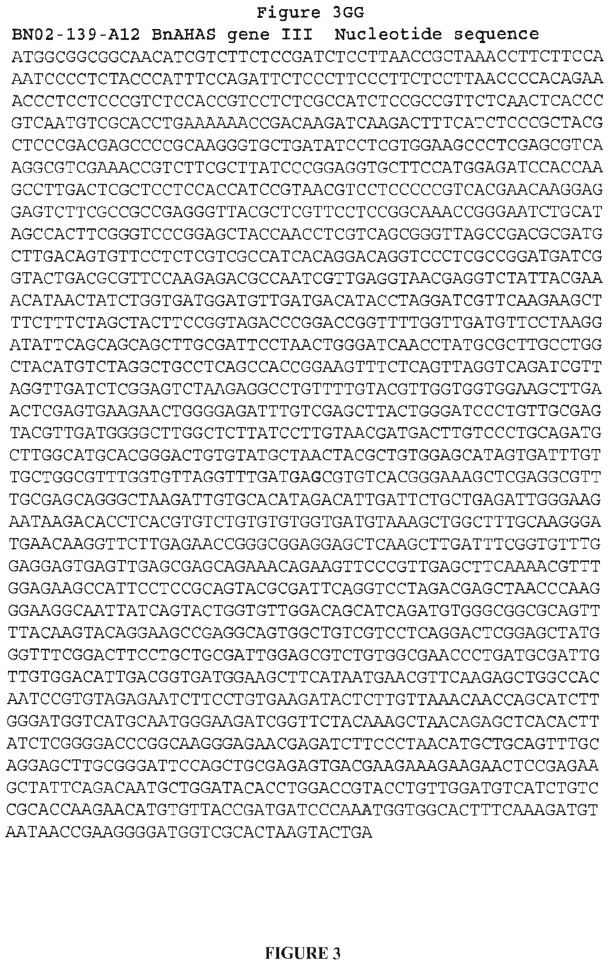

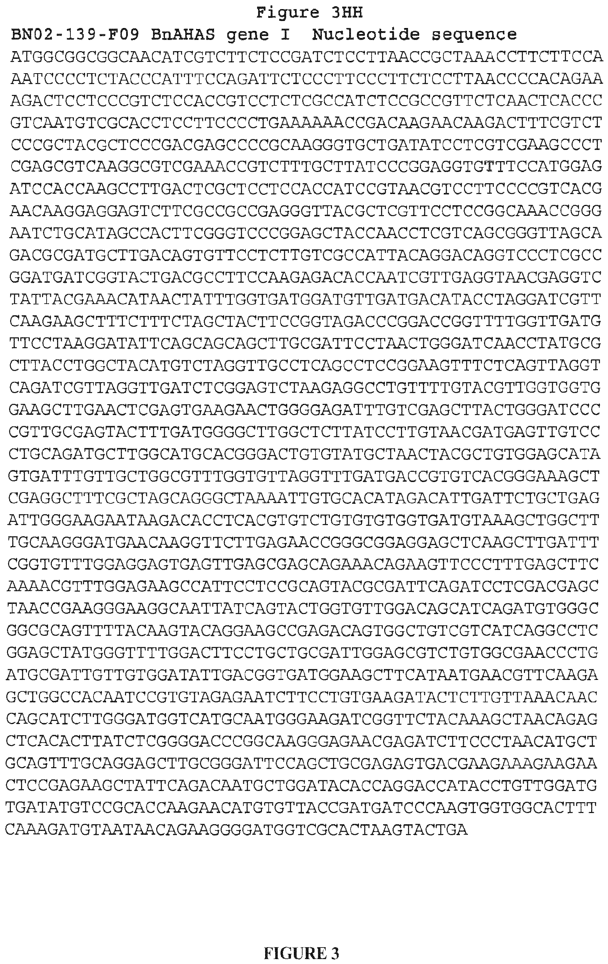

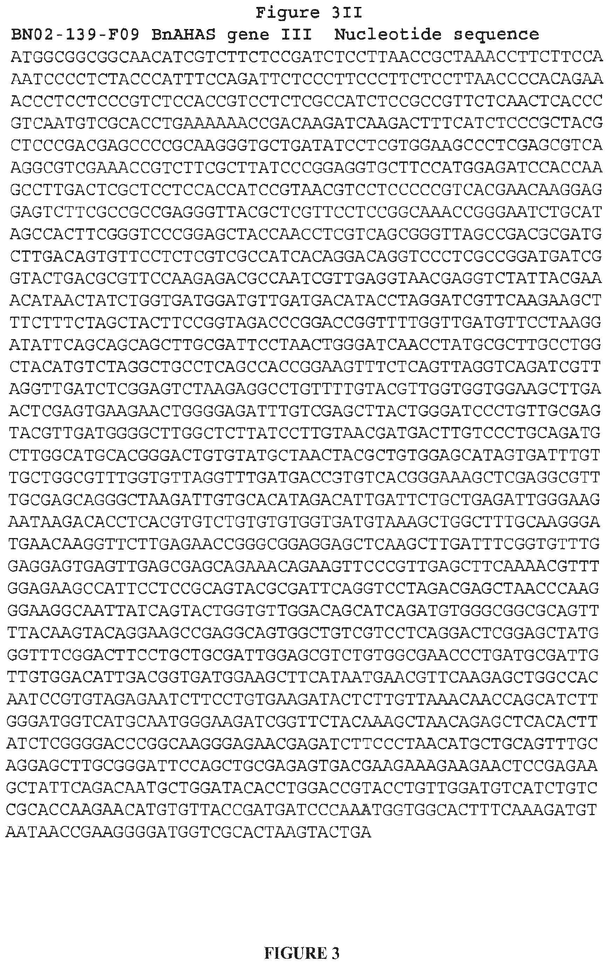

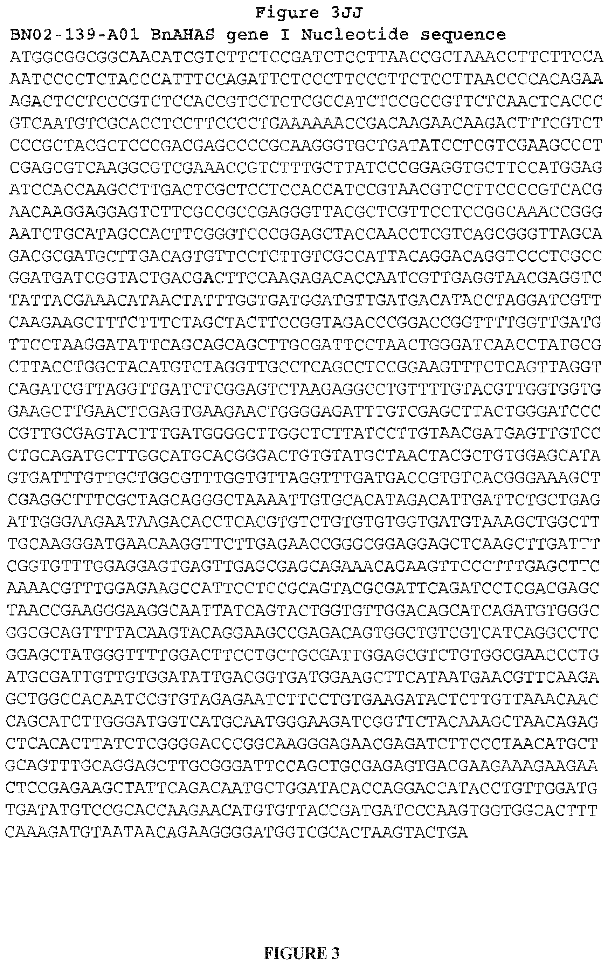

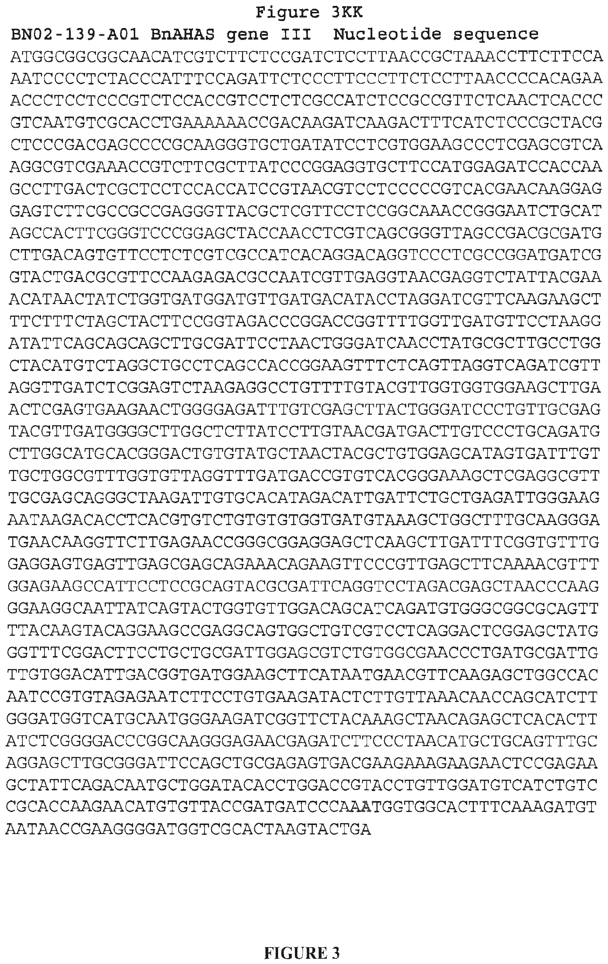

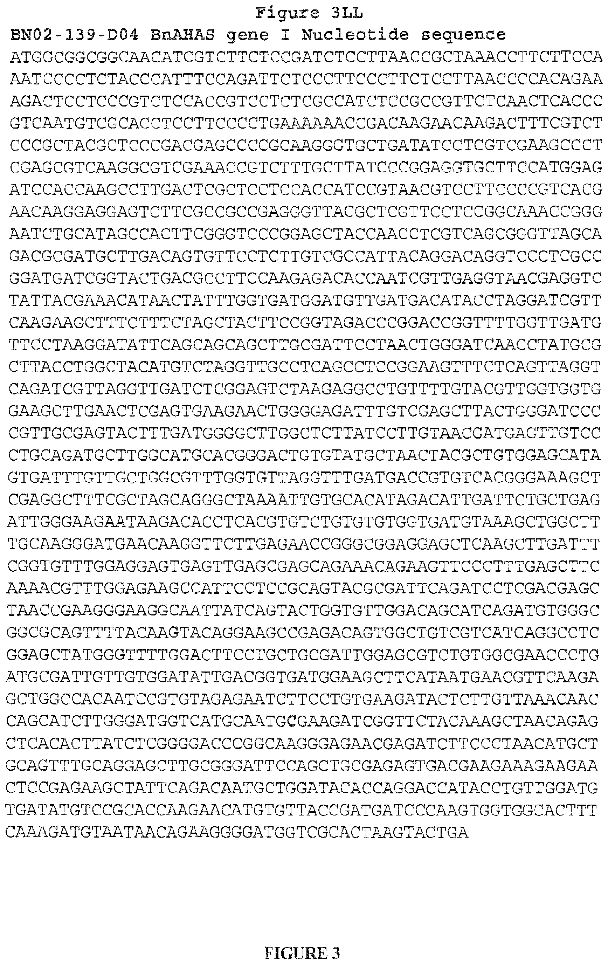

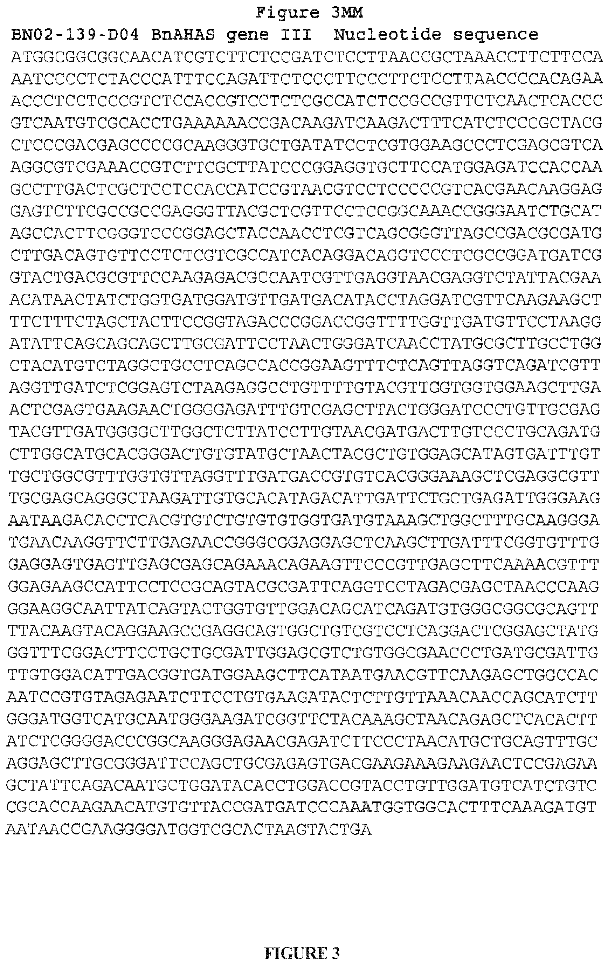

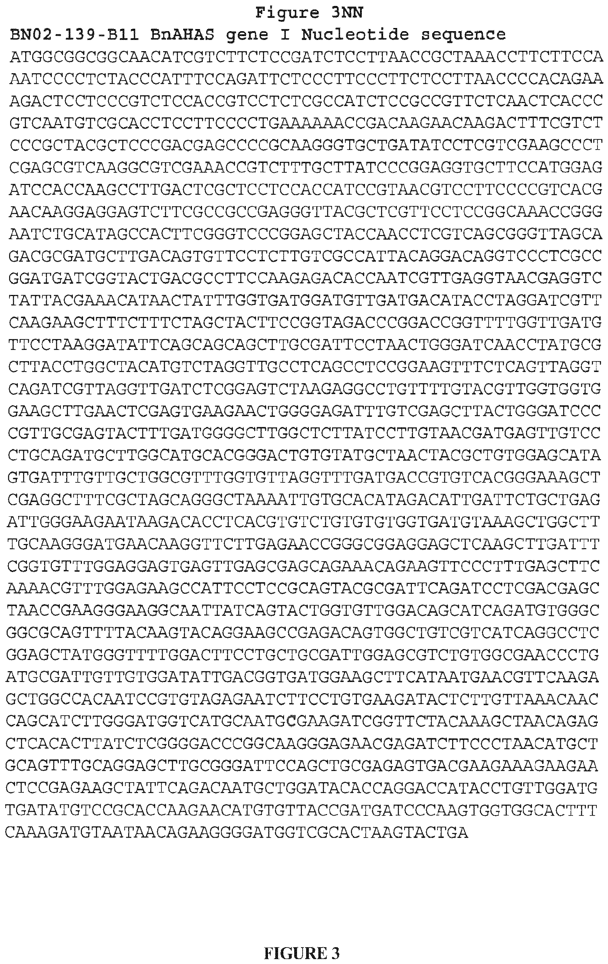

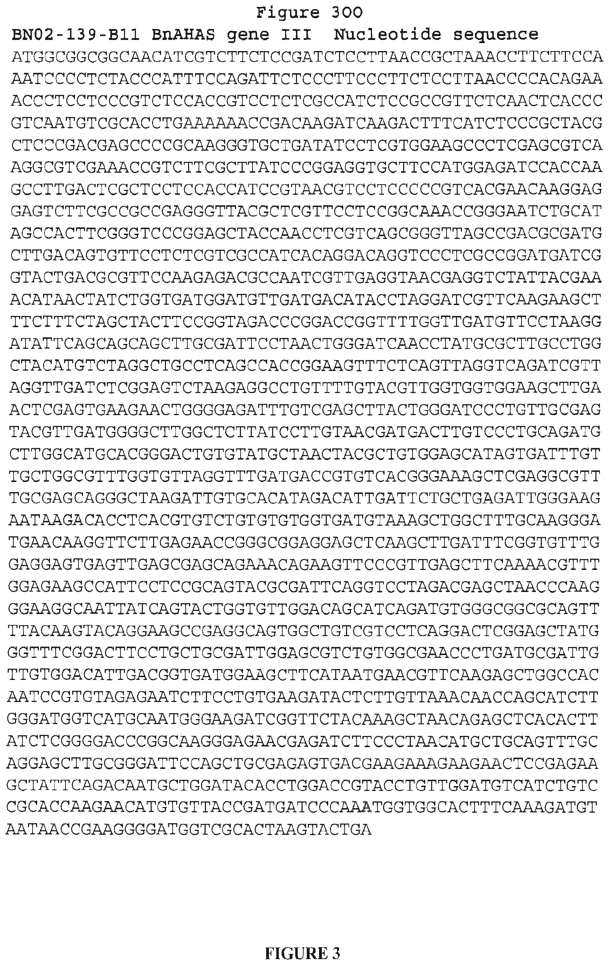

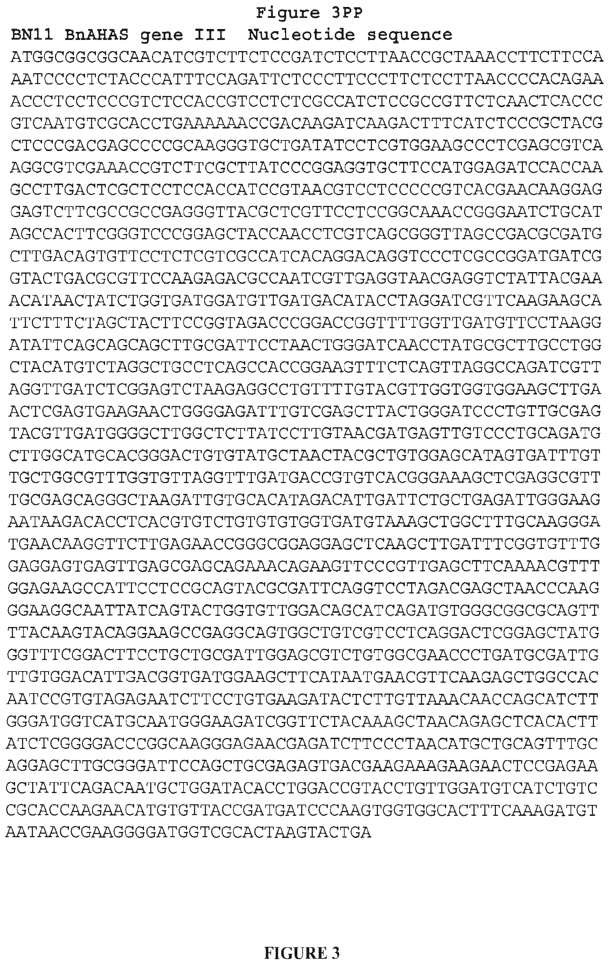

A "gene" refers to a DNA sequence that comprises control and coding sequences necessary for the production of an RNA, which may have a non-coding function (*e.g.,* a ribosomal or transfer RNA) or which may include a polypeptide or a polypeptide precursor. The RNA or polypeptide may be encoded by a full length coding sequence or by any portion of the coding sequence so long as the desired activity or function is retained. As used herein, the term "AHAS Gene" refers to a gene that has homology to a *Brassica* AHAS gene. In certain embodiments, the AHAS gene has 70%; 75%; 80%; 85%; 90%; 95%; 96%; 97%; 98%; 99%; or 100% identity to a specific *Brassica* AHAS gene, for example the *Brassica napus* AHAS gene I or the *Brassica napus* AHAS gene III. In certain embodiments, the AHAS gene has 60%; 70%; 75%; 80%; 85%; 90%; 95%; 96%; 97%; 98%; 99%; or 100% identity to a sequence selected from the sequences in Figure 3. In certain embodiments, the AHAS gene is modified with at least one mutation. In other embodiments, the AHAS gene is modified with at least two mutations. In some embodiments, the AHAS gene is modified with at least one mutation selected from the mutations shown in Table 2. In certain embodiments, the AHAS gene is modified with at least two mutations shown in Table 2. In certain embodiments, the mutation is a conserved mutation.

By "coding sequence" is meant a sequence of a nucleic acid or its complement, or a part thereof, that can be transcribed and/or translated to produce the mRNA for and/or the polypeptide or a fragment thereof. Coding sequences include exons in a genomic DNA or immature primary RNA transcripts, which are joined together by the cell's biochemical machinery to provide a mature mRNA. The anti-sense strand is the complement of such a nucleic acid, and the encoding sequence can be deduced therefrom.

By "non-coding sequence" is meant a sequence of a nucleic acid or its complement, or a part thereof, that is not transcribed into amino acid *in vivo*, or where tRNA does not interact to place or attempt to place an amino acid. Non-coding sequences include both intron sequences in genomic DNA or immature primary RNA transcripts, and gene-associated sequences such as promoters, enhancers, silencers, *etc.*

A nucleobase is a base, which in certain preferred embodiments is a purine, pyrimidine, or a derivative or analog thereof. Nucleosides are nucleobases that contain a pentosefuranosyl moiety, e.g., an optionally substituted riboside or 2'-deoxyriboside. Nucleosides can be linked by one of several linkage moieties, which may or may not contain phosphorus. Nucleosides that are linked by unsubstituted phosphodiester linkages are termed nucleotides. The term "nucleobase" as used herein includes peptide nucleobases, the subunits of peptide nucleic acids, and morpholine nucleobases as well as nucleosides and nucleotides.

An oligonucleobase is a polymer comprising nucleobases; preferably at least a portion of which can hybridize by Watson-Crick base pairing to a DNA having the complementary sequence. An oligonucleobase chain may have a single 5' and 3' terminus, which are the ultimate nucleobases of the polymer. A particular oligonucleobase chain can contain nucleobases of all types. An oligonucleobase compound is a compound comprising one or more oligonucleobase chains that may be complementary and hybridized by Watson-Crick base pairing. Ribo-type nucleobases include pentosefuranosyl containing nucleobases wherein the 2' carbon is a methylene substituted with a hydroxyl, alkyloxy or halogen. Deoxyribo-type nucleobases are nucleobases other than ribo-type nucleobases and include all nucleobases that do not contain a pentosefuranosyl moiety.

In certain embodiments, an oligonucleobase strand may include both oligonucleobase chains and segments or regions of oligonucleobase chains. An oligonucleobase strand may have a 3' end and a 5' end, and when an oligonucleobase strand is coextensive with a chain, the 3' and 5' ends of the strand are also 3' and 5' termini of the chain.

The term "gene repair oligonucleobase" as used herein denotes oligonucleobases, including mixed duplex oligonucleotides, non-nucleotide containing molecules, single stranded oligodeoxynucleotides and other gene repair molecules.

By "isolated", when referring to a nucleic acid (e.g., an oligonucleotide such as RNA, DNA, or a mixed polymer) is meant a nucleic acid that is apart from a substantial portion of the genome in which it naturally occurs and/or is substantially separated from other cellular components which naturally accompany such nucleic acid. For example, any nucleic acid that has been produced synthetically (e.g., by serial base condensation) is considered to be isolated. Likewise, nucleic acids that are recombinantly expressed, cloned, produced by a primer extension reaction (e.g., PCR), or otherwise excised from a genome are also considered to be isolated.

An "amino acid sequence" refers to a polypeptide or protein sequence. The convention "AAwt###AAmut" is used to indicate a mutation that results in the wild-type amino acid AAwt at position ### in the polypeptide being replaced with mutant AAmut.

By "complement" is meant the complementary sequence to a nucleic acid according to standard Watson/Crick pairing rules. A complement sequence can also be a sequence of RNA complementary to the DNA sequence or its complement sequence, and can also be a cDNA.

By "substantially complementary" is meant that two sequences hybridize under stringent hybridization conditions. The skilled artisan will understand that substantially complementary sequences need not hybridize along their entire length.

As used herein the term "codon" refers to a sequence of three adjacent nucleotides (either RNA or DNA) constituting the genetic code that determines the insertion of a specific amino acid in a polypeptide chain during protein synthesis or the signal to stop protein synthesis. The term "codon" is also used to refer to the corresponding (and complementary) sequences of three nucleotides in the messenger RNA into which the original DNA is transcribed.

As used herein, the term "AHAS Protein" refers to a protein that has homology to a *Brassica* AHAS protein. In certain embodiments, the AHAS protein has 70%; 75%; 80%; 85%; 90%; 95%; 96%; 97%; 98%; 99%; or 100% identity to a specific *Brassica* AHAS protein, such as for example, the *Brassica napus* AHAS protein I or the *Brassica napus* AHAS protein III. In certain embodiments, the AHAS protein has 70%; 75%; 80%; 85%; 90%; 95%; 96%; 97%; 98%; 99%; or 100% identity to a sequence selected from the sequences in Figure 2. In certain embodiments, the AHAS protein is modified with at least one mutation. In other embodiments, the AHAS protein is modified with at least two mutations. In some embodiments, the AHAS protein is modified with at least one mutation selected from the mutations shown in Table 2. In certain embodiments, the AHAS protein is modified with at least two mutations shown in Table 2. In certain embodiments, the mutation is a conserved mutation.

The term "wild-type" refers to a gene or a gene product that has the characteristics of that gene or gene product when isolated from a naturally occurring source. A wild-type gene is that which is most frequently observed in a population and is thus arbitrarily designated the "normal" or "wild-type" form of the gene. "Wild-type" may also refer to the sequence at a specific nucleotide position or positions, or the sequence at a particular codon position or positions, or the sequence at a particular amino acid position or positions.

As used herein, "mutant," or "modified" refers to a nucleic acid or protein which displays modifications in sequence and or functional properties (*i.e.,* altered characteristics) when compared to the wild-type gene or gene product. "Mutant," or "modified" also refers to the sequence at a specific nucleotide position or positions, or the sequence at a particular codon position or positions, or the sequence at a particular amino acid position or positions which displays modifications in sequence and or functional properties (*i.e.,* altered characteristics) when compared to the wild-type gene or gene product.

A "mutation" is meant to encompass at least a single nucleotide variation in a nucleic acid sequence or a single amino acid variation in a polypeptide relative to the normal sequence or wild-type sequence. A mutation may include a substitution, a deletion, an inversion or an insertion.

As used herein, the term "homology" refers to sequence similarity among proteins and DNA. The term "homology" or "homologous" refers to a degree of identity. There may be partial homology or complete homology. A partially homologous sequence is one that has less than 100% sequence identity when compared to another sequence.

"Heterozygous" refers to having different alleles at one or more genetic loci in homologous chromosome segments. As used herein "heterozygous" may also refer to a sample, a cell, a cell population or an organism in which different alleles at one or more genetic loci may be detected. Heterozygous samples may also be determined via methods known in the art such as, for example, nucleic acid sequencing. For example, if a sequencing electropherogram shows two peaks at a single locus and both peaks are roughly the same size, the sample may be characterized as heterozygous. Or, if one peak is smaller than another, but is at least about 25% the size of the larger peak, the sample may be characterized as heterozygous. In some embodiments, the smaller peak is at least about 15% of the larger peak. In other embodiments, the smaller peak is at least about 10% of the larger peak. In other embodiments, the smaller peak is at least about 5% of the larger peak. In other embodiments, a minimal amount of the smaller peak is detected.

As used herein, "homozygous" refers to having identical alleles at one or more genetic loci in homologous chromosome segments. "Homozygous" may also refer to a sample, a cell, a cell population or an organism in which the same alleles at one or more genetic loci may be detected. Homozygous samples may be determined via methods known in the art, such as, for example, nucleic acid sequencing. For example, if a sequencing electropherogram shows a single peak at a particular locus, the sample may be termed "homozygous" with respect to that locus.

The term "hemizygous" refers to a gene or gene segment being present only once in the genotype of a cell or an organism because the second allele is deleted. As used herein "hemizygous" may also refer to a sample, a cell, a cell population or an organism in which an allele at one or more genetic loci may be detected only once in the genotype.

The term "zygosity status" as used herein refers to a sample, a cell population, or an organism as appearing heterozygous, homozygous, or hemizygous as determined by testing methods known in the art and described herein. The term "zygosity status of a nucleic acid" means determining whether the source of nucleic acid appears heterozygous, homozygous, or hemizygous. The "zygosity status" may refer to differences in a single nucleotide in a sequence. In some methods, the zygosity status of a sample with respect to a single mutation may be categorized as homozygous wild-type, heterozygous (*i.e.,* one wild-type allele and one mutant allele), homozygous mutant, or hemizygous (*i.e.,* a single copy of either the wild-type or mutant allele).

As used herein, the term "RTDS" refers to The *Rapid Trait Development System*™ (RTDS) developed by Cibus. RTDS is a site-specific gene modification system that is effective at making precise changes in a gene sequence without the incorporation of foreign genes or control sequences.

The term "about" as used herein means in quantitative terms plus or minus 10%. For example, "about 3%" would encompass 2.7-3.3% and "about 10%" would encompass 9-11%.

### BRIEF DESCRIPTION OF THE FIGURES

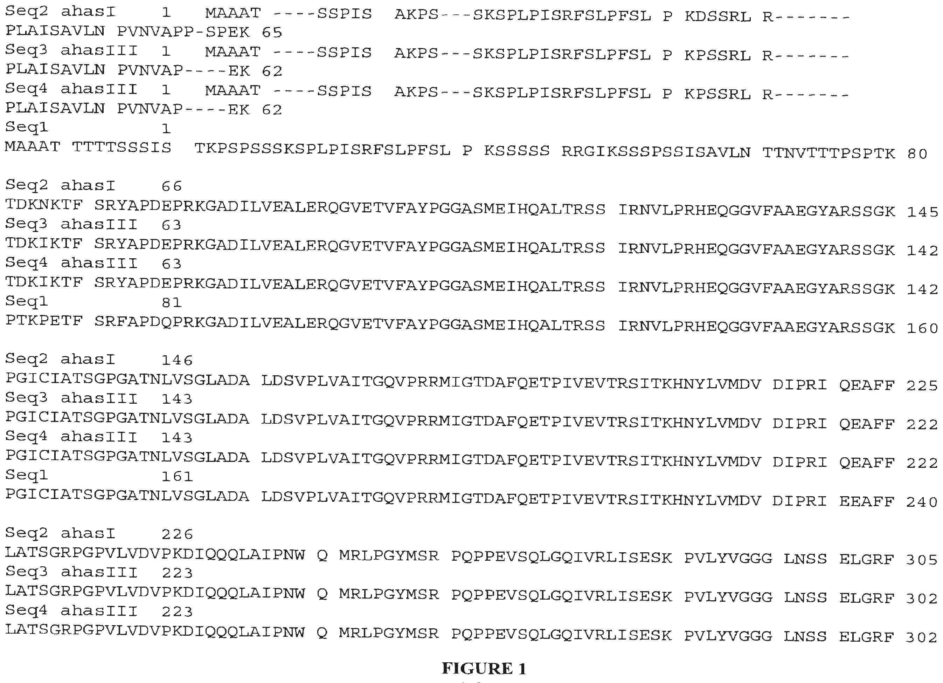

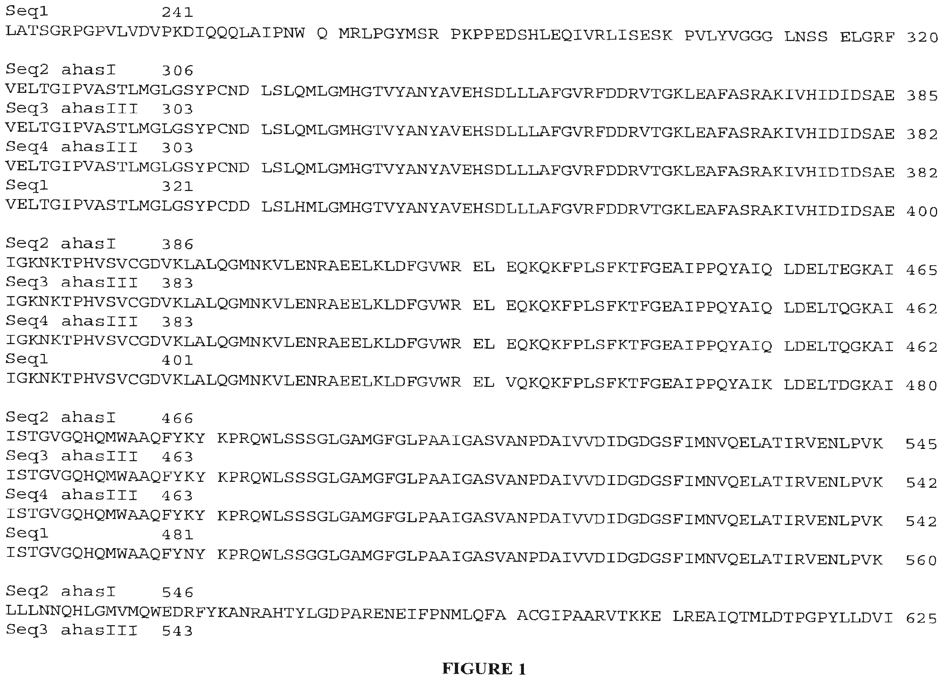

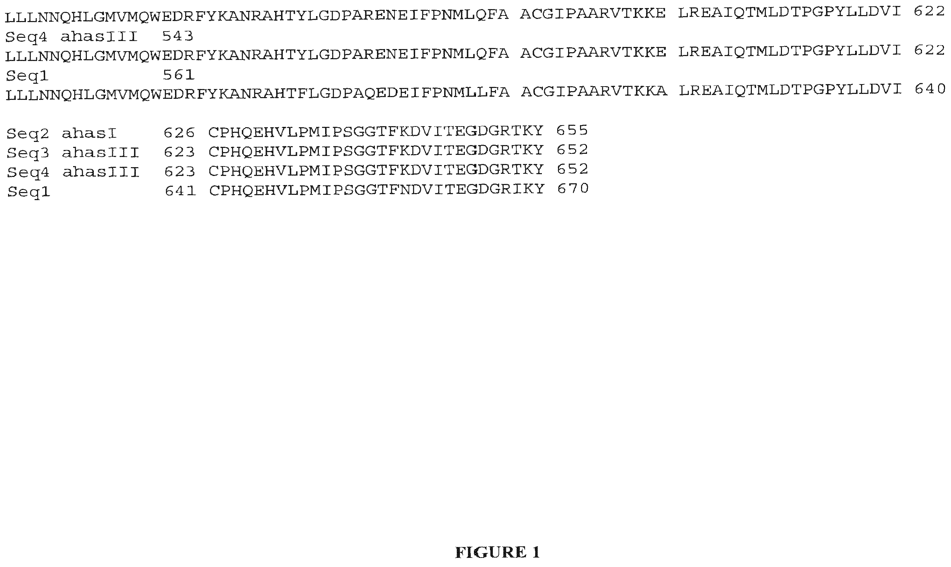

**FIGURE 1** shows an amino acid alignment of *Arabidopsis* acetohydroxyacid synthase AHAS (SEQ ID NO: 1), *Brassica napus* AHAS I (SEQ ID NO: 2), and *Brassica napus* AHAS III (SEQ ID NOs: 3 and 4). SEQ ID NO: 1 is the amino acid sequence of the *Arabidopsis* AHAS At3g48560 based on the annotated genomic DNA sequence Genbank accession number NC003074. SEQ ID NO: 2 is the amino acid sequence of *Brassica napus* AHAS I from Cibus elite lines BN-2 and BN-11. This sequence is identical to the translated product of Genbank accession Z11524. SEQ ID NO: 3 is the amino acid sequence of *Brassica napus* AHAS III from Cibus elite line BN-2. This sequence is identical to the translated product of Genbank accession Z11526, excepting a D325E substitution at amino acid 325. SEQ ID NO: 4 is the amino acid sequence of *Brassica napus* AHAS III from Cibus elite line BN-11. This sequence is identical to the translated product of SEQ ID NO: 3, excepting an E343 at amino acid 343 as in SEQ ID NO: 1.
Figure 2 shows amino acid sequences of translated genes referred to in Table 2. Amino acids indicated in bold represent the mutation.
Figure 3 shows nucleotide sequences referred to in Table 2. Nucleotides indicated in bold represent the mutation.
Figure 4 shows results of the spray trial described in Example 4.

### DETAILED DESCRIPTION OF THE INVENTION

Provided are compositions and methods related in part to the successful targeting of acetohydroxyacid synthase (AHAS) genes in *Brassica* using, for example, the Rapid Trait Development System *(**RTDS***™) technology developed by Cibus. In combination or alone, plants containing any of the mutations disclosed herein can form the basis of new herbicide-resistant products. Also provided are seeds produced from the mutated plants in which the AHAS genes are either homozygous or heterozygous for the mutations. The mutations disclosed herein can be in combination with any other mutation known or with mutations discovered in the future.

*RTDS* is based on altering a targeted gene by utilizing the cell's own gene repair system to specifically modify the gene sequence in situ and not insert foreign DNA and gene expression control sequences. This procedure effects a precise change in the genetic sequence while the rest of the genome is left unaltered. In contrast to conventional transgenic GMOs, there is no integration of foreign genetic material, nor is any foreign genetic material left in the plant. The changes in the genetic sequence introduced by *RTDS* are not randomly inserted. Since affected genes remain in their native location, no random, uncontrolled or adverse pattern of expression occurs.

The *RTDS* that effects this change is a chemically synthesized oligonucleotide which may be composed of both DNA and modified RNA bases as well as other chemical moieties, and is designed to hybridize at the targeted gene location to create a mismatched base-pair(s). This mismatched base-pair acts as a signal to attract the cell's own natural gene repair system to that site and correct (replace, insert or delete) the designated nucleotide(s) within the gene. Once the correction process is complete the *RTDS* molecule is degraded and the now-modified or repaired gene is expressed under that gene's normal endogenous control mechanisms.

The subject mutations in the AHAS I and III genes were described using the *Brassica napus* AHAS genes and proteins (see SEQ ID NOs: 2, 3 and 4). The compositions and methods also encompass mutant AHAS genes of other species (paralogs). However, due to variations in the AHAS genes of different species, the number of the amino acid residue to be changed in one species may be different in another species. Nevertheless, the analogous position is readily identified by one of skill in the art by sequence homology. For example, FIGURE 1 shows the aligned amino acid sequences of the *Arabidopsis* AHAS (SEQ ID NO:1) and *Brassica napus* AHAS I (SEQ ID NO:2) and AHAS III (SEQ ID NO:3 and SEQ ID NO: 4) paralogs. Thus, analogous positions in these and other paralogs can be identified and mutated.

The compositions and methods relate in part to mutations in an AHAS gene that render a plant resistant or tolerant to an herbicide of the AHAS-inhibiting or ALS-inhibiting family of herbicides. The compositions and methods also relate to the use of a gene repair oligonucleobase to make a desired mutation in the chromosomal or episomal sequences of a plant in the gene encoding for an AHAS protein. The mutated protein, which substantially maintains the catalytic activity of the wild-type protein, allows for increased resistance or tolerance of the plant to an herbicide of the AHAS-inhibiting family, and allows for the substantially normal growth or development of the plant, its organs, tissues, or cells as compared to the wild-type plant irrespective of the presence or absence of the herbicide. The compositions and methods also relate to a non-transgenic plant cell in which an AHAS gene has been mutated, a non-transgenic plant regenerated therefrom, as well as a plant resulting from a cross using a regenerated non-transgenic plant to a plant having a mutation in a different AHAS gene or to a plant having a mutated EPSPS gene, for example.

Imidazolinones are among the five chemical families of AHAS-inhibiting herbicides. The other four families are sulfonylureas, triazolopyrimidines, pyrimidinylthiobenzoates and sulfonylamino-carbonyltriazolinones (Tan *et al.,* 2005).

Also provided is a transgenic or non-transgenic plant or plant cell having one or more mutations in the AHAS gene, for example, such as disclosed herein. In certain embodiments, the plant or plant cell having one or more mutations in the AHAS gene has increased resistance or tolerance to a member of the AHAS-inhibiting. In certain embodiments, the plant or plant cell having one or more mutations in the AHAS gene may exhibit substantially normal growth or development of the plant, its organs, tissues or cells, as compared to the corresponding wild-type plant or cell. In particular aspects and embodiments provided are non-transgenic plants having a mutation in an AHAS gene, for example, such as disclosed herein, which in certain embodiments has increased resistance or tolerance to a member of the AHAS-inhibiting herbicide family and may exhibit substantially normal growth or development of the plant, its organs, tissues or cells, as compared to the corresponding wild-type plant or cell, i.e., in the presence of one or more herbicide such as for example, an imidazolinone and/or sulfonyl urea, the mutated AHAS protein has substantially the same catalytic activity as compared to the wild-type AHAS protein.

Further provided are methods for producing a plant having a mutated AHAS gene, for example, having one or more mutations as described herein; preferably the plant substantially maintains the catalytic activity of the wild-type protein irrespective of the presence or absence of a relevant herbicide. In certain embodiments, the methods include introducing into a plant cell a gene repair oligonucleobase with one or more targeted mutations in the AHAS gene (for example, such as disclosed herein) and identifying a cell, seed, or plant having a mutated AHAS gene.

In various embodiments, plants as disclosed herein can be of any species of dicotyledonous, monocotyledonous or gymnospermous plant, including any woody plant species that grows as a tree or shrub, any herbaceous species, or any species that produces edible fruits, seeds or vegetables, or any species that produces colorful or aromatic flowers. For example, the plant may be selected from a species of plant from the group consisting of canola, sunflower, tobacco, sugar beet, cotton, maize, wheat, barley, rice, sorghum, tomato, mango, peach, apple, pear, strawberry, banana, melon, potato, carrot, lettuce, onion, soya spp, sugar cane, pea, field beans, poplar, grape, citrus, alfalfa, rye, oats, turf and forage grasses, flax, oilseed rape, cucumber, morning glory, balsam, pepper, eggplant, marigold, lotus, cabbage, daisy, carnation, tulip, iris, lily, and nut producing plants insofar as they are not already specifically mentioned.

The gene repair oligonucleobase can be introduced into a plant cell using any method commonly used in the art, including but not limited to, microcarriers (biolistic delivery), microfibers, polyethylene glycol (PEG)-mediated uptake, electroporation, and microinjection.

Also provided are methods and compositions related to the culture of cells mutated according to methods as disclosed herein in order to obtain a plant that produces seeds, henceforth a "fertile plant", and the production of seeds and additional plants from such a fertile plant.

Also provided are methods of selectively controlling weeds in a field, the field comprising plants with the disclosed AHAS gene alterations and weeds, the method comprising application to the field of an herbicide to which the plants have been rendered resistant.

Also provided are mutations in the AHAS gene that confer resistance or tolerance to a member of the relevant herbicide to a plant or wherein the mutated AHAS gene has substantially the same enzymatic activity as compared to wild-type AHAS.

### Gene Repair Oligonucleobases

The methods and compositions disclosed herein can be practiced or made with "gene repair oligonucleobases" having the conformations and chemistries as described in detail below. The "gene repair oligonucleobases" as contemplated herein have also been described in published scientific and patent literature using other names including "recombinagenic oligonucleobases;" "RNA/DNA chimeric oligonucleotides;" "chimeric oligonucleotides;" "mixed duplex oligonucleotides" (MDONs); "RNA DNA oligonucleotides (RDOs);" "gene targeting oligonucleotides;" "genoplasts;" "single stranded modified oligonucleotides;" "Single stranded oligodeoxynucleotide mutational vectors" (SSOMVs); "duplex mutational vectors;" and "heteroduplex mutational vectors."

Oligonucleobases having the conformations and chemistries described in U.S. Pat. No. 5,565,350 by Kmiec (Kmiec I) and U.S. Pat. No. 5,731,181 by Kmiec (Kmiec II), hereby incorporated by reference, are suitable for use as "gene repair oligonucleobases" of the invention. The gene repair oligonucleobases in Kmiec I and/or Kmiec II contain two complementary strands, one of which contains at least one segment of RNA-type nucleotides (an "RNA segment") that are base paired to DNA-type nucleotides of the other strand.

Kmiec II discloses that purine and pyrimidine base-containing non-nucleotides can be substituted for nucleotides. Additional gene repair molecules that can be used for the present invention are described in U.S. Pat. Nos. 5,756,325; 5,871,984; 5,760,012; 5,888,983; 5,795,972; 5,780,296; 5,945,339; 6,004,804; and 6,010,907 and in International Patent No. PCT/US00/23457; and in International Patent Publication Nos. WO 98/49350; WO 99/07865; WO 99/58723; WO 99/58702; and WO 99/40789, which are each hereby incorporated in their entirety.

In one embodiment, the gene repair oligonucleobase is a mixed duplex oligonucleotides (MDON) in which the RNA-type nucleotides of the mixed duplex oligonucleotide are made RNase resistant by replacing the 2'-hydroxyl with a fluoro, chloro or bromo functionality or by placing a substituent on the 2'-0. Suitable substituents include the substituents taught by the Kmiec II. Alternative substituents include the substituents taught by U.S. Pat. No. 5,334,711 (Sproat) and the substituents taught by patent publications EP 629 387 and EP 679 657 (collectively, the Martin Applications), which are hereby incorporated by reference. As used herein, a 2'-fluoro, chloro or bromo derivative of a ribonucleotide or a ribonucleotide having a 2'-OH substituted with a substituent described in the Martin Applications or Sproat is termed a "2'-Substituted Ribonucleotide." As used herein the term "RNA-type nucleotide" means a 2'-hydroxyl or 2'-Substituted Nucleotide that is linked to other nucleotides of a mixed duplex oligonucleotide by an unsubstituted phosphodiester linkage or any of the non-natural linkages taught by Kmiec I or Kmiec II. As used herein the term "deoxyribo-type nucleotide" means a nucleotide having a 2'-H, which can be linked to other nucleotides of a gene repair oligonucleobase by an unsubstituted phosphodiester linkage or any of the non-natural linkages taught by Kmiec I or Kmiec II.

In a particular embodiment of the present invention, the gene repair oligonucleobase is a mixed duplex oligonucleotides (MDON) that is linked solely by unsubstituted phosphodiester bonds. In alternative embodiments, the linkage is by substituted phosphodiesters, phosphodiester derivatives and non-phosphorus-based linkages as taught by Kmiec II. In yet another embodiment, each RNA-type nucleotide in the mixed duplex oligonucleotide is a 2'-Substituted Nucleotide. Particular preferred embodiments of 2'-Substituted Ribonucleotides are 2'-fluoro, 2'-methoxy, 2'-propyloxy, 2'-allyloxy, 2'-hydroxylethyloxy, 2'-methoxyethyloxy, 2'-fluoropropyloxy and 2'-trifluoropropyloxy substituted ribonucleotides. More preferred embodiments of 2'-Substituted Ribonucleotides are 2'-fluoro, 2'-methoxy, 2'-methoxyethyloxy, and 2'-allyloxy substituted nucleotides. In another embodiment the mixed duplex oligonucleotide is linked by unsubstituted phosphodiester bonds.

Although mixed duplex oligonucleotides (MDONs) having only a single type of 2'-substituted RNA-type nucleotide are more conveniently synthesized, the methods of the invention can be practiced with mixed duplex oligonucleotides having two or more types of RNA-type nucleotides. The function of an RNA segment may not be affected by an interruption caused by the introduction of a deoxynucleotide between two RNA-type trinucleotides, accordingly, the term RNA segment encompasses terms such as "interrupted RNA segment." An uninterrupted RNA segment is termed a contiguous RNA segment. In an alternative embodiment an RNA segment can contain alternating RNase-resistant and unsubstituted 2'-OH nucleotides. The mixed duplex oligonucleotides preferably have fewer than 100 nucleotides and more preferably fewer than 85 nucleotides, but more than 50 nucleotides. The first and second strands are Watson-Crick base paired. In one embodiment the strands of the mixed duplex oligonucleotide are covalently bonded by a linker, such as a single stranded hexa, penta or tetranucleotide so that the first and second strands are segments of a single oligonucleotide chain having a single 3' and a single 5' end. The 3' and 5' ends can be protected by the addition of a "hairpin cap" whereby the 3' and 5' terminal nucleotides are Watson-Crick paired to adjacent nucleotides. A second hairpin cap can, additionally, be placed at the junction between the first and second strands distant from the 3' and 5' ends, so that the Watson-Crick pairing between the first and second strands is stabilized.

The first and second strands contain two regions that are homologous with two fragments of the target gene, i.e., have the same sequence as the target gene. A homologous region contains the nucleotides of an RNA segment and may contain one or more DNA-type nucleotides of connecting DNA segment and may also contain DNA-type nucleotides that are not within the intervening DNA segment. The two regions of homology are separated by, and each is adjacent to, a region having a sequence that differs from the sequence of the target gene, termed a "heterologous region." The heterologous region can contain one, two or three mismatched nucleotides. The mismatched nucleotides can be contiguous or alternatively can be separated by one or two nucleotides that are homologous with the target gene. Alternatively, the heterologous region can also contain an insertion or one, two, three or of five or fewer nucleotides. Alternatively, the sequence of the mixed duplex oligonucleotide may differ from the sequence of the target gene only by the deletion of one, two, three, or five or fewer nucleotides from the mixed duplex oligonucleotide. The length and position of the heterologous region is, in this case, deemed to be the length of the deletion, even though no nucleotides of the mixed duplex oligonucleotide are within the heterologous region. The distance between the fragments of the target gene that are complementary to the two homologous regions is identical to the length of the heterologous region where a substitution or substitutions is intended. When the heterologous region contains an insertion, the homologous regions are thereby separated in the mixed duplex oligonucleotide farther than their complementary homologous fragments are in the gene, and the converse is applicable when the heterologous region encodes a deletion.

The RNA segments of the mixed duplex oligonucleotides are each a part of a homologous region, i.e., a region that is identical in sequence to a fragment of the target gene, which segments together preferably contain at least 13 RNA-type nucleotides and preferably from 16 to 25 RNA-type nucleotides or yet more preferably 18-22 RNA-type nucleotides or most preferably 20 nucleotides. In one embodiment, RNA segments of the homology regions are separated by and adjacent to, i.e., "connected by" an intervening DNA segment. In one embodiment, each nucleotide of the heterologous region is a nucleotide of the intervening DNA segment. An intervening DNA segment that contains the heterologous region of a mixed duplex oligonucleotide is termed a "mutator segment."

In another embodiment of the present invention, the gene repair oligonucleobase (GRON) is a single stranded oligodeoxynucleotide mutational vector (SSOMV), which is disclosed in International Patent Application PCT/US00/23457, U.S. Pat. Nos. 6,271,360, 6,479,292, and 7,060,500 which is incorporated by reference in its entirety. The sequence of the SSOMV is based on the same principles as the mutational vectors described in U.S. Pat. Nos. 5,756,325; 5,871,984; 5,760,012; 5,888,983; 5,795,972; 5,780,296; 5,945,339; 6,004,804; and 6,010,907 and in International Publication Nos. WO 98/49350; WO 99/07865; WO 99/58723; WO 99/58702; and WO 99/40789. The sequence of the SSOMV contains two regions that are homologous with the target sequence separated by a region that contains the desired genetic alteration termed the mutator region. The mutator region can have a sequence that is the same length as the sequence that separates the homologous regions in the target sequence, but having a different sequence. Such a mutator region can cause a substitution. Alternatively, the homologous regions in the SSOMV can be contiguous to each other, while the regions in the target gene having the same sequence are separated by one, two or more nucleotides. Such an SSOMV causes a deletion from the target gene of the nucleotides that are absent from the SSOMV. Lastly, the sequence of the target gene that is identical to the homologous regions may be adjacent in the target gene but separated by one, two, or more nucleotides in the sequence of the SSOMV. Such an SSOMV causes an insertion in the sequence of the target gene.

The nucleotides of the SSOMV are deoxyribonucleotides that are linked by unmodified phosphodiester bonds except that the 3' terminal and/or 5' terminal internucleotide linkage or alternatively the two 3' terminal and/or 5' terminal internucleotide linkages can be a phosphorothioate or phosphoamidate. As used herein an internucleotide linkage is the linkage between nucleotides of the SSOMV and does not include the linkage between the 3' end nucleotide or 5' end nucleotide and a blocking substituent. In a specific embodiment the length of the SSOMV is between 21 and 55 deoxynucleotides and the lengths of the homology regions are, accordingly, a total length of at least 20 deoxynucleotides and at least two homology regions should each have lengths of at least 8 deoxynucleotides.

The SSOMV can be designed to be complementary to either the coding or the non-coding strand of the target gene. When the desired mutation is a substitution of a single base, it is preferred that both the mutator nucleotide and the targeted nucleotide be a pyrimidine. To the extent that is consistent with achieving the desired functional result, it is preferred that both the mutator nucleotide and the targeted nucleotide in the complementary strand be pyrimidines. Particularly preferred are SSOMVs that encode transversion mutations, i.e., a C or T mutator nucleotide is mismatched, respectively, with a C or T nucleotide in the complementary strand.

In addition to the oligodeoxynucleotide, the SSOMV can contain a 5' blocking substituent that is attached to the 5' terminal carbons through a linker. The chemistry of the linker is not critical other than its length, which should preferably be at least 6 atoms long and that the linker should be flexible. A variety of non-toxic substituents such as biotin, cholesterol or other steroids or a non-intercalating cationic fluorescent dye can be used. Particularly preferred reagents to make SSOMVs are the reagents sold as Cy3™ and Cy5™ by Glen Research, Sterling Va. (now GE Healthcare), which are blocked phosphoroamidites that upon incorporation into an oligonucleotide yield 3,3,3',3'-tetramethyl N,N'-isopropyl substituted indomonocarbocyanine and indodicarbocyanine dyes, respectively. Cy3 is particularly preferred. When the indocarbocyanine is N-oxyalkyl substituted it can be conveniently linked to the 5' terminal of the oligodeoxynucleotide as a phosphodiester with a 5' terminal phosphate. The chemistry of the dye linker between the dye and the oligodeoxynucleotide is not critical and is chosen for synthetic convenience. When the commercially available Cy3 phosphoramidite is used as directed, the resulting 5' modification consists of a blocking substituent and linker together which are a N-hydroxypropyl, N'-phosphatidylpropyl 3,3,3',3'-tetramethyl indomonocarbocyanine.

In a preferred embodiment the indocarbocyanine dye is tetra substituted at the 3 and 3' positions of the indole rings. Without limitations as to theory these substitutions prevent the dye from being an intercalating dye. The identity of the substituents at these positions is not critical. The SSOMV can in addition have a 3' blocking substituent. Again the chemistry of the 3' blocking substituent is not critical.

The mutations herein described might also be obtained by mutagenesis (random, somatic or directed) and other DNA editing or recombination technologies including, but not limited to, gene targeting using site-specific homologous recombination by zinc finger nucleases.

### Delivery of Gene Repair Oligonucleobases into Plant Cells

Any commonly known method used to transform a plant cell can be used for delivering the gene repair oligonucleobases. Illustrative methods are listed below.

### Microcarriers and Microfibers

The use of metallic microcarriers (microspheres) for introducing large fragments of DNA into plant cells having cellulose cell walls by projectile penetration is well known to those skilled in the relevant art (henceforth biolistic delivery). U.S. Pat. Nos. 4,945,050; 5,100,792 and 5,204,253 describe general techniques for selecting microcarriers and devices for projecting them.

Specific conditions for using microcarriers in the methods of the present invention are described in International Publication WO 99/07865. In an illustrative technique, ice cold microcarriers (60 mg/mL), mixed duplex oligonucleotide (60 mg/mL) 2.5 M CaCl₂ and 0.1 M spermidine are added in that order; the mixture gently agitated, e.g., by vortexing, for 10 minutes and then left at room temperature for 10 minutes, whereupon the microcarriers are diluted in 5 volumes of ethanol, centrifuged and resuspended in 100% ethanol. Good results can be obtained with a concentration in the adhering solution of 8-10 µg/µL microcarriers, 14-17 µg/mL mixed duplex oligonucleotide, 1.1-1.4 M CaCl₂ and 18-22 mM spermidine. Optimal results were observed under the conditions of 8 µg/µL microcarriers, 16.5 µg/mL mixed duplex oligonucleotide, 1.3 M CaCl₂ and 21 mM spermidine.

Gene repair oligonucleobases can also be introduced into plant cells for the practice of the present invention using microfibers to penetrate the cell wall and cell membrane. U.S. Pat. No. 5,302,523 to Coffee et al. describes the use of 30.times.0.5 µm and 10.times.0.3 µm silicon carbide fibers to facilitate transformation of suspension maize cultures of Black Mexican Sweet. Any mechanical technique that can be used to introduce DNA for transformation of a plant cell using microfibers can be used to deliver gene repair oligonucleobases for transmutation.

An illustrative technique for microfiber delivery of a gene repair oligonucleobase is as follows: Sterile microfibers (2 µg) are suspended in 150 µL of plant culture medium containing about 10 µg of a mixed duplex oligonucleotide. A suspension culture is allowed to settle and equal volumes of packed cells and the sterile fiber/nucleotide suspension are vortexed for 10 minutes and plated. Selective media are applied immediately or with a delay of up to about 120 h as is appropriate for the particular trait.

### Protoplast Electroporation

In an alternative embodiment, the gene repair oligonucleobases can be delivered to the plant cell by electroporation of a protoplast derived from a plant part. The protoplasts are formed by enzymatic treatment of a plant part, particularly a leaf, according to techniques well known to those skilled in the art. See, e.g., Gallois et al., 1996, in Methods in Molecular Biology 55:89-107, Humana Press, Totowa, N.J.; Kipp et al., 1999, in Methods in Molecular Biology 133:213-221, Humana Press, Totowa, N.J. The protoplasts need not be cultured in growth media prior to electroporation. Illustrative conditions for electroporation are 3.times.10.sup.5 protoplasts in a total volume of 0.3 mL with a concentration of gene repair oligonucleobase of between 0.6-4 µg/mL.

### Protoplast PEG-mediated DNA uptake

In an alternative embodiment, nucleic acids are taken up by plant protoplasts in the presence of the membrane-modifying agent polyethylene glycol, according to techniques well known to those skilled in the art (see, e.g., Gharti-Chhetri *et al.,* 1992; Datta *et al.,* 1992).

### Microinjection

In an alternative embodiment, the gene repair oligonucleobases can be delivered by injecting it with a microcapillary into plant cells or into protoplasts (see, e.g., Miki *et al.,* 1989; Schnorf *et al.,* 1991).

### Selection of Herbicide Resistant Plants and Application of Herbicide

Plants and plant cells can be tested for resistance or tolerance to an herbicide using commonly known methods in the art, e.g., by growing the plant or plant cell in the presence of an herbicide and measuring the rate of growth as compared to the growth rate in the absence of the herbicide.

As used herein, substantially normal growth of a plant, plant organ, plant tissue or plant cell is defined as a growth rate or rate of cell division of the plant, plant organ, plant tissue, or plant cell that is at least 35%, at least 50%, at least 60%, or at least 75% of the growth rate or rate of cell division in a corresponding plant, plant organ, plant tissue or plant cell expressing the wild-type AHAS protein.

As used herein, substantially normal development of a plant, plant organ, plant tissue or plant cell is defined as the occurrence of one or more development events in the plant, plant organ, plant tissue or plant cell that are substantially the same as those occurring in a corresponding plant, plant organ, plant tissue or plant cell expressing the wild-type AHAS protein.

In certain embodiments plant organs provided herein include, but are not limited to, leaves, stems, roots, vegetative buds, floral buds, meristems, embryos, cotyledons, endosperm, sepals, petals, pistils, carpels, stamens, anthers, microspores, pollen, pollen tubes, ovules, ovaries and fruits, or sections, slices or discs taken therefrom. Plant tissues include, but are not limited to, callus tissues, ground tissues, vascular tissues, storage tissues, meristematic tissues, leaf tissues, shoot tissues, root tissues, gall tissues, plant tumor tissues, and reproductive tissues. Plant cells include, but are not limited to, isolated cells with cell walls, variously sized aggregates thereof, and protoplasts.

Plants are substantially "tolerant" to a relevant herbicide when they are subjected to it and provide a dose/response curve which is shifted to the right when compared with that provided by similarly subjected non-tolerant like plant. Such dose/response curves have "dose" plotted on the X-axis and "percentage kill", "herbicidal effect", etc., plotted on the y-axis. Tolerant plants will require more herbicide than non-tolerant like plants in order to produce a given herbicidal effect. Plants that are substantially "resistant" to the herbicide exhibit few, if any, necrotic, lytic, chlorotic or other lesions, when subjected to herbicide at concentrations and rates which are typically employed by the agrochemical community to kill weeds in the field. Plants which are resistant to an herbicide are also tolerant of the herbicide.

### EXAMPLES

Following are examples, which illustrate procedures for practicing the invention. These examples should not be construed as limiting. All percentages are by weight and all solvent mixture proportions are by volume unless otherwise noted.

### Example 1: Preparation of Herbicide-Resistant Brassica Samples

Unless otherwise specified, as used herein, numbering of the gene(s) is based on the amino acid sequence of the *Arabidopsis* acetolactate synthase (ALS) or acetohydroxyacid synthase (AHAS) At3g48560 (SEQ ID NO:1). In laboratory notebook references prior to October 2005, the S653 position (based on the *Arabidopsis* amino acid sequence) is referred to as S621 based on the corn amino acid sequences ZmAHAS108 and ZmAHAS109 (Fang *et al.,* 1992).

One goal was to make the imazethapyr (Imi) resistant amino acid substitution S653N in either or both BnAHAS I and III of spring Canola (*Brassica napus,* Spring Oilseed Rape - designated BN-2) and Winter Oilseed Rape (WOSR, also *Brassica napus* - designated BN-11).

In order to amplify the target regions of BnAHAS I and III from *Brassica napus* (initially elite Canola line BN-2), the oligo pair BnALS1 and BnALS2 was designed (SEQ ID NOs: 9 and 10). Since BnAHAS I and III do not contain introns, BnALS1 and BnALS2 amplify the target regions of 284 bp from both genomic DNA and cDNA flanking the S653 site. This primer pair was also designed to enable amplification of the ALS target region from *Arabidopsis.* The primers were received and resuspended in sterile water.

Initially the BnALS1/BnALS2 primer pair was used to PCR amplify the BnAHAS I and III C-terminal target regions from BN-2. Later, the ALS target regions were amplified from additional BN-2 samples and cloned into pGEM-T easy. Cloned inserts from 12 colonies from each of 3 genomic DNA and cDNA samples grown in overnight cultures were plasmid prepped and sequenced, confirming the target sequence. Glycerol stocks were prepared for BN-2 ALS 2c-21 as the BnAHAS I representative and BN-2 ALS YB10-2g-14 as the BnAHAS III representative for these 284 bp target regions.

The genomic and cDNA target region BnALS sequences from line BN-2 were analyzed and PCR errors recorded. Based on the sequence of BnAHAS I and III from BN-2 a single GRON (Gene Repair Oligonucleotide) BnALS1621/C/41/5'Cy3/3'idC (SEQ ID NO: 5) was designed to make a serine to asparagine amino acid substitution (AGT -> AAT) at position 653 (See Table 1). The initial syntheses were resuspended and their concentrations determined. The resuspended oligos were stored frozen at -70°C. The non-coding version of this GRON was called BnALS1621/NC/41/5'Cy3/3'idC (SEQ ID NO: 6). At a later date, the BnALS1621/NC was compared with all Genbank sequences where the only sequences with >16 nucleotides of 41 hybridizing were BnAHAS I and III and where the oligo had the single mismatch G->A designed to introduce the S653N amino acid substitution.

**TABLE 1. GRON SEQUENCES**

| GRON | Sequence |
|---|---|
| BnALS 1621/C/41/5'Cy3/3'idC | VTGTGTTACCGATGATCCCAAATGGTGGCACTTTC AAAGATGH (SEQ ID NO: 5) |
| BnALS 1621/NC/41/5'Cy3/3'idC | VCATCTTTGAAAGTGCCACCATTTGGGATCATCGG TAACACAH (SEQ ID NO: 6) |
| BnALS1574/C/41/5'Cy3/3'idC | VCTTGGGATGGTCATGCAATTGGAAGATCGGTTCT ACAAAGCH (SEQ ID NO: 7) |
| BnALS1574/'NC/41/5'Cy3/3'idC | VGCTTTGTAGAACCGATCTTCCAATTGCATGACCA TCCCAAGH (SEQ ID NO: 8) |

| | |
|---|---|
| The converting base is shown in bold. V=CY3; H=3'DMT dC CPG | |

BnALS target regions, from a Cibus elite Winter Oil Seed Rape (WOSR) line BN-11, were amplified from genomic DNA and cDNA of single plants known to represent line BN-11. The BN-11 BnALS sequences were analyzed and PCR errors recorded.

In addition to characterizing the BnALS target regions from BN-2 and BN-11, the same target regions were also characterized in the commercial variety Clearfield Canola (BN-15). The Clearfield BnAHAS sequences were analyzed and PCR errors recorded showing the expected amino acid changes, S653N (A**G**T -> A**A**T) in BnAHAS I and W574L (T**G**G -> T**T**G) in BnAHAS III. Ultimately full coding sequences for BnAHAS I and III were amplified using BnALS3/BnALSOR2 (SEQ ID NOs: 11 and 12) and BnALS3/BnALSOR3 (SEQ ID NOs: 11 and 13) respectively, cloned and sequenced from lines BN-2, BN-11 and BN-15 to serve as reference sequences for comparative purposes

Imi resistant BN-2 (Canola) callus samples BnALS1621N-44 to 53, arising from various treatments, were extracted using the Edwards *et al.* (1991) method. Genomic DNA from this material was screened using an allele specific PCR (AS-PCR) mix (MM#23 composed of oligos BnALSOF1, BnALSOR2, BnALSOR3 and BnALSIR1A) (SEQ ID NOs: 14, 12, 13, and 15, respectively) to specifically detect the S653N change and allele specific PCR mix (MM#29 composed of oligos BnALSOF2, BnALSOR4 and BnALSIF1T) (SEQ ID NOs: 16, 17, and 18, respectively) to specifically detect the W574L change in either BnAHAS I or III. These initial AS-PCR results were inconclusive, therefore, additional samples BnALS1621 N-54 to 58 for most of samples BnALS1621 N-44 to 53 were received, and extracted using the Edwards *et al.* (1991) method with additional purification steps to obtain more pure genomic DNA.

At this point, samples BnALS1621N-54 to 58 were amplified with a BnALSOF2, BnALSOR2 and BnALSOR3 primer combination, and a small amount of amplified product was obtained. These products were cloned into pGEM-T easy and 12 white colonies per line setup as overnight cultures, plasmid prepped (using the Qiagen plasmid miniprep kit), and sequenced using BigDye 3.1 sequencing chemistry.

Preliminary sequence analysis determined that BnALS1621N-55-13 had an S653N mutation in BnAHAS III (A**G**T -> A**A**T) and two other clones from this line, BnALS1621N-55-15 and 19, were wildtype in BnAHAS III. Full sequence analysis was recorded. To confirm that this single positive did not result from a PCR error, an additional 38 colonies from this line were screened by AS-PCR. The strongest 8 positives from this AS-PCR screen BnALS1621N-55-1 to 8 were setup as overnight cultures, and plasmid DNA was isolated and sequenced. Seven of the 8 positive colonies, BnALS 1621N-55-2 to 8 by AS-PCR were positive by sequencing for the S653N mutation in BnAHAS III; the other BnALS1621N-55-1 was a wildtype BnAHAS I sequence. Together these results indicated that line BnALS1621N-55 is heterozygous for the S653N mutation in BnAHAS III.

Sample BnALS1621N-55 was a parallel callus sample from the same initial Imi resistant callus as BnALS1621N-49. Genomic DNA from these samples was amplified with the BnALSOF2, BnALSOR2 and BnALSOR3 primer combination, and the fragments cloned into pGEM-T easy and transformed. MM#23 was used to screen 38 white colonies for the S653N mutation, with 4 positive colonies being identified (BnALS1621N-49-9 to 12). These colonies were sequenced. Three of the four colonies (BnALS 1621N-49-9, 10 and 12) had the S653N mutation in BnAHAS III.

A similar method of target region cloning followed by AS-PCR with sequence confirmation was used to identify other lines with the S653N polymorphism. Among these was line BnALS-97. BnALS-97 was regenerated into a plant and allowed to set seed. Since this line was a prototype (as a plant), the full coding sequences of both BnAHAS I and III were cloned and sequenced. In this line, the only polymorphism compared to the BN-2 wildtype sequence was the AGT -> AAT codon change which results in the S653N amino acid substitution in BnAHAS III.

Preliminary sequence analysis of random cloned BnALSOF2/BnALSOR2 and BnALSOF2/BnALSOR3 amplicons from line BnALS1621N-57 indicated that both clones BnALS1621N-57-43 and 46 had the W574L mutation (TGG -> TTG) in BnAHAS I. Full sequence analysis was recorded, and indicated line 57 is heterozygous, since clones 38 and 41 were W574. Therefore, the BnALSOF2, BnALSOR2 and BnALSOR3 primer combination was used to amplify this fragment from BnALS1621N-57 and BnALS1621N-45, a parallel callus sample from the same initial Imi resistant callus as BnALS1621N-57, ligated into pGEM-T easy, transformed and plated with blue/white selection.

MM#29 was used to screen 19 white colonies for the W574L from each of the plates for BnALS1621N-57 and BnALS1621N-45, with 4 positive colonies for each (BnALS1621N-45-1, 2, 9 and 18, and BnALS1621N-57-1, 7, 13 and 16) being plasmid prepped and sequenced. Sequence analysis showed that all 8 colonies had the W574L mutation in BnAHAS I. The annealing temperature for MM#29 was optimized, and an additional 19 colonies screened using these new conditions. Three positive colonies (BnALS1621N-57-17, 18 and 19) were plasmid prepped and sequenced.

Additional Imi resistant Canola samples BnALS-68 to 91 were received and genomic DNA extracted using the Edwards *et al.* (1991) method. From each sample, the BnALSOF2, BnALSOR2 and BnALSOR3 primer combinations were used to amplify fragments of BnAHAS I and III and clone them into pGEM-T easy.

Using MM#23 in AS-PCR reactions to detect the S653N mutation, 12 colonies per line were screened, and results showed 2 positives from line BnALS-81 (BnALS-81-203 and 208) and 4 from BnALS-76 (BnALS-76-153, 154, 156 and 162) to contain the S653N mutation in BnAHAS III by sequencing. Full sequence analysis was recorded. By AS-PCR screening with MM#23, both PCR-amplified target regions or of bacterial colonies with single cloned inserts, line BnALS-159 (molecular biology sample 102; colony 655) was identified as having the S653N mutation in BnAHAS I, Cibus' initial S653N event in this gene.

Finally, in line BnALS-83 (molecular biology sample 91), wildtype (colony 408) and mutant (colonies 403, 405 and 406) were identified to indicate this line was heterozygous for the S653T mutation (AGT -> ACT) in BnAHAS I. As plants, line BnALS-83 was confirmed to be heterozygous in BnAHAS I for the S653T mutation. Further screening with MM#23 (S653N) and MM#29 (W574L) of new Imi resistance BN-2 Canola lines BnALS-76 and BnALS-123 was performed. Since the aforementioned expected mutations were not found in these samples, 12 BnALSOF2/OR2/3 were cloned and sequenced. All 7 clones of BnAHAS III for BnALS-123 (Colonies 5, 17-19, 21, 22, 26 and 28) were positive for the S653T mutation. However, seed from the R1 plants was genotyped as heterozygous.

Additionally, N-terminal PCR fragments of each of BnAHAS I and III were cloned and sequenced for BnALS-58, 68 and 69 using BnALS3/BnALS8 (SEQ ID NOs: 11 and 19 respectively) and BnALS3/BnALS9 (SEQ ID NOs: 11 and 20, respectively) oligo combinations respectively. A new tissue sample for BnALS-96 was obtained and N-terminal PCR fragments of BnAHAS I and III were cloned and sequenced to show line BnALS-96 had an A205V mutation (GCC -> GTC) in BnAHAS I. All four clones of the full length coding sequence BnALS3/OR2 amplicon from plant material derived from callus line BnALS-68 had an A205V change (GCG -> GTG) in BnAHAS III, identical to the change in BnALS-69.

Lines determined to have a mutation, the experiment they were derived from and the treatment provided are summarized in Table 2. Line BnALS-159 died as a callus line and did not regenerate shoots. Table 3 shows exemplary oligos used in the experiments disclosed herein.

**TABLE 2. MUTATIONS IN IMI RESISTANT TISSUE SAMPLES OF BN CANOLA.**

| Line (CS#) | Mutation | SNP | Gene | Line |
|---|---|---|---|---|
| BnALS-96 | A205V | G**C**C → G**T**C | I | BN2 |
| BnALS-68 | A205V | G**C**G → G**T**G | III | BN2 |
| BnALS-69 | A205V | G**C**G → G**T**G | III | BN2 |
| BnALS-58 | A205D | G**C**C → G**A**C | I | BN2 |
| BnALS-63 | W574C | TG**G** → TG**T** | III | BN2 |
| BnALS-57 | W574L | T**G**G → T**T**G | I | BN2 |
| BnALS-67 | W574L | T**G**G → T**T**G | I | BN2 |
| BN02-204-A01 | W574L;R577W | T**G**G → T**T**G; **C**GG → **T**GG | III | BN2 |
| BN02-224-C01 | W574L (HET) | T**G**G → T**T**G | III | BN2 |
| BN02-224-B01 | W574M (HET) | T**G**G → A**T**G | III | BN2 |
| BnALS-76 | W574S | T**G**G → TC**G** | III | BN2 |
| BnALS-159* | S653N | A**G**T → A**A**T | I | BN2 |
| BnALS-55 | S653N | A**G**T → A**A**T | III | BN2 |
| BnALS-97 | S653N | A**G**T → A**A**T | III | BN2 |
| BnALS-61 | S653N | A**G**T → A**A**T | III | BN2 |
| BnALS-84 | S653N | A**G**T → A**A**T | III | BN2 |
| BnALS-83 | S653T | A**G**T → A**C**T | I | BN2 |
| BnALS-123 | S653T | AG**T** → A**C**T | III | BN2 |
| BN02-139-E07 | W574C(het);S653N | TG**G** → TG**C**; A**G**T → A**A**T | III | BN2 |
| BN02-139-D05 | A205V;S653N | G**C**G → G**T**G;A**G**T → A**A**T | III | BN2 |
| BN02-139-F08 | A205V;S653N | G**C**G → G**T**G;A**G**T → A**A**T | III | BN2 |
| BN02-139-C03 | A205V(het);S653N | G**C**C → G**T**C;A**G**T → A**A**T | I;III | BN2 |
| BN02-139-D06 | W574C;S653N | TG**G** → TG**C**;A**G**T → A**A**T | I;III | . BN2 |
| BN02-139-E10 | W574L;S653N | T**G**G → T**T**G;A**G**T → A**A**T | I;III | BN2 |
| BN02-139-A13 | W574L(het);S653N | T**G**G → T**T**G;A**G**T → A**A**T | III | BN2 |
| BN02-139-A12 | D376E;S653N | GA**C** → GA**G**;A**G**T → A**A**T | III | BN2 |
| BN02-139-F09 | A122V;S653N | G**C**T → G**T**T;A**G**T → A**A**T | I;III | BN2 |
| BN02-139-A01 | A205D, S653N | G**C**G → G**A**C;A**G**T → A**A**T | I;III | BN2 |
| BN02-139-D-04 | W574C; S653N | TG**G** → TG**C**;A**G**T → A**A**T | I;III | BN2 |
| BN02-139-B11 | W574C; S653N | TG**G** → TG**C**;A**G**T → A**A**T | I;III | BN2 |

**TABLE 3: EXEMPLARY OLIGOS**

| Name | Length | Oligo Sequence |
|---|---|---|
| BnALS1 (SEQ ID NO: 9) | 24 | ATGCAATGGGAAGATCGGTTCTAC |
| BnALS2 (SEQ ID NO: 10) | 29 | CCATCYCCTTCKGTTATKACATCKTTGAA |
| BnALS3 (SEQ ID NO: 11) | 20 | CTAACCATGGCGGCGGCAAC |
| BnALSOR2 (SEQ ID NO: 12) | 28 | AGTCTGGGAACAAACCAAAAGCAGTACA |
| BnALSOR3 (SEQ ID NO: 13) | 28 | CGTCTGGGAACAACCAAAAGTAGTACAA |
| BnALSOF1 (SEQ ID NO: 14) | 28 | AGTGACGAAGAAAGAAGAACTCCGAGAA |
| BnALSIR1A (SEQ ID NO: 15) | 30 | CTGTTATTACATCTTTGAAAGTGCCACAAT |
| BnALSOF2 (SEQ ID NO: 16) | 28 | TGACGGTGATGGAAGCTTCATAATGAAC |
| BnALSOR4 (SEQ ID NO: 17) | 28 | GTCCWGGTGTATCCAGCATTGTCTGAAT |
| BnALSIF1T (SEQ ID NO: 18) | 26 | CAGCATCTTGGGATGGTCATGCAGTT |
| BnALS8 (SEQ ID NO: 19) | 21 | CCCATCAAAGTACTCGCACCG |
| BnALS9 (SEQ ID NO: 20) | 21 | CCCATCAACGTACTCGCACCA |

As shoots become available, crosses are being made in all permutations and combinations. Line BnALS-97 is the reference S653N in BnAHAS III. Line BnALS-57 is the reference W574L in BnAHAS I. Both BnALS-68 and 69 are being advanced as reference lines for A205V in BnAHAS III. Line BnALS-83 is the first line that was heterozygous for S653T in BnAHAS I as callus to also be heterozygous as a plant.

### Example 2: Materials and Methods

**Cell Culture Work Description.** Shoots derived both from seeds and from microspore-derived embryos were propagated under sterile conditions *in vitro.* Cuttings were subcultured every 2 - 4 weeks and cultured in petri dishes (25 mm x 90 mm) in a volume of 40 - 45 mL RS medium (Dovzhenko, 2001). The dishes were sealed with Micropore tape (3M Company). Young leaves were used for protoplast isolation.

**Protoplast isolation and purification.** About 600 mg of leaf tissue of 2 - 3 week-old *in vitro* shoots were cut into small strips with a scalpel in a petri dish with 5 mL medium B (Pelletier *et al.,* 1983), pH adjusted to 5.8. After approximately 1 h, medium B was replaced with enzyme solution, consisting of medium B in which 0.5% (w/v) Cellulase YC and 0.75% (w/v) Macerozyme R10 (both from Karlan Research Products, Cottonwood, Arizona), 1 g/L bovine serum albumin, and 1 g/L 2-morpholinoethanesulfonic acid were dissolved. The enzyme solution was vacuum-infiltrated into the leaf tissue, and the dish with leaf pieces in enzyme solution was incubated for at 25°C in darkness. Protoplast purification was performed using an iodixanol density gradient (adapted from Optiprep Application Sheet C18; Purification of Intact Plant Protoplasts; Axis-Shield USA, 10 Commerce Way, Norton, MA 02776). After the density gradient centrifugation, the band with purified protoplasts was removed together with about 5 mL W5 medium (Frigerio *et al.,* 1998). The protoplast yield was determined with a hemocytometer, and the protoplasts were stored for 2 h at 4°C.

**Gene Repair Oligonucleotide GRON introduction.** The protoplast suspension was mixed with an equal volume of W5 medium, transferred to a 50 mL centrifuge tube, and centrifuged for 5 min at the lowest setting of a clinical centrifuge (about 50 x g). The supernatant was removed and replaced with TM medium (Klaus, 2001), adjusting the protoplast density to 5 x 10⁶/mL. Aliquots of 100 µL containing 5 x 10⁶ protoplasts each were distributed into 12 mL round bottom centrifuge tubes. GRONs targeted at a mutation in one or both AHAS genes were then introduced into the protoplasts using a PEG treatment. To introduce the GRONs into the protoplasts, 12.5 µg GRON dissolved in 25 µL purified water and 125 µL of a polyethylene glycol solution (5 g PEG MW 1500, 638 mg mannitol, 207 mg CaNO₃ x 4H₂O and 8.75 mL purified water; pH adjusted to about 9.0) was added. After a 30 min incubation on ice, the protoplast-PEG suspension was washed with W5 medium and resuspended in medium B. The suspension was kept overnight in a refrigerator at about 4°C.

**Embedding of protoplasts in calcium alginate.** One day after the GRON introduction, protoplasts were embedded in calcium alginate. The embedding of protoplasts in gel substrates (e.g., agarose, alginate) has been shown to enhance protoplast survival and to increase division frequencies of protoplast-derived cells. The method applied was based on that described in Dovzhenko (2001).

**Protoplast culture and selection of imazethapyr-resistant calli.** The selection of imazethapyr-resistant calli was carried out using sequential subcultures of the alginates in media according to Pelletier *et al.* (1983). Selection was started one week after the PEG/GRON treatment at a concentration of 0.5 µM imazethapyr. The herbicide did not have an immediate effect. Initially, all microcolonies that had formed during the early culture phase without imazethapyr continued to grow, but slower than controls without added herbicide. One to two weeks after the onset of selection, the colonies slowed down in growth or stopped growing.

Before the end of the selection phase in liquid medium, cells and colonies were released from the alginate by treating them for 30 - 45 min with culture medium containing 50 mM sodium citrate. At the moment of transferring released colonies from liquid to solid medium, the majority of colonies were either dead, or consisted of a greenish center, covered with outer layers of dead cells. On the solidified selection medium E the majority of microcalli that still contained living cells stopped growing and turned brownish. Limited growth of individual calli continued occasionally, but all non-resistant calli eventually turned brown and died. Two to three weeks after the transfer to solidified selection medium (occasionally earlier), actively growing calli appeared among a background of brownish cells and microcalli.

Regeneration of plants from protoplast-derived, herbicide-tolerant calli with a confirmed mutation in an AHAS gene. Imi-tolerant calli that had developed on solidified selection medium and whose DNA upon analysis had shown the presence of a mutation were transferred to herbicide-free medium E (Pelletier *et al.,* 1983) to accelerate development. Individual callus lines varied in their growth rates and morphologies. In general, the development towards shoot regeneration followed these steps:
Undifferentiated, green callus -> callus with dark green areas -> development of roots -> development of shoot initials -> development of stunted shoots with hyperhydric (vitrified) leaves.

The development of individual callus line was variable, but through continuous subculture and multiplication on medium E or modifications of medium E with lower concentrations of alpha-naphthalene acetic acid (NAA) eventually many callus lines produced shoots.

Once shoots with three to four leaves had formed on medium E, they were transferred to RS medium (Dovzhenko, 2001). On this medium, over time shoot and leaf tissue developed that was morphologically 'normal' (i.e., non-hyperhydric). After *in vitro* plantlets had produced roots, standard protocols were used for the adaptation to greenhouse conditions.

### Example 4: Herbicide Spray Data

*B. napus* plants at the 5-6 leaf stage were sprayed with various AHAS inhibiting herbicides. *B. napus* plants sprayed included the parental line BN02 (or BN11 as required), BN15 (Clearfield, a commercial two gene check) and mutants as detailed below. Herbicides were sprayed in the presence of 0.25% AU391 surfactant at the following rates:
Imazamox (Beyond™) 0, 2, 4, 6, 8, 12, 16, 32 and 48 oz active ingredient/Acre (ai/A)
Thifensulfuron 0.028, 0.056, 0.112, 0.168 lb ai/A
Tribenuron 0.015, 0.03, 0.06, 0.12, 0.18 lb ai/A
Nicosulfuron lb 0.06, 0.120, 0.24, 0.36 ai/A
Rimsulfuron 0.015, 0.03, 0.06, 0.12, 0.18 lb ai/A
2:1 weight:weight Thifensulfuron/Tribenuron 0.056, 0.112, 0.224, 0.336 lb ai/A
2.22:1 Thifensulfuron/Nicosulfuron 0.058, 0.116, 0.232 lb ai/A
Primisulfuron 0.035, 0.070, 0.140 lb ai/A
Flumetsulam0.040, 0.080, 0.16 lb ai/A
Cloransulam0.039, 0.078, 0.156 lb ai/A

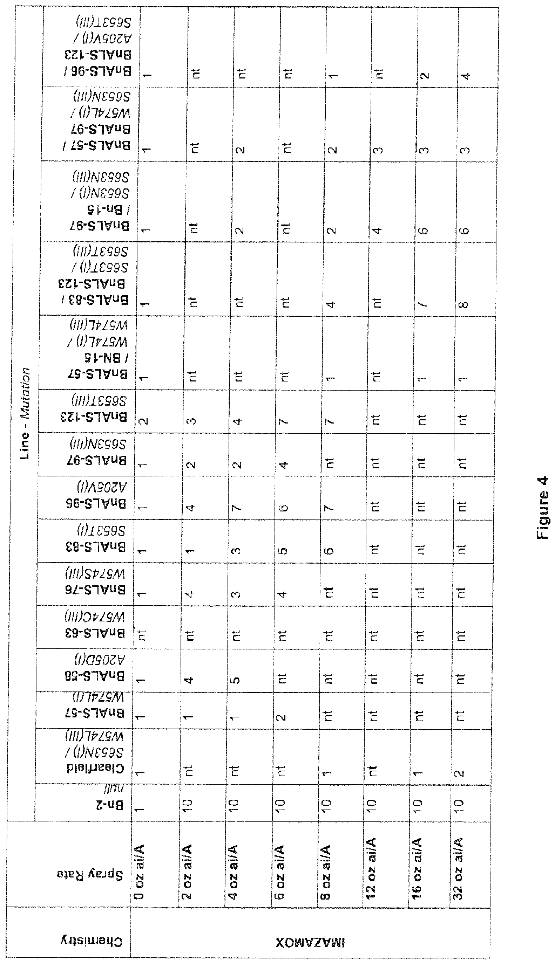

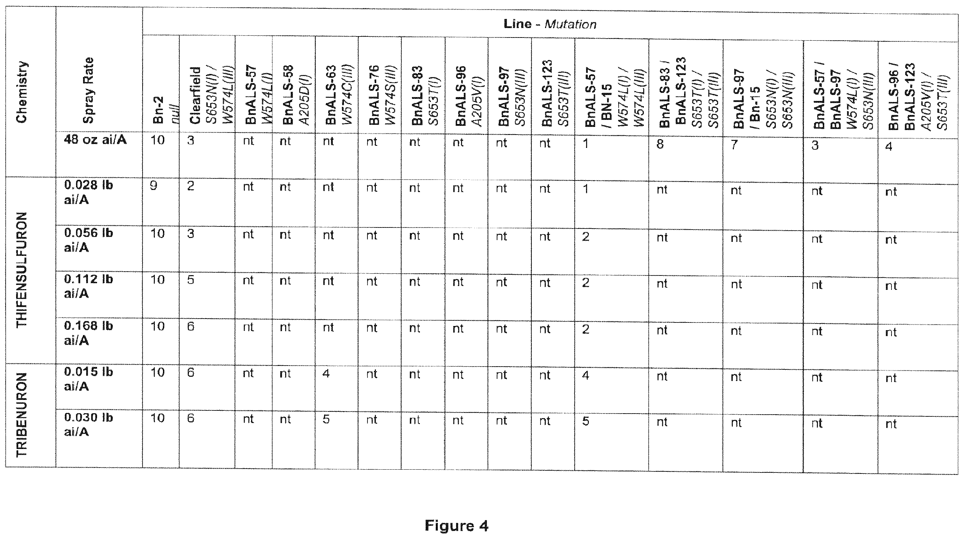

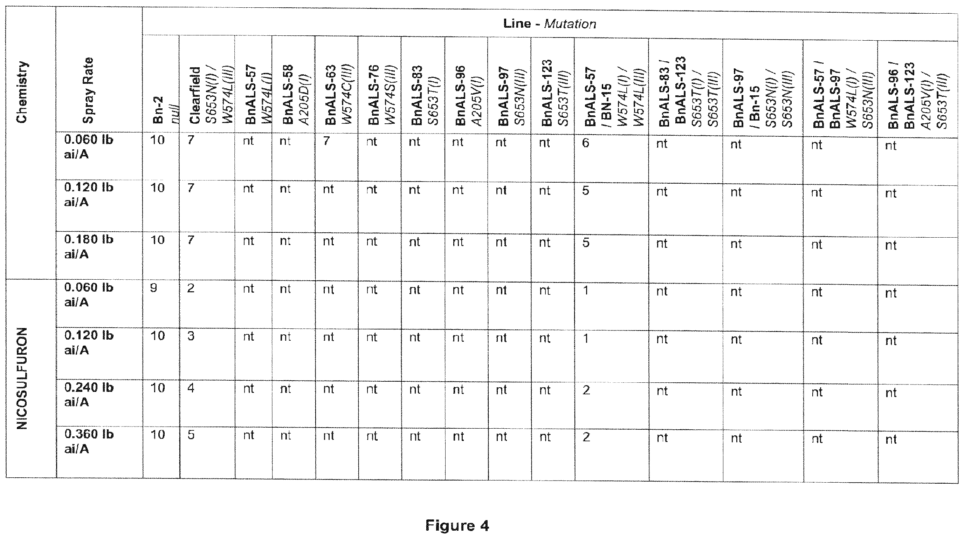

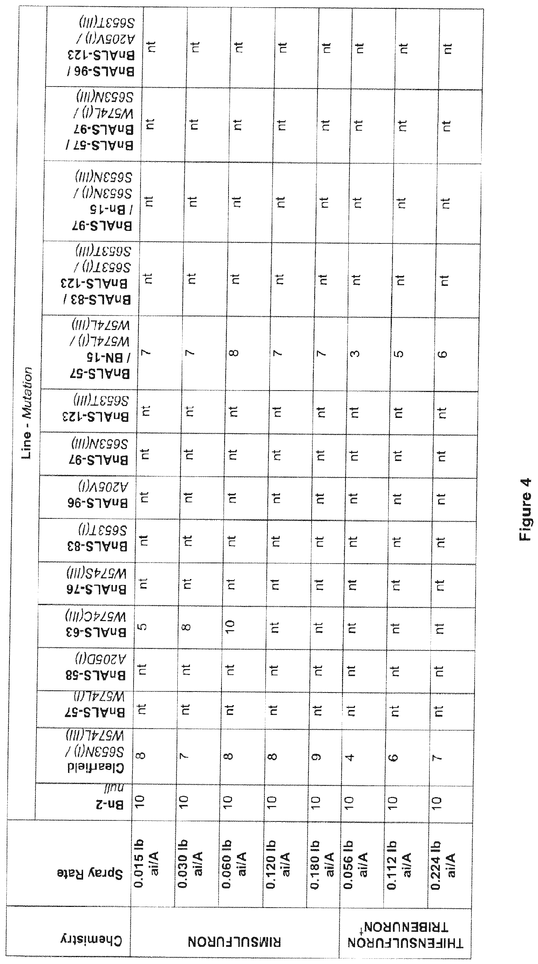

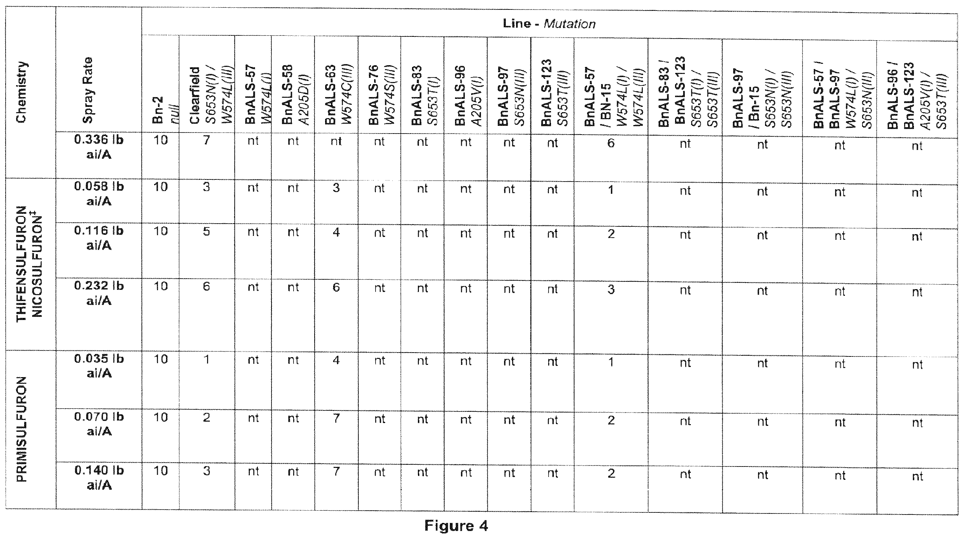

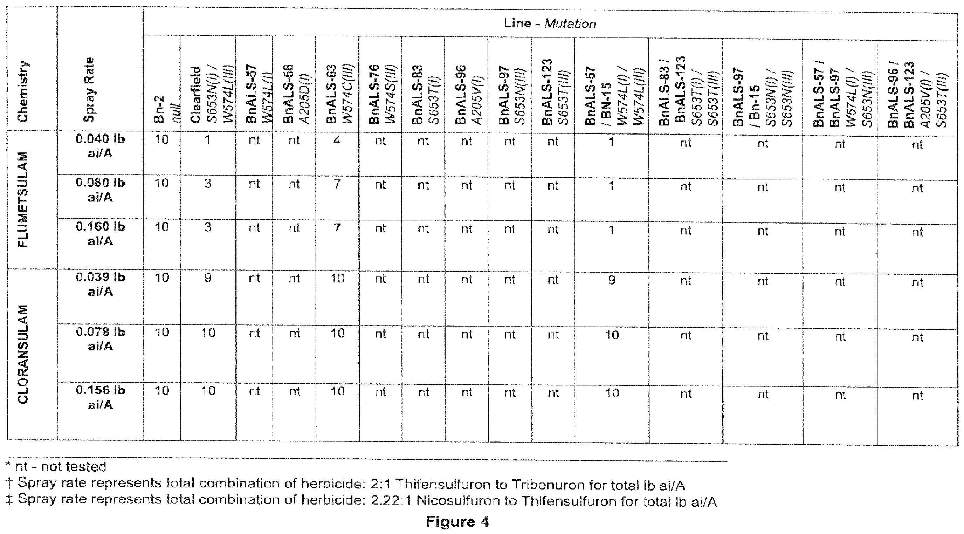

Herbicides were applied by foliar spray with control plants being left unsprayed. Excepting Imazamox, all AHAS inhibiting herbicide trials were evaluated 14 days post spraying using a damage scale of 1-10 with 10 being dead and 1 being the undamaged unsprayed controls. controls. Individual plant lines were scored at each spray rate compared to the performance of the controls at that particular rate. Results of the spray trial are presented in Figure 4.

Chemistry tested: Genotype (# of seedlings for all rates)
Thifensulfuron: BN2 (6), BN15 (6), BN15xBnALS-57 (18)
Tribenuron: BN2 (6), BN15 (6), BN15xBnALS-57 (18), 63 (9)
Nicosulfuron: BN2 (6), BN15 (6), BN15xBnALS-57 (18)
THI/TRI: BN2 (6), BN15 (6), BN15xBnALS-57 (18)
Rimsulfuron: BN2 (6), BN15 (6), BN15xBnALS-57 (18), 63 (9)
TH1/ Nic: BN2 (18), 63 (9), BN15xBnALS-57 (36), BN15 (18)
Primisulfuron: BN2 (9), 63 (9), BN15 (9), BN15xBnALS-57 (18)
Flumetsulam: BN2 (9), 63 (9), BN15 (9), BN15xBnALS-57 (18)
Cloransulam: BN2 (9), 63 (9), BN15 (9), BN15xBnALS-57 (18)

Trials with Imazamox were evaluated as follows:
10d score for: 8, 16, 32, and 48 oz/A of BnALS-83xBnALS-123, BnALS-96xBnALS-123, BnALS-97xBnALS-57, BN15xBnALS-57, BN2, BN15, BnALS-97xBN15
17d score for: 4 and 12 oz/A of BnALS-97xBnALS-57, BN15xBnALS-97, BN2
28d score for: 2, 4, 6, 8 oz/A for all the single factor mutations.

### Imazamox:

2 oz/A: BN2 (18), BnALS-123 (9), BnALS-96 (9), BnALS-97 (18), BnALS-83 (6), BnALS-76 (18), BnALS-58 (9), BnALS-57 (12)
4 oz/A: BN2 (18), BnALS-123 (9), BnALS-96 (9), BnALS-97 (18), BnALS-83 (9), BnALS-76 (18), BnALS-58 (9), BnALS-57 (12), BnALS-97xBN15 (15), BnALS-97xBnALS-57 (14) 6 oz/A: BN2 (9), BnALS-123 (9), BnALS-96 (12), BnALS-97 (9), BnALS-83 (12), BnALS-76 (9), BnALS-57 (12)
8 oz/A: BnALS-123 (9), BnALS-96 (12), BnALS-83 (12), BnALS-83xBnALS-123 (18), BnALS-96xBnALS-123 (18), BN2 (18), BN15 (18), BN15xBnALS-57 (15), BnALS-97xBnALS-57 (18), BN15xBnALS-97 (18)
12 oz/A: BnALS-97xBN15 (15), BnALS-97xBnALS-57 (14), BN2 (12)
16 oz/A: BnALS-83xBnALS-123 (18), BnALS-96xBnALS-123 (18), BN2 (15), BN15 (18), BN15xBnALS-57 (18), BnALS-97xBnALS-57 (18), BN15xBnALS-97 (18)
32 oz/A: BnALS-83xBnALS-123 (18), BnALS-96xBnALS-123 (18), BN2 (13), BN15 (18), BN15xBnALS-57 (18), BnALS-97xBnALS-57 (18), BN15xBnALS-97 (18)
48 oz/A: BnALS-83xBnALS-123 (18), BnALS-96xBnALS-123 (18), BN2 (6), BN15 (18), BN15xBnALS-57 (18), BnALS-97xBnALS-57 (18), BN15xBnALS-97 (18)

### REFERENCES

Datta SK, Datta K, Soltanifar N, Donn G, Potrykus I (1992) Herbicide-resistant Indica rice plants from IRRI breeding line IR72 after PEG-mediated transformation of protoplasts. Plant Molec. Biol. 20:619-629
Dovzhenko A (2001) Towards plastid transformation in rapeseed (Brassica napus L.) and sugarbeet (Beta vulgaris L.). PhD Dissertation, LMU Munich, Faculty of Biology
Edwards K, Johnstone C, Thompson C (1991) A simple and rapid method for the preparation of plant genomic DNA for PCR analysis. Nucleic Acids Res. 19:1349.
Frigerio L, Vitale A, Lord JM, Ceriotti A, Roberts LM (1998) Free ricin A chain, proricin, and native toxin have different cellular fates when expressed in tobacco protoplasts. J Biol Chem 273:14194-14199
Fang LY, Gross PR, Chen CH, Lillis M (1992) Sequence of two acetohydroxyacid synthase genes from Zea mays. Plant Mol Biol. 18(6):1185-7
Gharti-Chhetri GB, Cherdshewasart W, Dewulf J, Jacobs M, Negrutiu I (1992) Polyethylene glycol-mediated direct gene transfer in Nicotiana spp. Physiol. Plant. 85:345-351
Klaus S (2003) Markerfreie transplastome Tabakpflanzen (Marker-free transplastomic tobacco plants). PhD Dissertation, LMU Munich, Faculty of Biology
Miki B, Huang B, Bird S, Kemble R, Simmonds D, Keller W (1989) A procedure for the microinjection of plant cells and protoplasts. Meth. Cell Science 12:139-144
Pelletier G, Primard C, Vedel F, Chetrit P, Remy R, Rouselle P, Renard M (1983) Intergeneric cytoplasm hybridization in Cruciferae by protoplast fusion. Mol. Gen. Genet. 191: 244-250
Schnorf M, Neuhaus-Url G, Galli A, Iida S, Potrykus I, Neuhaus G (1991) An improved approach for transformation of plant cells by microinjection: molecular and genetic analysis. Transgen. Res. 1:23- 30
Tan S, Evans RR, Dahmer ML, Singh BK, Shaner DL (2005) Imidazolinone-tolerant crops: history, current status and future. Pest Manag Sci. 61(3):246-57,

Unless otherwise defined, all technical and scientific terms used herein have the same meaning as commonly understood by one of ordinary skill in the art to which this invention belongs.

The inventions illustratively described herein may suitably be practiced in the absence of any element or elements, limitation or limitations, not specifically disclosed herein. Thus, for example, the terms "comprising," "including," "containing," etc. shall be read expansively and without limitation. Additionally, the terms and expressions employed herein have been used as terms of description and not of limitation, and there is no intention in the use of such terms and expressions of excluding any equivalents of the features shown and described or portions thereof. It is recognized that various modifications are possible within the scope of the invention claimed.

Thus, it should be understood that although the present invention has been specifically disclosed by preferred embodiments and optional features, modification, improvement, and variation of the inventions disclosed may be resorted to by those skilled in the art, and that such modifications, improvements and variations are considered to be within the scope of this invention. The materials, methods, and examples provided here are representative of preferred embodiments, are exemplary, and are not intended as limitations on the scope of the invention.

The invention has been described broadly and generically herein. Each of the narrower species and subgeneric groupings falling within the generic disclosure also form part of the invention. This includes the generic description of the invention with a proviso or negative limitation removing any subject matter from the genus, regardless of whether or not the excised material is specifically recited herein.

In addition, where features or aspects of the invention are described in terms of Markush groups, those skilled in the art will recognize that the invention is also thereby described in terms of any individual member or subgroup of members of the Markush group.

All publications, patent applications, patents, and other references mentioned herein are expressly incorporated by reference in their entirety, to the same extent as if each were incorporated by reference individually. In case of conflict, the present specification, including definitions, will control.

Certain aspects of the present invention are described in the clauses below.
Clause 1. An isolated nucleic acid encoding an acetohydroxyacid synthase (AHAS) protein comprising a mutation at one or more amino acid positions corresponding to a position selected from the group consisting of A205, D376, W574, R577, and S653 of SEQ ID NO: 1.
Clause 2. The isolated nucleic acid of clause 1, wherein said AHAS protein encoded by said nucleic acid comprises one or more mutations selected from the group consisting of:
   an alanine to valine substitution at a position corresponding to position 205 of SEQ ID NO: 1,
   an alanine to aspartic acid substitution at a position corresponding to position 205 of SEQ ID NO: 1,
   an aspartic acid to glutamic acid substitution at a position corresponding to position 376 of SEQ ID NO: 1,
   a tryptophan to cysteine substitution at a position corresponding to position 574 of SEQ ID NO: 1,
   a tryptophan to leucine substitution at a position corresponding to position 574 of SEQ ID NO: 1, wherein said substitution is not W574L in *Brassica* AHAS III gene,
   a tryptophan to methionine substitution at a position corresponding to position 574 of SEQ ID NO: 1,
   a tryptophan to serine substitution at a position corresponding to position 574 of SEQ ID NO: 1,
   an arginine to tryptophan substitution at a position corresponding to position 577 of SEQ ID NO: 1,
   a serine to asparagine substitution at a position corresponding to position 653 of SEQ ID NO: 1, wherein said substitution is not S653N in *Brassica* AHAS I gene, and
   a serine to threonine substitution at a position corresponding to position 653 of SEQ ID NO: 1.
Clause 3. The isolated nucleic acid of clause 1 or 2, wherein the mutation comprises a serine to asparagine substitution at a position corresponding to position 653 of SEQ ID NO: 1, wherein said mutation is not S653N in *Brassica* AHAS I gene.
Clause 4. The isolated nucleic acid of clause 1 or 2, wherein the mutation comprises a serine to threonine substitution at a position corresponding to position 653 of SEQ ID NO: 1.
Clause 5. The isolated nucleic acid of clause 1 or 2, wherein the mutation comprises a tryptophan to leucine substitution at a position corresponding to position 574 of SEQ ID NO: 1, wherein said mutation is not W574L in *Brassica* AHAS III gene.
Clause 6. The isolated nucleic acid of clause 1 or 2, wherein the mutation comprises an alanine to valine substitution at a position corresponding to position 205 of SEQ ID NO: 1.
Clause 7. The isolated nucleic acid of clause 1 or 2, wherein the mutation comprises an alanine to aspartic acid substitution at a position corresponding to position 205 of SEQ ID NO: 1.
Clause 8. The isolated nucleic acid of clause 1 or 2, wherein said AHAS protein encoded by said nucleic acid comprises a mutation selected from the group consisting of the mutations shown in Table 2.
Clause 9. The isolated nucleic acid of clause 1 or 2, wherein said AHAS protein encoded by said nucleic acid comprises two or more mutations.
Clause 10. The isolated nucleic acid of clause 9 wherein said two or more mutations are selected from the group consisting of the mutations shown in Table 2.
Clause 11. The isolated nucleic acid of clause 9 or 10, wherein said AHAS protein encoded by said nucleic acid comprises a first mutation at a position corresponding to S653 of SEQ ID NO: 1 and a second mutation at one or more amino acid positions corresponding to a position selected from the group consisting of: A205, D376, W574, and R577 of SEQ ID NO: 1.
Clause 12. The isolated nucleic acid of clause 11, wherein said first mutation comprises a serine to asparagine substitution at a position corresponding to position 653 of SEQ ID NO: 1.
Clause 13. The isolated nucleic acid of clause 11, wherein said first mutation comprises a serine to threonine substitution at a position corresponding to position 653 of SEQ ID NO: 1.
Clause 14. The isolated nucleic acid of clause 11, 12, or 13 wherein said second mutation is selected from the group consisting of:
   an alanine to valine substitution at a position corresponding to position 205 of SEQ ID NO: 1,
   an alanine to aspartic acid substitution at a position corresponding to position 205 of SEQ ID NO: 1,
   an aspartic acid to glutamic acid substitution at a position corresponding to position 376 of SEQ ID NO: 1,
   a tryptophan to cysteine substitution at a position corresponding to position 574 of SEQ ID NO: 1,
   a tryptophan to leucine substitution at a position corresponding to position 574 of SEQ ID NO: 1, wherein said substitution is not W574L in *Brassica* AHAS III gene,
   a tryptophan to methionine substitution at a position corresponding to position 574 of SEQ ID NO: 1,
   a tryptophan to serine substitution at a position corresponding to position 574 of SEQ ID NO: 1,
   an arginine to tryptophan substitution at a position corresponding to position 577 of SEQ ID NO: 1,
   a serine to asparagine substitution at a position corresponding to position 653 of SEQ ID NO: 1, wherein said substitution is not S653N in *Brassica* AHAS I gene, and
   a serine to threonine substitution at a position corresponding to position 653 of SEQ ID NO: 1.
Clause 15. The isolated nucleic acid of clause 9 or 10, wherein said AHAS protein encoded by said nucleic acid comprises a first mutation at a position corresponding to W574 of SEQ ID NO: 1 and a second mutation at a position corresponding to R577 of SEQ ID NO: 1.
Clause 16. The isolated nucleic acid of clause 15, wherein said first mutation comprises a tryptophan to leucine substitution at a position corresponding to 574 of SEQ ID NO: 1.
Clause 17. The isolated nucleic acid of clause 15 or 16, wherein said second mutation comprises an arginine to tryptophan substitution at a position corresponding to position 577 of SEQ ID NO: 1.
Clause 18. The isolated nucleic acid of any one of clauses 1 to 17, wherein said nucleic acid encodes an AHAS protein that is resistant to inhibition by an AHAS-inhibiting herbicide.
Clause 19. The isolated nucleic acid of clause 18, wherein said AHAS-inhibiting herbicide is selected from the group consisting of herbicides of: imidazolinone, sulfonylurea, triazolopyrimidine, pyrimidinylthiobenzoate, sulfonylamino-carbonyltriazolinone, and mixtures thereof.
Clause 20. The isolated nucleic acid of clause 19, wherein said AHAS-inhibiting herbicide is an imidazolinone herbicide.
Clause 21. The isolated nucleic acid of clause 19, wherein said AHAS-inhibiting herbicide is a sulfonylurea herbicide.
Clause 22. The isolated nucleic acid of any one of clauses 1 to 21 wherein said nucleic acid encodes an AHAS protein comprising 70% or more identity to one or more of the amino acid sequences in Figure 2.
Clause 23. The isolated nucleic acid of any one of clauses 1 to 22, wherein said nucleic acid encodes a *Brassica* AHAS protein.
Clause 24. The isolated nucleic acid of clause any one of clauses 1 to 23, wherein said nucleic acid encodes a *Brassica napus* AHAS protein.
Clause 25. The isolated nucleic acid of any one of clauses 1 to 24, wherein said nucleic acid encodes a *Brassica napus* AHAS I protein.
Clause 26. The isolated nucleic acid of any one of clauses 1 to 24, wherein said nucleic acid encodes a *Brassica napus* AHAS III protein.
Clause 27. An isolated nucleic acid encoding an acetohydroxyacid synthase (AHAS) protein comprising a serine to asparagine substitution in *Brassica napus* AHAS III gene at a position corresponding to position 653 of SEQ ID NO: 1.
Clause 28. An expression vector comprising a nucleic acid encoding an acetohydroxyacid synthase (AHAS) protein comprising a mutation at one or more amino acid positions corresponding to a position selected from the group consisting of A205, D376, W574, R577, and S653 of SEQ ID NO: 1.
Clause 29. The expression vector of clause 28, wherein said AHAS protein encoded by said nucleic acid comprises one or more mutations selected from the group consisting of:
   an alanine to valine substitution at a position corresponding to position 205 of SEQ ID NO: 1,
   an alanine to aspartic acid substitution at a position corresponding to position 205 of SEQ ID NO: 1,
   an aspartic acid to glutamic acid substitution at a position corresponding to position 376 of SEQ ID NO: 1,
   a tryptophan to cysteine substitution at a position corresponding to position 574 of SEQ ID NO: 1,
   a tryptophan to leucine substitution at a position corresponding to position 574 of SEQ ID NO: 1, wherein said substitution is not W574L in *Brassica* AHAS III gene,
   a tryptophan to methionine substitution at a position corresponding to position 574 of SEQ ID NO: 1,
   a tryptophan to serine substitution at a position corresponding to position 574 of SEQ ID NO: 1,
   an arginine to tryptophan substitution at a position corresponding to position 577 of SEQ ID NO: 1,
   a serine to asparagine substitution at a position corresponding to position 653 of SEQ ID NO: 1, wherein said substitution is not S653N in *Brassica* AHAS I gene, and
   a serine to threonine substitution at a position corresponding to position 653 of SEQ ID NO: 1.
Clause 30. The expression vector of clause 28 or 29, wherein the mutation comprises a serine to asparagine substitution at a position corresponding to position 653 of SEQ ID NO: 1, wherein said mutation is not S653N in *Brassica* AHAS I gene.
Clause 31. The expression vector of clause 28 or 29, wherein the mutation comprises a serine to threonine substitution at a position corresponding to position 653 of SEQ ID NO: 1.
Clause 32. The expression vector of clause 28 or 29, wherein the mutation comprises a tryptophan to leucine substitution at a position corresponding to position 574 of SEQ ID NO: 1, wherein said mutation is not W574L in *Brassica* AHAS III gene.
Clause 33. The expression vector of clause 28 or 29, wherein the mutation comprises an alanine to valine substitution at a position corresponding to position 205 of SEQ ID NO: 1.
Clause 34. The expression vector of clause 28 or 29, wherein the mutation comprises an alanine to aspartic acid substitution at a position corresponding to position 205 of SEQ ID NO: 1.
Clause 35. An expression vector comprising a nucleic acid encoding an acetohydroxyacid synthase (AHAS) protein comprising a serine to asparagine substitution in *Brassica napus* AHAS III gene at a position corresponding to position 653 of SEQ ID NO: 1.
Clause 36. A plant comprising an acetohydroxyacid synthase (AHAS) gene, wherein said gene encodes a protein comprising a mutation at one or more amino acid positions corresponding to a position selected from the group consisting of A205, D376, W574, R577, and S653 of SEQ ID NO: 1.
Clause 37. A plant comprising an acetohydroxyacid synthase (AHAS) gene, wherein said plant is resistant to an AHAS-inhibiting herbicide, wherein said gene encodes a protein comprising a mutation at one or more amino acid positions corresponding to a position selected from the group consisting of A205, D376, W574, R577, and S653 of SEQ ID NO: 1.
Clause 38. The plant of clause 36 or 37, wherein said protein encoded by said AHAS gene comprises one or more mutations selected from the group consisting of:
   an alanine to valine substitution at a position corresponding to position 205 of SEQ ID NO: 1,
   an alanine to aspartic acid substitution at a position corresponding to position 205 of SEQ ID NO: 1,
   an aspartic acid to glutamic acid substitution at a position corresponding to position 376 of SEQ ID NO: 1,
   a tryptophan to cysteine substitution at a position corresponding to position 574 of SEQ ID NO: 1,
   a tryptophan to leucine substitution at a position corresponding to position 574 of SEQ ID NO: 1, wherein said substitution is not W574L in *Brassica* AHAS III gene,
   a tryptophan to methionine substitution at a position corresponding to position 574 of SEQ ID NO: 1,
   a tryptophan to serine substitution at a position corresponding to position 574 of SEQ ID NO: 1,
   an arginine to tryptophan substitution at a position corresponding to position 577 of SEQ ID NO: 1,
   a serine to asparagine substitution at a position corresponding to position 653 of SEQ ID NO: 1, wherein said substitution is not S653N in *Brassica* AHAS I gene, and
   a serine to threonine substitution at a position corresponding to position 653 of SEQ ID NO: 1.
Clause 39. The plant of any one of clauses 36 to 38, wherein the mutation comprises a serine to asparagine substitution at a position corresponding to position 653 of SEQ ID NO: 1, wherein said mutation is not S653N in *Brassica* AHAS I gene.
Clause 40. The plant of any one of clauses 36 to 38, wherein the mutation comprises a serine to threonine substitution at a position corresponding to position 653 of SEQ ID NO: 1.
Clause 41. The plant of any one of clauses 36 to 38, wherein the mutation comprises a tryptophan to leucine substitution at a position corresponding to position 574 of SEQ ID NO: 1, wherein said mutation is not W574L in *Brassica* AHAS III gene.
Clause 42. The plant of any one of clauses 36 to 38, wherein the mutation comprises an alanine to valine substitution at a position corresponding to position 205 of SEQ ID NO: 1.
Clause 43. The plant of any one of clauses 36 to 38, wherein the mutation comprises an alanine to aspartic acid substitution at a position corresponding to position 205 of SEQ ID NO: 1.
Clause 44. The plant of any one of clauses 36 to 38, wherein the mutation is selected from the group consisting of the mutations shown in Table 2.
Clause 45. The plant of any one of clauses 36 to 38, wherein said protein encoded by said AHAS gene comprises two or more mutations.
Clause 46. The plant of clause 45 wherein said two or more mutations are selected from the group consisting of the mutations shown in Table 2.
Clause 47. The plant of clause 45 or 46, wherein said protein encoded by said AHAS gene comprises a first mutation at a position corresponding to S653 of SEQ ID NO: 1 and a second mutation at one or more amino acid positions corresponding to a position selected from the group consisting of: A205, D376, W574, and R577 of SEQ ID NO: 1.
Clause 48. The plant of clause 47, wherein said first mutation comprises a serine to asparagine substitution at a position corresponding to position 653 of SEQ ID NO: 1.
Clause 49. The plant of clause 47, wherein said first mutation comprises a serine to threonine substitution at a position corresponding to position 653 of SEQ ID NO: 1.
Clause 50. The plant of clause 47, 48, or 49, wherein said second mutation is selected from the group consisting of:
   an alanine to valine substitution at a position corresponding to position 205 of SEQ ID NO: 1,
   an alanine to aspartic acid substitution at a position corresponding to position 205 of SEQ ID NO: 1,
   an aspartic acid to glutamic acid substitution at a position corresponding to position 376 of SEQ ID NO: 1,
   a tryptophan to cysteine substitution at a position corresponding to position 574 of SEQ ID NO: 1,
   a tryptophan to leucine substitution at a position corresponding to position 574 of SEQ ID NO: 1, wherein said substitution is not W574L in *Brassica* AHAS III gene,
   a tryptophan to methionine substitution at a position corresponding to position 574 of SEQ ID NO: 1,
   a tryptophan to serine substitution at a position corresponding to position 574 of SEQ ID NO: 1,
   an arginine to tryptophan substitution at a position corresponding to position 577 of SEQ ID NO: 1,
   a serine to asparagine substitution at a position corresponding to position 653 of SEQ ID NO: 1, wherein said substitution is not S653N in *Brassica* AHAS I gene, and
   a serine to threonine substitution at a position corresponding to position 653 of SEQ ID NO: 1.
Clause 51. The plant of clause 45 or 46, wherein said protein encoded by said AHAS gene comprises a first mutation at a position corresponding to W574 of SEQ ID NO: 1 and a second mutation at a position corresponding to R577 of SEQ ID NO: 1.
Clause 52. The plant of clause 51, wherein said first mutation comprises a tryptophan to leucine substitution at a position corresponding to 574 of SEQ ID NO: 1.
Clause 53. The plant of clause 51 or 52, wherein said second mutation comprises an arginine to tryptophan substitution at a position corresponding to position 577 of SEQ ID NO: 1.
Clause 54. The plant of any one of clauses 36 to 53, wherein said AHAS gene encodes a protein that is resistant to inhibition by an AHAS-inhibiting herbicide.
Clause 55. The plant of clause 54, wherein said AHAS-inhibiting herbicide is selected from the group consisting of herbicides of: imidazolinone, sulfonylurea, triazolopyrimidine, pyrimidinylthiobenzoate, sulfonylamino-carbonyltriazolinone, and mixtures thereof.
Clause 56. The plant of clause 55, wherein said AHAS-inhibiting herbicide is an imidazolinone herbicide.
Clause 57. The plant of clause 55, wherein said AHAS-inhibiting herbicide is a sulfonylurea herbicide.
Clause 58. The plant of any one of clauses 36 to 57 wherein said AHAS gene encodes a protein comprising 70% or more identity to one or more of the amino acid sequences in Figure 2.
Clause 59. The plant of any one of clauses 36 to 58, wherein said plant is resistant to the application of at least one AHAS-inhibiting herbicide.
Clause 60. The plant of any one of clauses 36 to 59, wherein said plant is resistant to the application of at least one AHAS-inhibiting herbicide selected from the group consisting of herbicides of: imidazolinone, sulfonylurea, triazolopyrimidine, pyrimidinylthiobenzoate, sulfonylamino-carbonyltriazolinone, and mixtures thereof.
Clause 61. The plant of clause any one of clauses 36 to 60, wherein said plant is a *Brassica* plant produced by growing a seed of a line selected from the lines listed in Table 2.
Clause 62. The plant of any one of clauses 36 to 61, wherein said plant is a *Brassica* species.
Clause 63. The plant of any one of clauses 36 to 62, wherein said plant is *Brassica napus.*
Clause 64. The plant of clause any one of clauses 36 to 63, wherein said AHAS gene encodes a *Brassica napus* AHAS protein.
Clause 65. The plant of clause any one of clauses 36 to 64, wherein said AHAS gene encodes a *Brassica napus* AHAS I protein.
Clause 66. The plant of clause any one of clauses 36 to 64, wherein said AHAS gene encodes a *Brassica napus* AHAS III protein.
Clause 67. The plant of clause any one of clauses 36 to 66, wherein said plant is non-transgenic.
Clause 68. A plant comprising an acetohydroxyacid synthase (AHAS) gene, wherein said gene encodes a protein comprising a serine to asparagine substitution in *Brassica napus* AHAS III gene at a position corresponding to position 653 of SEQ ID NO: 1.
Clause 69. A seed comprising an acetohydroxyacid synthase (AHAS) gene, wherein said gene encodes a protein comprising a mutation at one or more amino acid positions corresponding to a position selected from the group consisting of A205, D376, W574, R577, and S653 of SEQ ID NO: 1.
Clause 70. The seed of clause 69, wherein said protein encoded by said AHAS gene comprises one or more mutations selected from the group consisting of:
   an alanine to valine substitution at a position corresponding to position 205 of SEQ ID NO: 1,
   an alanine to aspartic acid substitution at a position corresponding to position 205 of SEQ ID NO: 1,
   an aspartic acid to glutamic acid substitution at a position corresponding to position 376 of SEQ ID NO: 1,
   a tryptophan to cysteine substitution at a position corresponding to position 574 of SEQ ID NO: 1,
   a tryptophan to leucine substitution at a position corresponding to position 574 of SEQ ID NO: 1, wherein said substitution is not W574L in *Brassica* AHAS III gene,
   a tryptophan to methionine substitution at a position corresponding to position 574 of SEQ ID NO: 1,
   a tryptophan to serine substitution at a position corresponding to position 574 of SEQ ID NO: 1,
   an arginine to tryptophan substitution at a position corresponding to position 577 of SEQ ID NO: 1,
   a serine to asparagine substitution at a position corresponding to position 653 of SEQ ID NO: 1, wherein said substitution is not S653N in *Brassica* AHAS I gene, and
   a serine to threonine substitution at a position corresponding to position 653 of SEQ ID NO: 1.
Clause 71. The seed of clause 69 or 70, wherein the mutation comprises a serine to asparagine substitution at a position corresponding to position 653 of SEQ ID NO: 1, wherein said mutation is not S653N in *Brassica* AHAS I gene.
Clause 72. The seed of clause 69 or 70, wherein the mutation comprises a serine to threonine substitution at a position corresponding to position 653 of SEQ ID NO: 1.
Clause 73. The seed of clause 69 or 70, wherein the mutation comprises a tryptophan to leucine substitution at a position corresponding to position 574 of SEQ ID NO: 1, wherein said mutation is not W574L in *Brassica* AHAS III gene.
Clause 74. The seed of clause 69 or 70, wherein the mutation comprises an alanine to valine substitution at a position corresponding to position 205 of SEQ ID NO: 1.
Clause 75. The seed of clause 69 or 70, wherein the mutation comprises an alanine to aspartic acid substitution at a position corresponding to position 205 of SEQ ID NO: 1.
Clause 76. The seed of clause 69 or 70, wherein the mutation is selected from the group consisting of the mutations shown in Table 2.
Clause 77. The seed of clause 69 or 70, wherein said protein encoded by said AHAS gene comprises two or more mutations.
Clause 78. The seed of clause 77 wherein said two or more mutations are selected from the group consisting of the mutations shown in Table 2.
Clause 79. The seed of clause 77 or 78, wherein said protein encoded by said AHAS gene comprises a first mutation at a position corresponding to S653 of SEQ ID NO: 1 and a second mutation at one or more amino acid positions corresponding to a position selected from the group consisting of: A205, D376, W574, and R577 of SEQ ID NO: 1.
Clause 80. The seed of clause 79, wherein said first mutation comprises a serine to asparagine substitution at a position corresponding to position 653 of SEQ ID NO: 1.
Clause 81. The seed of clause 79, wherein said first mutation comprises a serine to threonine substitution at a position corresponding to position 653 of SEQ ID NO: 1.
Clause 82. The seed of clause 79, 80, or 81, wherein said second mutation is selected from the group consisting of:
   an alanine to valine substitution at a position corresponding to position 205 of SEQ ID NO: 1,
   an alanine to aspartic acid substitution at a position corresponding to position 205 of SEQ ID NO: 1,
   an aspartic acid to glutamic acid substitution at a position corresponding to position 376 of SEQ ID NO: 1,
   a tryptophan to cysteine substitution at a position corresponding to position 574 of SEQ ID NO: 1,
   a tryptophan to leucine substitution at a position corresponding to position 574 of SEQ ID NO: 1, wherein said substitution is not W574L in *Brassica* AHAS III gene,
   a tryptophan to methionine substitution at a position corresponding to position 574 of SEQ ID NO: 1,
   a tryptophan to serine substitution at a position corresponding to position 574 of SEQ ID NO: 1,
   an arginine to tryptophan substitution at a position corresponding to position 577 of SEQ ID NO: 1,
   a serine to asparagine substitution at a position corresponding to position 653 of SEQ ID NO: 1, wherein said substitution is not S653N in *Brassica* AHAS I gene, and
   a serine to threonine substitution at a position corresponding to position 653 of SEQ ID NO: 1.
Clause 83. The seed of clause 77 or 78, wherein said protein encoded by said AHAS gene comprises a first mutation at a position corresponding to W574 of SEQ ID NO: 1 and a second mutation at a position corresponding to R577 of SEQ ID NO: 1.
Clause 84. The seed of clause 83, wherein said first mutation comprises a tryptophan to leucine substitution at a position corresponding to 574 of SEQ ID NO: 1.
Clause 85. The seed of clause 83 or 84, wherein said second mutation comprises an arginine to tryptophan substitution at a position corresponding to position 577 of SEQ ID NO: 1.
Clause 86. The seed of any one of clauses 69 to 85, wherein said AHAS gene encodes a protein that is resistant to inhibition by an AHAS-inhibiting herbicide.
Clause 87. The seed of clause 86, wherein said AHAS-inhibiting herbicide is selected from the group consisting of herbicides of: imidazolinone, sulfonylurea, triazolopyrimidine, pyrimidinylthiobenzoate, sulfonylamino-carbonyltriazolinone, and mixtures thereof.
Clause 88. The seed of clause 87, wherein said AHAS-inhibiting herbicide is an imidazolinone herbicide.
Clause 89. The seed of clause 87, wherein said AHAS-inhibiting herbicide is a sulfonylurea herbicide.
Clause 90. The seed of any one of clauses 69 to 89, wherein said AHAS gene encodes a protein comprising 70% or more identity to one or more of the amino acid sequences in Figure 2.
Clause 91. The seed of any one of clauses 69 to 90, wherein said AHAS gene encodes a *Brassica* protein.
Clause 92. The seed of any one of clauses 69 to 91, wherein said AHAS gene encodes a *Brassica napus* protein.
Clause 93. The seed of any one of clauses 69 to 92, wherein said AHAS gene encodes a *Brassica napus* AHAS I protein.
Clause 94. The seed of any one of clauses 69 to 92, wherein said *Brassica* AHAS gene encodes a *Brassica napus* AHAS III protein.
Clause 95. A plant grown from the seed of any one of clauses 69 to 94.
Clause 96. A plant element from the plant of clause 95, wherein the plant element is selected from the group consisting of pollen, protoplast, ovule, and cell.
Clause 97. A seed produced by a plant of any one of clauses 36 to 68.
Clause 98. A seed produced by a plant of any one of clauses 36 to 68, wherein said seed is a *Brassica* seed.
Clause 99. A seed produced by a plant of any one of clauses 36 to 68, wherein said seed is a seed of a *Brassica* plant line wherein said line is selected from the lines listed in Table 2.
Clause 100. A seed produced by a plant of any one of clauses 36 to 68, wherein said seed is non-transgenic.
Clause 101. A seed of an AHAS-inhibiting herbicide-resistant plant capable of producing a plant having AHAS herbicide resistance, wherein said seed is produced by a plant of any one of clauses 36 to 68.
Clause 102. A method for producing an herbicide-resistant plant, said method comprising:
   (a) introducing into a plant cell a gene repair oligonucleobase (GRON) with a targeted mutation in an acetohydroxyacid synthase (AHAS) gene to produce a plant cell with an AHAS gene that expresses an AHAS protein comprising a mutation at one or more amino acid positions corresponding to a position selected from the group consisting of A205, D376, W574, R577, and S653 of SEQ ID NO: 1;
   (b) identifying a plant cell having substantially normal growth and catalytic activity as compared to a corresponding wild-type plant cell in the presence of an AHAS-inhibiting herbicide; and
   (c) regenerating a non-transgenic herbicide-resistant plant having a mutated AHAS gene from said plant cell.
Clause 103. The method of clause 102, wherein said protein encoded by said AHAS gene comprises one or more mutations selected from the group consisting of:
   an alanine to valine substitution at a position corresponding to position 205 of SEQ ID NO: 1,
   an alanine to aspartic acid substitution at a position corresponding to position 205 of SEQ ID NO: 1,
   an aspartic acid to glutamic acid substitution at a position corresponding to position 376 of SEQ ID NO: 1,
   a tryptophan to cysteine substitution at a position corresponding to position 574 of SEQ ID NO: 1,
   a tryptophan to leucine substitution at a position corresponding to position 574 of SEQ ID NO: 1, wherein said substitution is not W574L in *Brassica* AHAS III gene,
   a tryptophan to methionine substitution at a position corresponding to position 574 of SEQ ID NO: 1,
   a tryptophan to serine substitution at a position corresponding to position 574 of SEQ ID NO: 1,
   an arginine to tryptophan substitution at a position corresponding to position 577 of SEQ ID NO: 1,
   a serine to asparagine substitution at a position corresponding to position 653 of SEQ ID NO: 1, wherein said substitution is not S653N in *Brassica* AHAS I gene, and
   a serine to threonine substitution at a position corresponding to position 653 of SEQ ID NO: 1.
Clause 104. The method of clause 102 or 103, wherein the mutation comprises a serine to asparagine substitution at a position corresponding to position 653 of SEQ ID NO: 1, wherein said mutation is not S653N in *Brassica* AHAS I gene.
Clause 105. The method of clause 102 or 103, wherein the mutation comprises a serine to threonine substitution at a position corresponding to position 653 of SEQ ID NO: 1.
Clause 106. The method of clause 102 or 103, wherein the mutation comprises a tryptophan to leucine substitution at a position corresponding to position 574 of SEQ ID NO: 1, wherein said mutation is not W574L in *Brassica* AHAS III gene.
Clause 107. The method of clause 102 or 103, wherein the mutation comprises an alanine to valine substitution at a position corresponding to position 205 of SEQ ID NO: 1.
Clause 108. The method of clause 102 or 103, wherein the mutation comprises an alanine to aspartic acid substitution at a position corresponding to position 205 of SEQ ID NO: 1.
Clause 109. The method of clause 102 or 103, wherein the mutation is selected from the group consisting of the mutations shown in Table 2.
Clause 110. The method of clause 102 or 103, wherein said protein encoded by said AHAS gene comprises two or more mutations.
Clause 111. The method of clause 110 wherein said two or more mutations are selected from the group consisting of the mutations shown in Table 2.
Clause 112. The method of clause 110 or 111, wherein said protein encoded by said AHAS gene comprises a first mutation at a position corresponding to S653 of SEQ ID NO: 1 and a second mutation at one or more amino acid positions corresponding to a position selected from the group consisting of: A205, D376, W574, and R577 of SEQ ID NO: 1.
Clause 113. The method of clause 112, wherein said first mutation comprises a serine to asparagine substitution at a position corresponding to position 653 of SEQ ID NO: 1.
Clause 114. The method of clause 112, wherein said first mutation comprises a serine to threonine substitution at a position corresponding to position 653 of SEQ ID NO: 1.
Clause 115. The method of clause 112 or 113, wherein said second mutation is selected from the group consisting of:
   an alanine to valine substitution at a position corresponding to position 205 of SEQ ID NO: 1,
   an alanine to aspartic acid substitution at a position corresponding to position 205 of SEQ ID NO: 1,
   an aspartic acid to glutamic acid substitution at a position corresponding to position 376 of SEQ ID NO: 1,
   a tryptophan to cysteine substitution at a position corresponding to position 574 of SEQ ID NO: 1,
   a tryptophan to leucine substitution at a position corresponding to position 574 of SEQ ID NO: 1, wherein said substitution is not W574L in *Brassica* AHAS III gene,
   a tryptophan to methionine substitution at a position corresponding to position 574 of SEQ ID NO: 1,
   a tryptophan to serine substitution at a position corresponding to position 574 of SEQ ID NO: 1,
   an arginine to tryptophan substitution at a position corresponding to position 577 of SEQ ID NO: 1,
   a serine to asparagine substitution at a position corresponding to position 653 of SEQ ID NO: 1, wherein said substitution is not S653N in *Brassica* AHAS I gene, and
   a serine to threonine substitution at a position corresponding to position 653 of SEQ ID NO: 1.
Clause 116. The method of clause 112 or 114, wherein said second mutation is selected from the group consisting of:
   an alanine to valine substitution at a position corresponding to position 205 of SEQ ID NO: 1,
   an alanine to aspartic acid substitution at a position corresponding to position 205 of SEQ ID NO: 1,
   an aspartic acid to glutamic acid substitution at a position corresponding to position 376 of SEQ ID NO: 1,
   a tryptophan to cysteine substitution at a position corresponding to position 574 of SEQ ID NO: 1,
   a tryptophan to leucine substitution at a position corresponding to position 574 of SEQ ID NO: 1, wherein said substitution is not W574L in *Brassica* AHAS III gene,
   a tryptophan to methionine substitution at a position corresponding to position 574 of SEQ ID NO: 1,
   a tryptophan to serine substitution at a position corresponding to position 574 of SEQ ID NO: 1,
   an arginine to tryptophan substitution at a position corresponding to position 577 of SEQ ID NO: 1,
   a serine to asparagine substitution at a position corresponding to position 653 of SEQ ID NO: 1, wherein said substitution is not S653N in *Brassica* AHAS I gene, and
   a serine to threonine substitution at a position corresponding to position 653 of SEQ ID NO: 1.
Clause 117. The method of clause 110 or 111, wherein said protein encoded by said AHAS gene comprises a first mutation at a position corresponding to W574 of SEQ ID NO: 1 and a second mutation at a position corresponding to R577 of SEQ ID NO: 1.
Clause 118. The method of clause 117, wherein said first mutation comprises a tryptophan to leucine substitution at a position corresponding to 574 of SEQ ID NO: 1.
Clause 119. The method of clause 117 or 118, wherein said second mutation comprises an arginine to tryptophan substitution at a position corresponding to position 577 of SEQ ID NO: 1.
Clause 120. The method of any one of clauses 102 to 119, wherein said AHAS gene encodes a protein that is resistant to inhibition by an AHAS-inhibiting herbicide.
Clause 121. The method of clause 120, wherein said AHAS-inhibiting herbicide is selected from the group consisting of herbicides of: imidazolinone, sulfonylurea, triazolopyrimidine, pyrimidinylthiobenzoate, sulfonylamino-carbonyltriazolinone, and mixtures thereof.
Clause 122. The method of clause 121, wherein said AHAS-inhibiting herbicide is an imidazolinone herbicide.
Clause 123. The method of clause 121, wherein said AHAS-inhibiting herbicide is a sulfonylurea herbicide.
Clause 124. The method of any one of clauses 102 to 123, wherein said AHAS gene encodes a protein comprising 70% or more identity to one or more of the amino acid sequences in Figure 2.
Clause 125. A method for increasing the herbicide-resistance of a plant comprising crossing a first *Brassica* plant to a second *Brassica* plant, wherein said first plant comprises an acetohydroxyacid synthase (AHAS) gene, wherein said gene encodes a protein comprising a mutation at one or more amino acid positions corresponding to a position selected from the group consisting of A205, D376, W574, R577, and S653 of SEQ ID NO: 1; screening a population resulting from the cross for increased AHAS herbicide-resistance; selecting a member resulting from the cross having increased AHAS herbicide-resistance; and producing seeds resulting from the cross.
Clause 126. The method of clause 125, wherein said protein encoded by said AHAS gene comprises one or more mutations selected from the group consisting of:
   an alanine to valine substitution at a position corresponding to position 205 of SEQ ID NO: 1,
   an alanine to aspartic acid substitution at a position corresponding to position 205 of SEQ ID NO: 1,
   an aspartic acid to glutamic acid substitution at a position corresponding to position 376 of SEQ ID NO: 1,
   a tryptophan to cysteine substitution at a position corresponding to position 574 of SEQ ID NO: 1,
   a tryptophan to leucine substitution at a position corresponding to position 574 of SEQ ID NO: 1, wherein said substitution is not W574L in *Brassica* AHAS III gene,
   a tryptophan to methionine substitution at a position corresponding to position 574 of SEQ ID NO: 1,
   a tryptophan to serine substitution at a position corresponding to position 574 of SEQ ID NO: 1,
   an arginine to tryptophan substitution at a position corresponding to position 577 of SEQ ID NO: 1,
   a serine to asparagine substitution at a position corresponding to position 653 of SEQ ID NO: 1, wherein said substitution is not S653N in *Brassica* AHAS I gene, and
   a serine to threonine substitution at a position corresponding to position 653 of SEQ ID NO: 1.
Clause 127. A hybrid *Brassica* seed produced by the method of clause 125 or 126.
Clause 128. The method of clause 125 or 126, further comprising growing plants from seed produced from the crossing of said first and second plants.
Clause 129. The method of clause 125 or 126, wherein said AHAS gene encodes a protein that is resistant to inhibition by an AHAS-inhibiting herbicide.
Clause 130. The method of clause 129, wherein said AHAS-inhibiting herbicide is selected from the group consisting of herbicides of: imidazolinone, sulfonylurea, triazolopyrimidine, pyrimidinylthiobenzoate, sulfonylamino-carbonyltriazolinone, and mixtures thereof.
Clause 131. The method of clause 130, wherein said AHAS-inhibiting herbicide is an imidazolinone herbicide.
Clause 132. The method of clause 130, wherein said AHAS-inhibiting herbicide is a sulfonylurea herbicide.
Clause 133. A method of controlling weeds in a field containing plants said method comprising applying an effective amount of at least one AHAS-inhibiting herbicide to said field containing said weeds and plants, said plant having an acetohydroxyacid synthase (AHAS) gene, wherein said gene encodes a protein comprising a mutation at one or more amino acid positions corresponding to a position selected from the group consisting of A205, D376, W574, R577, and S653 of SEQ ID NO: 1.
Clause 134. The method of clause 133, wherein said protein encoded by said AHAS gene comprises one or more mutations selected from the group consisting of:
   an alanine to valine substitution at a position corresponding to position 205 of SEQ ID NO: 1,
   an alanine to aspartic acid substitution at a position corresponding to position 205 of SEQ ID NO: 1,
   an aspartic acid to glutamic acid substitution at a position corresponding to position 376 of SEQ ID NO: 1,
   a tryptophan to cysteine substitution at a position corresponding to position 574 of SEQ ID NO: 1,
   a tryptophan to leucine substitution at a position corresponding to position 574 of SEQ ID NO: 1, wherein said substitution is not W574L in *Brassica* AHAS III gene,
   a tryptophan to methionine substitution at a position corresponding to position 574 of SEQ ID NO: 1,
   a tryptophan to serine substitution at a position corresponding to position 574 of SEQ ID NO: 1,
   an arginine to tryptophan substitution at a position corresponding to position 577 of SEQ ID NO: 1,
   a serine to asparagine substitution at a position corresponding to position 653 of SEQ ID NO: 1, wherein said substitution is not S653N in *Brassica* AHAS I gene, and
   a serine to threonine substitution at a position corresponding to position 653 of SEQ ID NO: 1.
Clause 135. The method of clause 133 or 134, wherein said AHAS-inhibiting herbicide is selected from the group consisting of herbicides of: imidazolinone, sulfonylurea, triazolopyrimidine, pyrimidinylthiobenzoate, sulfonylamino-carbonyltriazolinone, and mixtures thereof.
Clause 136. The method of clause 135, wherein said AHAS-inhibiting herbicide is an imidazolinone herbicide.
Clause 137. The method of clause 135, wherein said AHAS-inhibiting herbicide is a sulfonylurea herbicide.
Clause 138. A plant comprising an acetohydroxyacid synthase (AHAS) gene, wherein said gene encodes a protein comprising one or more mutations selected from the group consisting of:
   an alanine to valine substitution at a position corresponding to position 205 of SEQ ID NO: 1,
   an alanine to aspartic acid substitution at a position corresponding to position 205 of SEQ ID NO: 1,
   an aspartic acid to glutamic acid substitution at a position corresponding to position 376 of SEQ ID NO: 1,
   a tryptophan to cysteine substitution at a position corresponding to position 574 of SEQ ID NO: 1,
   a tryptophan to leucine substitution at a position corresponding to position 574 of SEQ ID NO: 1, wherein said substitution is not W574L in *Brassica* AHAS III gene,
   a tryptophan to methionine substitution at a position corresponding to position 574 of SEQ ID NO: 1,
   a tryptophan to serine substitution at a position corresponding to position 574 of SEQ ID NO: 1, and
   an arginine to tryptophan substitution at a position corresponding to position 577 of SEQ ID NO: 1;
   wherein said plant is selected from the group consisting of sugar beet, oilseed rape, and canola; and wherein said plant is resistant to a sulfonylurea herbicide.
Clause 139. The plant of clause 138, wherein the mutation comprises a tryptophan to leucine substitution at a position corresponding to position 574 of SEQ ID NO: 1, wherein said mutation is not W574L in *Brassica* AHAS III gene.
Clause 140. The plant of clause 138, wherein the mutation comprises an alanine to valine substitution at a position corresponding to position 205 of SEQ ID NO: 1.
Clause 141. The plant of clause 138, wherein the mutation comprises an alanine to aspartic acid substitution at a position corresponding to position 205 of SEQ ID NO: 1.
Clause 142. The plant of clause 138, wherein said protein encoded by said AHAS gene comprises two or more mutations.
Clause 143. The plant of clause 142, wherein said second mutation is selected from the group consisting of:
   an alanine to valine substitution at a position corresponding to position 205 of SEQ ID NO: 1,
   an alanine to aspartic acid substitution at a position corresponding to position 205 of SEQ ID NO: 1,
   an aspartic acid to glutamic acid substitution at a position corresponding to position 376 of SEQ ID NO: 1,
   a tryptophan to cysteine substitution at a position corresponding to position 574 of SEQ ID NO: 1,
   a tryptophan to leucine substitution at a position corresponding to position 574 of SEQ ID NO: 1, wherein said substitution is not W574L in *Brassica* AHAS III gene,
   a tryptophan to methionine substitution at a position corresponding to position 574 of SEQ ID NO: 1,
   a tryptophan to serine substitution at a position corresponding to position 574 of SEQ ID NO: 1, and
   an arginine to tryptophan substitution at a position corresponding to position 577 of SEQ ID NO: 1.
Clause 144. The plant of clause 142, wherein said protein encoded by said AHAS gene comprises a first mutation at a position corresponding to W574 of SEQ ID NO: 1 and a second mutation at a position corresponding to R577 of SEQ ID NO: 1.
Clause 145. The plant of clause 144, wherein said first mutation comprises a tryptophan to leucine substitution at a position corresponding to 574 of SEQ ID NO: 1.
Clause 146. The plant of clause 144 or 145, wherein said second mutation comprises an arginine to tryptophan substitution at a position corresponding to position 577 of SEQ ID NO: 1.
Clause 147. The plant of any one of clauses 138 to 146, wherein said AHAS gene encodes a protein comprising 70% or more identity to one or more of the amino acid sequences in Figure 2.
Clause 148. The plant of any one of clauses 138 to 147, wherein said AHAS gene encodes a *Brassica napus* AHAS I protein.
Clause 149. The plant of any one of clauses 138 to 147, wherein said AHAS gene encodes a *Brassica napus* AHAS III protein.
Clause 150. The plant of any one of clauses 138 to 149, wherein said plant is non-transgenic.
Clause 151. A seed comprising an acetohydroxyacid synthase (AHAS) gene, wherein said gene encodes a protein comprising one or more mutations selected from the group consisting of:
   an alanine to valine substitution at a position corresponding to position 205 of SEQ ID NO: 1,
   an alanine to aspartic acid substitution at a position corresponding to position 205 of SEQ ID NO: 1,
   an aspartic acid to glutamic acid substitution at a position corresponding to position 376 of SEQ ID NO: 1,
   a tryptophan to cysteine substitution at a position corresponding to position 574 of SEQ ID NO: 1,
   a tryptophan to leucine substitution at a position corresponding to position 574 of SEQ ID NO: 1, wherein said substitution is not W574L in *Brassica* AHAS III gene,
   a tryptophan to methionine substitution at a position corresponding to position 574 of SEQ ID NO: 1,
   a tryptophan to serine substitution at a position corresponding to position 574 of SEQ ID NO: 1, and
   an arginine to tryptophan substitution at a position corresponding to position 577 of SEQ ID NO: 1;
   wherein said seed is of a plant is selected from the group consisting of sugar beet, oilseed rape, and canola; and wherein said plant is resistant to a sulfonylurea herbicide.
Clause 152. The seed of clause 151, wherein the mutation comprises a tryptophan to leucine substitution at a position corresponding to position 574 of SEQ ID NO: 1, wherein said mutation is not W574L in *Brassica* AHAS III gene.
Clause 153. The seed of clause 151, wherein the mutation comprises an alanine to valine substitution at a position corresponding to position 205 of SEQ ID NO: 1.
Clause 154. The seed of clause 151, wherein the mutation comprises an alanine to aspartic acid substitution at a position corresponding to position 205 of SEQ ID NO: 1.
Clause 155. The seed of clause 151, wherein said protein encoded by said AHAS gene comprises two or more mutations.
Clause 156. The seed of clause 155, wherein said second mutation is selected from the group consisting of:
   an alanine to valine substitution at a position corresponding to position 205 of SEQ ID NO: 1,
   an alanine to aspartic acid substitution at a position corresponding to position 205 of SEQ ID NO: 1,
   an aspartic acid to glutamic acid substitution at a position corresponding to position 376 of SEQ ID NO: 1,
   a tryptophan to cysteine substitution at a position corresponding to position 574 of SEQ ID NO: 1,
   a tryptophan to leucine substitution at a position corresponding to position 574 of SEQ ID NO: 1, wherein said substitution is not W574L in *Brassica* AHAS III gene,
   a tryptophan to methionine substitution at a position corresponding to position 574 of SEQ ID NO: 1,
   a tryptophan to serine substitution at a position corresponding to position 574 of SEQ ID NO: 1, and
   an arginine to tryptophan substitution at a position corresponding to position 577 of SEQ ID NO: 1.
Clause 157. The seed of clause 155, wherein said protein encoded by said AHAS gene comprises a first mutation at a position corresponding to W574 of SEQ ID NO: 1 and a second mutation at a position corresponding to R577 of SEQ ID NO: 1.
Clause 158. The seed of clause 157, wherein said first mutation comprises a tryptophan to leucine substitution at a position corresponding to 574 of SEQ ID NO: 1.
Clause 159. The seed of clause 157 or 158, wherein said second mutation comprises an arginine to tryptophan substitution at a position corresponding to position 577 of SEQ ID NO: 1.
Clause 160. The seed of any one of clauses 151 to 159, wherein said AHAS gene encodes a protein comprising 70% or more identity to one or more of the amino acid sequences in Figure 2.
Clause 161. The seed of clause any one of clauses 151 to 160, wherein said AHAS gene encodes a *Brassica napus* protein.
Clause 162. The seed of clause any one of clauses 151 to 161, wherein said AHAS gene encodes a *Brassica napus* AHAS I protein.
Clause 163. The seed of clause any one of clauses 151 to 161, wherein said *Brassica* AHAS gene encodes a *Brassica napus* AHAS III protein.
Clause 164. A plant grown from the seed of clause any one of clauses 151 to 163.
Clause 165. A plant element from the plant of clause 164, wherein the plant element is selected from the group consisting of pollen, protoplast, ovule, and cell.
Clause 166. A seed produced by a plant of any one of clauses 138 to 150
Clause 167. A seed produced by a plant of any one of clauses 138 to 150, wherein said seed is non-transgenic.
Clause 168. A method for producing an herbicide-resistant plant, said method comprising:
   (a) introducing into a plant cell a gene repair oligonucleobase (GRON) with a targeted mutation in an acetohydroxyacid synthase (AHAS) gene to produce a plant cell with an AHAS gene that expresses an AHAS protein comprising one or more mutations selected from the group consisting of:
      an alanine to valine substitution at a position corresponding to position 205 of SEQ ID NO: 1,
      an alanine to aspartic acid substitution at a position corresponding to position 205 of SEQ ID NO: 1,
      an aspartic acid to glutamic acid substitution at a position corresponding to position 376 of SEQ ID NO: 1,
      a tryptophan to cysteine substitution at a position corresponding to position 574 of SEQ ID NO: 1,
      a tryptophan to leucine substitution at a position corresponding to position 574 of SEQ ID NO: 1, wherein said substitution is not W574L in *Brassica* AHAS III gene,
      a tryptophan to methionine substitution at a position corresponding to position 574 of SEQ ID NO: 1,
      a tryptophan to serine substitution at a position corresponding to position 574 of SEQ ID NO: 1, and
      an arginine to tryptophan substitution at a position corresponding to position 577 of SEQ ID NO: 1;
   (b) identifying a plant cell having substantially normal growth and catalytic activity as compared to a corresponding wild-type plant cell in the presence of an AHAS-inhibiting herbicide; and
   (c) regenerating a non-transgenic herbicide-resistant plant having a mutated AHAS gene from said plant cell;
   wherein said herbicide-resistant plant is selected from the group consisting of sugar beet, oilseed rape, and canola; and wherein said herbicide is a sulfonylurea herbicide.
Clause 169. The method of clause 168, wherein the mutation comprises a tryptophan to leucine substitution at a position corresponding to position 574 of SEQ ID NO: 1, wherein said mutation is not W574L in *Brassica* AHAS III gene.
Clause 170. The method of clause 168, wherein the mutation comprises an alanine to valine substitution at a position corresponding to position 205 of SEQ ID NO: 1.
Clause 171. The method of clause 168, wherein the mutation comprises an alanine to aspartic acid substitution at a position corresponding to position 205 of SEQ ID NO: 1.
Clause 172. The method of clause 168, wherein said protein encoded by said AHAS gene comprises two or more mutations.
Clause 173. The method of clause 172, wherein said second mutation is selected from the group consisting of:
   an alanine to valine substitution at a position corresponding to position 205 of SEQ ID NO: 1,
   an alanine to aspartic acid substitution at a position corresponding to position 205 of SEQ ID NO: 1,
   an aspartic acid to glutamic acid substitution at a position corresponding to position 376 of SEQ ID NO: 1,
   a tryptophan to cysteine substitution at a position corresponding to position 574 of SEQ ID NO: 1,
   a tryptophan to leucine substitution at a position corresponding to position 574 of SEQ ID NO: 1, wherein said substitution is not W574L in *Brassica* AHAS III gene,
   a tryptophan to methionine substitution at a position corresponding to position 574 of SEQ ID NO: 1,
   a tryptophan to serine substitution at a position corresponding to position 574 of SEQ ID NO: 1, and
   an arginine to tryptophan substitution at a position corresponding to position 577 of SEQ ID NO: 1.
Clause 174. The method of clause 172, wherein said protein encoded by said AHAS gene comprises a first mutation at a position corresponding to W574 of SEQ ID NO: 1 and a second mutation at a position corresponding to R577 of SEQ ID NO: 1.
Clause 175. The method of clause 174, wherein said first mutation comprises a tryptophan to leucine substitution at a position corresponding to 574 of SEQ ID NO: 1.
Clause 176. The method of clause 174 or 175, wherein said second mutation comprises an arginine to tryptophan substitution at a position corresponding to position 577 of SEQ ID NO: 1.
Clause 177. The method of any one of clauses 168 to 176, wherein said AHAS gene encodes a protein comprising 70% or more identity to one or more of the amino acid sequences in Figure 2.
Clause 178. A method of controlling weeds in a field containing plants said method comprising applying an effective amount of at least one AHAS-inhibiting herbicide to said field containing said weeds and plants, said plant having an acetohydroxyacid synthase (AHAS) gene, wherein said gene encodes a protein having one or more mutations selected from the group consisting of:
   an alanine to valine substitution at a position corresponding to position 205 of SEQ ID NO: 1,
   an alanine to aspartic acid substitution at a position corresponding to position 205 of SEQ ID NO: 1,
   an aspartic acid to glutamic acid substitution at a position corresponding to position 376 of SEQ ID NO: 1,
   a tryptophan to cysteine substitution at a position corresponding to position 574 of SEQ ID NO: 1,
   a tryptophan to leucine substitution at a position corresponding to position 574 of SEQ ID NO: 1, wherein said substitution is not W574L in *Brassica* AHAS III gene,
   a tryptophan to methionine substitution at a position corresponding to position 574 of SEQ ID NO: 1,
   a tryptophan to serine substitution at a position corresponding to position 574 of SEQ ID NO: 1, and
   an arginine to tryptophan substitution at a position corresponding to position 577 of SEQ ID NO: 1;
   wherein said plant is selected from the group consisting sugar beet, oilseed rape, and canola; and wherein said plant is resistant to a sulfonylurea herbicide.

## Claims

1. A *Brassica napus* plant comprising an acetohydroxyacid synthase I (AHAS I) gene, wherein said AHAS I gene encodes a AHAS I protein comprising a tryptophan to leucine substitution at the amino acid position corresponding to position W574 of SEQ ID NO: 1, said plant further comprising an acetohydroxyacid synthase III (AHAS III) gene, wherein said AHAS III gene encodes a AHAS III protein comprising a tryptophan to leucine substitution at the amino acid position corresponding to position W574 of SEQ ID NO: 1, wherein said AHAS I protein and said AHAS III protein are resistant to inhibition by an AHAS-inhibiting herbicide.

2. A *Brassica napus* seed comprising an acetohydroxyacid synthase I (AHAS I) gene, wherein said AHAS I gene encodes a AHAS I protein comprising a tryptophan to leucine substitution at the amino acid position corresponding to position W574 of SEQ ID NO: 1, said seed further comprising an acetohydroxyacid synthase III (AHAS III) gene, wherein said AHAS III gene encodes a AHAS III protein comprising a tryptophan to leucine substitution at the amino acid position corresponding to position W574 of SEQ ID NO: 1, wherein said AHAS I protein and said AHAS III protein are resistant to inhibition by an AHAS-inhibiting herbicide.

3. The plant of claim 1 or the seed of claim 2, wherein the plant or seed is non-transgenic.


SEQUENCE LISTING

<110> CIBUS LLC

<120> MUTATED ACETOHYDROXYACID SYNTHASE GENES IN BRASSICA

<130> 074267-0604

<140> PCT/US2008/078098
<141> 2008-10-03

<150> 60/977,944
<151> 2007-10-05

<160> 104

<170> PatentIn version 3.5

<210> 1
<211> 670
<212> PRT
<213> Arabidopsis thaliana

<400> 1
Met Ala Ala Ala Thr Thr Thr Thr Thr Thr Ser Ser Ser Ile Ser Phe
1               5                   10                  15

Ser Thr Lys Pro Ser Pro Ser Ser Ser Lys Ser Pro Leu Pro Ile Ser
            20                  25                  30

Arg Phe Ser Leu Pro Phe Ser Leu Asn Pro Asn Lys Ser Ser Ser Ser
        35                  40                  45

Ser Arg Arg Arg Gly Ile Lys Ser Ser Ser Pro Ser Ser Ile Ser Ala
    50                  55                  60

Val Leu Asn Thr Thr Thr Asn Val Thr Thr Thr Pro Ser Pro Thr Lys
65                  70                  75                  80

Pro Thr Lys Pro Glu Thr Phe Ile Ser Arg Phe Ala Pro Asp Gln Pro
                85                  90                  95

Arg Lys Gly Ala Asp Ile Leu Val Glu Ala Leu Glu Arg Gln Gly Val
            100                 105                 110

Glu Thr Val Phe Ala Tyr Pro Gly Gly Ala Ser Met Glu Ile His Gln
        115                 120                 125

Ala Leu Thr Arg Ser Ser Ser Ile Arg Asn Val Leu Pro Arg His Glu
    130                 135                 140

Gln Gly Gly Val Phe Ala Ala Glu Gly Tyr Ala Arg Ser Ser Gly Lys
145                 150                 155                 160

Pro Gly Ile Cys Ile Ala Thr Ser Gly Pro Gly Ala Thr Asn Leu Val
                165                 170                 175

Ser Gly Leu Ala Asp Ala Leu Leu Asp Ser Val Pro Leu Val Ala Ile
            180                 185                 190

Thr Gly Gln Val Pro Arg Arg Met Ile Gly Thr Asp Ala Phe Gln Glu
        195                 200                 205

Thr Pro Ile Val Glu Val Thr Arg Ser Ile Thr Lys His Asn Tyr Leu
    210                 215                 220

Val Met Asp Val Glu Asp Ile Pro Arg Ile Ile Glu Glu Ala Phe Phe
225                 230                 235                 240

Leu Ala Thr Ser Gly Arg Pro Gly Pro Val Leu Val Asp Val Pro Lys
                245                 250                 255

Asp Ile Gln Gln Gln Leu Ala Ile Pro Asn Trp Glu Gln Ala Met Arg
            260                 265                 270

Leu Pro Gly Tyr Met Ser Arg Met Pro Lys Pro Pro Glu Asp Ser His
        275                 280                 285

Leu Glu Gln Ile Val Arg Leu Ile Ser Glu Ser Lys Lys Pro Val Leu
    290                 295                 300

Tyr Val Gly Gly Gly Cys Leu Asn Ser Ser Asp Glu Leu Gly Arg Phe
305                 310                 315                 320

Val Glu Leu Thr Gly Ile Pro Val Ala Ser Thr Leu Met Gly Leu Gly
                325                 330                 335

Ser Tyr Pro Cys Asp Asp Glu Leu Ser Leu His Met Leu Gly Met His
            340                 345                 350

Gly Thr Val Tyr Ala Asn Tyr Ala Val Glu His Ser Asp Leu Leu Leu
        355                 360                 365

Ala Phe Gly Val Arg Phe Asp Asp Arg Val Thr Gly Lys Leu Glu Ala
    370                 375                 380

Phe Ala Ser Arg Ala Lys Ile Val His Ile Asp Ile Asp Ser Ala Glu
385                 390                 395                 400

Ile Gly Lys Asn Lys Thr Pro His Val Ser Val Cys Gly Asp Val Lys
                405                 410                 415

Leu Ala Leu Gln Gly Met Asn Lys Val Leu Glu Asn Arg Ala Glu Glu
            420                 425                 430

Leu Lys Leu Asp Phe Gly Val Trp Arg Asn Glu Leu Asn Val Gln Lys
        435                 440                 445

Gln Lys Phe Pro Leu Ser Phe Lys Thr Phe Gly Glu Ala Ile Pro Pro
    450                 455                 460

Gln Tyr Ala Ile Lys Val Leu Asp Glu Leu Thr Asp Gly Lys Ala Ile
465                 470                 475                 480

Ile Ser Thr Gly Val Gly Gln His Gln Met Trp Ala Ala Gln Phe Tyr
                485                 490                 495

Asn Tyr Lys Lys Pro Arg Gln Trp Leu Ser Ser Gly Gly Leu Gly Ala
            500                 505                 510

Met Gly Phe Gly Leu Pro Ala Ala Ile Gly Ala Ser Val Ala Asn Pro
        515                 520                 525

Asp Ala Ile Val Val Asp Ile Asp Gly Asp Gly Ser Phe Ile Met Asn
    530                 535                 540

Val Gln Glu Leu Ala Thr Ile Arg Val Glu Asn Leu Pro Val Lys Val
545                 550                 555                 560

Leu Leu Leu Asn Asn Gln His Leu Gly Met Val Met Gln Trp Glu Asp
                565                 570                 575

Arg Phe Tyr Lys Ala Asn Arg Ala His Thr Phe Leu Gly Asp Pro Ala
            580                 585                 590

Gln Glu Asp Glu Ile Phe Pro Asn Met Leu Leu Phe Ala Ala Ala Cys
        595                 600                 605

Gly Ile Pro Ala Ala Arg Val Thr Lys Lys Ala Asp Leu Arg Glu Ala
    610                 615                 620

Ile Gln Thr Met Leu Asp Thr Pro Gly Pro Tyr Leu Leu Asp Val Ile
625                 630                 635                 640

Cys Pro His Gln Glu His Val Leu Pro Met Ile Pro Ser Gly Gly Thr
                645                 650                 655

Phe Asn Asp Val Ile Thr Glu Gly Asp Gly Arg Ile Lys Tyr

660                 665                 670

<210> 2
<211> 655
<212> PRT
<213> Brassica napus

<400> 2
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Asp Ser Ser Arg Leu His Arg Pro Leu Ala Ile
        35                  40                  45

Ser Ala Val Leu Asn Ser Pro Val Asn Val Ala Pro Pro Ser Pro Glu
    50                  55                  60

Lys Thr Asp Lys Asn Lys Thr Phe Val Ser Arg Tyr Ala Pro Asp Glu
65                  70                  75                  80

Pro Arg Lys Gly Ala Asp Ile Leu Val Glu Ala Leu Glu Arg Gln Gly
                85                  90                  95

Val Glu Thr Val Phe Ala Tyr Pro Gly Gly Ala Ser Met Glu Ile His
            100                 105                 110

Gln Ala Leu Thr Arg Ser Ser Thr Ile Arg Asn Val Leu Pro Arg His
        115                 120                 125

Glu Gln Gly Gly Val Phe Ala Ala Glu Gly Tyr Ala Arg Ser Ser Gly
    130                 135                 140

Lys Pro Gly Ile Cys Ile Ala Thr Ser Gly Pro Gly Ala Thr Asn Leu
145                 150                 155                 160

Val Ser Gly Leu Ala Asp Ala Met Leu Asp Ser Val Pro Leu Val Ala
                165                 170                 175

Ile Thr Gly Gln Val Pro Arg Arg Met Ile Gly Thr Asp Ala Phe Gln
            180                 185                 190

Glu Thr Pro Ile Val Glu Val Thr Arg Ser Ile Thr Lys His Asn Tyr
        195                 200                 205

Leu Val Met Asp Val Asp Asp Ile Pro Arg Ile Val Gln Glu Ala Phe
    210                 215                 220

Phe Leu Ala Thr Ser Gly Arg Pro Gly Pro Val Leu Val Asp Val Pro
225                 230                 235                 240

Lys Asp Ile Gln Gln Gln Leu Ala Ile Pro Asn Trp Asp Gln Pro Met
                245                 250                 255

Arg Leu Pro Gly Tyr Met Ser Arg Leu Pro Gln Pro Pro Glu Val Ser
            260                 265                 270

Gln Leu Gly Gln Ile Val Arg Leu Ile Ser Glu Ser Lys Arg Pro Val
        275                 280                 285

Leu Tyr Val Gly Gly Gly Ser Leu Asn Ser Ser Glu Glu Leu Gly Arg
    290                 295                 300

Phe Val Glu Leu Thr Gly Ile Pro Val Ala Ser Thr Leu Met Gly Leu
305                 310                 315                 320

Gly Ser Tyr Pro Cys Asn Asp Glu Leu Ser Leu Gln Met Leu Gly Met
                325                 330                 335

His Gly Thr Val Tyr Ala Asn Tyr Ala Val Glu His Ser Asp Leu Leu
            340                 345                 350

Leu Ala Phe Gly Val Arg Phe Asp Asp Arg Val Thr Gly Lys Leu Glu
        355                 360                 365

Ala Phe Ala Ser Arg Ala Lys Ile Val His Ile Asp Ile Asp Ser Ala
    370                 375                 380

Glu Ile Gly Lys Asn Lys Thr Pro His Val Ser Val Cys Gly Asp Val
385                 390                 395                 400

Lys Leu Ala Leu Gln Gly Met Asn Lys Val Leu Glu Asn Arg Ala Glu
                405                 410                 415

Glu Leu Lys Leu Asp Phe Gly Val Trp Arg Ser Glu Leu Ser Glu Gln
            420                 425                 430

Lys Gln Lys Phe Pro Leu Ser Phe Lys Thr Phe Gly Glu Ala Ile Pro
        435                 440                 445

Pro Gln Tyr Ala Ile Gln Ile Leu Asp Glu Leu Thr Glu Gly Lys Ala
    450                 455                 460

Ile Ile Ser Thr Gly Val Gly Gln His Gln Met Trp Ala Ala Gln Phe

465                 470                 475                 480

Tyr Lys Tyr Arg Lys Pro Arg Gln Trp Leu Ser Ser Ser Gly Leu Gly
                485                 490                 495

Ala Met Gly Phe Gly Leu Pro Ala Ala Ile Gly Ala Ser Val Ala Asn
            500                 505                 510

Pro Asp Ala Ile Val Val Asp Ile Asp Gly Asp Gly Ser Phe Ile Met
        515                 520                 525

Asn Val Gln Glu Leu Ala Thr Ile Arg Val Glu Asn Leu Pro Val Lys
    530                 535                 540

Ile Leu Leu Leu Asn Asn Gln His Leu Gly Met Val Met Gln Trp Glu
545                 550                 555                 560

Asp Arg Phe Tyr Lys Ala Asn Arg Ala His Thr Tyr Leu Gly Asp Pro
                565                 570                 575

Ala Arg Glu Asn Glu Ile Phe Pro Asn Met Leu Gln Phe Ala Gly Ala
            580                 585                 590

Cys Gly Ile Pro Ala Ala Arg Val Thr Lys Lys Glu Glu Leu Arg Glu
        595                 600                 605

Ala Ile Gln Thr Met Leu Asp Thr Pro Gly Pro Tyr Leu Leu Asp Val
    610                 615                 620

Ile Cys Pro His Gln Glu His Val Leu Pro Met Ile Pro Ser Gly Gly
625                 630                 635                 640

Thr Phe Lys Asp Val Ile Thr Glu Gly Asp Gly Arg Thr Lys Tyr
                645                 650                 655

<210> 3
<211> 652
<212> PRT
<213> Brassica napus

<400> 3
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu

290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Trp Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Ser Gly Gly Thr Phe Lys
625                 630                 635                 640

Asp Val Ile Thr Glu Gly Asp Gly Arg Thr Lys Tyr
                645                 650

<210> 4
<211> 652
<212> PRT
<213> Brassica napus

<400> 4
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly

115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Glu Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Trp Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Ser Gly Gly Thr Phe Lys
625                 630                 635                 640

Asp Val Ile Thr Glu Gly Asp Gly Arg Thr Lys Tyr
                645                 650

<210> 5
<211> 41
<212> DNA
<213> Artificial Sequence

<220>
<223> Description of Artificial Sequence: Synthetic
      oligonucleotide

<400> 5
tgtgttaccg atgatcccaa atggtggcac tttcaaagat g                          41

<210> 6
<211> 41
<212> DNA
<213> Artificial Sequence

<220>
<223> Description of Artificial Sequence: Synthetic
      oligonucleotide

<400> 6
catctttgaa agtgccacca tttgggatca tcggtaacac a                          41

<210> 7
<211> 41
<212> DNA
<213> Artificial Sequence

<220>
<223> Description of Artificial Sequence: Synthetic
      oligonucleotide

<400> 7
cttgggatgg tcatgcaatt ggaagatcgg ttctacaaag c                          41

<210> 8
<211> 41
<212> DNA
<213> Artificial Sequence

<220>
<223> Description of Artificial Sequence: Synthetic
      oligonucleotide

<400> 8
gctttgtaga accgatcttc caattgcatg accatcccaa g                          41

<210> 9
<211> 24

<212> DNA
<213> Artificial Sequence

<220>
<223> Description of Artificial Sequence: Synthetic
      oligonucleotide

<400> 9
atgcaatggg aagatcggtt ctac                                              24


<210> 10
<211> 29
<212> DNA
<213> Artificial Sequence

<220>
<223> Description of Artificial Sequence: Synthetic
      oligonucleotide

<400> 10
ccatcycctt ckgttatkac atckttgaa                                         29


<210> 11
<211> 20
<212> DNA
<213> Artificial Sequence

<220>
<223> Description of Artificial Sequence: Synthetic
      oligonucleotide

<400> 11
ctaaccatgg cggcggcaac                                                   20


<210> 12
<211> 28
<212> DNA
<213> Artificial Sequence

<220>
<223> Description of Artificial Sequence: Synthetic
      oligonucleotide

<400> 12
agtctgggaa caaaccaaaa gcagtaca                                          28


<210> 13
<211> 28
<212> DNA
<213> Artificial Sequence

<220>
<223> Description of Artificial Sequence: Synthetic
      oligonucleotide

<400> 13
cgtctgggaa caaccaaaag tagtacaa                                          28

<210> 14
<211> 28
<212> DNA
<213> Artificial Sequence

<220>
<223> Description of Artificial Sequence: Synthetic
      oligonucleotide

<400> 14
agtgacgaag aaagaagaac tccgagaa                                          28


<210> 15
<211> 30
<212> DNA
<213> Artificial Sequence

<220>
<223> Description of Artificial Sequence: Synthetic
      oligonucleotide

<400> 15
ctgttattac atctttgaaa gtgccacaat                                        30


<210> 16
<211> 28
<212> DNA
<213> Artificial Sequence

<220>
<223> Description of Artificial Sequence: Synthetic
      oligonucleotide

<400> 16
tgacggtgat ggaagcttca taatgaac                                          28


<210> 17
<211> 28
<212> DNA
<213> Artificial Sequence

<220>
<223> Description of Artificial Sequence: Synthetic
      oligonucleotide

<400> 17
gtccwggtgt atccagcatt gtctgaat                                          28


<210> 18
<211> 26
<212> DNA
<213> Artificial Sequence

<220>
<223> Description of Artificial Sequence: Synthetic
      oligonucleotide

<400> 18
cagcatcttg ggatggtcat gcagtt                                            26

<210> 19
<211> 21
<212> DNA
<213> Artificial Sequence

<220>
<223> Description of Artificial Sequence: Synthetic
      oligonucleotide

<400> 19
cccatcaaag tactcgcacc g                                                21


<210> 20
<211> 21
<212> DNA
<213> Artificial Sequence

<220>
<223> Description of Artificial Sequence: Synthetic
      oligonucleotide

<400> 20
cccatcaacg tactcgcacc a                                                21


<210> 21
<211> 654
<212> PRT
<213> Artificial Sequence

<220>
<223> Description of Artificial Sequence: Synthetic
      polypeptide

<400> 21
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Asp Ser Ser Arg Leu His Arg Pro Leu Ala Ile
        35                  40                  45

Ser Ala Val Leu Asn Ser Pro Val Asn Val Ala Pro Pro Ser Pro Glu
    50                  55                  60

Lys Thr Asp Lys Asn Lys Thr Phe Val Ser Arg Tyr Ala Pro Asp Glu
65                  70                  75                  80

Pro Arg Lys Gly Ala Asp Ile Leu Val Glu Ala Leu Glu Arg Gln Gly
                85                  90                  95

Val Glu Thr Val Phe Ala Tyr Pro Gly Gly Ala Ser Met Glu Ile His

            100                 105                 110

Gln Ala Leu Thr Arg Ser Ser Thr Ile Arg Asn Val Leu Pro Arg His
        115                 120                 125

Glu Gln Gly Gly Val Phe Ala Ala Glu Gly Tyr Ala Arg Ser Ser Gly
    130                 135                 140

Lys Pro Gly Ile Cys Ile Ala Thr Ser Gly Pro Gly Ala Thr Asn Leu
145                 150                 155                 160

Val Ser Gly Leu Ala Asp Ala Met Leu Asp Ser Val Pro Leu Val Ala
                165                 170                 175

Ile Thr Gly Gln Val Pro Arg Arg Met Ile Gly Thr Asp Ala Phe Gln
            180                 185                 190

Glu Thr Pro Ile Val Glu Val Thr Arg Ser Ile Thr Lys His Asn Tyr
        195                 200                 205

Leu Val Met Asp Val Asp Asp Ile Pro Arg Ile Val Gln Glu Ala Phe
    210                 215                 220

Phe Leu Ala Thr Ser Gly Arg Pro Gly Pro Val Leu Val Asp Val Pro
225                 230                 235                 240

Lys Asp Ile Gln Gln Gln Leu Ala Ile Pro Asn Trp Asp Gln Pro Met
                245                 250                 255

Arg Leu Pro Gly Tyr Met Ser Arg Leu Pro Gln Pro Pro Glu Val Ser
            260                 265                 270

Gln Leu Gly Gln Ile Val Arg Leu Ile Ser Glu Ser Lys Arg Pro Val
        275                 280                 285

Leu Tyr Val Gly Gly Gly Ser Leu Asn Ser Ser Glu Glu Leu Gly Arg
    290                 295                 300

Phe Val Glu Leu Thr Gly Ile Pro Val Ala Ser Thr Leu Met Gly Leu
305                 310                 315                 320

Gly Ser Tyr Pro Cys Asn Asp Glu Leu Ser Leu Gln Met Leu Gly Met
                325                 330                 335

His Gly Thr Val Tyr Ala Asn Tyr Ala Val Glu His Ser Asp Leu Leu
            340                 345                 350

Leu Ala Phe Gly Val Arg Phe Asp Asp Arg Val Thr Gly Lys Leu Glu
        355                 360                 365

Ala Phe Ala Ser Arg Ala Lys Ile Val His Ile Asp Ile Asp Ser Ala
    370                 375                 380

Glu Ile Gly Lys Asn Lys Thr Pro His Val Ser Val Cys Gly Asp Val
385                 390                 395                 400

Lys Leu Ala Leu Gln Gly Met Asn Lys Val Leu Glu Asn Arg Ala Glu
                405                 410                 415

Glu Leu Lys Leu Asp Phe Gly Val Trp Arg Ser Glu Leu Ser Glu Gln
            420                 425                 430

Lys Gln Lys Phe Pro Leu Ser Phe Lys Thr Phe Gly Glu Ala Ile Pro
        435                 440                 445

Pro Gln Tyr Ala Ile Gln Ile Leu Asp Glu Leu Thr Glu Gly Lys Ala
    450                 455                 460

Ile Ile Ser Thr Gly Val Gly Gln His Gln Met Trp Ala Ala Gln Phe
465                 470                 475                 480

Tyr Lys Tyr Arg Lys Pro Arg Gln Trp Leu Ser Ser Ser Gly Leu Gly
                485                 490                 495

Ala Met Gly Phe Gly Leu Pro Ala Ala Ile Gly Ala Ser Val Ala Asn
            500                 505                 510

Pro Asp Ala Ile Val Val Asp Ile Asp Gly Asp Gly Ser Phe Ile Met
        515                 520                 525

Asn Val Gln Glu Leu Ala Thr Ile Arg Val Glu Asn Leu Pro Val Lys
    530                 535                 540

Ile Leu Leu Leu Asn Asn Gln His Leu Gly Met Val Met Gln Trp Glu
545                 550                 555                 560

Asp Arg Phe Tyr Lys Ala Asn Arg Ala His Thr Tyr Leu Gly Asp Pro
                565                 570                 575

Ala Arg Glu Asn Glu Ile Phe Pro Asn Met Leu Gln Phe Gly Ala Cys
            580                 585                 590

Gly Ile Pro Ala Ala Arg Val Thr Lys Lys Glu Glu Leu Arg Glu Ala
        595                 600                 605

Ile Gln Thr Met Leu Asp Thr Pro Gly Pro Tyr Leu Leu Asp Val Ile
    610                 615                 620

Cys Pro His Gln Glu His Val Leu Pro Met Ile Pro Ser Gly Gly Thr
625                 630                 635                 640

Phe Lys Asp Val Ile Thr Glu Gly Asp Gly Arg Thr Lys Tyr
                645                 650

<210> 22
<211> 652
<212> PRT
<213> Artificial Sequence

<220>
<223> Description of Artificial Sequence: Synthetic
      polypeptide

<400> 22
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Trp Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Ser Gly Gly Thr Phe Lys
625                 630                 635                 640

Asp Val Ile Thr Glu Gly Asp Gly Arg Thr Lys Tyr
                645                 650

<210> 23
<211> 654

<212> PRT
<213> Artificial Sequence

<220>
<223> Description of Artificial Sequence: Synthetic
      polypeptide

<400> 23
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Asp Ser Ser Arg Leu His Arg Pro Leu Ala Ile
        35                  40                  45

Ser Ala Val Leu Asn Ser Pro Val Asn Val Ala Pro Pro Ser Pro Glu
    50                  55                  60

Lys Thr Asp Lys Asn Lys Thr Phe Val Ser Arg Tyr Ala Pro Asp Glu
65                  70                  75                  80

Pro Arg Lys Gly Ala Asp Ile Leu Val Glu Ala Leu Glu Arg Gln Gly
                85                  90                  95

Val Glu Thr Val Phe Ala Tyr Pro Gly Gly Ala Ser Met Glu Ile His
            100                 105                 110

Gln Ala Leu Thr Arg Ser Ser Thr Ile Arg Asn Val Leu Pro Arg His
        115                 120                 125

Glu Gln Gly Gly Val Phe Ala Ala Glu Gly Tyr Ala Arg Ser Ser Gly
    130                 135                 140

Lys Pro Gly Ile Cys Ile Ala Thr Ser Gly Pro Gly Ala Thr Asn Leu
145                 150                 155                 160

Val Ser Gly Leu Ala Asp Ala Met Leu Asp Ser Val Pro Leu Val Ala
                165                 170                 175

Ile Thr Gly Gln Val Pro Arg Arg Met Ile Gly Thr Asp Val Phe Gln
            180                 185                 190

Glu Thr Pro Ile Val Glu Val Thr Arg Ser Ile Thr Lys His Asn Tyr
        195                 200                 205

Leu Val Met Asp Val Asp Asp Ile Pro Arg Ile Val Gln Glu Ala Phe
    210                 215                 220

Phe Leu Ala Thr Ser Gly Arg Pro Gly Pro Val Leu Val Asp Val Pro
225                 230                 235                 240

Lys Asp Ile Gln Gln Gln Leu Ala Ile Pro Asn Trp Asp Gln Pro Met
                245                 250                 255

Arg Leu Pro Gly Tyr Met Ser Arg Leu Pro Gln Pro Pro Glu Val Ser
            260                 265                 270

Gln Leu Gly Gln Ile Val Arg Leu Ile Ser Glu Ser Lys Arg Pro Val
        275                 280                 285

Leu Tyr Val Gly Gly Gly Ser Leu Asn Ser Ser Glu Glu Leu Gly Arg
    290                 295                 300

Phe Val Glu Leu Thr Gly Ile Pro Val Ala Ser Thr Leu Met Gly Leu
305                 310                 315                 320

Gly Ser Tyr Pro Cys Asn Asp Glu Leu Ser Leu Gln Met Leu Gly Met
                325                 330                 335

His Gly Thr Val Tyr Ala Asn Tyr Ala Val Glu His Ser Asp Leu Leu
            340                 345                 350

Leu Ala Phe Gly Val Arg Phe Asp Asp Arg Val Thr Gly Lys Leu Glu
        355                 360                 365

Ala Phe Ala Ser Arg Ala Lys Ile Val His Ile Asp Ile Asp Ser Ala
    370                 375                 380

Glu Ile Gly Lys Asn Lys Thr Pro His Val Ser Val Cys Gly Asp Val
385                 390                 395                 400

Lys Leu Ala Leu Gln Gly Met Asn Lys Val Leu Glu Asn Arg Ala Glu
                405                 410                 415

Glu Leu Lys Leu Asp Phe Gly Val Trp Arg Ser Glu Leu Ser Glu Gln
            420                 425                 430

Lys Gln Lys Phe Pro Leu Ser Phe Lys Thr Phe Gly Glu Ala Ile Pro
        435                 440                 445

Pro Gln Tyr Ala Ile Gln Ile Leu Asp Glu Leu Thr Glu Gly Lys Ala
    450                 455                 460

Ile Ile Ser Thr Gly Val Gly Gln His Gln Met Trp Ala Ala Gln Phe
465                 470                 475                 480

Tyr Lys Tyr Arg Lys Pro Arg Gln Trp Leu Ser Ser Ser Gly Leu Gly
                485                 490                 495

Ala Met Gly Phe Gly Leu Pro Ala Ala Ile Gly Ala Ser Val Ala Asn
            500                 505                 510

Pro Asp Ala Ile Val Val Asp Ile Asp Gly Asp Gly Ser Phe Ile Met
        515                 520                 525

Asn Val Gln Glu Leu Ala Thr Ile Arg Val Glu Asn Leu Pro Val Lys
    530                 535                 540

Ile Leu Leu Leu Asn Asn Gln His Leu Gly Met Val Met Gln Trp Glu
545                 550                 555                 560

Asp Arg Phe Tyr Lys Ala Asn Arg Ala His Thr Tyr Leu Gly Asp Pro
                565                 570                 575

Ala Arg Glu Asn Glu Ile Phe Pro Asn Met Leu Gln Phe Gly Ala Cys
            580                 585                 590

Gly Ile Pro Ala Ala Arg Val Thr Lys Lys Glu Glu Leu Arg Glu Ala
        595                 600                 605

Ile Gln Thr Met Leu Asp Thr Pro Gly Pro Tyr Leu Leu Asp Val Ile
    610                 615                 620

Cys Pro His Gln Glu His Val Leu Pro Met Ile Pro Ser Gly Gly Thr
625                 630                 635                 640

Phe Lys Asp Val Ile Thr Glu Gly Asp Gly Arg Thr Lys Tyr
                645                 650

<210> 24
<211> 652
<212> PRT
<213> Artificial Sequence

<220>
<223> Description of Artificial Sequence: Synthetic
      polypeptide

<400> 24
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Val Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu

530                 535                 540

Leu Asn Asn Gln His Leu Gly Met Val Met Gln Trp Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Ser Gly Gly Thr Phe Lys
625                 630                 635                 640

Asp Val Ile Thr Glu Gly Asp Gly Arg Thr Lys Tyr
                645                 650

<210> 25
<211> 652
<212> PRT
<213> Artificial Sequence

<220>
<223> Description of Artificial Sequence: Synthetic
      polypeptide

<400> 25
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Val Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe

            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Trp Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Ser Gly Gly Thr Phe Lys
625                 630                 635                 640

Asp Val Ile Thr Glu Gly Asp Gly Arg Thr Lys Tyr
                645                 650

<210> 26
<211> 654
<212> PRT
<213> Artificial Sequence

<220>
<223> Description of Artificial Sequence: Synthetic
      polypeptide

<400> 26
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Asp Ser Ser Arg Leu His Arg Pro Leu Ala Ile
        35                  40                  45

Ser Ala Val Leu Asn Ser Pro Val Asn Val Ala Pro Pro Ser Pro Glu
    50                  55                  60

Lys Thr Asp Lys Asn Lys Thr Phe Val Ser Arg Tyr Ala Pro Asp Glu
65                  70                  75                  80

Pro Arg Lys Gly Ala Asp Ile Leu Val Glu Ala Leu Glu Arg Gln Gly
                85                  90                  95

Val Glu Thr Val Phe Ala Tyr Pro Gly Gly Ala Ser Met Glu Ile His
            100                 105                 110

Gln Ala Leu Thr Arg Ser Ser Thr Ile Arg Asn Val Leu Pro Arg His
        115                 120                 125

Glu Gln Gly Gly Val Phe Ala Ala Glu Gly Tyr Ala Arg Ser Ser Gly
    130                 135                 140

Lys Pro Gly Ile Cys Ile Ala Thr Ser Gly Pro Gly Ala Thr Asn Leu

145                 150                 155                 160

Val Ser Gly Leu Ala Asp Ala Met Leu Asp Ser Val Pro Leu Val Ala
                165                 170                 175

Ile Thr Gly Gln Val Pro Arg Arg Met Ile Gly Thr Asp Asp Phe Gln
            180                 185                 190

Glu Thr Pro Ile Val Glu Val Thr Arg Ser Ile Thr Lys His Asn Tyr
        195                 200                 205

Leu Val Met Asp Val Asp Asp Ile Pro Arg Ile Val Gln Glu Ala Phe
    210                 215                 220

Phe Leu Ala Thr Ser Gly Arg Pro Gly Pro Val Leu Val Asp Val Pro
225                 230                 235                 240

Lys Asp Ile Gln Gln Gln Leu Ala Ile Pro Asn Trp Asp Gln Pro Met
                245                 250                 255

Arg Leu Pro Gly Tyr Met Ser Arg Leu Pro Gln Pro Pro Glu Val Ser
            260                 265                 270

Gln Leu Gly Gln Ile Val Arg Leu Ile Ser Glu Ser Lys Arg Pro Val
        275                 280                 285

Leu Tyr Val Gly Gly Gly Ser Leu Asn Ser Ser Glu Glu Leu Gly Arg
    290                 295                 300

Phe Val Glu Leu Thr Gly Ile Pro Val Ala Ser Thr Leu Met Gly Leu
305                 310                 315                 320

Gly Ser Tyr Pro Cys Asn Asp Glu Leu Ser Leu Gln Met Leu Gly Met
                325                 330                 335

His Gly Thr Val Tyr Ala Asn Tyr Ala Val Glu His Ser Asp Leu Leu
            340                 345                 350

Leu Ala Phe Gly Val Arg Phe Asp Asp Arg Val Thr Gly Lys Leu Glu
        355                 360                 365

Ala Phe Ala Ser Arg Ala Lys Ile Val His Ile Asp Ile Asp Ser Ala
    370                 375                 380

Glu Ile Gly Lys Asn Lys Thr Pro His Val Ser Val Cys Gly Asp Val
385                 390                 395                 400

Lys Leu Ala Leu Gln Gly Met Asn Lys Val Leu Glu Asn Arg Ala Glu
                405                 410                 415

Glu Leu Lys Leu Asp Phe Gly Val Trp Arg Ser Glu Leu Ser Glu Gln
            420                 425                 430

Lys Gln Lys Phe Pro Leu Ser Phe Lys Thr Phe Gly Glu Ala Ile Pro
        435                 440                 445

Pro Gln Tyr Ala Ile Gln Ile Leu Asp Glu Leu Thr Glu Gly Lys Ala
    450                 455                 460

Ile Ile Ser Thr Gly Val Gly Gln His Gln Met Trp Ala Ala Gln Phe
465                 470                 475                 480

Tyr Lys Tyr Arg Lys Pro Arg Gln Trp Leu Ser Ser Ser Gly Leu Gly
                485                 490                 495

Ala Met Gly Phe Gly Leu Pro Ala Ala Ile Gly Ala Ser Val Ala Asn
            500                 505                 510

Pro Asp Ala Ile Val Val Asp Ile Asp Gly Asp Gly Ser Phe Ile Met
        515                 520                 525

Asn Val Gln Glu Leu Ala Thr Ile Arg Val Glu Asn Leu Pro Val Lys
    530                 535                 540

Ile Leu Leu Leu Asn Asn Gln His Leu Gly Met Val Met Gln Trp Glu
545                 550                 555                 560

Asp Arg Phe Tyr Lys Ala Asn Arg Ala His Thr Tyr Leu Gly Asp Pro
                565                 570                 575

Ala Arg Glu Asn Glu Ile Phe Pro Asn Met Leu Gln Phe Gly Ala Cys
            580                 585                 590

Gly Ile Pro Ala Ala Arg Val Thr Lys Lys Glu Glu Leu Arg Glu Ala
        595                 600                 605

Ile Gln Thr Met Leu Asp Thr Pro Gly Pro Tyr Leu Leu Asp Val Ile
    610                 615                 620

Cys Pro His Gln Glu His Val Leu Pro Met Ile Pro Ser Gly Gly Thr
625                 630                 635                 640

Phe Lys Asp Val Ile Thr Glu Gly Asp Gly Arg Thr Lys Tyr
                645                 650

<210> 27
<211> 654
<212> PRT
<213> Artificial Sequence

<220>
<223> Description of Artificial Sequence: Synthetic
      polypeptide

<400> 27
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Asp Ser Ser Arg Leu His Arg Pro Leu Ala Ile
        35                  40                  45

Ser Ala Val Leu Asn Ser Pro Val Asn Val Ala Pro Pro Ser Pro Glu
    50                  55                  60

Lys Thr Asp Lys Asn Lys Thr Phe Val Ser Arg Tyr Ala Pro Asp Glu
65                  70                  75                  80

Pro Arg Lys Gly Ala Asp Ile Leu Val Glu Ala Leu Glu Arg Gln Gly
                85                  90                  95

Val Glu Thr Val Phe Ala Tyr Pro Gly Gly Ala Ser Met Glu Ile His
            100                 105                 110

Gln Ala Leu Thr Arg Ser Ser Thr Ile Arg Asn Val Leu Pro Arg His
        115                 120                 125

Glu Gln Gly Gly Val Phe Ala Ala Glu Gly Tyr Ala Arg Ser Ser Gly
    130                 135                 140

Lys Pro Gly Ile Cys Ile Ala Thr Ser Gly Pro Gly Ala Thr Asn Leu
145                 150                 155                 160

Val Ser Gly Leu Ala Asp Ala Met Leu Asp Ser Val Pro Leu Val Ala
                165                 170                 175

Ile Thr Gly Gln Val Pro Arg Arg Met Ile Gly Thr Asp Ala Phe Gln
            180                 185                 190

Glu Thr Pro Ile Val Glu Val Thr Arg Ser Ile Thr Lys His Asn Tyr
        195                 200                 205

Leu Val Met Asp Val Asp Asp Ile Pro Arg Ile Val Gln Glu Ala Phe
    210                 215                 220

Phe Leu Ala Thr Ser Gly Arg Pro Gly Pro Val Leu Val Asp Val Pro
225                 230                 235                 240

Lys Asp Ile Gln Gln Gln Leu Ala Ile Pro Asn Trp Asp Gln Pro Met
                245                 250                 255

Arg Leu Pro Gly Tyr Met Ser Arg Leu Pro Gln Pro Pro Glu Val Ser
            260                 265                 270

Gln Leu Gly Gln Ile Val Arg Leu Ile Ser Glu Ser Lys Arg Pro Val
        275                 280                 285

Leu Tyr Val Gly Gly Gly Ser Leu Asn Ser Ser Glu Glu Leu Gly Arg
    290                 295                 300

Phe Val Glu Leu Thr Gly Ile Pro Val Ala Ser Thr Leu Met Gly Leu
305                 310                 315                 320

Gly Ser Tyr Pro Cys Asn Asp Glu Leu Ser Leu Gln Met Leu Gly Met
                325                 330                 335

His Gly Thr Val Tyr Ala Asn Tyr Ala Val Glu His Ser Asp Leu Leu
            340                 345                 350

Leu Ala Phe Gly Val Arg Phe Asp Asp Arg Val Thr Gly Lys Leu Glu
        355                 360                 365

Ala Phe Ala Ser Arg Ala Lys Ile Val His Ile Asp Ile Asp Ser Ala
    370                 375                 380

Glu Ile Gly Lys Asn Lys Thr Pro His Val Ser Val Cys Gly Asp Val
385                 390                 395                 400

Lys Leu Ala Leu Gln Gly Met Asn Lys Val Leu Glu Asn Arg Ala Glu
                405                 410                 415

Glu Leu Lys Leu Asp Phe Gly Val Trp Arg Ser Glu Leu Ser Glu Gln
            420                 425                 430

Lys Gln Lys Phe Pro Leu Ser Phe Lys Thr Phe Gly Glu Ala Ile Pro
        435                 440                 445

Pro Gln Tyr Ala Ile Gln Ile Leu Asp Glu Leu Thr Glu Gly Lys Ala
    450                 455                 460

Ile Ile Ser Thr Gly Val Gly Gln His Gln Met Trp Ala Ala Gln Phe
465                 470                 475                 480

Tyr Lys Tyr Arg Lys Pro Arg Gln Trp Leu Ser Ser Ser Gly Leu Gly
                485                 490                 495

Ala Met Gly Phe Gly Leu Pro Ala Ala Ile Gly Ala Ser Val Ala Asn
            500                 505                 510

Pro Asp Ala Ile Val Val Asp Ile Asp Gly Asp Gly Ser Phe Ile Met
        515                 520                 525

Asn Val Gln Glu Leu Ala Thr Ile Arg Val Glu Asn Leu Pro Val Lys
    530                 535                 540

Ile Leu Leu Leu Asn Asn Gln His Leu Gly Met Val Met Gln Leu Glu
545                 550                 555                 560

Asp Arg Phe Tyr Lys Ala Asn Arg Ala His Thr Tyr Leu Gly Asp Pro
                565                 570                 575

Ala Arg Glu Asn Glu Ile Phe Pro Asn Met Leu Gln Phe Gly Ala Cys
            580                 585                 590

Gly Ile Pro Ala Ala Arg Val Thr Lys Lys Glu Glu Leu Arg Glu Ala
        595                 600                 605

Ile Gln Thr Met Leu Asp Thr Pro Gly Pro Tyr Leu Leu Asp Val Ile
    610                 615                 620

Cys Pro His Gln Glu His Val Leu Pro Met Ile Pro Ser Gly Gly Thr
625                 630                 635                 640

Phe Lys Asp Val Ile Thr Glu Gly Asp Gly Arg Thr Lys Tyr
                645                 650

<210> 28
<211> 654
<212> PRT
<213> Artificial Sequence

<220>
<223> Description of Artificial Sequence: Synthetic
      polypeptide

<400> 28
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Asp Ser Ser Arg Leu His Arg Pro Leu Ala Ile
        35                  40                  45

Ser Ala Val Leu Asn Ser Pro Val Asn Val Ala Pro Pro Ser Pro Glu
    50                  55                  60

Lys Thr Asp Lys Asn Lys Thr Phe Val Ser Arg Tyr Ala Pro Asp Glu
65                  70                  75                  80

Pro Arg Lys Gly Ala Asp Ile Leu Val Glu Ala Leu Glu Arg Gln Gly
                85                  90                  95

Val Glu Thr Val Phe Ala Tyr Pro Gly Gly Ala Ser Met Glu Ile His
            100                 105                 110

Gln Ala Leu Thr Arg Ser Ser Thr Ile Arg Asn Val Leu Pro Arg His
        115                 120                 125

Glu Gln Gly Gly Val Phe Ala Ala Glu Gly Tyr Ala Arg Ser Ser Gly
    130                 135                 140

Lys Pro Gly Ile Cys Ile Ala Thr Ser Gly Pro Gly Ala Thr Asn Leu
145                 150                 155                 160

Val Ser Gly Leu Ala Asp Ala Met Leu Asp Ser Val Pro Leu Val Ala
                165                 170                 175

Ile Thr Gly Gln Val Pro Arg Arg Met Ile Gly Thr Asp Ala Phe Gln
            180                 185                 190

Glu Thr Pro Ile Val Glu Val Thr Arg Ser Ile Thr Lys His Asn Tyr
        195                 200                 205

Leu Val Met Asp Val Asp Asp Ile Pro Arg Ile Val Gln Glu Ala Phe
    210                 215                 220

Phe Leu Ala Thr Ser Gly Arg Pro Gly Pro Val Leu Val Asp Val Pro
225                 230                 235                 240

Lys Asp Ile Gln Gln Gln Leu Ala Ile Pro Asn Trp Asp Gln Pro Met
                245                 250                 255

Arg Leu Pro Gly Tyr Met Ser Arg Leu Pro Gln Pro Pro Glu Val Ser
            260                 265                 270

Gln Leu Gly Gln Ile Val Arg Leu Ile Ser Glu Ser Lys Arg Pro Val
        275                 280                 285

Leu Tyr Val Gly Gly Gly Ser Leu Asn Ser Ser Glu Glu Leu Gly Arg
    290                 295                 300

Phe Val Glu Leu Thr Gly Ile Pro Val Ala Ser Thr Leu Met Gly Leu
305                 310                 315                 320

Gly Ser Tyr Pro Cys Asn Asp Glu Leu Ser Leu Gln Met Leu Gly Met
                325                 330                 335

His Gly Thr Val Tyr Ala Asn Tyr Ala Val Glu His Ser Asp Leu Leu
            340                 345                 350

Leu Ala Phe Gly Val Arg Phe Asp Asp Arg Val Thr Gly Lys Leu Glu
        355                 360                 365

Ala Phe Ala Ser Arg Ala Lys Ile Val His Ile Asp Ile Asp Ser Ala
    370                 375                 380

Glu Ile Gly Lys Asn Lys Thr Pro His Val Ser Val Cys Gly Asp Val
385                 390                 395                 400

Lys Leu Ala Leu Gln Gly Met Asn Lys Val Leu Glu Asn Arg Ala Glu
                405                 410                 415

Glu Leu Lys Leu Asp Phe Gly Val Trp Arg Ser Glu Leu Ser Glu Gln
            420                 425                 430

Lys Gln Lys Phe Pro Leu Ser Phe Lys Thr Phe Gly Glu Ala Ile Pro
        435                 440                 445

Pro Gln Tyr Ala Ile Gln Ile Leu Asp Glu Leu Thr Glu Gly Lys Ala
    450                 455                 460

Ile Ile Ser Thr Gly Val Gly Gln His Gln Met Trp Ala Ala Gln Phe
465                 470                 475                 480

Tyr Lys Tyr Arg Lys Pro Arg Gln Trp Leu Ser Ser Ser Gly Leu Gly
                485                 490                 495

Ala Met Gly Phe Gly Leu Pro Ala Ala Ile Gly Ala Ser Val Ala Asn
            500                 505                 510

Pro Asp Ala Ile Val Val Asp Ile Asp Gly Asp Gly Ser Phe Ile Met
        515                 520                 525

Asn Val Gln Glu Leu Ala Thr Ile Arg Val Glu Asn Leu Pro Val Lys
    530                 535                 540

Ile Leu Leu Leu Asn Asn Gln His Leu Gly Met Val Met Gln Leu Glu
545                 550                 555                 560

Asp Arg Phe Tyr Lys Ala Asn Arg Ala His Thr Tyr Leu Gly Asp Pro
                565                 570                 575

Ala Arg Glu Asn Glu Ile Phe Pro Asn Met Leu Gln Phe Gly Ala Cys
            580                 585                 590

Gly Ile Pro Ala Ala Arg Val Thr Lys Lys Glu Glu Leu Arg Glu Ala
        595                 600                 605

Ile Gln Thr Met Leu Asp Thr Pro Gly Pro Tyr Leu Leu Asp Val Ile
    610                 615                 620

Cys Pro His Gln Glu His Val Leu Pro Met Ile Pro Ser Gly Gly Thr
625                 630                 635                 640

Phe Lys Asp Val Ile Thr Glu Gly Asp Gly Arg Thr Lys Tyr
                645                 650

<210> 29
<211> 652
<212> PRT
<213> Artificial Sequence

<220>
<223> Description of Artificial Sequence: Synthetic
      polypeptide

<400> 29
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Leu Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile

580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Ser Gly Gly Thr Phe Lys
625                 630                 635                 640

Asp Val Ile Thr Glu Gly Asp Gly Arg Thr Lys Tyr
                645                 650

<210> 30
<211> 652
<212> PRT
<213> Artificial Sequence

<220>
<223> Description of Artificial Sequence: Synthetic
      polypeptide

<400> 30
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala

385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Met Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Ser Gly Gly Thr Phe Lys
625                 630                 635                 640

Asp Val Ile Thr Glu Gly Asp Gly Arg Thr Lys Tyr
                645                 650

<210> 31
<211> 652
<212> PRT
<213> Artificial Sequence

<220>
<223> Description of Artificial Sequence: Synthetic
      polypeptide

<400> 31
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met

        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Ser Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Ser Gly Gly Thr Phe Lys
625                 630                 635                 640

Asp Val Ile Thr Glu Gly Asp Gly Arg Thr Lys Tyr
                645                 650

<210> 32
<211> 654
<212> PRT
<213> Artificial Sequence

<220>
<223> Description of Artificial Sequence: Synthetic
      polypeptide

<400> 32
Met Ala Ala Ala Thr Ser Ser Ser Pro Ile Ser Leu Thr Ala Lys Pro

1               5                   10                  15

Ser Ser Lys Ser Pro Leu Pro Ile Ser Arg Phe Ser Leu Pro Phe Ser
            20                  25                  30

Leu Thr Pro Gln Lys Asp Ser Ser Arg Leu His Arg Pro Leu Ala Ile
        35                  40                  45

Ser Ala Val Leu Asn Ser Pro Val Asn Val Ala Pro Pro Ser Pro Glu
    50                  55                  60

Lys Thr Asp Lys Asn Lys Thr Phe Val Ser Arg Tyr Ala Pro Asp Glu
65                  70                  75                  80

Pro Arg Lys Gly Ala Asp Ile Leu Val Glu Ala Leu Glu Arg Gln Gly
                85                  90                  95

Val Glu Thr Val Phe Ala Tyr Pro Gly Gly Ala Ser Met Glu Ile His
            100                 105                 110

Gln Ala Leu Thr Arg Ser Ser Thr Ile Arg Asn Val Leu Pro Arg His
        115                 120                 125

Glu Gln Gly Gly Val Phe Ala Ala Glu Gly Tyr Ala Arg Ser Ser Gly
    130                 135                 140

Lys Pro Gly Ile Cys Ile Ala Thr Ser Gly Pro Gly Ala Thr Asn Leu
145                 150                 155                 160

Val Ser Gly Leu Ala Asp Ala Met Leu Asp Ser Val Pro Leu Val Ala
                165                 170                 175

Ile Thr Gly Gln Val Pro Arg Arg Met Ile Gly Thr Asp Ala Phe Gln
            180                 185                 190

Glu Thr Pro Ile Val Glu Val Thr Arg Ser Ile Thr Lys His Asn Tyr
        195                 200                 205

Leu Val Met Asp Val Asp Asp Ile Pro Arg Ile Val Gln Glu Ala Phe
    210                 215                 220

Phe Leu Ala Thr Ser Gly Arg Pro Gly Pro Val Leu Val Asp Val Pro
225                 230                 235                 240

Lys Asp Ile Gln Gln Gln Leu Ala Ile Pro Asn Trp Asp Gln Pro Met
                245                 250                 255

Arg Leu Pro Gly Tyr Met Ser Arg Leu Pro Gln Pro Pro Glu Val Ser
            260                 265                 270

Gln Leu Gly Gln Ile Val Arg Leu Ile Ser Glu Ser Lys Arg Pro Val
        275                 280                 285

Leu Tyr Val Gly Gly Gly Ser Leu Asn Ser Ser Glu Glu Leu Gly Arg
    290                 295                 300

Phe Val Glu Leu Thr Gly Ile Pro Val Ala Ser Thr Leu Met Gly Leu
305                 310                 315                 320

Gly Ser Tyr Pro Cys Asn Asp Glu Leu Ser Leu Gln Met Leu Gly Met
                325                 330                 335

His Gly Thr Val Tyr Ala Asn Tyr Ala Val Glu His Ser Asp Leu Leu
            340                 345                 350

Leu Ala Phe Gly Val Arg Phe Asp Asp Arg Val Thr Gly Lys Leu Glu
        355                 360                 365

Ala Phe Ala Ser Arg Ala Lys Ile Val His Ile Asp Ile Asp Ser Ala
    370                 375                 380

Glu Ile Gly Lys Asn Lys Thr Pro His Val Ser Val Cys Gly Asp Val
385                 390                 395                 400

Lys Leu Ala Leu Gln Gly Met Asn Lys Val Leu Glu Asn Arg Ala Glu
                405                 410                 415

Glu Leu Lys Leu Asp Phe Gly Val Trp Arg Ser Glu Leu Ser Glu Gln
            420                 425                 430

Lys Gln Lys Phe Pro Leu Ser Phe Lys Thr Phe Gly Glu Ala Ile Pro
        435                 440                 445

Pro Gln Tyr Ala Ile Gln Ile Leu Asp Glu Leu Thr Glu Gly Lys Ala
    450                 455                 460

Ile Ile Ser Thr Gly Val Gly Gln His Gln Met Trp Ala Ala Gln Phe
465                 470                 475                 480

Tyr Lys Tyr Arg Lys Pro Arg Gln Trp Leu Ser Ser Ser Gly Leu Gly
                485                 490                 495

Ala Met Gly Phe Gly Leu Pro Ala Ala Ile Gly Ala Ser Val Ala Asn
            500                 505                 510

Pro Asp Ala Ile Val Val Asp Ile Asp Gly Asp Gly Ser Phe Ile Met
        515                 520                 525

Asn Val Gln Glu Leu Ala Thr Ile Arg Val Glu Asn Leu Pro Val Lys
    530                 535                 540

Ile Leu Leu Leu Asn Asn Gln His Leu Gly Met Val Met Gln Trp Glu
545                 550                 555                 560

Asp Arg Phe Tyr Lys Ala Asn Arg Ala His Thr Tyr Leu Gly Asp Pro
                565                 570                 575

Ala Arg Glu Asn Glu Ile Phe Pro Asn Met Leu Gln Phe Gly Ala Cys
            580                 585                 590

Gly Ile Pro Ala Ala Arg Val Thr Lys Lys Glu Glu Leu Arg Glu Ala
        595                 600                 605

Ile Gln Thr Met Leu Asp Thr Pro Gly Pro Tyr Leu Leu Asp Val Ile
    610                 615                 620

Cys Pro His Gln Glu His Val Leu Pro Met Ile Pro Asn Gly Gly Thr
625                 630                 635                 640

Phe Lys Asp Val Ile Thr Glu Gly Asp Gly Arg Thr Lys Tyr
                645                 650

<210> 33
<211> 652
<212> PRT
<213> Artificial Sequence

<220>
<223> Description of Artificial Sequence: Synthetic
      polypeptide

<400> 33
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Trp Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Asn Gly Gly Thr Phe Lys
625                 630                 635                 640

Asp Val Ile Thr Glu Gly Asp Gly Arg Thr Lys Tyr
                645                 650

<210> 34
<211> 652
<212> PRT
<213> Artificial Sequence

<220>
<223> Description of Artificial Sequence: Synthetic
polypeptide

<400> 34
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Trp Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Asn Gly Gly Thr Phe Lys

625                 630                 635                 640

Asp Val Ile Thr Glu Gly Asp Gly Arg Thr Lys Tyr
                645                 650

<210> 35
<211> 652
<212> PRT
<213> Artificial Sequence

<220>
<223> Description of Artificial Sequence: Synthetic
      polypeptide

<400> 35
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr

435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Trp Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Asn Gly Gly Thr Phe Lys
625                 630                 635                 640

Asp Val Ile Thr Glu Gly Asp Gly Arg Thr Lys Tyr
                645                 650

<210> 36
<211> 652
<212> PRT
<213> Artificial Sequence

<220>
<223> Description of Artificial Sequence: Synthetic
      polypeptide

<400> 36
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro

245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Trp Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Asn Gly Gly Thr Phe Lys
625                 630                 635                 640

Asp Val Ile Thr Glu Gly Asp Gly Arg Thr Lys Tyr
                645                 650

<210> 37
<211> 652
<212> PRT
<213> Artificial Sequence

<220>
<223> Description of Artificial Sequence: Synthetic
      polypeptide

<400> 37
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp

50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Cys Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Ser Gly Gly Thr Phe Lys
625                 630                 635                 640

Asp Val Ile Thr Glu Gly Asp Gly Arg Thr Lys Tyr
                645                 650

<210> 38
<211> 654
<212> PRT
<213> Artificial Sequence

<220>
<223> Description of Artificial Sequence: Synthetic
      polypeptide

<400> 38
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Asp Ser Ser Arg Leu His Arg Pro Leu Ala Ile
        35                  40                  45

Ser Ala Val Leu Asn Ser Pro Val Asn Val Ala Pro Pro Ser Pro Glu
    50                  55                  60

Lys Thr Asp Lys Asn Lys Thr Phe Val Ser Arg Tyr Ala Pro Asp Glu
65                  70                  75                  80

Pro Arg Lys Gly Ala Asp Ile Leu Val Glu Ala Leu Glu Arg Gln Gly
                85                  90                  95

Val Glu Thr Val Phe Ala Tyr Pro Gly Gly Ala Ser Met Glu Ile His
            100                 105                 110

Gln Ala Leu Thr Arg Ser Ser Thr Ile Arg Asn Val Leu Pro Arg His
        115                 120                 125

Glu Gln Gly Gly Val Phe Ala Ala Glu Gly Tyr Ala Arg Ser Ser Gly
    130                 135                 140

Lys Pro Gly Ile Cys Ile Ala Thr Ser Gly Pro Gly Ala Thr Asn Leu
145                 150                 155                 160

Val Ser Gly Leu Ala Asp Ala Met Leu Asp Ser Val Pro Leu Val Ala
                165                 170                 175

Ile Thr Gly Gln Val Pro Arg Arg Met Ile Gly Thr Asp Ala Phe Gln
            180                 185                 190

Glu Thr Pro Ile Val Glu Val Thr Arg Ser Ile Thr Lys His Asn Tyr
        195                 200                 205

Leu Val Met Asp Val Asp Asp Ile Pro Arg Ile Val Gln Glu Ala Phe
    210                 215                 220

Phe Leu Ala Thr Ser Gly Arg Pro Gly Pro Val Leu Val Asp Val Pro
225                 230                 235                 240

Lys Asp Ile Gln Gln Gln Leu Ala Ile Pro Asn Trp Asp Gln Pro Met
                245                 250                 255

Arg Leu Pro Gly Tyr Met Ser Arg Leu Pro Gln Pro Pro Glu Val Ser
            260                 265                 270

Gln Leu Gly Gln Ile Val Arg Leu Ile Ser Glu Ser Lys Arg Pro Val
        275                 280                 285

Leu Tyr Val Gly Gly Gly Ser Leu Asn Ser Ser Glu Glu Leu Gly Arg
    290                 295                 300

Phe Val Glu Leu Thr Gly Ile Pro Val Ala Ser Thr Leu Met Gly Leu
305                 310                 315                 320

Gly Ser Tyr Pro Cys Asn Asp Glu Leu Ser Leu Gln Met Leu Gly Met
                325                 330                 335

His Gly Thr Val Tyr Ala Asn Tyr Ala Val Glu His Ser Asp Leu Leu
            340                 345                 350

Leu Ala Phe Gly Val Arg Phe Asp Asp Arg Val Thr Gly Lys Leu Glu
        355                 360                 365

Ala Phe Ala Ser Arg Ala Lys Ile Val His Ile Asp Ile Asp Ser Ala
    370                 375                 380

Glu Ile Gly Lys Asn Lys Thr Pro His Val Ser Val Cys Gly Asp Val
385                 390                 395                 400

Lys Leu Ala Leu Gln Gly Met Asn Lys Val Leu Glu Asn Arg Ala Glu
                405                 410                 415

Glu Leu Lys Leu Asp Phe Gly Val Trp Arg Ser Glu Leu Ser Glu Gln
            420                 425                 430

Lys Gln Lys Phe Pro Leu Ser Phe Lys Thr Phe Gly Glu Ala Ile Pro
        435                 440                 445

Pro Gln Tyr Ala Ile Gln Ile Leu Asp Glu Leu Thr Glu Gly Lys Ala
    450                 455                 460

Ile Ile Ser Thr Gly Val Gly Gln His Gln Met Trp Ala Ala Gln Phe
465                 470                 475                 480

Tyr Lys Tyr Arg Lys Pro Arg Gln Trp Leu Ser Ser Ser Gly Leu Gly
                485                 490                 495

Ala Met Gly Phe Gly Leu Pro Ala Ala Ile Gly Ala Ser Val Ala Asn
            500                 505                 510

Pro Asp Ala Ile Val Val Asp Ile Asp Gly Asp Gly Ser Phe Ile Met
        515                 520                 525

Asn Val Gln Glu Leu Ala Thr Ile Arg Val Glu Asn Leu Pro Val Lys
    530                 535                 540

Ile Leu Leu Leu Asn Asn Gln His Leu Gly Met Val Met Gln Trp Glu
545                 550                 555                 560

Asp Arg Phe Tyr Lys Ala Asn Arg Ala His Thr Tyr Leu Gly Asp Pro
                565                 570                 575

Ala Arg Glu Asn Glu Ile Phe Pro Asn Met Leu Gln Phe Gly Ala Cys
            580                 585                 590

Gly Ile Pro Ala Ala Arg Val Thr Lys Lys Glu Glu Leu Arg Glu Ala
        595                 600                 605

Ile Gln Thr Met Leu Asp Thr Pro Gly Pro Tyr Leu Leu Asp Val Ile
    610                 615                 620

Cys Pro His Gln Glu His Val Leu Pro Met Ile Pro Thr Gly Gly Thr
625                 630                 635                 640

Phe Lys Asp Val Ile Thr Glu Gly Asp Gly Arg Thr Lys Tyr
                645                 650

<210> 39
<211> 652
<212> PRT
<213> Artificial Sequence

<220>
<223> Description of Artificial Sequence: Synthetic
      polypeptide

<400> 39
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Trp Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Thr Gly Gly Thr Phe Lys
625                 630                 635                 640

Asp Val Ile Thr Glu Gly Asp Gly Arg Thr Lys Tyr
                645                 650

<210> 40
<211> 654
<212> PRT
<213> Artificial Sequence

<220>
<223> Description of Artificial Sequence: Synthetic
      polypeptide

<400> 40
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Asp Ser Ser Arg Leu His Arg Pro Leu Ala Ile
        35                  40                  45

Ser Ala Val Leu Asn Ser Pro Val Asn Val Ala Pro Pro Ser Pro Glu
    50                  55                  60

Lys Thr Asp Lys Asn Lys Thr Phe Val Ser Arg Tyr Ala Pro Asp Glu
65                  70                  75                  80

Pro Arg Lys Gly Ala Asp Ile Leu Val Glu Ala Leu Glu Arg Gln Gly
                85                  90                  95

Val Glu Thr Val Phe Ala Tyr Pro Gly Gly Ala Ser Met Glu Ile His
            100                 105                 110

Gln Ala Leu Thr Arg Ser Ser Thr Ile Arg Asn Val Leu Pro Arg His
        115                 120                 125

Glu Gln Gly Gly Val Phe Ala Ala Glu Gly Tyr Ala Arg Ser Ser Gly
    130                 135                 140

Lys Pro Gly Ile Cys Ile Ala Thr Ser Gly Pro Gly Ala Thr Asn Leu
145                 150                 155                 160

Val Ser Gly Leu Ala Asp Ala Met Leu Asp Ser Val Pro Leu Val Ala
                165                 170                 175

Ile Thr Gly Gln Val Pro Arg Arg Met Ile Gly Thr Asp Ala Phe Gln
            180                 185                 190

Glu Thr Pro Ile Val Glu Val Thr Arg Ser Ile Thr Lys His Asn Tyr
        195                 200                 205

Leu Val Met Asp Val Asp Asp Ile Pro Arg Ile Val Gln Glu Ala Phe
    210                 215                 220

Phe Leu Ala Thr Ser Gly Arg Pro Gly Pro Val Leu Val Asp Val Pro
225                 230                 235                 240

Lys Asp Ile Gln Gln Gln Leu Ala Ile Pro Asn Trp Asp Gln Pro Met
                245                 250                 255

Arg Leu Pro Gly Tyr Met Ser Arg Leu Pro Gln Pro Pro Glu Val Ser
            260                 265                 270

Gln Leu Gly Gln Ile Val Arg Leu Ile Ser Glu Ser Lys Arg Pro Val
        275                 280                 285

Leu Tyr Val Gly Gly Gly Ser Leu Asn Ser Ser Glu Glu Leu Gly Arg
    290                 295                 300

Phe Val Glu Leu Thr Gly Ile Pro Val Ala Ser Thr Leu Met Gly Leu
305                 310                 315                 320

Gly Ser Tyr Pro Cys Asn Asp Glu Leu Ser Leu Gln Met Leu Gly Met
                325                 330                 335

His Gly Thr Val Tyr Ala Asn Tyr Ala Val Glu His Ser Asp Leu Leu
            340                 345                 350

Leu Ala Phe Gly Val Arg Phe Asp Asp Arg Val Thr Gly Lys Leu Glu
        355                 360                 365

Ala Phe Ala Ser Arg Ala Lys Ile Val His Ile Asp Ile Asp Ser Ala
    370                 375                 380

Glu Ile Gly Lys Asn Lys Thr Pro His Val Ser Val Cys Gly Asp Val
385                 390                 395                 400

Lys Leu Ala Leu Gln Gly Met Asn Lys Val Leu Glu Asn Arg Ala Glu
                405                 410                 415

Glu Leu Lys Leu Asp Phe Gly Val Trp Arg Ser Glu Leu Ser Glu Gln
            420                 425                 430

Lys Gln Lys Phe Pro Leu Ser Phe Lys Thr Phe Gly Glu Ala Ile Pro
        435                 440                 445

Pro Gln Tyr Ala Ile Gln Ile Leu Asp Glu Leu Thr Glu Gly Lys Ala
    450                 455                 460

Ile Ile Ser Thr Gly Val Gly Gln His Gln Met Trp Ala Ala Gln Phe
465                 470                 475                 480

Tyr Lys Tyr Arg Lys Pro Arg Gln Trp Leu Ser Ser Ser Gly Leu Gly

485                 490                 495

Ala Met Gly Phe Gly Leu Pro Ala Ala Ile Gly Ala Ser Val Ala Asn
            500                 505                 510

Pro Asp Ala Ile Val Val Asp Ile Asp Gly Asp Gly Ser Phe Ile Met
        515                 520                 525

Asn Val Gln Glu Leu Ala Thr Ile Arg Val Glu Asn Leu Pro Val Lys
    530                 535                 540

Ile Leu Leu Leu Asn Asn Gln His Leu Gly Met Val Met Gln Trp Glu
545                 550                 555                 560

Asp Arg Phe Tyr Lys Ala Asn Arg Ala His Thr Tyr Leu Gly Asp Pro
                565                 570                 575

Ala Arg Glu Asn Glu Ile Phe Pro Asn Met Leu Gln Phe Gly Ala Cys
            580                 585                 590

Gly Ile Pro Ala Ala Arg Val Thr Lys Lys Glu Glu Leu Arg Glu Ala
        595                 600                 605

Ile Gln Thr Met Leu Asp Thr Pro Gly Pro Tyr Leu Leu Asp Val Ile
    610                 615                 620

Cys Pro His Gln Glu His Val Leu Pro Met Ile Pro Thr Gly Gly Thr
625                 630                 635                 640

Phe Lys Asp Val Ile Thr Glu Gly Asp Gly Arg Thr Lys Tyr
                645                 650

<210> 41
<211> 654
<212> PRT
<213> Artificial Sequence

<220>
<223> Description of Artificial Sequence: Synthetic
      polypeptide

<400> 41
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Asp Ser Ser Arg Leu His Arg Pro Leu Ala Ile
        35                  40                  45

Ser Ala Val Leu Asn Ser Pro Val Asn Val Ala Pro Pro Ser Pro Glu
    50                  55                  60

Lys Thr Asp Lys Asn Lys Thr Phe Val Ser Arg Tyr Ala Pro Asp Glu
65                  70                  75                  80

Pro Arg Lys Gly Ala Asp Ile Leu Val Glu Ala Leu Glu Arg Gln Gly
                85                  90                  95

Val Glu Thr Val Phe Ala Tyr Pro Gly Gly Ala Ser Met Glu Ile His
            100                 105                 110

Gln Ala Leu Thr Arg Ser Ser Thr Ile Arg Asn Val Leu Pro Arg His
        115                 120                 125

Glu Gln Gly Gly Val Phe Ala Ala Glu Gly Tyr Ala Arg Ser Ser Gly
    130                 135                 140

Lys Pro Gly Ile Cys Ile Ala Thr Ser Gly Pro Gly Ala Thr Asn Leu
145                 150                 155                 160

Val Ser Gly Leu Ala Asp Ala Met Leu Asp Ser Val Pro Leu Val Ala
                165                 170                 175

Ile Thr Gly Gln Val Pro Arg Arg Met Ile Gly Thr Asp Ala Phe Gln
            180                 185                 190

Glu Thr Pro Ile Val Glu Val Thr Arg Ser Ile Thr Lys His Asn Tyr
        195                 200                 205

Leu Val Met Asp Val Asp Asp Ile Pro Arg Ile Val Gln Glu Ala Phe
    210                 215                 220

Phe Leu Ala Thr Ser Gly Arg Pro Gly Pro Val Leu Val Asp Val Pro
225                 230                 235                 240

Lys Asp Ile Gln Gln Gln Leu Ala Ile Pro Asn Trp Asp Gln Pro Met
                245                 250                 255

Arg Leu Pro Gly Tyr Met Ser Arg Leu Pro Gln Pro Pro Glu Val Ser
            260                 265                 270

Gln Leu Gly Gln Ile Val Arg Leu Ile Ser Glu Ser Lys Arg Pro Val
        275                 280                 285

Leu Tyr Val Gly Gly Gly Ser Leu Asn Ser Ser Glu Glu Leu Gly Arg

290                 295                 300

Phe Val Glu Leu Thr Gly Ile Pro Val Ala Ser Thr Leu Met Gly Leu
305                 310                 315                 320

Gly Ser Tyr Pro Cys Asn Asp Glu Leu Ser Leu Gln Met Leu Gly Met
                325                 330                 335

His Gly Thr Val Tyr Ala Asn Tyr Ala Val Glu His Ser Asp Leu Leu
            340                 345                 350

Leu Ala Phe Gly Val Arg Phe Asp Asp Arg Val Thr Gly Lys Leu Glu
        355                 360                 365

Ala Phe Ala Ser Arg Ala Lys Ile Val His Ile Asp Ile Asp Ser Ala
    370                 375                 380

Glu Ile Gly Lys Asn Lys Thr Pro His Val Ser Val Cys Gly Asp Val
385                 390                 395                 400

Lys Leu Ala Leu Gln Gly Met Asn Lys Val Leu Glu Asn Arg Ala Glu
                405                 410                 415

Glu Leu Lys Leu Asp Phe Gly Val Trp Arg Ser Glu Leu Ser Glu Gln
            420                 425                 430

Lys Gln Lys Phe Pro Leu Ser Phe Lys Thr Phe Gly Glu Ala Ile Pro
        435                 440                 445

Pro Gln Tyr Ala Ile Gln Ile Leu Asp Glu Leu Thr Glu Gly Lys Ala
    450                 455                 460

Ile Ile Ser Thr Gly Val Gly Gln His Gln Met Trp Ala Ala Gln Phe
465                 470                 475                 480

Tyr Lys Tyr Arg Lys Pro Arg Gln Trp Leu Ser Ser Ser Gly Leu Gly
                485                 490                 495

Ala Met Gly Phe Gly Leu Pro Ala Ala Ile Gly Ala Ser Val Ala Asn
            500                 505                 510

Pro Asp Ala Ile Val Val Asp Ile Asp Gly Asp Gly Ser Phe Ile Met
        515                 520                 525

Asn Val Gln Glu Leu Ala Thr Ile Arg Val Glu Asn Leu Pro Val Lys
    530                 535                 540

Ile Leu Leu Leu Asn Asn Gln His Leu Gly Met Val Met Gln Trp Glu
545                 550                 555                 560

Asp Arg Phe Tyr Lys Ala Asn Arg Ala His Thr Tyr Leu Gly Asp Pro
                565                 570                 575

Ala Arg Glu Asn Glu Ile Phe Pro Asn Met Leu Gln Phe Gly Ala Cys
            580                 585                 590

Gly Ile Pro Ala Ala Arg Val Thr Lys Lys Glu Glu Leu Arg Glu Ala
        595                 600                 605

Ile Gln Thr Met Leu Asp Thr Pro Gly Pro Tyr Leu Leu Asp Val Ile
    610                 615                 620

Cys Pro His Gln Glu His Val Leu Pro Met Ile Pro Asn Gly Gly Thr
625                 630                 635                 640

Phe Lys Asp Val Ile Thr Glu Gly Asp Gly Arg Thr Lys Tyr
                645                 650

<210> 42
<211> 654
<212> PRT
<213> Artificial Sequence

<220>
<223> Description of Artificial Sequence: Synthetic
      polypeptide

<400> 42
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Asp Ser Ser Arg Leu His Arg Pro Leu Ala Ile
        35                  40                  45

Ser Ala Val Leu Asn Ser Pro Val Asn Val Ala Pro Pro Ser Pro Glu
    50                  55                  60

Lys Thr Asp Lys Asn Lys Thr Phe Val Ser Arg Tyr Ala Pro Asp Glu
65                  70                  75                  80

Pro Arg Lys Gly Ala Asp Ile Leu Val Glu Ala Leu Glu Arg Gln Gly
                85                  90                  95

Val Glu Thr Val Phe Ala Tyr Pro Gly Gly Ala Ser Met Glu Ile His

            100                 105                 110

Gln Ala Leu Thr Arg Ser Ser Thr Ile Arg Asn Val Leu Pro Arg His
        115                 120                 125

Glu Gln Gly Gly Val Phe Ala Ala Glu Gly Tyr Ala Arg Ser Ser Gly
    130                 135                 140

Lys Pro Gly Ile Cys Ile Ala Thr Ser Gly Pro Gly Ala Thr Asn Leu
145                 150                 155                 160

Val Ser Gly Leu Ala Asp Ala Met Leu Asp Ser Val Pro Leu Val Ala
                165                 170                 175

Ile Thr Gly Gln Val Pro Arg Arg Met Ile Gly Thr Asp Ala Phe Gln
            180                 185                 190

Glu Thr Pro Ile Val Glu Val Thr Arg Ser Ile Thr Lys His Asn Tyr
        195                 200                 205

Leu Val Met Asp Val Asp Asp Ile Pro Arg Ile Val Gln Glu Ala Phe
    210                 215                 220

Phe Leu Ala Thr Ser Gly Arg Pro Gly Pro Val Leu Val Asp Val Pro
225                 230                 235                 240

Lys Asp Ile Gln Gln Gln Leu Ala Ile Pro Asn Trp Asp Gln Pro Met
                245                 250                 255

Arg Leu Pro Gly Tyr Met Ser Arg Leu Pro Gln Pro Pro Glu Val Ser
            260                 265                 270

Gln Leu Gly Gln Ile Val Arg Leu Ile Ser Glu Ser Lys Arg Pro Val
        275                 280                 285

Leu Tyr Val Gly Gly Gly Ser Leu Asn Ser Ser Glu Glu Leu Gly Arg
    290                 295                 300

Phe Val Glu Leu Thr Gly Ile Pro Val Ala Ser Thr Leu Met Gly Leu
305                 310                 315                 320

Gly Ser Tyr Pro Cys Asn Asp Glu Leu Ser Leu Gln Met Leu Gly Met
                325                 330                 335

His Gly Thr Val Tyr Ala Asn Tyr Ala Val Glu His Ser Asp Leu Leu
            340                 345                 350

Leu Ala Phe Gly Val Arg Phe Asp Asp Arg Val Thr Gly Lys Leu Glu
        355                 360                 365

Ala Phe Ala Ser Arg Ala Lys Ile Val His Ile Asp Ile Asp Ser Ala
    370                 375                 380

Glu Ile Gly Lys Asn Lys Thr Pro His Val Ser Val Cys Gly Asp Val
385                 390                 395                 400

Lys Leu Ala Leu Gln Gly Met Asn Lys Val Leu Glu Asn Arg Ala Glu
                405                 410                 415

Glu Leu Lys Leu Asp Phe Gly Val Trp Arg Ser Glu Leu Ser Glu Gln
            420                 425                 430

Lys Gln Lys Phe Pro Leu Ser Phe Lys Thr Phe Gly Glu Ala Ile Pro
        435                 440                 445

Pro Gln Tyr Ala Ile Gln Ile Leu Asp Glu Leu Thr Glu Gly Lys Ala
    450                 455                 460

Ile Ile Ser Thr Gly Val Gly Gln His Gln Met Trp Ala Ala Gln Phe
465                 470                 475                 480

Tyr Lys Tyr Arg Lys Pro Arg Gln Trp Leu Ser Ser Ser Gly Leu Gly
                485                 490                 495

Ala Met Gly Phe Gly Leu Pro Ala Ala Ile Gly Ala Ser Val Ala Asn
            500                 505                 510

Pro Asp Ala Ile Val Val Asp Ile Asp Gly Asp Gly Ser Phe Ile Met
        515                 520                 525

Asn Val Gln Glu Leu Ala Thr Ile Arg Val Glu Asn Leu Pro Val Lys
    530                 535                 540

Ile Leu Leu Leu Asn Asn Gln His Leu Gly Met Val Met Gln Trp Glu
545                 550                 555                 560

Asp Arg Phe Tyr Lys Ala Asn Arg Ala His Thr Tyr Leu Gly Asp Pro
                565                 570                 575

Ala Arg Glu Asn Glu Ile Phe Pro Asn Met Leu Gln Phe Gly Ala Cys
            580                 585                 590

Gly Ile Pro Ala Ala Arg Val Thr Lys Lys Glu Glu Leu Arg Glu Ala
        595                 600                 605

Ile Gln Thr Met Leu Asp Thr Pro Gly Pro Tyr Leu Leu Asp Val Ile
    610                 615                 620

Cys Pro His Gln Glu His Val Leu Pro Met Ile Pro Asn Gly Gly Thr
625                 630                 635                 640

Phe Lys Asp Val Ile Thr Glu Gly Asp Gly Arg Thr Lys Tyr
                645                 650

<210> 43
<211> 652
<212> PRT
<213> Artificial Sequence

<220>
<223> Description of Artificial Sequence: Synthetic
      polypeptide

<400> 43
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Cys Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Asn Gly Gly Thr Phe Lys
625                 630                 635                 640

Asp Val Ile Thr Glu Gly Asp Gly Arg Thr Lys Tyr
                645                 650

<210> 44
<211> 652

<212> PRT
<213> Artificial Sequence

<220>
<223> Description of Artificial Sequence: Synthetic
      polypeptide

<400> 44
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Val Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Trp Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Asn Gly Gly Thr Phe Lys
625                 630                 635                 640

Asp Val Ile Thr Glu Gly Asp Gly Arg Thr Lys Tyr
                645                 650

<210> 45
<211> 652
<212> PRT
<213> Artificial Sequence

<220>
<223> Description of Artificial Sequence: Synthetic
      polypeptide

<400> 45
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Val Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu

530                 535                 540

Leu Asn Asn Gln His Leu Gly Met Val Met Gln Trp Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Asn Gly Gly Thr Phe Lys
625                 630                 635                 640

Asp Val Ile Thr Glu Gly Asp Gly Arg Thr Lys Tyr
                645                 650

<210> 46
<211> 654
<212> PRT
<213> Artificial Sequence

<220>
<223> Description of Artificial Sequence: Synthetic
      polypeptide

<400> 46
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Asp Ser Ser Arg Leu His Arg Pro Leu Ala Ile
        35                  40                  45

Ser Ala Val Leu Asn Ser Pro Val Asn Val Ala Pro Pro Ser Pro Glu
    50                  55                  60

Lys Thr Asp Lys Asn Lys Thr Phe Val Ser Arg Tyr Ala Pro Asp Glu
65                  70                  75                  80

Pro Arg Lys Gly Ala Asp Ile Leu Val Glu Ala Leu Glu Arg Gln Gly
                85                  90                  95

Val Glu Thr Val Phe Ala Tyr Pro Gly Gly Ala Ser Met Glu Ile His
            100                 105                 110

Gln Ala Leu Thr Arg Ser Ser Thr Ile Arg Asn Val Leu Pro Arg His
        115                 120                 125

Glu Gln Gly Gly Val Phe Ala Ala Glu Gly Tyr Ala Arg Ser Ser Gly
    130                 135                 140

Lys Pro Gly Ile Cys Ile Ala Thr Ser Gly Pro Gly Ala Thr Asn Leu
145                 150                 155                 160

Val Ser Gly Leu Ala Asp Ala Met Leu Asp Ser Val Pro Leu Val Ala
                165                 170                 175

Ile Thr Gly Gln Val Pro Arg Arg Met Ile Gly Thr Asp Val Phe Gln
            180                 185                 190

Glu Thr Pro Ile Val Glu Val Thr Arg Ser Ile Thr Lys His Asn Tyr
        195                 200                 205

Leu Val Met Asp Val Asp Asp Ile Pro Arg Ile Val Gln Glu Ala Phe
    210                 215                 220

Phe Leu Ala Thr Ser Gly Arg Pro Gly Pro Val Leu Val Asp Val Pro
225                 230                 235                 240

Lys Asp Ile Gln Gln Gln Leu Ala Ile Pro Asn Trp Asp Gln Pro Met
                245                 250                 255

Arg Leu Pro Gly Tyr Met Ser Arg Leu Pro Gln Pro Pro Glu Val Ser
            260                 265                 270

Gln Leu Gly Gln Ile Val Arg Leu Ile Ser Glu Ser Lys Arg Pro Val
        275                 280                 285

Leu Tyr Val Gly Gly Gly Ser Leu Asn Ser Ser Glu Glu Leu Gly Arg
    290                 295                 300

Phe Val Glu Leu Thr Gly Ile Pro Val Ala Ser Thr Leu Met Gly Leu
305                 310                 315                 320

Gly Ser Tyr Pro Cys Asn Asp Glu Leu Ser Leu Gln Met Leu Gly Met
                325                 330                 335

His Gly Thr Val Tyr Ala Asn Tyr Ala Val Glu His Ser Asp Leu Leu

340                 345                 350

Leu Ala Phe Gly Val Arg Phe Asp Asp Arg Val Thr Gly Lys Leu Glu
        355                 360                 365

Ala Phe Ala Ser Arg Ala Lys Ile Val His Ile Asp Ile Asp Ser Ala
    370                 375                 380

Glu Ile Gly Lys Asn Lys Thr Pro His Val Ser Val Cys Gly Asp Val
385                 390                 395                 400

Lys Leu Ala Leu Gln Gly Met Asn Lys Val Leu Glu Asn Arg Ala Glu
                405                 410                 415

Glu Leu Lys Leu Asp Phe Gly Val Trp Arg Ser Glu Leu Ser Glu Gln
            420                 425                 430

Lys Gln Lys Phe Pro Leu Ser Phe Lys Thr Phe Gly Glu Ala Ile Pro
        435                 440                 445

Pro Gln Tyr Ala Ile Gln Ile Leu Asp Glu Leu Thr Glu Gly Lys Ala
    450                 455                 460

Ile Ile Ser Thr Gly Val Gly Gln His Gln Met Trp Ala Ala Gln Phe
465                 470                 475                 480

Tyr Lys Tyr Arg Lys Pro Arg Gln Trp Leu Ser Ser Ser Gly Leu Gly
                485                 490                 495

Ala Met Gly Phe Gly Leu Pro Ala Ala Ile Gly Ala Ser Val Ala Asn
            500                 505                 510

Pro Asp Ala Ile Val Val Asp Ile Asp Gly Asp Gly Ser Phe Ile Met
        515                 520                 525

Asn Val Gln Glu Leu Ala Thr Ile Arg Val Glu Asn Leu Pro Val Lys
    530                 535                 540

Ile Leu Leu Leu Asn Asn Gln His Leu Gly Met Val Met Gln Trp Glu
545                 550                 555                 560

Asp Arg Phe Tyr Lys Ala Asn Arg Ala His Thr Tyr Leu Gly Asp Pro
                565                 570                 575

Ala Arg Glu Asn Glu Ile Phe Pro Asn Met Leu Gln Phe Gly Ala Cys
            580                 585                 590

Gly Ile Pro Ala Ala Arg Val Thr Lys Lys Glu Glu Leu Arg Glu Ala
        595                 600                 605

Ile Gln Thr Met Leu Asp Thr Pro Gly Pro Tyr Leu Leu Asp Val Ile
    610                 615                 620

Cys Pro His Gln Glu His Val Leu Pro Met Ile Pro Ser Gly Gly Thr
625                 630                 635                 640

Phe Lys Asp Val Ile Thr Glu Gly Asp Gly Arg Thr Lys Tyr
                645                 650

<210> 47
<211> 652
<212> PRT
<213> Artificial Sequence

<220>
<223> Description of Artificial Sequence: Synthetic
      polypeptide

<400> 47
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly

145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Trp Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Asn Gly Gly Thr Phe Lys
625                 630                 635                 640

Asp Val Ile Thr Glu Gly Asp Gly Arg Thr Lys Tyr
                645                 650

<210> 48
<211> 654
<212> PRT
<213> Artificial Sequence

<220>
<223> Description of Artificial Sequence: Synthetic
      polypeptide

<400> 48
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Asp Ser Ser Arg Leu His Arg Pro Leu Ala Ile
        35                  40                  45

Ser Ala Val Leu Asn Ser Pro Val Asn Val Ala Pro Pro Ser Pro Glu
    50                  55                  60

Lys Thr Asp Lys Asn Lys Thr Phe Val Ser Arg Tyr Ala Pro Asp Glu
65                  70                  75                  80

Pro Arg Lys Gly Ala Asp Ile Leu Val Glu Ala Leu Glu Arg Gln Gly
                85                  90                  95

Val Glu Thr Val Phe Ala Tyr Pro Gly Gly Ala Ser Met Glu Ile His
            100                 105                 110

Gln Ala Leu Thr Arg Ser Ser Thr Ile Arg Asn Val Leu Pro Arg His
        115                 120                 125

Glu Gln Gly Gly Val Phe Ala Ala Glu Gly Tyr Ala Arg Ser Ser Gly
    130                 135                 140

Lys Pro Gly Ile Cys Ile Ala Thr Ser Gly Pro Gly Ala Thr Asn Leu
145                 150                 155                 160

Val Ser Gly Leu Ala Asp Ala Met Leu Asp Ser Val Pro Leu Val Ala
                165                 170                 175

Ile Thr Gly Gln Val Pro Arg Arg Met Ile Gly Thr Asp Ala Phe Gln
            180                 185                 190

Glu Thr Pro Ile Val Glu Val Thr Arg Ser Ile Thr Lys His Asn Tyr
        195                 200                 205

Leu Val Met Asp Val Asp Asp Ile Pro Arg Ile Val Gln Glu Ala Phe
    210                 215                 220

Phe Leu Ala Thr Ser Gly Arg Pro Gly Pro Val Leu Val Asp Val Pro
225                 230                 235                 240

Lys Asp Ile Gln Gln Gln Leu Ala Ile Pro Asn Trp Asp Gln Pro Met
                245                 250                 255

Arg Leu Pro Gly Tyr Met Ser Arg Leu Pro Gln Pro Pro Glu Val Ser
            260                 265                 270

Gln Leu Gly Gln Ile Val Arg Leu Ile Ser Glu Ser Lys Arg Pro Val
        275                 280                 285

Leu Tyr Val Gly Gly Gly Ser Leu Asn Ser Ser Glu Glu Leu Gly Arg
    290                 295                 300

Phe Val Glu Leu Thr Gly Ile Pro Val Ala Ser Thr Leu Met Gly Leu
305                 310                 315                 320

Gly Ser Tyr Pro Cys Asn Asp Glu Leu Ser Leu Gln Met Leu Gly Met
                325                 330                 335

His Gly Thr Val Tyr Ala Asn Tyr Ala Val Glu His Ser Asp Leu Leu
            340                 345                 350

Leu Ala Phe Gly Val Arg Phe Asp Asp Arg Val Thr Gly Lys Leu Glu
        355                 360                 365

Ala Phe Ala Ser Arg Ala Lys Ile Val His Ile Asp Ile Asp Ser Ala
    370                 375                 380

Glu Ile Gly Lys Asn Lys Thr Pro His Val Ser Val Cys Gly Asp Val
385                 390                 395                 400

Lys Leu Ala Leu Gln Gly Met Asn Lys Val Leu Glu Asn Arg Ala Glu
                405                 410                 415

Glu Leu Lys Leu Asp Phe Gly Val Trp Arg Ser Glu Leu Ser Glu Gln
            420                 425                 430

Lys Gln Lys Phe Pro Leu Ser Phe Lys Thr Phe Gly Glu Ala Ile Pro
        435                 440                 445

Pro Gln Tyr Ala Ile Gln Ile Leu Asp Glu Leu Thr Glu Gly Lys Ala
    450                 455                 460

Ile Ile Ser Thr Gly Val Gly Gln His Gln Met Trp Ala Ala Gln Phe
465                 470                 475                 480

Tyr Lys Tyr Arg Lys Pro Arg Gln Trp Leu Ser Ser Ser Gly Leu Gly
                485                 490                 495

Ala Met Gly Phe Gly Leu Pro Ala Ala Ile Gly Ala Ser Val Ala Asn
            500                 505                 510

Pro Asp Ala Ile Val Val Asp Ile Asp Gly Asp Gly Ser Phe Ile Met
        515                 520                 525

Asn Val Gln Glu Leu Ala Thr Ile Arg Val Glu Asn Leu Pro Val Lys
    530                 535                 540

Ile Leu Leu Leu Asn Asn Gln His Leu Gly Met Val Met Gln Cys Glu
545                 550                 555                 560

Asp Arg Phe Tyr Lys Ala Asn Arg Ala His Thr Tyr Leu Gly Asp Pro
                565                 570                 575

Ala Arg Glu Asn Glu Ile Phe Pro Asn Met Leu Gln Phe Gly Ala Cys
            580                 585                 590

Gly Ile Pro Ala Ala Arg Val Thr Lys Lys Glu Glu Leu Arg Glu Ala
        595                 600                 605

Ile Gln Thr Met Leu Asp Thr Pro Gly Pro Tyr Leu Leu Asp Val Ile
    610                 615                 620

Cys Pro His Gln Glu His Val Leu Pro Met Ile Pro Ser Gly Gly Thr
625                 630                 635                 640

Phe Lys Asp Val Ile Thr Glu Gly Asp Gly Arg Thr Lys Tyr
                645                 650

<210> 49
<211> 652
<212> PRT
<213> Artificial Sequence

<220>
<223> Description of Artificial Sequence: Synthetic
      polypeptide

<400> 49
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Trp Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Asn Gly Gly Thr Phe Lys
625                 630                 635                 640

Asp Val Ile Thr Glu Gly Asp Gly Arg Thr Lys Tyr
                645                 650

<210> 50
<211> 654
<212> PRT
<213> Artificial Sequence

<220>
<223> Description of Artificial Sequence: Synthetic
      polypeptide

<400> 50
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Asp Ser Ser Arg Leu His Arg Pro Leu Ala Ile
        35                  40                  45

Ser Ala Val Leu Asn Ser Pro Val Asn Val Ala Pro Pro Ser Pro Glu
    50                  55                  60

Lys Thr Asp Lys Asn Lys Thr Phe Val Ser Arg Tyr Ala Pro Asp Glu
65                  70                  75                  80

Pro Arg Lys Gly Ala Asp Ile Leu Val Glu Ala Leu Glu Arg Gln Gly
                85                  90                  95

Val Glu Thr Val Phe Ala Tyr Pro Gly Gly Ala Ser Met Glu Ile His
            100                 105                 110

Gln Ala Leu Thr Arg Ser Ser Thr Ile Arg Asn Val Leu Pro Arg His
        115                 120                 125

Glu Gln Gly Gly Val Phe Ala Ala Glu Gly Tyr Ala Arg Ser Ser Gly
    130                 135                 140

Lys Pro Gly Ile Cys Ile Ala Thr Ser Gly Pro Gly Ala Thr Asn Leu
145                 150                 155                 160

Val Ser Gly Leu Ala Asp Ala Met Leu Asp Ser Val Pro Leu Val Ala
                165                 170                 175

Ile Thr Gly Gln Val Pro Arg Arg Met Ile Gly Thr Asp Ala Phe Gln
            180                 185                 190

Glu Thr Pro Ile Val Glu Val Thr Arg Ser Ile Thr Lys His Asn Tyr
        195                 200                 205

Leu Val Met Asp Val Asp Asp Ile Pro Arg Ile Val Gln Glu Ala Phe
    210                 215                 220

Phe Leu Ala Thr Ser Gly Arg Pro Gly Pro Val Leu Val Asp Val Pro
225                 230                 235                 240

Lys Asp Ile Gln Gln Gln Leu Ala Ile Pro Asn Trp Asp Gln Pro Met
                245                 250                 255

Arg Leu Pro Gly Tyr Met Ser Arg Leu Pro Gln Pro Pro Glu Val Ser
            260                 265                 270

Gln Leu Gly Gln Ile Val Arg Leu Ile Ser Glu Ser Lys Arg Pro Val
        275                 280                 285

Leu Tyr Val Gly Gly Gly Ser Leu Asn Ser Ser Glu Glu Leu Gly Arg
    290                 295                 300

Phe Val Glu Leu Thr Gly Ile Pro Val Ala Ser Thr Leu Met Gly Leu
305                 310                 315                 320

Gly Ser Tyr Pro Cys Asn Asp Glu Leu Ser Leu Gln Met Leu Gly Met
                325                 330                 335

His Gly Thr Val Tyr Ala Asn Tyr Ala Val Glu His Ser Asp Leu Leu
            340                 345                 350

Leu Ala Phe Gly Val Arg Phe Asp Asp Arg Val Thr Gly Lys Leu Glu
        355                 360                 365

Ala Phe Ala Ser Arg Ala Lys Ile Val His Ile Asp Ile Asp Ser Ala
    370                 375                 380

Glu Ile Gly Lys Asn Lys Thr Pro His Val Ser Val Cys Gly Asp Val
385                 390                 395                 400

Lys Leu Ala Leu Gln Gly Met Asn Lys Val Leu Glu Asn Arg Ala Glu
                405                 410                 415

Glu Leu Lys Leu Asp Phe Gly Val Trp Arg Ser Glu Leu Ser Glu Gln
            420                 425                 430

Lys Gln Lys Phe Pro Leu Ser Phe Lys Thr Phe Gly Glu Ala Ile Pro
        435                 440                 445

Pro Gln Tyr Ala Ile Gln Ile Leu Asp Glu Leu Thr Glu Gly Lys Ala
    450                 455                 460

Ile Ile Ser Thr Gly Val Gly Gln His Gln Met Trp Ala Ala Gln Phe
465                 470                 475                 480

Tyr Lys Tyr Arg Lys Pro Arg Gln Trp Leu Ser Ser Ser Gly Leu Gly
                485                 490                 495

Ala Met Gly Phe Gly Leu Pro Ala Ala Ile Gly Ala Ser Val Ala Asn
            500                 505                 510

Pro Asp Ala Ile Val Val Asp Ile Asp Gly Asp Gly Ser Phe Ile Met
        515                 520                 525

Asn Val Gln Glu Leu Ala Thr Ile Arg Val Glu Asn Leu Pro Val Lys
    530                 535                 540

Ile Leu Leu Leu Asn Asn Gln His Leu Gly Met Val Met Gln Leu Glu
545                 550                 555                 560

Asp Arg Phe Tyr Lys Ala Asn Arg Ala His Thr Tyr Leu Gly Asp Pro
                565                 570                 575

Ala Arg Glu Asn Glu Ile Phe Pro Asn Met Leu Gln Phe Gly Ala Cys

580                 585                 590

Gly Ile Pro Ala Ala Arg Val Thr Lys Lys Glu Glu Leu Arg Glu Ala
        595                 600                 605

Ile Gln Thr Met Leu Asp Thr Pro Gly Pro Tyr Leu Leu Asp Val Ile
    610                 615                 620

Cys Pro His Gln Glu His Val Leu Pro Met Ile Pro Ser Gly Gly Thr
625                 630                 635                 640

Phe Lys Asp Val Ile Thr Glu Gly Asp Gly Arg Thr Lys Tyr
                645                 650

<210> 51
<211> 652
<212> PRT
<213> Artificial Sequence

<220>
<223> Description of Artificial Sequence: Synthetic
      polypeptide

<400> 51
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala

385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Trp Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Asn Gly Gly Thr Phe Lys
625                 630                 635                 640

Asp Val Ile Thr Glu Gly Asp Gly Arg Thr Lys Tyr
                645                 650

<210> 52
<211> 652
<212> PRT
<213> Artificial Sequence

<220>
<223> Description of Artificial Sequence: Synthetic
      polypeptide

<400> 52
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met

        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Leu Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Asn Gly Gly Thr Phe Lys
625                 630                 635                 640

Asp Val Ile Thr Glu Gly Asp Gly Arg Thr Lys Tyr
                645                 650

<210> 53
<211> 652
<212> PRT
<213> Artificial Sequence

<220>
<223> Description of Artificial Sequence: Synthetic
      polypeptide

<400> 53
Met Ala Ala Ala Thr Ser Ser Ser Pro Ile Ser Leu Thr Ala Lys Pro

1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Glu Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Trp Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Asn Gly Gly Thr Phe Lys
625                 630                 635                 640

Asp Val Ile Thr Glu Gly Asp Gly Arg Thr Lys Tyr
                645                 650

<210> 54
<211> 654
<212> PRT
<213> Artificial Sequence

<220>
<223> Description of Artificial Sequence: Synthetic
      polypeptide

<400> 54
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Asp Ser Ser Arg Leu His Arg Pro Leu Ala Ile
        35                  40                  45

Ser Ala Val Leu Asn Ser Pro Val Asn Val Ala Pro Pro Ser Pro Glu
    50                  55                  60

Lys Thr Asp Lys Asn Lys Thr Phe Val Ser Arg Tyr Ala Pro Asp Glu
65                  70                  75                  80

Pro Arg Lys Gly Ala Asp Ile Leu Val Glu Ala Leu Glu Arg Gln Gly
                85                  90                  95

Val Glu Thr Val Phe Ala Tyr Pro Gly Gly Val Ser Met Glu Ile His
            100                 105                 110

Gln Ala Leu Thr Arg Ser Ser Thr Ile Arg Asn Val Leu Pro Arg His
        115                 120                 125

Glu Gln Gly Gly Val Phe Ala Ala Glu Gly Tyr Ala Arg Ser Ser Gly
    130                 135                 140

Lys Pro Gly Ile Cys Ile Ala Thr Ser Gly Pro Gly Ala Thr Asn Leu
145                 150                 155                 160

Val Ser Gly Leu Ala Asp Ala Met Leu Asp Ser Val Pro Leu Val Ala
                165                 170                 175

Ile Thr Gly Gln Val Pro Arg Arg Met Ile Gly Thr Asp Ala Phe Gln
            180                 185                 190

Glu Thr Pro Ile Val Glu Val Thr Arg Ser Ile Thr Lys His Asn Tyr
        195                 200                 205

Leu Val Met Asp Val Asp Asp Ile Pro Arg Ile Val Gln Glu Ala Phe
    210                 215                 220

Phe Leu Ala Thr Ser Gly Arg Pro Gly Pro Val Leu Val Asp Val Pro
225                 230                 235                 240

Lys Asp Ile Gln Gln Gln Leu Ala Ile Pro Asn Trp Asp Gln Pro Met
                245                 250                 255

Arg Leu Pro Gly Tyr Met Ser Arg Leu Pro Gln Pro Pro Glu Val Ser
            260                 265                 270

Gln Leu Gly Gln Ile Val Arg Leu Ile Ser Glu Ser Lys Arg Pro Val
        275                 280                 285

Leu Tyr Val Gly Gly Gly Ser Leu Asn Ser Ser Glu Glu Leu Gly Arg
    290                 295                 300

Phe Val Glu Leu Thr Gly Ile Pro Val Ala Ser Thr Leu Met Gly Leu
305                 310                 315                 320

Gly Ser Tyr Pro Cys Asn Asp Glu Leu Ser Leu Gln Met Leu Gly Met
                325                 330                 335

His Gly Thr Val Tyr Ala Asn Tyr Ala Val Glu His Ser Asp Leu Leu
            340                 345                 350

Leu Ala Phe Gly Val Arg Phe Asp Asp Arg Val Thr Gly Lys Leu Glu
        355                 360                 365

Ala Phe Ala Ser Arg Ala Lys Ile Val His Ile Asp Ile Asp Ser Ala
    370                 375                 380

Glu Ile Gly Lys Asn Lys Thr Pro His Val Ser Val Cys Gly Asp Val
385                 390                 395                 400

Lys Leu Ala Leu Gln Gly Met Asn Lys Val Leu Glu Asn Arg Ala Glu
                405                 410                 415

Glu Leu Lys Leu Asp Phe Gly Val Trp Arg Ser Glu Leu Ser Glu Gln
            420                 425                 430

Lys Gln Lys Phe Pro Leu Ser Phe Lys Thr Phe Gly Glu Ala Ile Pro
        435                 440                 445

Pro Gln Tyr Ala Ile Gln Ile Leu Asp Glu Leu Thr Glu Gly Lys Ala
    450                 455                 460

Ile Ile Ser Thr Gly Val Gly Gln His Gln Met Trp Ala Ala Gln Phe
465                 470                 475                 480

Tyr Lys Tyr Arg Lys Pro Arg Gln Trp Leu Ser Ser Ser Gly Leu Gly
                485                 490                 495

Ala Met Gly Phe Gly Leu Pro Ala Ala Ile Gly Ala Ser Val Ala Asn
            500                 505                 510

Pro Asp Ala Ile Val Val Asp Ile Asp Gly Asp Gly Ser Phe Ile Met
        515                 520                 525

Asn Val Gln Glu Leu Ala Thr Ile Arg Val Glu Asn Leu Pro Val Lys
    530                 535                 540

Ile Leu Leu Leu Asn Asn Gln His Leu Gly Met Val Met Gln Trp Glu
545                 550                 555                 560

Asp Arg Phe Tyr Lys Ala Asn Arg Ala His Thr Tyr Leu Gly Asp Pro
                565                 570                 575

Ala Arg Glu Asn Glu Ile Phe Pro Asn Met Leu Gln Phe Gly Ala Cys
            580                 585                 590

Gly Ile Pro Ala Ala Arg Val Thr Lys Lys Glu Glu Leu Arg Glu Ala
        595                 600                 605

Ile Gln Thr Met Leu Asp Thr Pro Gly Pro Tyr Leu Leu Asp Val Ile
    610                 615                 620

Cys Pro His Gln Glu His Val Leu Pro Met Ile Pro Ser Gly Gly Thr
625                 630                 635                 640

Phe Lys Asp Val Ile Thr Glu Gly Asp Gly Arg Thr Lys Tyr
                645                 650

<210> 55
<211> 652
<212> PRT
<213> Artificial Sequence

<220>
<223> Description of Artificial Sequence: Synthetic
      polypeptide

<400> 55
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Trp Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Asn Gly Gly Thr Phe Lys

625                 630                 635                 640

Asp Val Ile Thr Glu Gly Asp Gly Arg Thr Lys Tyr
                645                 650

<210> 56
<211> 654
<212> PRT
<213> Artificial Sequence

<220>
<223> Description of Artificial Sequence: Synthetic
      polypeptide

<400> 56
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Asp Ser Ser Arg Leu His Arg Pro Leu Ala Ile
        35                  40                  45

Ser Ala Val Leu Asn Ser Pro Val Asn Val Ala Pro Pro Ser Pro Glu
    50                  55                  60

Lys Thr Asp Lys Asn Lys Thr Phe Val Ser Arg Tyr Ala Pro Asp Glu
65                  70                  75                  80

Pro Arg Lys Gly Ala Asp Ile Leu Val Glu Ala Leu Glu Arg Gln Gly
                85                  90                  95

Val Glu Thr Val Phe Ala Tyr Pro Gly Gly Ala Ser Met Glu Ile His
            100                 105                 110

Gln Ala Leu Thr Arg Ser Ser Thr Ile Arg Asn Val Leu Pro Arg His
        115                 120                 125

Glu Gln Gly Gly Val Phe Ala Ala Glu Gly Tyr Ala Arg Ser Ser Gly
    130                 135                 140

Lys Pro Gly Ile Cys Ile Ala Thr Ser Gly Pro Gly Ala Thr Asn Leu
145                 150                 155                 160

Val Ser Gly Leu Ala Asp Ala Met Leu Asp Ser Val Pro Leu Val Ala
                165                 170                 175

Ile Thr Gly Gln Val Pro Arg Arg Met Ile Gly Thr Asp Asp Phe Gln
            180                 185                 190

Glu Thr Pro Ile Val Glu Val Thr Arg Ser Ile Thr Lys His Asn Tyr
        195                 200                 205

Leu Val Met Asp Val Asp Asp Ile Pro Arg Ile Val Gln Glu Ala Phe
    210                 215                 220

Phe Leu Ala Thr Ser Gly Arg Pro Gly Pro Val Leu Val Asp Val Pro
225                 230                 235                 240

Lys Asp Ile Gln Gln Gln Leu Ala Ile Pro Asn Trp Asp Gln Pro Met
                245                 250                 255

Arg Leu Pro Gly Tyr Met Ser Arg Leu Pro Gln Pro Pro Glu Val Ser
            260                 265                 270

Gln Leu Gly Gln Ile Val Arg Leu Ile Ser Glu Ser Lys Arg Pro Val
        275                 280                 285

Leu Tyr Val Gly Gly Gly Ser Leu Asn Ser Ser Glu Glu Leu Gly Arg
    290                 295                 300

Phe Val Glu Leu Thr Gly Ile Pro Val Ala Ser Thr Leu Met Gly Leu
305                 310                 315                 320

Gly Ser Tyr Pro Cys Asn Asp Glu Leu Ser Leu Gln Met Leu Gly Met
                325                 330                 335

His Gly Thr Val Tyr Ala Asn Tyr Ala Val Glu His Ser Asp Leu Leu
            340                 345                 350

Leu Ala Phe Gly Val Arg Phe Asp Asp Arg Val Thr Gly Lys Leu Glu
        355                 360                 365

Ala Phe Ala Ser Arg Ala Lys Ile Val His Ile Asp Ile Asp Ser Ala
    370                 375                 380

Glu Ile Gly Lys Asn Lys Thr Pro His Val Ser Val Cys Gly Asp Val
385                 390                 395                 400

Lys Leu Ala Leu Gln Gly Met Asn Lys Val Leu Glu Asn Arg Ala Glu
                405                 410                 415

Glu Leu Lys Leu Asp Phe Gly Val Trp Arg Ser Glu Leu Ser Glu Gln
            420                 425                 430

Lys Gln Lys Phe Pro Leu Ser Phe Lys Thr Phe Gly Glu Ala Ile Pro

435                 440                 445

Pro Gln Tyr Ala Ile Gln Ile Leu Asp Glu Leu Thr Glu Gly Lys Ala
    450                 455                 460

Ile Ile Ser Thr Gly Val Gly Gln His Gln Met Trp Ala Ala Gln Phe
465                 470                 475                 480

Tyr Lys Tyr Arg Lys Pro Arg Gln Trp Leu Ser Ser Ser Gly Leu Gly
                485                 490                 495

Ala Met Gly Phe Gly Leu Pro Ala Ala Ile Gly Ala Ser Val Ala Asn
            500                 505                 510

Pro Asp Ala Ile Val Val Asp Ile Asp Gly Asp Gly Ser Phe Ile Met
        515                 520                 525

Asn Val Gln Glu Leu Ala Thr Ile Arg Val Glu Asn Leu Pro Val Lys
    530                 535                 540

Ile Leu Leu Leu Asn Asn Gln His Leu Gly Met Val Met Gln Trp Glu
545                 550                 555                 560

Asp Arg Phe Tyr Lys Ala Asn Arg Ala His Thr Tyr Leu Gly Asp Pro
                565                 570                 575

Ala Arg Glu Asn Glu Ile Phe Pro Asn Met Leu Gln Phe Gly Ala Cys
            580                 585                 590

Gly Ile Pro Ala Ala Arg Val Thr Lys Lys Glu Glu Leu Arg Glu Ala
        595                 600                 605

Ile Gln Thr Met Leu Asp Thr Pro Gly Pro Tyr Leu Leu Asp Val Ile
    610                 615                 620

Cys Pro His Gln Glu His Val Leu Pro Met Ile Pro Ser Gly Gly Thr
625                 630                 635                 640

Phe Lys Asp Val Ile Thr Glu Gly Asp Gly Arg Thr Lys Tyr
                645                 650

<210> 57
<211> 652
<212> PRT
<213> Artificial Sequence

<220>
<223> Description of Artificial Sequence: Synthetic
      polypeptide

<400> 57
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro

245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Trp Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Asn Gly Gly Thr Phe Lys
625                 630                 635                 640

Asp Val Ile Thr Glu Gly Asp Gly Arg Thr Lys Tyr
                645                 650

<210> 58
<211> 654
<212> PRT
<213> Artificial Sequence

<220>
<223> Description of Artificial Sequence: Synthetic
      polypeptide

<400> 58
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Asp Ser Ser Arg Leu His Arg Pro Leu Ala Ile
        35                  40                  45

Ser Ala Val Leu Asn Ser Pro Val Asn Val Ala Pro Pro Ser Pro Glu

50                  55                  60

Lys Thr Asp Lys Asn Lys Thr Phe Val Ser Arg Tyr Ala Pro Asp Glu
65                  70                  75                  80

Pro Arg Lys Gly Ala Asp Ile Leu Val Glu Ala Leu Glu Arg Gln Gly
                85                  90                  95

Val Glu Thr Val Phe Ala Tyr Pro Gly Gly Ala Ser Met Glu Ile His
            100                 105                 110

Gln Ala Leu Thr Arg Ser Ser Thr Ile Arg Asn Val Leu Pro Arg His
        115                 120                 125

Glu Gln Gly Gly Val Phe Ala Ala Glu Gly Tyr Ala Arg Ser Ser Gly
    130                 135                 140

Lys Pro Gly Ile Cys Ile Ala Thr Ser Gly Pro Gly Ala Thr Asn Leu
145                 150                 155                 160

Val Ser Gly Leu Ala Asp Ala Met Leu Asp Ser Val Pro Leu Val Ala
                165                 170                 175

Ile Thr Gly Gln Val Pro Arg Arg Met Ile Gly Thr Asp Ala Phe Gln
            180                 185                 190

Glu Thr Pro Ile Val Glu Val Thr Arg Ser Ile Thr Lys His Asn Tyr
        195                 200                 205

Leu Val Met Asp Val Asp Asp Ile Pro Arg Ile Val Gln Glu Ala Phe
    210                 215                 220

Phe Leu Ala Thr Ser Gly Arg Pro Gly Pro Val Leu Val Asp Val Pro
225                 230                 235                 240

Lys Asp Ile Gln Gln Gln Leu Ala Ile Pro Asn Trp Asp Gln Pro Met
                245                 250                 255

Arg Leu Pro Gly Tyr Met Ser Arg Leu Pro Gln Pro Pro Glu Val Ser
            260                 265                 270

Gln Leu Gly Gln Ile Val Arg Leu Ile Ser Glu Ser Lys Arg Pro Val
        275                 280                 285

Leu Tyr Val Gly Gly Gly Ser Leu Asn Ser Ser Glu Glu Leu Gly Arg
    290                 295                 300

Phe Val Glu Leu Thr Gly Ile Pro Val Ala Ser Thr Leu Met Gly Leu
305                 310                 315                 320

Gly Ser Tyr Pro Cys Asn Asp Glu Leu Ser Leu Gln Met Leu Gly Met
                325                 330                 335

His Gly Thr Val Tyr Ala Asn Tyr Ala Val Glu His Ser Asp Leu Leu
            340                 345                 350

Leu Ala Phe Gly Val Arg Phe Asp Asp Arg Val Thr Gly Lys Leu Glu
        355                 360                 365

Ala Phe Ala Ser Arg Ala Lys Ile Val His Ile Asp Ile Asp Ser Ala
    370                 375                 380

Glu Ile Gly Lys Asn Lys Thr Pro His Val Ser Val Cys Gly Asp Val
385                 390                 395                 400

Lys Leu Ala Leu Gln Gly Met Asn Lys Val Leu Glu Asn Arg Ala Glu
                405                 410                 415

Glu Leu Lys Leu Asp Phe Gly Val Trp Arg Ser Glu Leu Ser Glu Gln
            420                 425                 430

Lys Gln Lys Phe Pro Leu Ser Phe Lys Thr Phe Gly Glu Ala Ile Pro
        435                 440                 445

Pro Gln Tyr Ala Ile Gln Ile Leu Asp Glu Leu Thr Glu Gly Lys Ala
    450                 455                 460

Ile Ile Ser Thr Gly Val Gly Gln His Gln Met Trp Ala Ala Gln Phe
465                 470                 475                 480

Tyr Lys Tyr Arg Lys Pro Arg Gln Trp Leu Ser Ser Ser Gly Leu Gly
                485                 490                 495

Ala Met Gly Phe Gly Leu Pro Ala Ala Ile Gly Ala Ser Val Ala Asn
            500                 505                 510

Pro Asp Ala Ile Val Val Asp Ile Asp Gly Asp Gly Ser Phe Ile Met
        515                 520                 525

Asn Val Gln Glu Leu Ala Thr Ile Arg Val Glu Asn Leu Pro Val Lys
    530                 535                 540

Ile Leu Leu Leu Asn Asn Gln His Leu Gly Met Val Met Gln Cys Glu
545                 550                 555                 560

Asp Arg Phe Tyr Lys Ala Asn Arg Ala His Thr Tyr Leu Gly Asp Pro
                565                 570                 575

Ala Arg Glu Asn Glu Ile Phe Pro Asn Met Leu Gln Phe Gly Ala Cys
            580                 585                 590

Gly Ile Pro Ala Ala Arg Val Thr Lys Lys Glu Glu Leu Arg Glu Ala
        595                 600                 605

Ile Gln Thr Met Leu Asp Thr Pro Gly Pro Tyr Leu Leu Asp Val Ile
    610                 615                 620

Cys Pro His Gln Glu His Val Leu Pro Met Ile Pro Ser Gly Gly Thr
625                 630                 635                 640

Phe Lys Asp Val Ile Thr Glu Gly Asp Gly Arg Thr Lys Tyr
                645                 650

<210> 59
<211> 652
<212> PRT
<213> Artificial Sequence

<220>
<223> Description of Artificial Sequence: Synthetic
      polypeptide

<400> 59
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Trp Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Asn Gly Gly Thr Phe Lys
625                 630                 635                 640

Asp Val Ile Thr Glu Gly Asp Gly Arg Thr Lys Tyr
                645                 650

<210> 60
<211> 654
<212> PRT
<213> Artificial Sequence

<220>
<223> Description of Artificial Sequence: Synthetic
      polypeptide

<400> 60
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Asp Ser Ser Arg Leu His Arg Pro Leu Ala Ile
        35                  40                  45

Ser Ala Val Leu Asn Ser Pro Val Asn Val Ala Pro Pro Ser Pro Glu
    50                  55                  60

Lys Thr Asp Lys Asn Lys Thr Phe Val Ser Arg Tyr Ala Pro Asp Glu
65                  70                  75                  80

Pro Arg Lys Gly Ala Asp Ile Leu Val Glu Ala Leu Glu Arg Gln Gly
                85                  90                  95

Val Glu Thr Val Phe Ala Tyr Pro Gly Gly Ala Ser Met Glu Ile His
            100                 105                 110

Gln Ala Leu Thr Arg Ser Ser Thr Ile Arg Asn Val Leu Pro Arg His
        115                 120                 125

Glu Gln Gly Gly Val Phe Ala Ala Glu Gly Tyr Ala Arg Ser Ser Gly
    130                 135                 140

Lys Pro Gly Ile Cys Ile Ala Thr Ser Gly Pro Gly Ala Thr Asn Leu
145                 150                 155                 160

Val Ser Gly Leu Ala Asp Ala Met Leu Asp Ser Val Pro Leu Val Ala
                165                 170                 175

Ile Thr Gly Gln Val Pro Arg Arg Met Ile Gly Thr Asp Ala Phe Gln
        180                 185                 190

Glu Thr Pro Ile Val Glu Val Thr Arg Ser Ile Thr Lys His Asn Tyr
    195                 200                 205

Leu Val Met Asp Val Asp Asp Ile Pro Arg Ile Val Gln Glu Ala Phe
    210                 215                 220

Phe Leu Ala Thr Ser Gly Arg Pro Gly Pro Val Leu Val Asp Val Pro
225                 230                 235                 240

Lys Asp Ile Gln Gln Gln Leu Ala Ile Pro Asn Trp Asp Gln Pro Met
                245                 250                 255

Arg Leu Pro Gly Tyr Met Ser Arg Leu Pro Gln Pro Pro Glu Val Ser
            260                 265                 270

Gln Leu Gly Gln Ile Val Arg Leu Ile Ser Glu Ser Lys Arg Pro Val
        275                 280                 285

Leu Tyr Val Gly Gly Gly Ser Leu Asn Ser Ser Glu Glu Leu Gly Arg
    290                 295                 300

Phe Val Glu Leu Thr Gly Ile Pro Val Ala Ser Thr Leu Met Gly Leu
305                 310                 315                 320

Gly Ser Tyr Pro Cys Asn Asp Glu Leu Ser Leu Gln Met Leu Gly Met
                325                 330                 335

His Gly Thr Val Tyr Ala Asn Tyr Ala Val Glu His Ser Asp Leu Leu
            340                 345                 350

Leu Ala Phe Gly Val Arg Phe Asp Asp Arg Val Thr Gly Lys Leu Glu
        355                 360                 365

Ala Phe Ala Ser Arg Ala Lys Ile Val His Ile Asp Ile Asp Ser Ala
    370                 375                 380

Glu Ile Gly Lys Asn Lys Thr Pro His Val Ser Val Cys Gly Asp Val
385                 390                 395                 400

Lys Leu Ala Leu Gln Gly Met Asn Lys Val Leu Glu Asn Arg Ala Glu
                405                 410                 415

Glu Leu Lys Leu Asp Phe Gly Val Trp Arg Ser Glu Leu Ser Glu Gln
            420                 425                 430

Lys Gln Lys Phe Pro Leu Ser Phe Lys Thr Phe Gly Glu Ala Ile Pro
        435                 440                 445

Pro Gln Tyr Ala Ile Gln Ile Leu Asp Glu Leu Thr Glu Gly Lys Ala
    450                 455                 460

Ile Ile Ser Thr Gly Val Gly Gln His Gln Met Trp Ala Ala Gln Phe
465                 470                 475                 480

Tyr Lys Tyr Arg Lys Pro Arg Gln Trp Leu Ser Ser Ser Gly Leu Gly
                485                 490                 495

Ala Met Gly Phe Gly Leu Pro Ala Ala Ile Gly Ala Ser Val Ala Asn
            500                 505                 510

Pro Asp Ala Ile Val Val Asp Ile Asp Gly Asp Gly Ser Phe Ile Met
        515                 520                 525

Asn Val Gln Glu Leu Ala Thr Ile Arg Val Glu Asn Leu Pro Val Lys
    530                 535                 540

Ile Leu Leu Leu Asn Asn Gln His Leu Gly Met Val Met Gln Cys Glu
545                 550                 555                 560

Asp Arg Phe Tyr Lys Ala Asn Arg Ala His Thr Tyr Leu Gly Asp Pro
                565                 570                 575

Ala Arg Glu Asn Glu Ile Phe Pro Asn Met Leu Gln Phe Gly Ala Cys
            580                 585                 590

Gly Ile Pro Ala Ala Arg Val Thr Lys Lys Glu Glu Leu Arg Glu Ala
        595                 600                 605

Ile Gln Thr Met Leu Asp Thr Pro Gly Pro Tyr Leu Leu Asp Val Ile
    610                 615                 620

Cys Pro His Gln Glu His Val Leu Pro Met Ile Pro Ser Gly Gly Thr
625                 630                 635                 640

Phe Lys Asp Val Ile Thr Glu Gly Asp Gly Arg Thr Lys Tyr
                645                 650

<210> 61
<211> 652
<212> PRT
<213> Artificial Sequence

<220>
<223> Description of Artificial Sequence: Synthetic
      polypeptide

<400> 61
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu
    290                 295                 300

Leu Thr Gly Ile Pro Val Ala Ser Thr Leu Met Gly Leu Gly Ser Tyr
305                 310                 315                 320

Pro Cys Asn Asp Asp Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly

                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Trp Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Asn Gly Gly Thr Phe Lys
625                 630                 635                 640

Asp Val Ile Thr Glu Gly Asp Gly Arg Thr Lys Tyr
                645                 650

<210> 62
<211> 652
<212> PRT
<213> Artificial Sequence

<220>
<223> Description of Artificial Sequence: Synthetic
polypeptide

<400> 62
Met Ala Ala Ala Thr Ser Ser Ser Pro Ile Ser Leu Thr Ala Lys Pro
1               5                   10                  15

Ser Ser Lys Ser Pro Leu Pro Ile Ser Arg Phe Ser Leu Pro Phe Ser
            20                  25                  30

Leu Thr Pro Gln Lys Pro Ser Ser Arg Leu His Arg Pro Leu Ala Ile
        35                  40                  45

Ser Ala Val Leu Asn Ser Pro Val Asn Val Ala Pro Glu Lys Thr Asp
    50                  55                  60

Lys Ile Lys Thr Phe Ile Ser Arg Tyr Ala Pro Asp Glu Pro Arg Lys
65                  70                  75                  80

Gly Ala Asp Ile Leu Val Glu Ala Leu Glu Arg Gln Gly Val Glu Thr
                85                  90                  95

Val Phe Ala Tyr Pro Gly Gly Ala Ser Met Glu Ile His Gln Ala Leu
            100                 105                 110

Thr Arg Ser Ser Thr Ile Arg Asn Val Leu Pro Arg His Glu Gln Gly
        115                 120                 125

Gly Val Phe Ala Ala Glu Gly Tyr Ala Arg Ser Ser Gly Lys Pro Gly
    130                 135                 140

Ile Cys Ile Ala Thr Ser Gly Pro Gly Ala Thr Asn Leu Val Ser Gly
145                 150                 155                 160

Leu Ala Asp Ala Met Leu Asp Ser Val Pro Leu Val Ala Ile Thr Gly
                165                 170                 175

Gln Val Pro Arg Arg Met Ile Gly Thr Asp Ala Phe Gln Glu Thr Pro
            180                 185                 190

Ile Val Glu Val Thr Arg Ser Ile Thr Lys His Asn Tyr Leu Val Met
        195                 200                 205

Asp Val Asp Asp Ile Pro Arg Ile Val Gln Glu Ala Phe Phe Leu Ala
    210                 215                 220

Thr Ser Gly Arg Pro Gly Pro Val Leu Val Asp Val Pro Lys Asp Ile
225                 230                 235                 240

Gln Gln Gln Leu Ala Ile Pro Asn Trp Asp Gln Pro Met Arg Leu Pro
                245                 250                 255

Gly Tyr Met Ser Arg Leu Pro Gln Pro Pro Glu Val Ser Gln Leu Gly
            260                 265                 270

Gln Ile Val Arg Leu Ile Ser Glu Ser Lys Arg Pro Val Leu Tyr Val
        275                 280                 285

Gly Gly Gly Ser Leu Asn Ser Ser Glu Glu Leu Gly Arg Phe Val Glu

290                 295                 300

Leu Thr Gly Ile Pro Val Ala Ser Thr Leu Met Gly Leu Gly Ser Tyr
305                 310                 315                 320

Pro Cys Asn Asp Glu Leu Ser Leu Gln Met Leu Gly Met His Gly Thr
                325                 330                 335

Val Tyr Ala Asn Tyr Ala Val Glu His Ser Asp Leu Leu Leu Ala Phe
            340                 345                 350

Gly Val Arg Phe Asp Asp Arg Val Thr Gly Lys Leu Glu Ala Phe Ala
        355                 360                 365

Ser Arg Ala Lys Ile Val His Ile Asp Ile Asp Ser Ala Glu Ile Gly
    370                 375                 380

Lys Asn Lys Thr Pro His Val Ser Val Cys Gly Asp Val Lys Leu Ala
385                 390                 395                 400

Leu Gln Gly Met Asn Lys Val Leu Glu Asn Arg Ala Glu Glu Leu Lys
                405                 410                 415

Leu Asp Phe Gly Val Trp Arg Ser Glu Leu Ser Glu Gln Lys Gln Lys
            420                 425                 430

Phe Pro Leu Ser Phe Lys Thr Phe Gly Glu Ala Ile Pro Pro Gln Tyr
        435                 440                 445

Ala Ile Gln Val Leu Asp Glu Leu Thr Gln Gly Lys Ala Ile Ile Ser
    450                 455                 460

Thr Gly Val Gly Gln His Gln Met Trp Ala Ala Gln Phe Tyr Lys Tyr
465                 470                 475                 480

Arg Lys Pro Arg Gln Trp Leu Ser Ser Ser Gly Leu Gly Ala Met Gly
                485                 490                 495

Phe Gly Leu Pro Ala Ala Ile Gly Ala Ser Val Ala Asn Pro Asp Ala
            500                 505                 510

Ile Val Val Asp Ile Asp Gly Asp Gly Ser Phe Ile Met Asn Val Gln
        515                 520                 525

Glu Leu Ala Thr Ile Arg Val Glu Asn Leu Pro Val Lys Ile Leu Leu
    530                 535                 540

Leu Asn Asn Gln His Leu Gly Met Val Met Gln Trp Glu Asp Arg Phe
545                 550                 555                 560

Tyr Lys Ala Asn Arg Ala His Thr Tyr Leu Gly Asp Pro Ala Arg Glu
                565                 570                 575

Asn Glu Ile Phe Pro Asn Met Leu Gln Phe Ala Gly Ala Cys Gly Ile
            580                 585                 590

Pro Ala Ala Arg Val Thr Lys Lys Glu Glu Leu Arg Glu Ala Ile Gln
        595                 600                 605

Thr Met Leu Asp Thr Pro Gly Pro Tyr Leu Leu Asp Val Ile Cys Pro
    610                 615                 620

His Gln Glu His Val Leu Pro Met Ile Pro Ser Gly Gly Thr Phe Lys
625                 630                 635                 640

Asp Val Ile Thr Glu Gly Asp Gly Arg Thr Lys Tyr
                645                 650

<210> 63
<211> 1968
<212> DNA
<213> Artificial Sequence

<220>
<223> Description of Artificial Sequence: Synthetic
      polynucleotide

<400> 63
atggcggcgg caacatcgtc ttctccgatc tccttaaccg ctaaaccttc ttccaaatcc      60

cctctaccca tttccagatt ctcccttccc ttctccttaa ccccacagaa agactcctcc     120

cgtctccacc gtcctctcgc catctccgcc gttctcaact cacccgtcaa tgtcgcacct     180

ccttcccctg aaaaaaccga caagaacaag actttcgtct cccgctacgc tcccgacgag     240

ccccgcaagg gtgctgatat cctcgtcgaa gccctcgagc gtcaaggcgt cgaaaccgtc     300

tttgcttatc ccggaggtgc ttccatggag atccaccaag ccttgactcg ctcctccacc     360

atccgtaacg tccttccccg tcacgaacaa ggaggagtct tcgccgccga gggttacgct     420

cgttcctccg gcaaaccggg aatctgcata gccacttcgg gtcccggagc taccaacctc     480

gtcagcgggt tagcagacgc gatgcttgac agtgttcctc ttgtcgccat tacaggacag     540

gtccctcgcc ggatgatcgg tactgacgcc ttccaagaga caccaatcgt tgaggtaacg     600

aggtctatta cgaaacataa ctatttggtg atggatgttg atgacatacc taggatcgtt     660

caagaagctt tctttctagc tacttccggt agacccggac cggttttggt tgatgttcct     720

aaggatattc agcagcagct tgcgattcct aactgggatc aacctatgcg cttacctggc     780

tacatgtcta ggttgcctca gcctccggaa gtttctcagt taggtcagat cgttaggttg 840

atctcggagt ctaagaggcc tgttttgtac gttggtggtg gaagcttgaa ctcgagtgaa 900

gaactgggga gatttgtcga gcttactggg atccccgttg cgagtacttt gatggggctt 960

ggctcttatc cttgtaacga tgagttgtcc ctgcagatgc ttggcatgca cgggactgtg 1020

tatgctaact acgctgtgga gcatagtgat ttgttgctgg cgtttggtgt taggtttgat 1080

gaccgtgtca cgggaaagct cgaggctttc gctagcaggg ctaaaattgt gcacatagac 1140

attgattctg ctgagattgg gaagaataag acacctcacg tgtctgtgtg tggtgatgta 1200

aagctggctt tgcaagggat gaacaaggtt cttgagaacc gggcggagga gctcaagctt 1260

gatttcggtg tttggaggag tgagttgagc gagcagaaac agaagttccc tttgagcttc 1320

aaaacgtttg gagaagccat tcctccgcag tacgcgattc agatcctcga cgagctaacc 1380

gaagggaagg caattatcag tactggtgtt ggacagcatc agatgtgggc ggcgcagttt 1440

tacaagtaca ggaagccgag acagtggctg tcgtcatcag gcctcggagc tatgggtttt 1500

ggacttcctg ctgcgattgg agcgtctgtg gcgaaccctg atgcgattgt tgtggatatt 1560

gacggtgatg gaagcttcat aatgaacgtt caagagctgg ccacaatccg tgtagagaat 1620

cttcctgtga agatactctt gttaaacaac cagcatcttg ggatggtcat gcaatgggaa 1680

gatcggttct acaaagctaa cagagctcac acttatctcg gggacccggc aagggagaac 1740

gagatcttcc ctaacatgct gcagtttgca ggagcttgcg ggattccagc tgcgagagtg 1800

acgaagaaag aagaactccg agaagctatt cagacaatgc tggatacacc aggaccatac 1860

ctgttggatg tgatatgtcc gcaccaagaa catgtgttac cgatgatccc aagtggtggc 1920

actttcaaag atgtaataac agaaggggat ggtcgcacta agtactga 1968

<210> 64
<211> 1959
<212> DNA
<213> Artificial Sequence

<220>
<223> Description of Artificial Sequence: Synthetic
      polynucleotide

<400> 64
atggcggcgg caacatcgtc ttctccgatc tccttaaccg ctaaaccttc ttccaaatcc 60

cctctaccca tttccagatt ctcccttccc ttctccttaa ccccacagaa accctcctcc 120

cgtctccacc gtcctctcgc catctccgcc gttctcaact cacccgtcaa tgtcgcacct 180

gaaaaaaccg acaagatcaa gactttcatc tcccgctacg ctcccgacga gccccgcaag 240

ggtgctgata tcctcgtgga agccctcgag cgtcaaggcg tcgaaaccgt cttcgcttat 300

cccggaggtg cttccatgga gatccaccaa gccttgactc gctcctccac catccgtaac 360

gtcctccccc gtcacgaaca aggaggagtc ttcgccgccg agggttacgc tcgttcctcc 420

ggcaaaccgg gaatctgcat agccacttcg ggtcccggag ctaccaacct cgtcagcggg 480

ttagccgacg cgatgcttga cagtgttcct ctcgtcgcca tcacaggaca ggtccctcgc 540

cggatgatcg gtactgacgc gttccaagag acgccaatcg ttgaggtaac gaggtctatt 600

acgaaacata actatctggt gatggatgtt gatgacatac ctaggatcgt tcaagaagct 660

ttctttctag ctacttccgg tagacccgga ccggttttgg ttgatgttcc taaggatatt 720

cagcagcagc ttgcgattcc taactgggat caacctatgc gcttgcctgg ctacatgtct 780

aggctgcctc agccaccgga agtttctcag ttaggtcaga tcgttaggtt gatctcggag 840

tctaagaggc ctgttttgta cgttggtggt ggaagcttga actcgagtga agaactgggg 900

agatttgtcg agcttactgg gatccctgtt gcgagtacgt tgatggggct tggctcttat 960

ccttgtaacg atgacttgtc cctgcagatg cttggcatgc acgggactgt gtatgctaac 1020

tacgctgtgg agcatagtga tttgttgctg gcgtttggtg ttaggtttga tgaccgtgtc 1080

acgggaaagc tcgaggcgtt tgcgagcagg gctaagattg tgcacataga cattgattct 1140

gctgagattg ggaagaataa gacacctcac gtgtctgtgt gtggtgatgt aaagctggct 1200

ttgcaaggga tgaacaaggt tcttgagaac cgggcggagg agctcaagct tgatttcggt 1260

gtttggagga gtgagttgag cgagcagaaa cagaagttcc cgttgagctt caaaacgttt 1320

ggagaagcca ttcctccgca gtacgcgatt caggtcctag acgagctaac ccaagggaag 1380

gcaattatca gtactggtgt tggacagcat cagatgtggg cggcgcagtt ttacaagtac 1440

aggaagccga ggcagtggct gtcgtcctca ggactcggag ctatgggttt cggacttcct 1500

gctgcgattg gagcgtctgt ggcgaaccct gatgcgattg ttgtggacat tgacggtgat 1560

ggaagcttca taatgaacgt tcaagagctg gccacaatcc gtgtagagaa tcttcctgtg 1620

aagatactct tgttaaacaa ccagcatctt gggatggtca tgcaatggga agatcggttc 1680

tacaaagcta acagagctca cacttatctc ggggacccgg caagggagaa cgagatcttc 1740

cctaacatgc tgcagtttgc aggagcttgc gggattccag ctgcgagagt gacgaagaaa 1800

gaagaactcc gagaagctat tcagacaatg ctggatacac ctggaccgta cctgttggat 1860

gtcatctgtc cgcaccaaga acatgtgtta ccgatgatcc caagtggtgg cactttcaaa 1920

gatgtaataa ccgaagggga tggtcgcact aagtactga 1959

<210> 65
<211> 1968
<212> DNA
<213> Artificial Sequence

<220>
<223> Description of Artificial Sequence: Synthetic

polynucleotide

<400> 65
atggcggcgg caacatcgtc ttctccgatc tccttaaccg ctaaaccttc ttccaaatcc      60

cctctaccca tttccagatt ctcccttccc ttctccttaa ccccacagaa agactcctcc     120

cgtctccacc gtcctctcgc catctccgcc gttctcaact cacccgtcaa tgtcgcacct     180

ccttcccctg aaaaaaccga caagaacaag actttcgtct cccgctacgc tcccgacgag     240

ccccgcaagg gtgctgatat cctcgtcgaa gccctcgagc gtcaaggcgt cgaaaccgtc     300

tttgcttatc ccggaggtgc ttccatggag atccaccaag ccttgactcg ctcctccacc     360

atccgtaacg tccttccccg tcacgaacaa ggaggagtct tcgccgccga gggttacgct     420

cgttcctccg gcaaaccggg aatctgcata gccacttcgg gtcccggagc taccaacctc     480

gtcagcgggt tagcagacgc gatgcttgac agtgttcctc ttgtcgccat tacaggacag     540

gtccctcgcc ggatgatcgg tactgacgtc ttccaagaga caccaatcgt tgaggtaacg     600

aggtctatta cgaaacataa ctatttggtg atggatgttg atgacatacc taggatcgtt     660

caagaagctt tctttctagc tacttccggt agacccggac cggttttggt tgatgttcct     720

aaggatattc agcagcagct tgcgattcct aactgggatc aacctatgcg cttacctggc     780

tacatgtcta ggttgcctca gcctccggaa gtttctcagt taggtcagat cgttaggttg     840

atctcggagt ctaagaggcc tgttttgtac gttggtggtg gaagcttgaa ctcgagtgaa     900

gaactgggga gatttgtcga gcttactggg atccccgttg cgagtacttt gatggggctt     960

ggctcttatc cttgtaacga tgagttgtcc ctgcagatgc ttggcatgca cgggactgtg    1020

tatgctaact acgctgtgga gcatagtgat ttgttgctgg cgtttggtgt taggtttgat    1080

gaccgtgtca cgggaaagct cgaggctttc gctagcaggg ctaaaattgt gcacatagac    1140

attgattctg ctgagattgg gaagaataag acacctcacg tgtctgtgtg tggtgatgta    1200

aagctggctt tgcaagggat gaacaaggtt cttgagaacc gggcggagga gctcaagctt    1260

gatttcggtg tttggaggag tgagttgagc gagcagaaac agaagttccc tttgagcttc    1320

aaaacgtttg gagaagccat tcctccgcag tacgcgattc agatcctcga cgagctaacc    1380

gaagggaagg caattatcag tactggtgtt ggacagcatc agatgtgggc ggcgcagttt    1440

tacaagtaca ggaagccgag acagtggctg tcgtcatcag gcctcggagc tatgggtttt    1500

ggacttcctg ctgcgattgg agcgtctgtg gcgaaccctg atgcgattgt tgtggatatt    1560

gacggtgatg gaagcttcat aatgaacgtt caagagctgg ccacaatccg tgtagagaat    1620

cttcctgtga agatactctt gttaaacaac cagcatcttg ggatggtcat gcaatgggaa    1680

gatcggttct acaaagctaa cagagctcac acttatctcg gggacccggc aagggagaac    1740

gagatcttcc ctaacatgct gcagtttgca ggagcttgcg ggattccagc tgcgagagtg    1800

acgaagaaag aagaactccg agaagctatt cagacaatgc tggatacacc aggaccatac 1860

ctgttggatg tgatatgtcc gcaccaagaa catgtgttac cgatgatccc aagtggtggc 1920

actttcaaag atgtaataac agaaggggat ggtcgcacta agtactga 1968

<210> 66
<211> 1959
<212> DNA
<213> Artificial Sequence

<220>
<223> Description of Artificial Sequence: Synthetic
      polynucleotide

<400> 66
atggcggcgg caacatcgtc ttctccgatc tccttaaccg ctaaaccttc ttccaaatcc 60

cctctaccca tttccagatt ctcccttccc ttctccttaa ccccacagaa accctcctcc 120

cgtctccacc gtcctctcgc catctccgcc gttctcaact cacccgtcaa tgtcgcacct 180

gaaaaaaccg acaagatcaa gactttcatc tcccgctacg ctcccgacga gccccgcaag 240

ggtgctgata tcctcgtgga agccctcgag cgtcaaggcg tcgaaaccgt cttcgcttat 300

cccggaggtg cttccatgga gatccaccaa gccttgactc gctcctccac catccgtaac 360

gtcctccccc gtcacgaaca aggaggagtc ttcgccgccg agggttacgc tcgttcctcc 420

ggcaaaccgg gaatctgcat agccacttcg ggtcccggag ctaccaacct cgtcagcggg 480

ttagccgacg cgatgcttga cagtgttcct ctcgtcgcca tcacaggaca ggtccctcgc 540

cggatgatcg gtactgacgt gttccaagag acgccaatcg ttgaggtaac gaggtctatt 600

acgaaacata actatctggt gatggatgtt gatgacatac ctaggatcgt tcaagaagct 660

ttctttctag ctacttccgg tagacccgga ccggttttgg ttgatgttcc taaggatatt 720

cagcagcagc ttgcgattcc taactgggat caacctatgc gcttgcctgg ctacatgtct 780

aggctgcctc agccaccgga agtttctcag ttaggtcaga tcgttaggtt gatctcggag 840

tctaagaggc ctgttttgta cgttggtggt ggaagcttga actcgagtga agaactgggg 900

agatttgtcg agcttactgg gatccctgtt gcgagtacgt tgatggggct tggctcttat 960

ccttgtaacg atgacttgtc cctgcagatg cttggcatgc acgggactgt gtatgctaac 1020

tacgctgtgg agcatagtga tttgttgctg gcgtttggtg ttaggtttga tgaccgtgtc 1080

acgggaaagc tcgaggcgtt tgcgagcagg gctaagattg tgcacataga cattgattct 1140

gctgagattg ggaagaataa gacacctcac gtgtctgtgt gtggtgatgt aaagctggct 1200

ttgcaaggga tgaacaaggt tcttgagaac cgggcggagg agctcaagct tgatttcggt 1260

gtttggagga gtgagttgag cgagcagaaa cagaagttcc cgttgagctt caaaacgttt 1320

ggagaagcca ttcctccgca gtacgcgatt caggtcctag acgagctaac ccaagggaag 1380

gcaattatca gtactggtgt tggacagcat cagatgtggg cggcgcagtt ttacaagtac 1440

aggaagccga ggcagtggct gtcgtcctca ggactcggag ctatgggttt cggacttcct 1500

gctgcgattg gagcgtctgt ggcgaaccct gatgcgattg ttgtggacat tgacggtgat 1560

ggaagcttca taatgaacgt tcaagagctg gccacaatcc gtgtagagaa tcttcctgtg 1620

aagatactct tgttaaacaa ccagcatctt gggatggtca tgcaatggga agatcggttc 1680

tacaaagcta acagagctca cacttatctc ggggacccgg caagggagaa cgagatcttc 1740

cctaacatgc tgcagtttgc aggagcttgc gggattccag ctgcgagagt gacgaagaaa 1800

gaagaactcc gagaagctat tcagacaatg ctggatacac ctggaccgta cctgttggat 1860

gtcatctgtc cgcaccaaga acatgtgtta ccgatgatcc caagtggtgg cactttcaaa 1920

gatgtaataa ccgaagggga tggtcgcact aagtactga 1959

<210> 67
<211> 1959
<212> DNA
<213> Artificial Sequence

<220>
<223> Description of Artificial Sequence: Synthetic
      polynucleotide

<400> 67
atggcggcgg caacatcgtc ttctccgatc tccttaaccg ctaaaccttc ttccaaatcc 60

cctctaccca tttccagatt ctcccttccc ttctccttaa ccccacagaa accctcctcc 120

cgtctccacc gtcctctcgc catctccgcc gttctcaact cacccgtcaa tgtcgcacct 180

gaaaaaaccg acaagatcaa gactttcatc tcccgctacg ctcccgacga gccccgcaag 240

ggtgctgata tcctcgtgga agccctcgag cgtcaaggcg tcgaaaccgt cttcgcttat 300

cccggaggtg cttccatgga gatccaccaa gccttgactc gctcctccac catccgtaac 360

gtcctccccc gtcacgaaca aggaggagtc ttcgccgccg agggttacgc tcgttcctcc 420

ggcaaaccgg gaatctgcat agccacttcg ggtcccggag ctaccaacct cgtcagcggg 480

ttagccgacg cgatgcttga cagtgttcct ctcgtcgcca tcacaggaca ggtccctcgc 540

cggatgatcg gtactgacgt gttccaagag acgccaatcg ttgaggtaac gaggtctatt 600

acgaaacata actatctggt gatggatgtt gatgacatac ctaggatcgt tcaagaagct 660

ttctttctag ctacttccgg tagacccgga ccggttttgg ttgatgttcc taaggatatt 720

cagcagcagc ttgcgattcc taactgggat caacctatgc gcttgcctgg ctacatgtct 780

aggctgcctc agccaccgga agtttctcag ttaggtcaga tcgttaggtt gatctcggag 840

tctaagaggc ctgttttgta cgttggtggt ggaagcttga actcgagtga agaactgggg 900

agatttgtcg agcttactgg gatccctgtt gcgagtacgt tgatggggct tggctcttat 960

ccttgtaacg atgacttgtc cctgcagatg cttggcatgc acgggactgt gtatgctaac 1020

tacgctgtgg agcatagtga tttgttgctg gcgtttggtg ttaggtttga tgaccgtgtc 1080

acgggaaagc tcgaggcgtt tgcgagcagg gctaagattg tgcacataga cattgattct 1140

gctgagattg ggaagaataa gacacctcac gtgtctgtgt gtggtgatgt aaagctggct 1200

ttgcaaggga tgaacaaggt tcttgagaac cgggcggagg agctcaagct tgatttcggt 1260

gtttggagga gtgagttgag cgagcagaaa cagaagttcc cgttgagctt caaaacgttt 1320

ggagaagcca ttcctccgca gtacgcgatt caggtcctag acgagctaac ccaagggaag 1380

gcaattatca gtactggtgt tggacagcat cagatgtggg cggcgcagtt ttacaagtac 1440

aggaagccga ggcagtggct gtcgtcctca ggactcggag ctatgggttt cggacttcct 1500

gctgcgattg gagcgtctgt ggcgaaccct gatgcgattg ttgtggacat tgacggtgat 1560

ggaagcttca taatgaacgt tcaagagctg gccacaatcc gtgtagagaa tcttcctgtg 1620

aagatactct tgttaaacaa ccagcatctt gggatggtca tgcaatggga agatcggttc 1680

tacaaagcta acagagctca cacttatctc ggggacccgg caagggagaa cgagatcttc 1740

cctaacatgc tgcagtttgc aggagcttgc gggattccag ctgcgagagt gacgaagaaa 1800

gaagaactcc gagaagctat tcagacaatg ctggatacac ctggaccgta cctgttggat 1860

gtcatctgtc cgcaccaaga acatgtgtta ccgatgatcc caagtggtgg cactttcaaa 1920

gatgtaataa ccgaagggga tggtcgcact aagtactga 1959

<210> 68
<211> 1968
<212> DNA
<213> Artificial Sequence

<220>
<223> Description of Artificial Sequence: Synthetic
      polynucleotide

<400> 68
atggcggcgg caacatcgtc ttctccgatc tccttaaccg ctaaaccttc ttccaaatcc 60

cctctaccca tttccagatt ctcccttccc ttctccttaa ccccacagaa agactcctcc 120

cgtctccacc gtcctctcgc catctccgcc gttctcaact cacccgtcaa tgtcgcacct 180

ccttcccctg aaaaaaccga caagaacaag actttcgtct cccgctacgc tcccgacgag 240

ccccgcaagg gtgctgatat cctcgtcgaa gccctcgagc gtcaaggcgt cgaaaccgtc 300

tttgcttatc ccggaggtgc ttccatggag atccaccaag ccttgactcg ctcctccacc 360

atccgtaacg tccttccccg tcacgaacaa ggaggagtct tcgccgccga gggttacgct 420

cgttcctccg gcaaaccggg aatctgcata gccacttcgg gtcccggagc taccaacctc 480

gtcagcgggt tagcagacgc gatgcttgac agtgttcctc ttgtcgccat tacaggacag 540

gtccctcgcc ggatgatcgg tactgacgac ttccaagaga caccaatcgt tgaggtaacg 600

aggtctatta cgaaacataa ctatttggtg atggatgttg atgacatacc taggatcgtt 660

caagaagctt tctttctagc tacttccggt agacccggac cggttttggt tgatgttcct 720

aaggatattc agcagcagct tgcgattcct aactgggatc aacctatgcg cttacctggc 780

tacatgtcta ggttgcctca gcctccggaa gtttctcagt taggtcagat cgttaggttg 840

atctcggagt ctaagaggcc tgttttgtac gttggtggtg gaagcttgaa ctcgagtgaa 900

gaactgggga gatttgtcga gcttactggg atccccgttg cgagtacttt gatggggctt 960

ggctcttatc cttgtaacga tgagttgtcc ctgcagatgc ttggcatgca cgggactgtg 1020

tatgctaact acgctgtgga gcatagtgat ttgttgctgg cgtttggtgt taggtttgat 1080

gaccgtgtca cgggaaagct cgaggctttc gctagcaggg ctaaaattgt gcacatagac 1140

attgattctg ctgagattgg gaagaataag acacctcacg tgtctgtgtg tggtgatgta 1200

aagctggctt tgcaagggat gaacaaggtt cttgagaacc gggcggagga gctcaagctt 1260

gatttcggtg tttggaggag tgagttgagc gagcagaaac agaagttccc tttgagcttc 1320

aaaacgtttg gagaagccat tcctccgcag tacgcgattc agatcctcga cgagctaacc 1380

gaagggaagg caattatcag tactggtgtt ggacagcatc agatgtgggc ggcgcagttt 1440

tacaagtaca ggaagccgag acagtggctg tcgtcatcag gcctcggagc tatgggtttt 1500

ggacttcctg ctgcgattgg agcgtctgtg gcgaaccctg atgcgattgt tgtggatatt 1560

gacggtgatg gaagcttcat aatgaacgtt caagagctgg ccacaatccg tgtagagaat 1620

cttcctgtga agatactctt gttaaacaac cagcatcttg ggatggtcat gcaatgggaa 1680

gatcggttct acaaagctaa cagagctcac acttatctcg gggacccggc aagggagaac 1740

gagatcttcc ctaacatgct gcagtttgca ggagcttgcg ggattccagc tgcgagagtg 1800

acgaagaaag aagaactccg agaagctatt cagacaatgc tggatacacc aggaccatac 1860

ctgttggatg tgatatgtcc gcaccaagaa catgtgttac cgatgatccc aagtggtggc 1920

actttcaaag atgtaataac agaaggggat ggtcgcacta agtactga 1968

<210> 69
<211> 1968
<212> DNA
<213> Artificial Sequence

<220>
<223> Description of Artificial Sequence: Synthetic
      polynucleotide

<400> 69
atggcggcgg caacatcgtc ttctccgatc tccttaaccg ctaaaccttc ttccaaatcc 60

cctctaccca tttccagatt ctcccttccc ttctccttaa ccccacagaa agactcctcc 120

cgtctccacc gtcctctcgc catctccgcc gttctcaact cacccgtcaa tgtcgcacct 180

ccttcccctg aaaaaaccga caagaacaag actttcgtct cccgctacgc tcccgacgag 240

ccccgcaagg gtgctgatat cctcgtcgaa gccctcgagc gtcaaggcgt cgaaaccgtc 300

tttgcttatc ccggaggtgc ttccatggag atccaccaag ccttgactcg ctcctccacc 360

atccgtaacg tccttccccg tcacgaacaa ggaggagtct tcgccgccga gggttacgct 420

cgttcctccg gcaaaccggg aatctgcata gccacttcgg gtcccggagc taccaacctc 480

gtcagcgggt tagcagacgc gatgcttgac agtgttcctc ttgtcgccat tacaggacag 540

gtccctcgcc ggatgatcgg tactgacgcc ttccaagaga caccaatcgt tgaggtaacg 600

aggtctatta cgaaacataa ctatttggtg atggatgttg atgacatacc taggatcgtt 660

caagaagctt tctttctagc tacttccggt agacccggac cggttttggt tgatgttcct 720

aaggatattc agcagcagct tgcgattcct aactgggatc aacctatgcg cttacctggc 780

tacatgtcta ggttgcctca gcctccggaa gtttctcagt taggtcagat cgttaggttg 840

atctcggagt ctaagaggcc tgttttgtac gttggtggtg gaagcttgaa ctcgagtgaa 900

gaactgggga gatttgtcga gcttactggg atccccgttg cgagtacttt gatggggctt 960

ggctcttatc cttgtaacga tgagttgtcc ctgcagatgc ttggcatgca cgggactgtg 1020

tatgctaact acgctgtgga gcatagtgat ttgttgctgg cgtttggtgt taggtttgat 1080

gaccgtgtca cgggaaagct cgaggctttc gctagcaggg ctaaaattgt gcacatagac 1140

attgattctg ctgagattgg gaagaataag acacctcacg tgtctgtgtg tggtgatgta 1200

aagctggctt tgcaagggat gaacaaggtt cttgagaacc gggcggagga gctcaagctt 1260

gatttcggtg tttggaggag tgagttgagc gagcagaaac agaagttccc tttgagcttc 1320

aaaacgtttg gagaagccat tcctccgcag tacgcgattc agatcctcga cgagctaacc 1380

gaagggaagg caattatcag tactggtgtt ggacagcatc agatgtgggc ggcgcagttt 1440

tacaagtaca ggaagccgag acagtggctg tcgtcatcag gcctcggagc tatgggtttt 1500

ggacttcctg ctgcgattgg agcgtctgtg gcgaaccctg atgcgattgt tgtggatatt 1560

gacggtgatg gaagcttcat aatgaacgtt caagagctgg ccacaatccg tgtagagaat 1620

cttcctgtga agatactctt gttaaacaac cagcatcttg ggatggtcat gcaattggaa 1680

gatcggttct acaaagctaa cagagctcac acttatctcg gggacccggc aagggagaac 1740

gagatcttcc ctaacatgct gcagtttgca ggagcttgcg ggattccagc tgcgagagtg 1800

acgaagaaag aagaactccg agaagctatt cagacaatgc tggatacacc aggaccatac 1860

ctgttggatg tgatatgtcc gcaccaagaa catgtgttac cgatgatccc aagtggtggc 1920

actttcaaag atgtaataac agaaggggat ggtcgcacta agtactga 1968

<210> 70
<211> 1968
<212> DNA
<213> Artificial Sequence

<220>
<223> Description of Artificial Sequence: Synthetic
      polynucleotide

<400> 70
atggcggcgg caacatcgtc ttctccgatc tccttaaccg ctaaaccttc ttccaaatcc      60

cctctaccca tttccagatt ctcccttccc ttctccttaa ccccacagaa agactcctcc     120

cgtctccacc gtcctctcgc catctccgcc gttctcaact cacccgtcaa tgtcgcacct     180

ccttcccctg aaaaaaccga caagaacaag actttcgtct cccgctacgc tcccgacgag     240

ccccgcaagg gtgctgatat cctcgtcgaa gccctcgagc gtcaaggcgt cgaaaccgtc     300

tttgcttatc ccggaggtgc ttccatggag atccaccaag ccttgactcg ctcctccacc     360

atccgtaacg tccttccccg tcacgaacaa ggaggagtct tcgccgccga gggttacgct     420

cgttcctccg gcaaaccggg aatctgcata gccacttcgg gtcccggagc taccaacctc     480

gtcagcgggt tagcagacgc gatgcttgac agtgttcctc ttgtcgccat tacaggacag     540

gtccctcgcc ggatgatcgg tactgacgcc ttccaagaga caccaatcgt tgaggtaacg     600

aggtctatta cgaaacataa ctatttggtg atggatgttg atgacatacc taggatcgtt     660

caagaagctt tctttctagc tacttccggt agacccggac cggttttggt tgatgttcct     720

aaggatattc agcagcagct tgcgattcct aactgggatc aacctatgcg cttacctggc     780

tacatgtcta ggttgcctca gcctccggaa gtttctcagt taggtcagat cgttaggttg     840

atctcggagt ctaagaggcc tgttttgtac gttggtggtg gaagcttgaa ctcgagtgaa     900

gaactgggga gatttgtcga gcttactggg atccccgttg cgagtacttt gatggggctt     960

ggctcttatc cttgtaacga tgagttgtcc ctgcagatgc ttggcatgca cgggactgtg    1020

tatgctaact acgctgtgga gcatagtgat ttgttgctgg cgtttggtgt taggtttgat    1080

gaccgtgtca cgggaaagct cgaggctttc gctagcaggg ctaaaattgt gcacatagac    1140

attgattctg ctgagattgg gaagaataag acacctcacg tgtctgtgtg tggtgatgta    1200

aagctggctt tgcaagggat gaacaaggtt cttgagaacc gggcggagga gctcaagctt    1260

gatttcggtg tttggaggag tgagttgagc gagcagaaac agaagttccc tttgagcttc    1320

aaaacgtttg gagaagccat tcctccgcag tacgcgattc agatcctcga cgagctaacc    1380

gaagggaagg caattatcag tactggtgtt ggacagcatc agatgtgggc ggcgcagttt    1440

tacaagtaca ggaagccgag acagtggctg tcgtcatcag gcctcggagc tatgggtttt    1500

ggacttcctg ctgcgattgg agcgtctgtg gcgaaccctg atgcgattgt tgtggatatt    1560

gacggtgatg gaagcttcat aatgaacgtt caagagctgg ccacaatccg tgtagagaat    1620

cttcctgtga agatactctt gttaaacaac cagcatcttg ggatggtcat gcaattggaa 1680

gatcggttct acaaagctaa cagagctcac acttatctcg gggacccggc aagggagaac 1740

gagatcttcc ctaacatgct gcagtttgca ggagcttgcg ggattccagc tgcgagagtg 1800

acgaagaaag aagaactccg agaagctatt cagacaatgc tggatacacc aggaccatac 1860

ctgttggatg tgatatgtcc gcaccaagaa catgtgttac cgatgatccc aagtggtggc 1920

actttcaaag atgtaataac agaaggggat ggtcgcacta agtactga 1968

<210> 71
<211> 1959
<212> DNA
<213> Artificial Sequence

<220>
<223> Description of Artificial Sequence: Synthetic
      polynucleotide

<400> 71
atggcggcgg caacatcgtc ttctccgatc tccttaaccg ctaaaccttc ttccaaatcc 60

cctctaccca tttccagatt ctcccttccc ttctccttaa ccccacagaa accctcctcc 120

cgtctccacc gtcctctcgc catctccgcc gttctcaact cacccgtcaa tgtcgcacct 180

gaaaaaaccg acaagatcaa gactttcatc tcccgctacg ctcccgacga gccccgcaag 240

ggtgctgata tcctcgtgga agccctcgag cgtcaaggcg tcgaaaccgt cttcgcttat 300

cccggaggtg cttccatgga gatccaccaa gccttgactc gctcctccac catccgtaac 360

gtcctccccc gtcacgaaca aggaggagtc ttcgccgccg agggttacgc tcgttcctcc 420

ggcaaaccgg gaatctgcat agccacttcg ggtcccggag ctaccaacct cgtcagcggg 480

ttagccgacg cgatgcttga cagtgttcct ctcgtcgcca tcacaggaca ggtccctcgc 540

cggatgatcg gtactgacgc gttccaagag acgccaatcg ttgaggtaac gaggtctatt 600

acgaaacata actatctggt gatggatgtt gatgacatac ctaggatcgt tcaagaagct 660

ttctttctag ctacttccgg tagacccgga ccggttttgg ttgatgttcc taaggatatt 720

cagcagcagc ttgcgattcc taactgggat caacctatgc gcttgcctgg ctacatgtct 780

aggctgcctc agccaccgga agtttctcag ttaggtcaga tcgttaggtt gatctcggag 840

tctaagaggc ctgttttgta cgttggtggt ggaagcttga actcgagtga agaactgggg 900

agatttgtcg agcttactgg gatccctgtt gcgagtacgt tgatggggct tggctcttat 960

ccttgtaacg atgacttgtc cctgcagatg cttggcatgc acgggactgt gtatgctaac 1020

tacgctgtgg agcatagtga tttgttgctg gcgtttggtg ttaggtttga tgaccgtgtc 1080

acgggaaagc tcgaggcgtt tgcgagcagg gctaagattg tgcacataga cattgattct 1140

gctgagattg ggaagaataa gacacctcac gtgtctgtgt gtggtgatgt aaagctggct 1200

ttgcaaggga tgaacaaggt tcttgagaac cgggcggagg agctcaagct tgatttcggt 1260

gtttggagga gtgagttgag cgagcagaaa cagaagttcc cgttgagctt caaaacgttt 1320

ggagaagcca ttcctccgca gtacgcgatt caggtcctag acgagctaac ccaagggaag 1380

gcaattatca gtactggtgt tggacagcat cagatgtggg cggcgcagtt ttacaagtac 1440

aggaagccga ggcagtggct gtcgtcctca ggactcggag ctatgggttt cggacttcct 1500

gctgcgattg gagcgtctgt ggcgaaccct gatgcgattg ttgtggacat tgacggtgat 1560

ggaagcttca taatgaacgt tcaagagctg gccacaatcc gtgtagagaa tcttcctgtg 1620

aagatactct tgttaaacaa ccagcatctt gggatggtca tgcaattgga agatcggttc 1680

tacaaagcta acagagctca cacttatctc ggggacccgg caaggagaga cgagatcttc 1740

cctaacatgc tgcagtttgc aggagcttgc gggattccag ctgcgagagt gacgaagaaa 1800

gaagaactcc gagaagctat tcagacaatg ctggatacac ctggaccgta cctgttggat 1860

gtcatctgtc cgcaccaaga acatgtgtta ccgatgatcc caagtggtgg cactttcaaa 1920

gatgtaataa ccgaagggga tggtcgcact aagtactga 1959

<210> 72
<211> 1959
<212> DNA
<213> Artificial Sequence

<220>
<223> Description of Artificial Sequence: Synthetic
      polynucleotide

<400> 72
atggcggcgg caacatcgtc ttctccgatc tccttaaccg ctaaaccttc ttccaaatcc 60

cctctaccca tttccagatt ctcccttccc ttctccttaa ccccacagaa accctcctcc 120

cgtctccacc gtcctctcgc catctccgcc gttctcaact cacccgtcaa tgtcgcacct 180

gaaaaaaccg acaagatcaa gactttcatc tcccgctacg ctcccgacga gccccgcaag 240

ggtgctgata tcctcgtgga agccctcgag cgtcaaggcg tcgaaaccgt cttcgcttat 300

cccggaggtg cttccatgga gatccaccaa gccttgactc gctcctccac catccgtaac 360

gtcctccccc gtcacgaaca aggaggagtc ttcgccgccg agggttacgc tcgttcctcc 420

ggcaaaccgg gaatctgcat agccacttcg ggtcccggag ctaccaacct cgtcagcggg 480

ttagccgacg cgatgcttga cagtgttcct ctcgtcgcca tcacaggaca ggtccctcgc 540

cggatgatcg gtactgacgc gttccaagag acgccaatcg ttgaggtaac gaggtctatt 600

acgaaacata actatctggt gatggatgtt gatgacatac ctaggatcgt tcaagaagct 660

ttctttctag ctacttccgg tagacccgga ccggttttgg ttgatgttcc taaggatatt 720

cagcagcagc ttgcgattcc taactgggat caacctatgc gcttgcctgg ctacatgtct 780

aggctgcctc agccaccgga agtttctcag ttaggtcaga tcgttaggtt gatctcggag 840

tctaagaggc ctgttttgta cgttggtggt ggaagcttga actcgagtga agaactgggg 900

agatttgtcg agcttactgg gatccctgtt gcgagtacgt tgatggggct tggctcttat 960

ccttgtaacg atgacttgtc cctgcagatg cttggcatgc acgggactgt gtatgctaac 1020

tacgctgtgg agcatagtga tttgttgctg gcgtttggtg ttaggtttga tgaccgtgtc 1080

acgggaaagc tcgaggcgtt tgcgagcagg gctaagattg tgcacataga cattgattct 1140

gctgagattg ggaagaataa gacacctcac gtgtctgtgt gtggtgatgt aaagctggct 1200

ttgcaaggga tgaacaaggt tcttgagaac cgggcggagg agctcaagct tgatttcggt 1260

gtttggagga gtgagttgag cgagcagaaa cagaagttcc cgttgagctt caaaacgttt 1320

ggagaagcca ttcctccgca gtacgcgatt caggtcctag acgagctaac ccaagggaag 1380

gcaattatca gtactggtgt tggacagcat cagatgtggg cggcgcagtt ttacaagtac 1440

aggaagccga ggcagtggct gtcgtcctca ggactcggag ctatgggttt cggacttcct 1500

gctgcgattg gagcgtctgt ggcgaaccct gatgcgattg ttgtggacat tgacggtgat 1560

ggaagcttca taatgaacgt tcaagagctg gccacaatcc gtgtagagaa tcttcctgtg 1620

aagatactct tgttaaacaa ccagcatctt gggatggtca tgcaaatgga agatcggttc 1680

tacaaagcta acagagctca cacttatctc ggggacccgg caagggagaa cgagatcttc 1740

cctaacatgc tgcagtttgc aggagcttgc gggattccag ctgcgagagt gacgaagaaa 1800

gaagaactcc gagaagctat tcagacaatg ctggatacac ctggaccgta cctgttggat 1860

gtcatctgtc cgcaccaaga acatgtgtta ccgatgatcc caagtggtgg cactttcaaa 1920

gatgtaataa ccgaagggga tggtcgcact aagtactga 1959

<210> 73
<211> 1959
<212> DNA
<213> Artificial Sequence

<220>
<223> Description of Artificial Sequence: Synthetic
      polynucleotide

<400> 73
atggcggcgg caacatcgtc ttctccgatc tccttaaccg ctaaaccttc ttccaaatcc 60

cctctaccca tttccagatt ctcccttccc ttctccttaa ccccacagaa accctcctcc 120

cgtctccacc gtcctctcgc catctccgcc gttctcaact cacccgtcaa tgtcgcacct 180

gaaaaaaccg acaagatcaa gactttcatc tcccgctacg ctcccgacga gccccgcaag 240

ggtgctgata tcctcgtgga agccctcgag cgtcaaggcg tcgaaaccgt cttcgcttat 300

cccggaggtg cttccatgga gatccaccaa gccttgactc gctcctccac catccgtaac 360

gtcctccccc gtcacgaaca aggaggagtc ttcgccgccg agggttacgc tcgttcctcc 420

ggcaaaccgg gaatctgcat agccacttcg ggtcccggag ctaccaacct cgtcagcggg 480

ttagccgacg cgatgcttga cagtgttcct ctcgtcgcca tcacaggaca ggtccctcgc 540

cggatgatcg gtactgacgc gttccaagag acgccaatcg ttgaggtaac gaggtctatt 600

acgaaacata actatctggt gatggatgtt gatgacatac ctaggatcgt tcaagaagct 660

ttctttctag ctacttccgg tagacccgga ccggttttgg ttgatgttcc taaggatatt 720

cagcagcagc ttgcgattcc taactgggat caacctatgc gcttgcctgg ctacatgtct 780

aggctgcctc agccaccgga agtttctcag ttaggtcaga tcgttaggtt gatctcggag 840

tctaagaggc ctgttttgta cgttggtggt ggaagcttga actcgagtga agaactgggg 900

agatttgtcg agcttactgg gatccctgtt gcgagtacgt tgatggggct tggctcttat 960

ccttgtaacg atgacttgtc cctgcagatg cttggcatgc acgggactgt gtatgctaac 1020

tacgctgtgg agcatagtga tttgttgctg gcgtttggtg ttaggtttga tgaccgtgtc 1080

acgggaaagc tcgaggcgtt tgcgagcagg gctaagattg tgcacataga cattgattct 1140

gctgagattg ggaagaataa gacacctcac gtgtctgtgt gtggtgatgt aaagctggct 1200

ttgcaaggga tgaacaaggt tcttgagaac cgggcggagg agctcaagct tgatttcggt 1260

gtttggagga gtgagttgag cgagcagaaa cagaagttcc cgttgagctt caaaacgttt 1320

ggagaagcca ttcctccgca gtacgcgatt caggtcctag acgagctaac ccaagggaag 1380

gcaattatca gtactggtgt tggacagcat cagatgtggg cggcgcagtt ttacaagtac 1440

aggaagccga ggcagtggct gtcgtcctca ggactcggag ctatgggttt cggacttcct 1500

gctgcgattg gagcgtctgt ggcgaaccct gatgcgattg ttgtggacat tgacggtgat 1560

ggaagcttca taatgaacgt tcaagagctg gccacaatcc gtgtagagaa tcttcctgtg 1620

aagatactct tgttaaacaa ccagcatctt gggatggtca tgcaatcgga agatcggttc 1680

tacaaagcta acagagctca cacttatctc ggggacccgg caagggagaa cgagatcttc 1740

cctaacatgc tgcagtttgc aggagcttgc gggattccag ctgcgagagt gacgaagaaa 1800

gaagaactcc gagaagctat tcagacaatg ctggatacac ctggaccgta cctgttggat 1860

gtcatctgtc cgcaccaaga acatgtgtta ccgatgatcc caagtggtgg cactttcaaa 1920

gatgtaataa ccgaagggga tggtcgcact aagtactga 1959

<210> 74
<211> 1968
<212> DNA
<213> Artificial Sequence

<220>
<223> Description of Artificial Sequence: Synthetic

polynucleotide

<400> 74
atggcggcgg caacatcgtc ttctccgatc tccttaaccg ctaaaccttc ttccaaatcc      60

cctctaccca tttccagatt ctcccttccc ttctccttaa ccccacagaa agactcctcc     120

cgtctccacc gtcctctcgc catctccgcc gttctcaact cacccgtcaa tgtcgcacct     180

ccttcccctg aaaaaaccga caagaacaag actttcgtct cccgctacgc tcccgacgag     240

ccccgcaagg gtgctgatat cctcgtcgaa gccctcgagc gtcaaggcgt cgaaaccgtc     300

tttgcttatc ccggaggtgc ttccatggag atccaccaag ccttgactcg ctcctccacc     360

atccgtaacg tccttccccg tcacgaacaa ggaggagtct tcgccgccga gggttacgct     420

cgttcctccg gcaaaccggg aatctgcata gccacttcgg gtcccggagc taccaacctc     480

gtcagcgggt tagcagacgc gatgcttgac agtgttcctc ttgtcgccat tacaggacag     540

gtccctcgcc ggatgatcgg tactgacgcc ttccaagaga caccaatcgt tgaggtaacg     600

aggtctatta cgaaacataa ctatttggtg atggatgttg atgacatacc taggatcgtt     660

caagaagctt tctttctagc tacttccggt agacccggac cggttttggt tgatgttcct     720

aaggatattc agcagcagct tgcgattcct aactgggatc aacctatgcg cttacctggc     780

tacatgtcta ggttgcctca gcctccggaa gtttctcagt taggtcagat cgttaggttg     840

atctcggagt ctaagaggcc tgttttgtac gttggtggtg gaagcttgaa ctcgagtgaa     900

gaactgggga gatttgtcga gcttactggg atccccgttg cgagtacttt gatggggctt     960

ggctcttatc cttgtaacga tgagttgtcc ctgcagatgc ttggcatgca cgggactgtg    1020

tatgctaact acgctgtgga gcatagtgat ttgttgctgg cgtttggtgt taggtttgat    1080

gaccgtgtca cgggaaagct cgaggctttc gctagcaggg ctaaaattgt gcacatagac    1140

attgattctg ctgagattgg gaagaataag acacctcacg tgtctgtgtg tggtgatgta    1200

aagctggctt tgcaagggat gaacaaggtt cttgagaacc gggcggagga gctcaagctt    1260

gatttcggtg tttggaggag tgagttgagc gagcagaaac agaagttccc tttgagcttc    1320

aaaacgtttg gagaagccat tcctccgcag tacgcgattc agatcctcga cgagctaacc    1380

gaagggaagg caattatcag tactggtgtt ggacagcatc agatgtgggc ggcgcagttt    1440

tacaagtaca ggaagccgag acagtggctg tcgtcatcag gcctcggagc tatgggtttt    1500

ggacttcctg ctgcgattgg agcgtctgtg gcgaaccctg atgcgattgt tgtggatatt    1560

gacggtgatg gaagcttcat aatgaacgtt caagagctgg ccacaatccg tgtagagaat    1620

cttcctgtga agatactctt gttaaacaac cagcatcttg ggatggtcat gcaatgggaa    1680

gatcggttct acaaagctaa cagagctcac acttatctcg gggacccggc aagggagaac    1740

gagatcttcc ctaacatgct gcagtttgca ggagcttgcg ggattccagc tgcgagagtg    1800

acgaagaaag aagaactccg agaagctatt cagacaatgc tggatacacc aggaccatac 1860

ctgttggatg tgatatgtcc gcaccaagaa catgtgttac cgatgatccc aaatggtggc 1920

actttcaaag atgtaataac agaaggggat ggtcgcacta agtactga 1968

<210> 75
<211> 1959
<212> DNA
<213> Artificial Sequence

<220>
<223> Description of Artificial Sequence: Synthetic
      polynucleotide

<400> 75
atggcggcgg caacatcgtc ttctccgatc tccttaaccg ctaaaccttc ttccaaatcc 60

cctctaccca tttccagatt ctcccttccc ttctccttaa ccccacagaa accctcctcc 120

cgtctccacc gtcctctcgc catctccgcc gttctcaact cacccgtcaa tgtcgcacct 180

gaaaaaaccg acaagatcaa gactttcatc tcccgctacg ctcccgacga gccccgcaag 240

ggtgctgata tcctcgtgga agccctcgag cgtcaaggcg tcgaaaccgt cttcgcttat 300

cccggaggtg cttccatgga gatccaccaa gccttgactc gctcctccac catccgtaac 360

gtcctccccc gtcacgaaca aggaggagtc ttcgccgccg agggttacgc tcgttcctcc 420

ggcaaaccgg gaatctgcat agccacttcg ggtcccggag ctaccaacct cgtcagcggg 480

ttagccgacg cgatgcttga cagtgttcct ctcgtcgcca tcacaggaca ggtccctcgc 540

cggatgatcg gtactgacgc gttccaagag acgccaatcg ttgaggtaac gaggtctatt 600

acgaaacata actatctggt gatggatgtt gatgacatac ctaggatcgt tcaagaagct 660

ttctttctag ctacttccgg tagacccgga ccggttttgg ttgatgttcc taaggatatt 720

cagcagcagc ttgcgattcc taactgggat caacctatgc gcttgcctgg ctacatgtct 780

aggctgcctc agccaccgga agtttctcag ttaggtcaga tcgttaggtt gatctcggag 840

tctaagaggc ctgttttgta cgttggtggt ggaagcttga actcgagtga agaactgggg 900

agatttgtcg agcttactgg gatccctgtt gcgagtacgt tgatggggct tggctcttat 960

ccttgtaacg atgacttgtc cctgcagatg cttggcatgc acgggactgt gtatgctaac 1020

tacgctgtgg agcatagtga tttgttgctg gcgtttggtg ttaggtttga tgaccgtgtc 1080

acgggaaagc tcgaggcgtt tgcgagcagg gctaagattg tgcacataga cattgattct 1140

gctgagattg ggaagaataa gacacctcac gtgtctgtgt gtggtgatgt aaagctggct 1200

ttgcaaggga tgaacaaggt tcttgagaac cgggcggagg agctcaagct tgatttcggt 1260

gtttggagga gtgagttgag cgagcagaaa cagaagttcc cgttgagctt caaaacgttt 1320

ggagaagcca ttcctccgca gtacgcgatt caggtcctag acgagctaac ccaagggaag 1380

gcaattatca gtactggtgt tggacagcat cagatgtggg cggcgcagtt ttacaagtac 1440

aggaagccga ggcagtggct gtcgtcctca ggactcggag ctatgggttt cggacttcct 1500

gctgcgattg gagcgtctgt ggcgaaccct gatgcgattg ttgtggacat tgacggtgat 1560

ggaagcttca taatgaacgt tcaagagctg gccacaatcc gtgtagagaa tcttcctgtg 1620

aagatactct tgttaaacaa ccagcatctt gggatggtca tgcaatggga agatcggttc 1680

tacaaagcta acagagctca cacttatctc ggggacccgg caagggagaa cgagatcttc 1740

cctaacatgc tgcagtttgc aggagcttgc gggattccag ctgcgagagt gacgaagaaa 1800

gaagaactcc gagaagctat tcagacaatg ctggatacac ctggaccgta cctgttggat 1860

gtcatctgtc cgcaccaaga acatgtgtta ccgatgatcc caaatggtgg cactttcaaa 1920

gatgtaataa ccgaagggga tggtcgcact aagtactga 1959

<210> 76
<211> 1959
<212> DNA
<213> Artificial Sequence

<220>
<223> Description of Artificial Sequence: Synthetic
      polynucleotide

<400> 76
atggcggcgg caacatcgtc ttctccgatc tccttaaccg ctaaaccttc ttccaaatcc 60

cctctaccca tttccagatt ctcccttccc ttctccttaa ccccacagaa accctcctcc 120

cgtctccacc gtcctctcgc catctccgcc gttctcaact cacccgtcaa tgtcgcacct 180

gaaaaaaccg acaagatcaa gactttcatc tcccgctacg ctcccgacga gccccgcaag 240

ggtgctgata tcctcgtgga agccctcgag cgtcaaggcg tcgaaaccgt cttcgcttat 300

cccggaggtg cttccatgga gatccaccaa gccttgactc gctcctccac catccgtaac 360

gtcctccccc gtcacgaaca aggaggagtc ttcgccgccg agggttacgc tcgttcctcc 420

ggcaaaccgg gaatctgcat agccacttcg ggtcccggag ctaccaacct cgtcagcggg 480

ttagccgacg cgatgcttga cagtgttcct ctcgtcgcca tcacaggaca ggtccctcgc 540

cggatgatcg gtactgacgc gttccaagag acgccaatcg ttgaggtaac gaggtctatt 600

acgaaacata actatctggt gatggatgtt gatgacatac ctaggatcgt tcaagaagct 660

ttctttctag ctacttccgg tagacccgga ccggttttgg ttgatgttcc taaggatatt 720

cagcagcagc ttgcgattcc taactgggat caacctatgc gcttgcctgg ctacatgtct 780

aggctgcctc agccaccgga agtttctcag ttaggtcaga tcgttaggtt gatctcggag 840

tctaagaggc ctgttttgta cgttggtggt ggaagcttga actcgagtga agaactgggg 900

agatttgtcg agcttactgg gatccctgtt gcgagtacgt tgatggggct tggctcttat 960

ccttgtaacg atgacttgtc cctgcagatg cttggcatgc acgggactgt gtatgctaac 1020

tacgctgtgg agcatagtga tttgttgctg gcgtttggtg ttaggtttga tgaccgtgtc 1080

acgggaaagc tcgaggcgtt tgcgagcagg gctaagattg tgcacataga cattgattct 1140

gctgagattg ggaagaataa gacacctcac gtgtctgtgt gtggtgatgt aaagctggct 1200

ttgcaaggga tgaacaaggt tcttgagaac cgggcggagg agctcaagct tgatttcggt 1260

gtttggagga gtgagttgag cgagcagaaa cagaagttcc cgttgagctt caaaacgttt 1320

ggagaagcca ttcctccgca gtacgcgatt caggtcctag acgagctaac ccaagggaag 1380

gcaattatca gtactggtgt tggacagcat cagatgtggg cggcgcagtt ttacaagtac 1440

aggaagccga ggcagtggct gtcgtcctca ggactcggag ctatgggttt cggacttcct 1500

gctgcgattg gagcgtctgt ggcgaaccct gatgcgattg ttgtggacat tgacggtgat 1560

ggaagcttca taatgaacgt tcaagagctg gccacaatcc gtgtagagaa tcttcctgtg 1620

aagatactct tgttaaacaa ccagcatctt gggatggtca tgcaatggga agatcggttc 1680

tacaaagcta acagagctca cacttatctc ggggacccgg caagggagaa cgagatcttc 1740

cctaacatgc tgcagtttgc aggagcttgc gggattccag ctgcgagagt gacgaagaaa 1800

gaagaactcc gagaagctat tcagacaatg ctggatacac ctggaccgta cctgttggat 1860

gtcatctgtc cgcaccaaga acatgtgtta ccgatgatcc caaatggtgg cactttcaaa 1920

gatgtaataa ccgaagggga tggtcgcact aagtactga 1959

<210> 77
<211> 1959
<212> DNA
<213> Artificial Sequence

<220>
<223> Description of Artificial Sequence: Synthetic
      polynucleotide

<400> 77
atggcggcgg caacatcgtc ttctccgatc tccttaaccg ctaaaccttc ttccaaatcc 60

cctctaccca tttccagatt ctcccttccc ttctccttaa ccccacagaa accctcctcc 120

cgtctccacc gtcctctcgc catctccgcc gttctcaact cacccgtcaa tgtcgcacct 180

gaaaaaaccg acaagatcaa gactttcatc tcccgctacg ctcccgacga gccccgcaag 240

ggtgctgata tcctcgtgga agccctcgag cgtcaaggcg tcgaaaccgt cttcgcttat 300

cccggaggtg cttccatgga gatccaccaa gccttgactc gctcctccac catccgtaac 360

gtcctccccc gtcacgaaca aggaggagtc ttcgccgccg agggttacgc tcgttcctcc 420

ggcaaaccgg gaatctgcat agccacttcg ggtcccggag ctaccaacct cgtcagcggg 480

ttagccgacg cgatgcttga cagtgttcct ctcgtcgcca tcacaggaca ggtccctcgc 540

cggatgatcg gtactgacgc gttccaagag acgccaatcg ttgaggtaac gaggtctatt 600

acgaaacata actatctggt gatggatgtt gatgacatac ctaggatcgt tcaagaagct 660

ttctttctag ctacttccgg tagacccgga ccggttttgg ttgatgttcc taaggatatt 720

cagcagcagc ttgcgattcc taactgggat caacctatgc gcttgcctgg ctacatgtct 780

aggctgcctc agccaccgga agtttctcag ttaggtcaga tcgttaggtt gatctcggag 840

tctaagaggc ctgttttgta cgttggtggt ggaagcttga actcgagtga agaactgggg 900

agatttgtcg agcttactgg gatccctgtt gcgagtacgt tgatggggct tggctcttat 960

ccttgtaacg atgacttgtc cctgcagatg cttggcatgc acgggactgt gtatgctaac 1020

tacgctgtgg agcatagtga tttgttgctg gcgtttggtg ttaggtttga tgaccgtgtc 1080

acgggaaagc tcgaggcgtt tgcgagcagg gctaagattg tgcacataga cattgattct 1140

gctgagattg ggaagaataa gacacctcac gtgtctgtgt gtggtgatgt aaagctggct 1200

ttgcaaggga tgaacaaggt tcttgagaac cgggcggagg agctcaagct tgatttcggt 1260

gtttggagga gtgagttgag cgagcagaaa cagaagttcc cgttgagctt caaaacgttt 1320

ggagaagcca ttcctccgca gtacgcgatt caggtcctag acgagctaac ccaagggaag 1380

gcaattatca gtactggtgt tggacagcat cagatgtggg cggcgcagtt ttacaagtac 1440

aggaagccga ggcagtggct gtcgtcctca ggactcggag ctatgggttt cggacttcct 1500

gctgcgattg gagcgtctgt ggcgaaccct gatgcgattg ttgtggacat tgacggtgat 1560

ggaagcttca taatgaacgt tcaagagctg gccacaatcc gtgtagagaa tcttcctgtg 1620

aagatactct tgttaaacaa ccagcatctt gggatggtca tgcaatggga agatcggttc 1680

tacaaagcta acagagctca cacttatctc ggggacccgg caagggagaa cgagatcttc 1740

cctaacatgc tgcagtttgc aggagcttgc gggattccag ctgcgagagt gacgaagaaa 1800

gaagaactcc gagaagctat tcagacaatg ctggatacac ctggaccgta cctgttggat 1860

gtcatctgtc cgcaccaaga acatgtgtta ccgatgatcc caaatggtgg cactttcaaa 1920

gatgtaataa ccgaagggga tggtcgcact aagtactga 1959

<210> 78
<211> 1959
<212> DNA
<213> Artificial Sequence

<220>
<223> Description of Artificial Sequence: Synthetic
      polynucleotide

<400> 78
atggcggcgg caacatcgtc ttctccgatc tccttaaccg ctaaaccttc ttccaaatcc 60

cctctaccca tttccagatt ctcccttccc ttctccttaa ccccacagaa accctcctcc 120

cgtctccacc gtcctctcgc catctccgcc gttctcaact cacccgtcaa tgtcgcacct 180

gaaaaaaccg acaagatcaa gactttcatc tcccgctacg ctcccgacga gccccgcaag 240

ggtgctgata tcctcgtgga agccctcgag cgtcaaggcg tcgaaaccgt cttcgcttat 300

cccggaggtg cttccatgga gatccaccaa gccttgactc gctcctccac catccgtaac 360

gtcctccccc gtcacgaaca aggaggagtc ttcgccgccg agggttacgc tcgttcctcc 420

ggcaaaccgg gaatctgcat agccacttcg ggtcccggag ctaccaacct cgtcagcggg 480

ttagccgacg cgatgcttga cagtgttcct ctcgtcgcca tcacaggaca ggtccctcgc 540

cggatgatcg gtactgacgc gttccaagag acgccaatcg ttgaggtaac gaggtctatt 600

acgaaacata actatctggt gatggatgtt gatgacatac ctaggatcgt tcaagaagct 660

ttctttctag ctacttccgg tagacccgga ccggttttgg ttgatgttcc taaggatatt 720

cagcagcagc ttgcgattcc taactgggat caacctatgc gcttgcctgg ctacatgtct 780

aggctgcctc agccaccgga agtttctcag ttaggtcaga tcgttaggtt gatctcggag 840

tctaagaggc ctgttttgta cgttggtggt ggaagcttga actcgagtga agaactgggg 900

agatttgtcg agcttactgg gatccctgtt gcgagtacgt tgatggggct tggctcttat 960

ccttgtaacg atgacttgtc cctgcagatg cttggcatgc acgggactgt gtatgctaac 1020

tacgctgtgg agcatagtga tttgttgctg gcgtttggtg ttaggtttga tgaccgtgtc 1080

acgggaaagc tcgaggcgtt tgcgagcagg gctaagattg tgcacataga cattgattct 1140

gctgagattg ggaagaataa gacacctcac gtgtctgtgt gtggtgatgt aaagctggct 1200

ttgcaaggga tgaacaaggt tcttgagaac cgggcggagg agctcaagct tgatttcggt 1260

gtttggagga gtgagttgag cgagcagaaa cagaagttcc cgttgagctt caaaacgttt 1320

ggagaagcca ttcctccgca gtacgcgatt caggtcctag acgagctaac ccaagggaag 1380

gcaattatca gtactggtgt tggacagcat cagatgtggg cggcgcagtt ttacaagtac 1440

aggaagccga ggcagtggct gtcgtcctca ggactcggag ctatgggttt cggacttcct 1500

gctgcgattg gagcgtctgt ggcgaaccct gatgcgattg ttgtggacat tgacggtgat 1560

ggaagcttca taatgaacgt tcaagagctg gccacaatcc gtgtagagaa tcttcctgtg 1620

aagatactct tgttaaacaa ccagcatctt gggatggtca tgcaatggga agatcggttc 1680

tacaaagcta acagagctca cacttatctc ggggacccgg caagggagaa cgagatcttc 1740

cctaacatgc tgcagtttgc aggagcttgc gggattccag ctgcgagagt gacgaagaaa 1800

gaagaactcc gagaagctat tcagacaatg ctggatacac ctggaccgta cctgttggat 1860

gtcatctgtc cgcaccaaga acatgtgtta ccgatgatcc caaatggtgg cactttcaaa 1920

gatgtaataa ccgaagggga tggtcgcact aagtactga 1959

<210> 79
<211> 1959
<212> DNA
<213> Artificial Sequence

<220>
<223> Description of Artificial Sequence: Synthetic
      polynucleotide

<400> 79
atggcggcgg caacatcgtc ttctccgatc tccttaaccg ctaaaccttc ttccaaatcc      60

cctctaccca tttccagatt ctcccttccc ttctccttaa ccccacagaa accctcctcc     120

cgtctccacc gtcctctcgc catctccgcc gttctcaact cacccgtcaa tgtcgcacct     180

gaaaaaaccg acaagatcaa gactttcatc tcccgctacg ctcccgacga gccccgcaag     240

ggtgctgata tcctcgtgga agccctcgag cgtcaaggcg tcgaaaccgt cttcgcttat     300

cccggaggtg cttccatgga gatccaccaa gccttgactc gctcctccac catccgtaac     360

gtcctccccc gtcacgaaca aggaggagtc ttcgccgccg agggttacgc tcgttcctcc     420

ggcaaaccgg gaatctgcat agccacttcg ggtcccggag ctaccaacct cgtcagcggg     480

ttagccgacg cgatgcttga cagtgttcct ctcgtcgcca tcacaggaca ggtccctcgc     540

cggatgatcg gtactgacgc gttccaagag acgccaatcg ttgaggtaac gaggtctatt     600

acgaaacata actatctggt gatggatgtt gatgacatac ctaggatcgt tcaagaagct     660

ttctttctag ctacttccgg tagacccgga ccggttttgg ttgatgttcc taaggatatt     720

cagcagcagc ttgcgattcc taactgggat caacctatgc gcttgcctgg ctacatgtct     780

aggctgcctc agccaccgga agtttctcag ttaggtcaga tcgttaggtt gatctcggag     840

tctaagaggc ctgttttgta cgttggtggt ggaagcttga actcgagtga agaactgggg     900

agatttgtcg agcttactgg gatccctgtt gcgagtacgt tgatggggct tggctcttat     960

ccttgtaacg atgacttgtc cctgcagatg cttggcatgc acgggactgt gtatgctaac    1020

tacgctgtgg agcatagtga tttgttgctg gcgtttggtg ttaggtttga tgaccgtgtc    1080

acgggaaagc tcgaggcgtt tgcgagcagg gctaagattg tgcacataga cattgattct    1140

gctgagattg ggaagaataa gacacctcac gtgtctgtgt gtggtgatgt aaagctggct    1200

ttgcaaggga tgaacaaggt tcttgagaac cgggcggagg agctcaagct tgatttcggt    1260

gtttggagga gtgagttgag cgagcagaaa cagaagttcc cgttgagctt caaaacgttt    1320

ggagaagcca ttcctccgca gtacgcgatt caggtcctag acgagctaac ccaaggaaag    1380

gcaattatca gtactggtgt tggacagcat cagatgtggg cggcgcagtt ttacaagtac    1440

aggaagccga ggcagtggct gtcgtcctca ggactcggag ctatgggttt cggacttcct    1500

gctgcgattg gagcgtctgt ggcgaaccct gatgcgattg ttgtggacat tgacggtgat    1560

ggaagcttca taatgaacgt tcaagagctg gccacaatcc gtgtagagaa tcttcctgtg    1620

aagatactct tgttaaacaa ccagcatctt gggatggtca tgcaatgcga agatcggttc 1680

tacaaagcta acagagctca cacttatctc ggggacccgg caagggagaa cgagatcttc 1740

cctaacatgc tgcagtttgc aggagcttgc gggattccag ctgcgagagt gacgaagaaa 1800

gaagaactcc gagaagctat tcagacaatg ctggatacac ctggaccgta cctgttggat 1860

gtcatctgtc cgcaccaaga acatgtgtta ccgatgatcc caagtggtgg cactttcaaa 1920

gatgtaataa ccgaagggga tggtcgcact aagtactga 1959

<210> 80
<211> 1968
<212> DNA
<213> Artificial Sequence

<220>
<223> Description of Artificial Sequence: Synthetic
      polynucleotide

<400> 80
atggcggcgg caacatcgtc ttctccgatc tccttaaccg ctaaaccttc ttccaaatcc 60

cctctaccca tttccagatt ctcccttccc ttctccttaa ccccacagaa agactcctcc 120

cgtctccacc gtcctctcgc catctccgcc gttctcaact cacccgtcaa tgtcgcacct 180

ccttcccctg aaaaaaccga caagaacaag actttcgtct cccgctacgc tcccgacgag 240

ccccgcaagg gtgctgatat cctcgtcgaa gccctcgagc gtcaaggcgt cgaaaccgtc 300

tttgcttatc ccggaggtgc ttccatggag atccaccaag ccttgactcg ctcctccacc 360

atccgtaacg tccttccccg tcacgaacaa ggaggagtct tcgccgccga gggttacgct 420

cgttcctccg gcaaaccggg aatctgcata gccacttcgg gtcccggagc taccaacctc 480

gtcagcgggt tagcagacgc gatgcttgac agtgttcctc ttgtcgccat tacaggacag 540

gtccctcgcc ggatgatcgg tactgacgcc ttccaagaga caccaatcgt tgaggtaacg 600

aggtctatta cgaaacataa ctatttggtg atggatgttg atgacatacc taggatcgtt 660

caagaagctt tctttctagc tacttccggt agacccggac cggttttggt tgatgttcct 720

aaggatattc agcagcagct tgcgattcct aactgggatc aacctatgcg cttacctggc 780

tacatgtcta ggttgcctca gcctccggaa gtttctcagt taggtcagat cgttaggttg 840

atctcggagt ctaagaggcc tgttttgtac gttggtggtg gaagcttgaa ctcgagtgaa 900

gaactgggga gatttgtcga gcttactggg atccccgttg cgagtacttt gatggggctt 960

ggctcttatc cttgtaacga tgagttgtcc ctgcagatgc ttggcatgca cgggactgtg 1020

tatgctaact acgctgtgga gcatagtgat ttgttgctgg cgtttggtgt taggtttgat 1080

gaccgtgtca cgggaaagct cgaggctttc gctagcaggg ctaaaattgt gcacatagac 1140

attgattctg ctgagattgg gaagaataag acacctcacg tgtctgtgtg tggtgatgta 1200

aagctggctt tgcaagggat gaacaaggtt cttgagaacc gggcggagga gctcaagctt 1260

gatttcggtg tttggaggag tgagttgagc gagcagaaac agaagttccc tttgagcttc 1320

aaaacgtttg gagaagccat tcctccgcag tacgcgattc agatcctcga cgagctaacc 1380

gaagggaagg caattatcag tactggtgtt ggacagcatc agatgtgggc ggcgcagttt 1440

tacaagtaca ggaagccgag acagtggctg tcgtcatcag gcctcggagc tatgggtttt 1500

ggacttcctg ctgcgattgg agcgtctgtg gcgaaccctg atgcgattgt tgtggatatt 1560

gacggtgatg gaagcttcat aatgaacgtt caagagctgg ccacaatccg tgtagagaat 1620

cttcctgtga agatactctt gttaaacaac cagcatcttg ggatggtcat gcaatgggaa 1680

gatcggttct acaaagctaa cagagctcac acttatctcg gggacccggc aagggagaac 1740

gagatcttcc ctaacatgct gcagtttgca ggagcttgcg ggattccagc tgcgagagtg 1800

acgaagaaag aagaactccg agaagctatt cagacaatgc tggatacacc aggaccatac 1860

ctgttggatg tgatatgtcc gcaccaagaa catgtgttac cgatgatccc aactggtggc 1920

actttcaaag atgtaataac agaaggggat ggtcgcacta agtactga 1968

<210> 81
<211> 1959
<212> DNA
<213> Artificial Sequence

<220>
<223> Description of Artificial Sequence: Synthetic
      polynucleotide

<400> 81
atggcggcgg caacatcgtc ttctccgatc tccttaaccg ctaaaccttc ttccaaatcc 60

cctctaccca tttccagatt ctcccttccc ttctccttaa ccccacagaa accctcctcc 120

cgtctccacc gtcctctcgc catctccgcc gttctcaact cacccgtcaa tgtcgcacct 180

gaaaaaaccg acaagatcaa gactttcatc tcccgctacg ctcccgacga gccccgcaag 240

ggtgctgata tcctcgtgga agccctcgag cgtcaaggcg tcgaaaccgt cttcgcttat 300

cccggaggtg cttccatgga gatccaccaa gccttgactc gctcctccac catccgtaac 360

gtcctccccc gtcacgaaca aggaggagtc ttcgccgccg agggttacgc tcgttcctcc 420

ggcaaaccgg gaatctgcat agccacttcg ggtcccggag ctaccaacct cgtcagcggg 480

ttagccgacg cgatgcttga cagtgttcct ctcgtcgcca tcacaggaca ggtccctcgc 540

cggatgatcg gtactgacgc gttccaagag acgccaatcg ttgaggtaac gaggtctatt 600

acgaaacata actatctggt gatggatgtt gatgacatac ctaggatcgt tcaagaagct 660

ttctttctag ctacttccgg tagacccgga ccggttttgg ttgatgttcc taaggatatt 720

cagcagcagc ttgcgattcc taactgggat caacctatgc gcttgcctgg ctacatgtct 780

aggctgcctc agccaccgga agtttctcag ttaggtcaga tcgttaggtt gatctcggag 840

tctaagaggc ctgttttgta cgttggtggt ggaagcttga actcgagtga agaactgggg 900

agatttgtcg agcttactgg gatccctgtt gcgagtacgt tgatggggct tggctcttat 960

ccttgtaacg atgacttgtc cctgcagatg cttggcatgc acgggactgt gtatgctaac 1020

tacgctgtgg agcatagtga tttgttgctg gcgtttggtg ttaggtttga tgaccgtgtc 1080

acgggaaagc tcgaggcgtt tgcgagcagg gctaagattg tgcacataga cattgattct 1140

gctgagattg ggaagaataa gacacctcac gtgtctgtgt gtggtgatgt aaagctggct 1200

ttgcaaggga tgaacaaggt tcttgagaac cgggcggagg agctcaagct tgatttcggt 1260

gtttggagga gtgagttgag cgagcagaaa cagaagttcc cgttgagctt caaaacgttt 1320

ggagaagcca ttcctccgca gtacgcgatt caggtcctag acgagctaac ccaagggaag 1380

gcaattatca gtactggtgt tggacagcat cagatgtggg cggcgcagtt ttacaagtac 1440

aggaagccga ggcagtggct gtcgtcctca ggactcggag ctatgggttt cggacttcct 1500

gctgcgattg gagcgtctgt ggcgaaccct gatgcgattg ttgtggacat tgacggtgat 1560

ggaagcttca taatgaacgt tcaagagctg gccacaatcc gtgtagagaa tcttcctgtg 1620

aagatactct tgttaaacaa ccagcatctt gggatggtca tgcaatggga agatcggttc 1680

tacaaagcta acagagctca cacttatctc ggggacccgg caagggagaa cgagatcttc 1740

cctaacatgc tgcagtttgc aggagcttgc gggattccag ctgcgagagt gacgaagaaa 1800

gaagaactcc gagaagctat tcagacaatg ctggatacac ctggaccgta cctgttggat 1860

gtcatctgtc cgcaccaaga acatgtgtta ccgatgatcc caactggtgg cactttcaaa 1920

gatgtaataa ccgaagggga tggtcgcact aagtactga 1959

<210> 82
<211> 1968
<212> DNA
<213> Artificial Sequence

<220>
<223> Description of Artificial Sequence: Synthetic
      polynucleotide

<400> 82
atggcggcgg caacatcgtc ttctccgatc tccttaaccg ctaaaccttc ttccaaatcc 60

cctctaccca tttccagatt ctcccttccc ttctccttaa ccccacagaa agactcctcc 120

cgtctccacc gtcctctcgc catctccgcc gttctcaact cacccgtcaa tgtcgcacct 180

ccttcccctg aaaaaaccga caagaacaag actttcgtct cccgctacgc tcccgacgag 240

ccccgcaagg gtgctgatat cctcgtcgaa gccctcgagc gtcaaggcgt cgaaaccgtc 300

tttgcttatc ccggaggtgc ttccatggag atccaccaag ccttgactcg ctcctccacc 360

atccgtaacg tccttccccg tcacgaacaa ggaggagtct tcgccgccga gggttacgct 420

cgttcctccg gcaaaccggg aatctgcata gccacttcgg gtcccggagc taccaacctc 480

gtcagcgggt tagcagacgc gatgcttgac agtgttcctc ttgtcgccat tacaggacag 540

gtccctcgcc ggatgatcgg tactgacgcc ttccaagaga caccaatcgt tgaggtaacg 600

aggtctatta cgaaacataa ctatttggtg atggatgttg atgacatacc taggatcgtt 660

caagaagctt tctttctagc tacttccggt agacccggac cggttttggt tgatgttcct 720

aaggatattc agcagcagct tgcgattcct aactgggatc aacctatgcg cttacctggc 780

tacatgtcta ggttgcctca gcctccggaa gtttctcagt taggtcagat cgttaggttg 840

atctcggagt ctaagaggcc tgttttgtac gttggtggtg gaagcttgaa ctcgagtgaa 900

gaactgggga gatttgtcga gcttactggg atccccgttg cgagtacttt gatggggctt 960

ggctcttatc cttgtaacga tgagttgtcc ctgcagatgc ttggcatgca cgggactgtg 1020

tatgctaact acgctgtgga gcatagtgat ttgttgctgg cgtttggtgt taggtttgat 1080

gaccgtgtca cgggaaagct cgaggctttc gctagcaggg ctaaaattgt gcacatagac 1140

attgattctg ctgagattgg gaagaataag acacctcacg tgtctgtgtg tggtgatgta 1200

aagctggctt tgcaagggat gaacaaggtt cttgagaacc gggcggagga gctcaagctt 1260

gatttcggtg tttggaggag tgagttgagc gagcagaaac agaagttccc tttgagcttc 1320

aaaacgtttg gagaagccat tcctccgcag tacgcgattc agatcctcga cgagctaacc 1380

gaagggaagg caattatcag tactggtgtt ggacagcatc agatgtgggc ggcgcagttt 1440

tacaagtaca ggaagccgag acagtggctg tcgtcatcag gcctcggagc tatgggtttt 1500

ggacttcctg ctgcgattgg agcgtctgtg gcgaaccctg atgcgattgt tgtggatatt 1560

gacggtgatg gaagcttcat aatgaacgtt caagagctgg ccacaatccg tgtagagaat 1620

cttcctgtga agatactctt gttaaacaac cagcatcttg ggatggtcat gcaatgggaa 1680

gatcggttct acaaagctaa cagagctcac acttatctcg gggacccggc aagggagaac 1740

gagatcttcc ctaacatgct gcagtttgca ggagcttgcg ggattccagc tgcgagagtg 1800

acgaagaaag aagaactccg agaagctatt cagacaatgc tggatacacc aggaccatac 1860

ctgttggatg tgatatgtcc gcaccaagaa catgtgttac cgatgatccc aactggtggc 1920

actttcaaag atgtaataac agaaggggat ggtcgcacta agtactga 1968

<210> 83
<211> 1968
<212> DNA
<213> Artificial Sequence

<220>
<223> Description of Artificial Sequence: Synthetic

polynucleotide

<400> 83
atggcggcgg caacatcgtc ttctccgatc tccttaaccg ctaaaccttc ttccaaatcc 60

cctctaccca tttccagatt ctcccttccc ttctccttaa ccccacagaa agactcctcc 120

cgtctccacc gtcctctcgc catctccgcc gttctcaact cacccgtcaa tgtcgcacct 180

ccttcccctg aaaaaaccga caagaacaag actttcgtct cccgctacgc tcccgacgag 240

ccccgcaagg gtgctgatat cctcgtcgaa gccctcgagc gtcaaggcgt cgaaaccgtc 300

tttgcttatc ccggaggtgc ttccatggag atccaccaag ccttgactcg ctcctccacc 360

atccgtaacg tccttccccg tcacgaacaa ggaggagtct tcgccgccga gggttacgct 420

cgttcctccg gcaaaccggg aatctgcata gccacttcgg gtcccggagc taccaacctc 480

gtcagcgggt tagcagacgc gatgcttgac agtgttcctc ttgtcgccat tacaggacag 540

gtccctcgcc ggatgatcgg tactgacgcc ttccaagaga caccaatcgt tgaggtaacg 600

aggtctatta cgaaacataa ctatttggtg atggatgttg atgacatacc taggatcgtt 660

caagaagctt tctttctagc tacttccggt agacccggac cggttttggt tgatgttcct 720

aaggatattc agcagcagct tgcgattcct aactgggatc aacctatgcg cttacctggc 780

tacatgtcta ggttgcctca gcctccggaa gtttctcagt taggtcagat cgttaggttg 840

atctcggagt ctaagaggcc tgttttgtac gttggtggtg gaagcttgaa ctcgagtgaa 900

gaactgggga gatttgtcga gcttactggg atccccgttg cgagtacttt gatggggctt 960

ggctcttatc cttgtaacga tgagttgtcc ctgcagatgc ttggcatgca cgggactgtg 1020

tatgctaact acgctgtgga gcatagtgat ttgttgctgg cgtttggtgt taggtttgat 1080

gaccgtgtca cgggaaagct cgaggctttc gctagcaggg ctaaaattgt gcacatagac 1140

attgattctg ctgagattgg gaagaataag acacctcacg tgtctgtgtg tggtgatgta 1200

aagctggctt tgcaagggat gaacaaggtt cttgagaacc gggcggagga gctcaagctt 1260

gatttcggtg tttggaggag tgagttgagc gagcagaaac agaagttccc tttgagcttc 1320

aaaacgtttg gagaagccat tcctccgcag tacgcgattc agatcctcga cgagctaacc 1380

gaagggaagg caattatcag tactggtgtt ggacagcatc agatgtgggc ggcgcagttt 1440

tacaagtaca ggaagccgag acagtggctg tcgtcatcag gcctcggagc tatgggtttt 1500

ggacttcctg ctgcgattgg agcgtctgtg gcgaaccctg atgcgattgt tgtggatatt 1560

gacggtgatg gaagcttcat aatgaacgtt caagagctgg ccacaatccg tgtagagaat 1620

cttcctgtga agatactctt gttaaacaac cagcatcttg ggatggtcat gcaatgggaa 1680

gatcggttct acaaagctaa cagagctcac acttatctcg gggacccggc aagggagaac 1740

gagatcttcc ctaacatgct gcagtttgca ggagcttgcg ggattccagc tgcgagagtg 1800

acgaagaaag aagaactccg agaagctatt cagacaatgc tggatacacc aggaccatac 1860

ctgttggatg tgatatgtcc gcaccaagaa catgtgttac cgatgatccc aaatggtggc 1920

actttcaaag atgtaataac agaaggggat ggtcgcacta agtactga 1968

<210> 84
<211> 1968
<212> DNA
<213> Artificial Sequence

<220>
<223> Description of Artificial Sequence: Synthetic
      polynucleotide

<400> 84
atggcggcgg caacatcgtc ttctccgatc tccttaaccg ctaaaccttc ttccaaatcc 60

cctctaccca tttccagatt ctcccttccc ttctccttaa ccccacagaa agactcctcc 120

cgtctccacc gtcctctcgc catctccgcc gttctcaact cacccgtcaa tgtcgcacct 180

ccttcccctg aaaaaaccga caagaacaag actttcgtct cccgctacgc tcccgacgag 240

ccccgcaagg gtgctgatat cctcgtcgaa gccctcgagc gtcaaggcgt cgaaaccgtc 300

tttgcttatc ccggaggtgc ttccatggag atccaccaag ccttgactcg ctcctccacc 360

atccgtaacg tccttccccg tcacgaacaa ggaggagtct tcgccgccga gggttacgct 420

cgttcctccg gcaaaccggg aatctgcata gccacttcgg gtcccggagc taccaacctc 480

gtcagcgggt tagcagacgc gatgcttgac agtgttcctc ttgtcgccat tacaggacag 540

gtccctcgcc ggatgatcgg tactgacgcc ttccaagaga caccaatcgt tgaggtaacg 600

aggtctatta cgaaacataa ctatttggtg atggatgttg atgacatacc taggatcgtt 660

caagaagctt tctttctagc tacttccggt agacccggac cggttttggt tgatgttcct 720

aaggatattc agcagcagct tgcgattcct aactgggatc aacctatgcg cttacctggc 780

tacatgtcta ggttgcctca gcctccggaa gtttctcagt taggtcagat cgttaggttg 840

atctcggagt ctaagaggcc tgttttgtac gttggtggtg gaagcttgaa ctcgagtgaa 900

gaactgggga gatttgtcga gcttactggg atccccgttg cgagtacttt gatggggctt 960

ggctcttatc cttgtaacga tgagttgtcc ctgcagatgc ttggcatgca cgggactgtg 1020

tatgctaact acgctgtgga gcatagtgat ttgttgctgg cgtttggtgt taggtttgat 1080

gaccgtgtca cgggaaagct cgaggctttc gctagcaggg ctaaaattgt gcacatagac 1140

attgattctg ctgagattgg gaagaataag acacctcacg tgtctgtgtg tggtgatgta 1200

aagctggctt tgcaagggat gaacaaggtt cttgagaacc gggcggagga gctcaagctt 1260

gatttcggtg tttggaggag tgagttgagc gagcagaaac agaagttccc tttgagcttc 1320

aaaacgtttg gagaagccat tcctccgcag tacgcgattc agatcctcga cgagctaacc 1380

gaagggaagg caattatcag tactggtgtt ggacagcatc agatgtgggc ggcgcagttt 1440

tacaagtaca ggaagccgag acagtggctg tcgtcatcag gcctcggagc tatgggtttt 1500

ggacttcctg ctgcgattgg agcgtctgtg gcgaaccctg atgcgattgt tgtggatatt 1560

gacggtgatg gaagcttcat aatgaacgtt caagagctgg ccacaatccg tgtagagaat 1620

cttcctgtga agatactctt gttaaacaac cagcatcttg ggatggtcat gcaatgggaa 1680

gatcggttct acaaagctaa cagagctcac acttatctcg gggacccggc aagggagaac 1740

gagatcttcc ctaacatgct gcagtttgca ggagcttgcg ggattccagc tgcgagagtg 1800

acgaagaaag aagaactccg agaagctatt cagacaatgc tggatacacc aggaccatac 1860

ctgttggatg tgatatgtcc gcaccaagaa catgtgttac cgatgatccc aaatggtggc 1920

actttcaaag atgtaataac agaaggggat ggtcgcacta agtactga 1968

<210> 85
<211> 1959
<212> DNA
<213> Artificial Sequence

<220>
<223> Description of Artificial Sequence: Synthetic
      polynucleotide

<400> 85
atggcggcgg caacatcgtc ttctccgatc tccttaaccg ctaaaccttc ttccaaatcc 60

cctctaccca tttccagatt ctcccttccc ttctccttaa ccccacagaa accctcctcc 120

cgtctccacc gtcctctcgc catctccgcc gttctcaact cacccgtcaa tgtcgcacct 180

gaaaaaaccg acaagatcaa gactttcatc tcccgctacg ctcccgacga gccccgcaag 240

ggtgctgata tcctcgtgga agccctcgag cgtcaaggcg tcgaaaccgt cttcgcttat 300

cccggaggtg cttccatgga gatccaccaa gccttgactc gctcctccac catccgtaac 360

gtcctccccc gtcacgaaca aggaggagtc ttcgccgccg agggttacgc tcgttcctcc 420

ggcaaaccgg gaatctgcat agccacttcg ggtcccggag ctaccaacct cgtcagcggg 480

ttagccgacg cgatgcttga cagtgttcct ctcgtcgcca tcacaggaca ggtccctcgc 540

cggatgatcg gtactgacgc gttccaagag acgccaatcg ttgaggtaac gaggtctatt 600

acgaaacata actatctggt gatggatgtt gatgacatac ctaggatcgt tcaagaagct 660

ttctttctag ctacttccgg tagacccgga ccggttttgg ttgatgttcc taaggatatt 720

cagcagcagc ttgcgattcc taactgggat caacctatgc gcttgcctgg ctacatgtct 780

aggctgcctc agccaccgga agtttctcag ttaggtcaga tcgttaggtt gatctcggag 840

tctaagaggc ctgttttgta cgttggtggt ggaagcttga actcgagtga agaactgggg 900

agatttgtcg agcttactgg gatccctgtt gcgagtacgt tgatggggct tggctcttat 960

ccttgtaacg atgacttgtc cctgcagatg cttggcatgc acgggactgt gtatgctaac 1020

tacgctgtgg agcatagtga tttgttgctg gcgtttggtg ttaggtttga tgaccgtgtc 1080

acgggaaagc tcgaggcgtt tgcgagcagg gctaagattg tgcacataga cattgattct 1140

gctgagattg ggaagaataa gacacctcac gtgtctgtgt gtggtgatgt aaagctggct 1200

ttgcaaggga tgaacaaggt tcttgagaac cgggcggagg agctcaagct tgatttcggt 1260

gtttggagga gtgagttgag cgagcagaaa cagaagttcc cgttgagctt caaaacgttt 1320

ggagaagcca ttcctccgca gtacgcgatt caggtcctag acgagctaac ccaagggaag 1380

gcaattatca gtactggtgt tggacagcat cagatgtggg cggcgcagtt ttacaagtac 1440

aggaagccga ggcagtggct gtcgtcctca ggactcggag ctatgggttt cggacttcct 1500

gctgcgattg gagcgtctgt ggcgaaccct gatgcgattg ttgtggacat tgacggtgat 1560

ggaagcttca taatgaacgt tcaagagctg gccacaatcc gtgtagagaa tcttcctgtg 1620

aagatactct tgttaaacaa ccagcatctt gggatggtca tgcaatgcga agatcggttc 1680

tacaaagcta acagagctca cacttatctc ggggacccgg caagggagaa cgagatcttc 1740

cctaacatgc tgcagtttgc aggagcttgc gggattccag ctgcgagagt gacgaagaaa 1800

gaagaactcc gagaagctat tcagacaatg ctggatacac ctggaccgta cctgttggat 1860

gtcatctgtc cgcaccaaga acatgtgtta ccgatgatcc caaatggtgg cactttcaaa 1920

gatgtaataa ccgaagggga tggtcgcact aagtactga 1959

<210> 86
<211> 1959
<212> DNA
<213> Artificial Sequence

<220>
<223> Description of Artificial Sequence: Synthetic
      polynucleotide

<400> 86
atggcggcgg caacatcgtc ttctccgatc tccttaaccg ctaaaccttc ttccaaatcc 60

cctctaccca tttccagatt ctcccttccc ttctccttaa ccccacagaa accctcctcc 120

cgtctccacc gtcctctcgc catctccgcc gttctcaact cacccgtcaa tgtcgcacct 180

gaaaaaaccg acaagatcaa gactttcatc tcccgctacg ctcccgacga gccccgcaag 240

ggtgctgata tcctcgtgga agccctcgag cgtcaaggcg tcgaaaccgt cttcgcttat 300

cccggaggtg cttccatgga gatccaccaa gccttgactc gctcctccac catccgtaac 360

gtcctccccc gtcacgaaca aggaggagtc ttcgccgccg agggttacgc tcgttcctcc 420

ggcaaaccgg gaatctgcat agccacttcg ggtcccggag ctaccaacct cgtcagcggg 480

ttagccgacg cgatgcttga cagtgttcct ctcgtcgcca tcacaggaca ggtccctcgc 540

cggatgatcg gtactgacgt gttccaagag acgccaatcg ttgaggtaac gaggtctatt 600

acgaaacata actatctggt gatggatgtt gatgacatac ctaggatcgt tcaagaagct 660

ttctttctag ctacttccgg tagacccgga ccggttttgg ttgatgttcc taaggatatt 720

cagcagcagc ttgcgattcc taactgggat caacctatgc gcttgcctgg ctacatgtct 780

aggctgcctc agccaccgga agtttctcag ttaggtcaga tcgttaggtt gatctcggag 840

tctaagaggc ctgttttgta cgttggtggt ggaagcttga actcgagtga agaactgggg 900

agatttgtcg agcttactgg gatccctgtt gcgagtacgt tgatggggct tggctcttat 960

ccttgtaacg atgacttgtc cctgcagatg cttggcatgc acgggactgt gtatgctaac 1020

tacgctgtgg agcatagtga tttgttgctg gcgtttggtg ttaggtttga tgaccgtgtc 1080

acgggaaagc tcgaggcgtt tgcgagcagg gctaagattg tgcacataga cattgattct 1140

gctgagattg ggaagaataa gacacctcac gtgtctgtgt gtggtgatgt aaagctggct 1200

ttgcaaggga tgaacaaggt tcttgagaac cgggcggagg agctcaagct tgatttcggt 1260

gtttggagga gtgagttgag cgagcagaaa cagaagttcc cgttgagctt caaaacgttt 1320

ggagaagcca ttcctccgca gtacgcgatt caggtcctag acgagctaac ccaagggaag 1380

gcaattatca gtactggtgt tggacagcat cagatgtggg cggcgcagtt ttacaagtac 1440

aggaagccga ggcagtggct gtcgtcctca ggactcggag ctatgggttt cggacttcct 1500

gctgcgattg gagcgtctgt ggcgaaccct gatgcgattg ttgtggacat tgacggtgat 1560

ggaagcttca taatgaacgt tcaagagctg gccacaatcc gtgtagagaa tcttcctgtg 1620

aagatactct tgttaaacaa ccagcatctt gggatggtca tgcaatggga agatcggttc 1680

tacaaagcta acagagctca cacttatctc ggggacccgg caagggagaa cgagatcttc 1740

cctaacatgc tgcagtttgc aggagcttgc gggattccag ctgcgagagt gacgaagaaa 1800

gaagaactcc gagaagctat tcagacaatg ctggatacac ctggaccgta cctgttggat 1860

gtcatctgtc cgcaccaaga acatgtgtta ccgatgatcc caaatggtgg cactttcaaa 1920

gatgtaataa ccgaagggga tggtcgcact aagtactga 1959

<210> 87
<211> 1959
<212> DNA
<213> Artificial Sequence

<220>
<223> Description of Artificial Sequence: Synthetic
      polynucleotide

<400> 87
   atggcggcgg caacatcgtc ttctccgatc tccttaaccg ctaaaccttc ttccaaatcc 60
   cctctaccca tttccagatt ctcccttccc ttctccttaa ccccacagaa accctcctcc 120
   cgtctccacc gtcctctcgc catctccgcc gttctcaact cacccgtcaa tgtcgcacct 180
   gaaaaaaccg acaagatcaa gactttcatc tcccgctacg ctcccgacga gccccgcaag 240
   ggtgctgata tcctcgtgga agccctcgag cgtcaaggcg tcgaaaccgt cttcgcttat 300
   cccggaggtg cttccatgga gatccaccaa gccttgactc gctcctccac catccgtaac 360
   gtcctccccc gtcacgaaca aggaggagtc ttcgccgccg agggttacgc tcgttcctcc 420
   ggcaaaccgg gaatctgcat agccacttcg ggtcccggag ctaccaacct cgtcagcggg 480
   ttagccgacg cgatgcttga cagtgttcct ctcgtcgcca tcacaggaca ggtccctcgc 540
   cggatgatcg gtactgacgt gttccaagag acgccaatcg ttgaggtaac gaggtctatt 600
   acgaaacata actatctggt gatggatgtt gatgacatac ctaggatcgt tcaagaagct 660
   ttctttctag ctacttccgg tagacccgga ccggttttgg ttgatgttcc taaggatatt 720
   cagcagcagc ttgcgattcc taactgggat caacctatgc gcttgcctgg ctacatgtct 780
   aggctgcctc agccaccgga agtttctcag ttaggtcaga tcgttaggtt gatctcggag 840
   tctaagaggc ctgttttgta cgttggtggt ggaagcttga actcgagtga agaactgggg 900
   agatttgtcg agcttactgg gatccctgtt gcgagtacgt tgatggggct tggctcttat 960
   ccttgtaacg atgacttgtc cctgcagatg cttggcatgc acgggactgt gtatgctaac 1020
   tacgctgtgg agcatagtga tttgttgctg gcgtttggtg ttaggtttga tgaccgtgtc 1080
   acgggaaagc tcgaggcgtt tgcgagcagg gctaagattg tgcacataga cattgattct 1140
   gctgagattg ggaagaataa gacacctcac gtgtctgtgt gtggtgatgt aaagctggct 1200
   ttgcaaggga tgaacaaggt tcttgagaac cgggcggagg agctcaagct tgatttcggt 1260
   gtttggagga gtgagttgag cgagcagaaa cagaagttcc cgttgagctt caaaacgttt 1320
   ggagaagcca ttcctccgca gtacgcgatt caggtcctag acgagctaac ccaagggaag 1380
   gcaattatca gtactggtgt tggacagcat cagatgtggg cggcgcagtt ttacaagtac 1440
   aggaagccga ggcagtggct gtcgtcctca ggactcggag ctatgggttt cggacttcct 1500
   gctgcgattg gagcgtctgt ggcgaaccct gatgcgattg ttgtggacat tgacggtgat 1560
   ggaagcttca taatgaacgt tcaagagctg gccacaatcc gtgtagagaa tcttcctgtg 1620
   aagatactct tgttaaacaa ccagcatctt gggatggtca tgcaatggga agatcggttc 1680
   tacaaagcta acagagctca cacttatctc ggggacccgg caagggagaa cgagatcttc 1740
   cctaacatgc tgcagtttgc aggagcttgc gggattccag ctgcgagagt gacgaagaaa 1800
   gaagaactcc gagaagctat tcagacaatg ctggatacac ctggaccgta cctgttggat 1860
   gtcatctgtc cgcaccaaga acatgtgtta ccgatgatcc caaatggtgg cactttcaaa 1920
   gatgtaataa ccgaagggga tggtcgcact aagtactga 1959

<210> 88
<211> 1968
<212> DNA
<213> Artificial Sequence

<220>
<223> Description of Artificial Sequence: Synthetic
      polynucleotide

<400> 88
atggcggcgg caacatcgtc ttctccgatc tccttaaccg ctaaaccttc ttccaaatcc      60

cctctaccca tttccagatt ctcccttccc ttctccttaa ccccacagaa agactcctcc     120

cgtctccacc gtcctctcgc catctccgcc gttctcaact cacccgtcaa tgtcgcacct     180

ccttcccctg aaaaaaccga caagaacaag actttcgtct cccgctacgc tcccgacgag     240

ccccgcaagg gtgctgatat cctcgtcgaa gccctcgagc gtcaaggcgt cgaaaccgtc     300

tttgcttatc ccggaggtgc ttccatggag atccaccaag ccttgactcg ctcctccacc     360

atccgtaacg tccttccccg tcacgaacaa ggaggagtct tcgccgccga gggttacgct     420

cgttcctccg gcaaaccggg aatctgcata gccacttcgg gtcccggagc taccaacctc     480

gtcagcgggt tagcagacgc gatgcttgac agtgttcctc ttgtcgccat tacaggacag     540

gtccctcgcc ggatgatcgg tactgacgtc ttccaagaga caccaatcgt tgaggtaacg     600

aggtctatta cgaaacataa ctatttggtg atggatgttg atgacatacc taggatcgtt     660

caagaagctt tctttctagc tacttccggt agacccggac cggttttggt tgatgttcct     720

aaggatattc agcagcagct tgcgattcct aactgggatc aacctatgcg cttacctggc     780

tacatgtcta ggttgcctca gcctccggaa gtttctcagt taggtcagat cgttaggttg     840

atctcggagt ctaagaggcc tgttttgtac gttggtggtg gaagcttgaa ctcgagtgaa     900

gaactgggga gatttgtcga gcttactggg atccccgttg cgagtacttt gatggggctt     960

ggctcttatc cttgtaacga tgagttgtcc ctgcagatgc ttggcatgca cgggactgtg    1020

tatgctaact acgctgtgga gcatagtgat ttgttgctgg cgtttggtgt taggtttgat    1080

gaccgtgtca cgggaaagct cgaggctttc gctagcaggg ctaaaattgt gcacatagac    1140

attgattctg ctgagattgg gaagaataag acacctcacg tgtctgtgtg tggtgatgta    1200

aagctggctt tgcaagggat gaacaaggtt cttgagaacc gggcggagga gctcaagctt    1260

gatttcggtg tttggaggag tgagttgagc gagcagaaac agaagttccc tttgagcttc    1320

aaaacgtttg gagaagccat tcctccgcag tacgcgattc agatcctcga cgagctaacc    1380

gaagggaagg caattatcag tactggtgtt ggacagcatc agatgtgggc ggcgcagttt    1440

tacaagtaca ggaagccgag acagtggctg tcgtcatcag gcctcggagc tatgggtttt    1500

ggacttcctg ctgcgattgg agcgtctgtg gcgaaccctg atgcgattgt tgtggatatt    1560

gacggtgatg gaagcttcat aatgaacgtt caagagctgg ccacaatccg tgtagagaat    1620

cttcctgtga agatactctt gttaaacaac cagcatcttg ggatggtcat gcaatgggaa 1680

gatcggttct acaaagctaa cagagctcac acttatctcg gggacccggc aagggagaac 1740

gagatcttcc ctaacatgct gcagtttgca ggagcttgcg ggattccagc tgcgagagtg 1800

acgaagaaag aagaactccg agaagctatt cagacaatgc tggatacacc aggaccatac 1860

ctgttggatg tgatatgtcc gcaccaagaa catgtgttac cgatgatccc aagtggtggc 1920

actttcaaag atgtaataac agaaggggat ggtcgcacta agtactga 1968

<210> 89
<211> 1959
<212> DNA
<213> Artificial Sequence

<220>
<223> Description of Artificial Sequence: Synthetic
      polynucleotide

<400> 89
atggcggcgg caacatcgtc ttctccgatc tccttaaccg ctaaaccttc ttccaaatcc 60

cctctaccca tttccagatt ctcccttccc ttctccttaa ccccacagaa accctcctcc 120

cgtctccacc gtcctctcgc catctccgcc gttctcaact cacccgtcaa tgtcgcacct 180

gaaaaaaccg acaagatcaa gactttcatc tcccgctacg ctcccgacga gccccgcaag 240

ggtgctgata tcctcgtgga agccctcgag cgtcaaggcg tcgaaaccgt cttcgcttat 300

cccggaggtg cttccatgga gatccaccaa gccttgactc gctcctccac catccgtaac 360

gtcctccccc gtcacgaaca aggaggagtc ttcgccgccg agggttacgc tcgttcctcc 420

ggcaaaccgg gaatctgcat agccacttcg ggtcccggag ctaccaacct cgtcagcggg 480

ttagccgacg cgatgcttga cagtgttcct ctcgtcgcca tcacaggaca ggtccctcgc 540

cggatgatcg gtactgacgc gttccaagag acgccaatcg ttgaggtaac gaggtctatt 600

acgaaacata actatctggt gatggatgtt gatgacatac ctaggatcgt tcaagaagct 660

ttctttctag ctacttccgg tagacccgga ccggttttgg ttgatgttcc taaggatatt 720

cagcagcagc ttgcgattcc taactgggat caacctatgc gcttgcctgg ctacatgtct 780

aggctgcctc agccaccgga agtttctcag ttaggtcaga tcgttaggtt gatctcggag 840

tctaagaggc ctgttttgta cgttggtggt ggaagcttga actcgagtga agaactgggg 900

agatttgtcg agcttactgg gatccctgtt gcgagtacgt tgatggggct tggctcttat 960

ccttgtaacg atgacttgtc cctgcagatg cttggcatgc acgggactgt gtatgctaac 1020

tacgctgtgg agcatagtga tttgttgctg gcgtttggtg ttaggtttga tgaccgtgtc 1080

acgggaaagc tcgaggcgtt tgcgagcagg gctaagattg tgcacataga cattgattct 1140

gctgagattg ggaagaataa gacacctcac gtgtctgtgt gtggtgatgt aaagctggct 1200

ttgcaaggga tgaacaaggt tcttgagaac cgggcggagg agctcaagct tgatttcggt 1260

gtttggagga gtgagttgag cgagcagaaa cagaagttcc cgttgagctt caaaacgttt 1320

ggagaagcca ttcctccgca gtacgcgatt caggtcctag acgagctaac ccaagggaag 1380

gcaattatca gtactggtgt tggacagcat cagatgtggg cggcgcagtt ttacaagtac 1440

aggaagccga ggcagtggct gtcgtcctca ggactcggag ctatgggttt cggacttcct 1500

gctgcgattg gagcgtctgt ggcgaaccct gatgcgattg ttgtggacat tgacggtgat 1560

ggaagcttca taatgaacgt tcaagagctg gccacaatcc gtgtagagaa tcttcctgtg 1620

aagatactct tgttaaacaa ccagcatctt gggatggtca tgcaatggga agatcggttc 1680

tacaaagcta acagagctca cacttatctc ggggacccgg caaggagaga cgagatcttc 1740

cctaacatgc tgcagtttgc aggagcttgc gggattccag ctgcgagagt gacgaagaaa 1800

gaagaactcc gagaagctat tcagacaatg ctggatacac ctggaccgta cctgttggat 1860

gtcatctgtc cgcaccaaga acatgtgtta ccgatgatcc caaatggtgg cactttcaaa 1920

gatgtaataa ccgaagggga tggtcgcact aagtactga 1959

<210> 90
<211> 1968
<212> DNA
<213> Artificial Sequence

<220>
<223> Description of Artificial Sequence: Synthetic
      polynucleotide

<400> 90
atggcggcgg caacatcgtc ttctccgatc tccttaaccg ctaaaccttc ttccaaatcc 60

cctctaccca tttccagatt ctcccttccc ttctccttaa ccccacagaa agactcctcc 120

cgtctccacc gtcctctcgc catctccgcc gttctcaact cacccgtcaa tgtcgcacct 180

ccttcccctg aaaaaaccga caagaacaag actttcgtct cccgctacgc tcccgacgag 240

ccccgcaagg gtgctgatat cctcgtcgaa gccctcgagc gtcaaggcgt cgaaaccgtc 300

tttgcttatc ccggaggtgc ttccatggag atccaccaag ccttgactcg ctcctccacc 360

atccgtaacg tccttccccg tcacgaacaa ggaggagtct tcgccgccga gggttacgct 420

cgttcctccg gcaaaccggg aatctgcata gccacttcgg gtcccggagc taccaacctc 480

gtcagcgggt tagcagacgc gatgcttgac agtgttcctc ttgtcgccat tacaggacag 540

gtccctcgcc ggatgatcgg tactgacgcc ttccaagaga caccaatcgt tgaggtaacg 600

aggtctatta cgaaacataa ctatttggtg atggatgttg atgacatacc taggatcgtt 660

caagaagctt tctttctagc tacttccggt agacccggac cggttttggt tgatgttcct 720

aaggatattc agcagcagct tgcgattcct aactgggatc aacctatgcg cttacctggc 780

tacatgtcta ggttgcctca gcctccggaa gtttctcagt taggtcagat cgttaggttg 840

atctcggagt ctaagaggcc tgttttgtac gttggtggtg gaagcttgaa ctcgagtgaa 900

gaactgggga gatttgtcga gcttactggg atccccgttg cgagtacttt gatggggctt 960

ggctcttatc cttgtaacga tgagttgtcc ctgcagatgc ttggcatgca cgggactgtg 1020

tatgctaact acgctgtgga gcatagtgat ttgttgctgg cgtttggtgt taggtttgat 1080

gaccgtgtca cgggaaagct cgaggctttc gctagcaggg ctaaaattgt gcacatagac 1140

attgattctg ctgagattgg gaagaataag acacctcacg tgtctgtgtg tggtgatgta 1200

aagctggctt tgcaagggat gaacaaggtt cttgagaacc gggcggagga gctcaagctt 1260

gatttcggtg tttggaggag tgagttgagc gagcagaaac agaagttccc tttgagcttc 1320

aaaacgtttg gagaagccat tcctccgcag tacgcgattc agatcctcga cgagctaacc 1380

gaagggaagg caattatcag tactggtgtt ggacagcatc agatgtgggc ggcgcagttt 1440

tacaagtaca ggaagccgag acagtggctg tcgtcatcag gcctcggagc tatgggtttt 1500

ggacttcctg ctgcgattgg agcgtctgtg gcgaaccctg atgcgattgt tgtggatatt 1560

gacggtgatg gaagcttcat aatgaacgtt caagagctgg ccacaatccg tgtagagaat 1620

cttcctgtga agatactctt gttaaacaac cagcatcttg ggatggtcat gcaatgcgaa 1680

gatcggttct acaaagctaa cagagctcac acttatctcg gggacccggc aagggagaac 1740

gagatcttcc ctaacatgct gcagtttgca ggagcttgcg ggattccagc tgcgagagtg 1800

acgaagaaag aagaactccg agaagctatt cagacaatgc tggatacacc aggaccatac 1860

ctgttggatg tgatatgtcc gcaccaagaa catgtgttac cgatgatccc aagtggtggc 1920

actttcaaag atgtaataac agaaggggat ggtcgcacta agtactga 1968

<210> 91
<211> 1959
<212> DNA
<213> Artificial Sequence

<220>
<223> Description of Artificial Sequence: Synthetic
      polynucleotide

<400> 91
atggcggcgg caacatcgtc ttctccgatc tccttaaccg ctaaaccttc ttccaaatcc 60

cctctaccca tttccagatt ctcccttccc ttctccttaa ccccacagaa accctcctcc 120

cgtctccacc gtcctctcgc catctccgcc gttctcaact cacccgtcaa tgtcgcacct 180

gaaaaaaccg acaagatcaa gactttcatc tcccgctacg ctcccgacga gccccgcaag 240

ggtgctgata tcctcgtgga agccctcgag cgtcaaggcg tcgaaaccgt cttcgcttat 300

cccggaggtg cttccatgga gatccaccaa gccttgactc gctcctccac catccgtaac 360

gtcctccccc gtcacgaaca aggaggagtc ttcgccgccg agggttacgc tcgttcctcc 420

ggcaaaccgg gaatctgcat agccacttcg ggtcccggag ctaccaacct cgtcagcggg 480

ttagccgacg cgatgcttga cagtgttcct ctcgtcgcca tcacaggaca ggtccctcgc 540

cggatgatcg gtactgacgc gttccaagag acgccaatcg ttgaggtaac gaggtctatt 600

acgaaacata actatctggt gatggatgtt gatgacatac ctaggatcgt tcaagaagct 660

ttctttctag ctacttccgg tagacccgga ccggttttgg ttgatgttcc taaggatatt 720

cagcagcagc ttgcgattcc taactgggat caacctatgc gcttgcctgg ctacatgtct 780

aggctgcctc agccaccgga agtttctcag ttaggtcaga tcgttaggtt gatctcggag 840

tctaagaggc ctgttttgta cgttggtggt ggaagcttga actcgagtga agaactgggg 900

agatttgtcg agcttactgg gatccctgtt gcgagtacgt tgatggggct tggctcttat 960

ccttgtaacg atgacttgtc cctgcagatg cttggcatgc acgggactgt gtatgctaac 1020

tacgctgtgg agcatagtga tttgttgctg gcgtttggtg ttaggtttga tgaccgtgtc 1080

acgggaaagc tcgaggcgtt tgcgagcagg gctaagattg tgcacataga cattgattct 1140

gctgagattg ggaagaataa gacacctcac gtgtctgtgt gtggtgatgt aaagctggct 1200

ttgcaaggga tgaacaaggt tcttgagaac cgggcggagg agctcaagct tgatttcggt 1260

gtttggagga gtgagttgag cgagcagaaa cagaagttcc cgttgagctt caaaacgttt 1320

ggagaagcca ttcctccgca gtacgcgatt caggtcctag acgagctaac ccaagggaag 1380

gcaattatca gtactggtgt tggacagcat cagatgtggg cggcgcagtt ttacaagtac 1440

aggaagccga ggcagtggct gtcgtcctca ggactcggag ctatgggttt cggacttcct 1500

gctgcgattg gagcgtctgt ggcgaaccct gatgcgattg ttgtggacat tgacggtgat 1560

ggaagcttca taatgaacgt tcaagagctg gccacaatcc gtgtagagaa tcttcctgtg 1620

aagatactct tgttaaacaa ccagcatctt gggatggtca tgcaatggga agatcggttc 1680

tacaaagcta acagagctca cacttatctc ggggacccgg caagggagaa cgagatcttc 1740

cctaacatgc tgcagtttgc aggagcttgc gggattccag ctgcgagagt gacgaagaaa 1800

gaagaactcc gagaagctat tcagacaatg ctggatacac ctggaccgta cctgttggat 1860

gtcatctgtc cgcaccaaga acatgtgtta ccgatgatcc caaatggtgg cactttcaaa 1920

gatgtaataa ccgaagggga tggtcgcact aagtactga 1959

<210> 92
<211> 1968
<212> DNA
<213> Artificial Sequence

<220>
<223> Description of Artificial Sequence: Synthetic

polynucleotide

<400> 92
atggcggcgg caacatcgtc ttctccgatc tccttaaccg ctaaaccttc ttccaaatcc 60

cctctaccca tttccagatt ctcccttccc ttctccttaa ccccacagaa agactcctcc 120

cgtctccacc gtcctctcgc catctccgcc gttctcaact cacccgtcaa tgtcgcacct 180

ccttcccctg aaaaaaccga caagaacaag actttcgtct cccgctacgc tcccgacgag 240

ccccgcaagg gtgctgatat cctcgtcgaa gccctcgagc gtcaaggcgt cgaaaccgtc 300

tttgcttatc ccggaggtgc ttccatggag atccaccaag ccttgactcg ctcctccacc 360

atccgtaacg tccttccccg tcacgaacaa ggaggagtct tcgccgccga gggttacgct 420

cgttcctccg gcaaaccggg aatctgcata gccacttcgg gtcccggagc taccaacctc 480

gtcagcgggt tagcagacgc gatgcttgac agtgttcctc ttgtcgccat tacaggacag 540

gtccctcgcc ggatgatcgg tactgacgcc ttccaagaga caccaatcgt tgaggtaacg 600

aggtctatta cgaaacataa ctatttggtg atggatgttg atgacatacc taggatcgtt 660

caagaagctt tctttctagc tacttccggt agacccggac cggttttggt tgatgttcct 720

aaggatattc agcagcagct tgcgattcct aactgggatc aacctatgcg cttacctggc 780

tacatgtcta ggttgcctca gcctccggaa gtttctcagt taggtcagat cgttaggttg 840

atctcggagt ctaagaggcc tgttttgtac gttggtggtg gaagcttgaa ctcgagtgaa 900

gaactgggga gatttgtcga gcttactggg atccccgttg cgagtacttt gatggggctt 960

ggctcttatc cttgtaacga tgagttgtcc ctgcagatgc ttggcatgca cgggactgtg 1020

tatgctaact acgctgtgga gcatagtgat ttgttgctgg cgtttggtgt taggtttgat 1080

gaccgtgtca cgggaaagct cgaggctttc gctagcaggg ctaaaattgt gcacatagac 1140

attgattctg ctgagattgg gaagaataag acacctcacg tgtctgtgtg tggtgatgta 1200

aagctggctt tgcaagggat gaacaaggtt cttgagaacc gggcggagga gctcaagctt 1260

gatttcggtg tttggaggag tgagttgagc gagcagaaac agaagttccc tttgagcttc 1320

aaaacgtttg gagaagccat tcctccgcag tacgcgattc agatcctcga cgagctaacc 1380

gaagggaagg caattatcag tactggtgtt ggacagcatc agatgtgggc ggcgcagttt 1440

tacaagtaca ggaagccgag acagtggctg tcgtcatcag gcctcggagc tatgggtttt 1500

ggacttcctg ctgcgattgg agcgtctgtg gcgaaccctg atgcgattgt tgtggatatt 1560

gacggtgatg gaagcttcat aatgaacgtt caagagctgg ccacaatccg tgtagagaat 1620

cttcctgtga agatactctt gttaaacaac cagcatcttg ggatggtcat gcaattggaa 1680

gatcggttct acaaagctaa cagagctcac acttatctcg gggacccggc aagggagaac 1740

gagatcttcc ctaacatgct gcagtttgca ggagcttgcg ggattccagc tgcgagagtg 1800

acgaagaaag aagaactccg agaagctatt cagacaatgc tggatacacc aggaccatac 1860

ctgttggatg tgatatgtcc gcaccaagaa catgtgttac cgatgatccc aagtggtggc 1920

actttcaaag atgtaataac agaaggggat ggtcgcacta agtactga 1968

<210> 93
<211> 1959
<212> DNA
<213> Artificial Sequence

<220>
<223> Description of Artificial Sequence: Synthetic
      polynucleotide

<400> 93
atggcggcgg caacatcgtc ttctccgatc tccttaaccg ctaaaccttc ttccaaatcc 60

cctctaccca tttccagatt ctcccttccc ttctccttaa ccccacagaa accctcctcc 120

cgtctccacc gtcctctcgc catctccgcc gttctcaact cacccgtcaa tgtcgcacct 180

gaaaaaaccg acaagatcaa gactttcatc tcccgctacg ctcccgacga gccccgcaag 240

ggtgctgata tcctcgtgga agccctcgag cgtcaaggcg tcgaaaccgt cttcgcttat 300

cccggaggtg cttccatgga gatccaccaa gccttgactc gctcctccac catccgtaac 360

gtcctccccc gtcacgaaca aggaggagtc ttcgccgccg agggttacgc tcgttcctcc 420

ggcaaaccgg gaatctgcat agccacttcg ggtcccggag ctaccaacct cgtcagcggg 480

ttagccgacg cgatgcttga cagtgttcct ctcgtcgcca tcacaggaca ggtccctcgc 540

cggatgatcg gtactgacgc gttccaagag acgccaatcg ttgaggtaac gaggtctatt 600

acgaaacata actatctggt gatggatgtt gatgacatac ctaggatcgt tcaagaagct 660

ttctttctag ctacttccgg tagacccgga ccggttttgg ttgatgttcc taaggatatt 720

cagcagcagc ttgcgattcc taactgggat caacctatgc gcttgcctgg ctacatgtct 780

aggctgcctc agccaccgga agtttctcag ttaggtcaga tcgttaggtt gatctcggag 840

tctaagaggc ctgttttgta cgttggtggt ggaagcttga actcgagtga agaactgggg 900

agatttgtcg agcttactgg gatccctgtt gcgagtacgt tgatggggct tggctcttat 960

ccttgtaacg atgacttgtc cctgcagatg cttggcatgc acgggactgt gtatgctaac 1020

tacgctgtgg agcatagtga tttgttgctg gcgtttggtg ttaggtttga tgaccgtgtc 1080

acgggaaagc tcgaggcgtt tgcgagcagg gctaagattg tgcacataga cattgattct 1140

gctgagattg ggaagaataa gacacctcac gtgtctgtgt gtggtgatgt aaagctggct 1200

ttgcaaggga tgaacaaggt tcttgagaac cgggcggagg agctcaagct tgatttcggt 1260

gtttggagga gtgagttgag cgagcagaaa cagaagttcc cgttgagctt caaaacgttt 1320

ggagaagcca ttcctccgca gtacgcgatt caggtcctag acgagctaac ccaagggaag 1380

gcaattatca gtactggtgt tggacagcat cagatgtggg cggcgcagtt ttacaagtac 1440

aggaagccga ggcagtggct gtcgtcctca ggactcggag ctatgggttt cggacttcct 1500

gctgcgattg gagcgtctgt ggcgaaccct gatgcgattg ttgtggacat tgacggtgat 1560

ggaagcttca taatgaacgt tcaagagctg gccacaatcc gtgtagagaa tcttcctgtg 1620

aagatactct tgttaaacaa ccagcatctt gggatggtca tgcaatggga agatcggttc 1680

tacaaagcta acagagctca cacttatctc ggggacccgg caagggagaa cgagatcttc 1740

cctaacatgc tgcagtttgc aggagcttgc gggattccag ctgcgagagt gacgaagaaa 1800

gaagaactcc gagaagctat tcagacaatg ctggatacac ctggaccgta cctgttggat 1860

gtcatctgtc cgcaccaaga acatgtgtta ccgatgatcc caaatggtgg cactttcaaa 1920

gatgtaataa ccgaagggga tggtcgcact aagtactga 1959

<210> 94
<211> 1959
<212> DNA
<213> Artificial Sequence

<220>
<223> Description of Artificial Sequence: Synthetic
      polynucleotide

<400> 94
atggcggcgg caacatcgtc ttctccgatc tccttaaccg ctaaaccttc ttccaaatcc 60

cctctaccca tttccagatt ctcccttccc ttctccttaa ccccacagaa accctcctcc 120

cgtctccacc gtcctctcgc catctccgcc gttctcaact cacccgtcaa tgtcgcacct 180

gaaaaaaccg acaagatcaa gactttcatc tcccgctacg ctcccgacga gccccgcaag 240

ggtgctgata tcctcgtgga agccctcgag cgtcaaggcg tcgaaaccgt cttcgcttat 300

cccggaggtg cttccatgga gatccaccaa gccttgactc gctcctccac catccgtaac 360

gtcctccccc gtcacgaaca aggaggagtc ttcgccgccg agggttacgc tcgttcctcc 420

ggcaaaccgg gaatctgcat agccacttcg ggtcccggag ctaccaacct cgtcagcggg 480

ttagccgacg cgatgcttga cagtgttcct ctcgtcgcca tcacaggaca ggtccctcgc 540

cggatgatcg gtactgacgc gttccaagag acgccaatcg ttgaggtaac gaggtctatt 600

acgaaacata actatctggt gatggatgtt gatgacatac ctaggatcgt tcaagaagct 660

ttctttctag ctacttccgg tagacccgga ccggttttgg ttgatgttcc taaggatatt 720

cagcagcagc ttgcgattcc taactgggat caacctatgc gcttgcctgg ctacatgtct 780

aggctgcctc agccaccgga agtttctcag ttaggtcaga tcgttaggtt gatctcggag 840

tctaagaggc ctgttttgta cgttggtggt ggaagcttga actcgagtga agaactgggg 900

agatttgtcg agcttactgg gatccctgtt gcgagtacgt tgatggggct tggctcttat 960

ccttgtaacg atgacttgtc cctgcagatg cttggcatgc acgggactgt gtatgctaac 1020

tacgctgtgg agcatagtga tttgttgctg gcgtttggtg ttaggtttga tgaccgtgtc 1080

acgggaaagc tcgaggcgtt tgcgagcagg gctaagattg tgcacataga cattgattct 1140

gctgagattg ggaagaataa gacacctcac gtgtctgtgt gtggtgatgt aaagctggct 1200

ttgcaaggga tgaacaaggt tcttgagaac cgggcggagg agctcaagct tgatttcggt 1260

gtttggagga gtgagttgag cgagcagaaa cagaagttcc cgttgagctt caaaacgttt 1320

ggagaagcca ttcctccgca gtacgcgatt caggtcctag acgagctaac ccaagggaag 1380

gcaattatca gtactggtgt tggacagcat cagatgtggg cggcgcagtt ttacaagtac 1440

aggaagccga ggcagtggct gtcgtcctca ggactcggag ctatgggttt cggacttcct 1500

gctgcgattg gagcgtctgt ggcgaaccct gatgcgattg ttgtggacat tgacggtgat 1560

ggaagcttca taatgaacgt tcaagagctg gccacaatcc gtgtagagaa tcttcctgtg 1620

aagatactct tgttaaacaa ccagcatctt gggatggtca tgcaattgga agatcggttc 1680

tacaaagcta acagagctca cacttatctc ggggacccgg caagggagaa cgagatcttc 1740

cctaacatgc tgcagtttgc aggagcttgc gggattccag ctgcgagagt gacgaagaaa 1800

gaagaactcc gagaagctat tcagacaatg ctggatacac ctggaccgta cctgttggat 1860

gtcatctgtc cgcaccaaga acatgtgtta ccgatgatcc caaatggtgg cactttcaaa 1920

gatgtaataa ccgaagggga tggtcgcact aagtactga 1959

<210> 95
<211> 1959
<212> DNA
<213> Artificial Sequence

<220>
<223> Description of Artificial Sequence: Synthetic
      polynucleotide

<400> 95
atggcggcgg caacatcgtc ttctccgatc tccttaaccg ctaaaccttc ttccaaatcc 60

cctctaccca tttccagatt ctcccttccc ttctccttaa ccccacagaa accctcctcc 120

cgtctccacc gtcctctcgc catctccgcc gttctcaact cacccgtcaa tgtcgcacct 180

gaaaaaaccg acaagatcaa gactttcatc tcccgctacg ctcccgacga gccccgcaag 240

ggtgctgata tcctcgtgga agccctcgag cgtcaaggcg tcgaaaccgt cttcgcttat 300

cccggaggtg cttccatgga gatccaccaa gccttgactc gctcctccac catccgtaac 360

gtcctccccc gtcacgaaca aggaggagtc ttcgccgccg agggttacgc tcgttcctcc 420

ggcaaaccgg gaatctgcat agccacttcg ggtcccggag ctaccaacct cgtcagcggg 480

ttagccgacg cgatgcttga cagtgttcct ctcgtcgcca tcacaggaca ggtccctcgc 540

cggatgatcg gtactgacgc gttccaagag acgccaatcg ttgaggtaac gaggtctatt 600

acgaaacata actatctggt gatggatgtt gatgacatac ctaggatcgt tcaagaagct 660

ttctttctag ctacttccgg tagacccgga ccggttttgg ttgatgttcc taaggatatt 720

cagcagcagc ttgcgattcc taactgggat caacctatgc gcttgcctgg ctacatgtct 780

aggctgcctc agccaccgga agtttctcag ttaggtcaga tcgttaggtt gatctcggag 840

tctaagaggc ctgttttgta cgttggtggt ggaagcttga actcgagtga agaactgggg 900

agatttgtcg agcttactgg gatccctgtt gcgagtacgt tgatggggct tggctcttat 960

ccttgtaacg atgacttgtc cctgcagatg cttggcatgc acgggactgt gtatgctaac 1020

tacgctgtgg agcatagtga tttgttgctg gcgtttggtg ttaggtttga tgagcgtgtc 1080

acgggaaagc tcgaggcgtt tgcgagcagg gctaagattg tgcacataga cattgattct 1140

gctgagattg ggaagaataa gacacctcac gtgtctgtgt gtggtgatgt aaagctggct 1200

ttgcaaggga tgaacaaggt tcttgagaac cgggcggagg agctcaagct tgatttcggt 1260

gtttggagga gtgagttgag cgagcagaaa cagaagttcc cgttgagctt caaaacgttt 1320

ggagaagcca ttcctccgca gtacgcgatt caggtcctag acgagctaac ccaagggaag 1380

gcaattatca gtactggtgt tggacagcat cagatgtggg cggcgcagtt ttacaagtac 1440

aggaagccga ggcagtggct gtcgtcctca ggactcggag ctatgggttt cggacttcct 1500

gctgcgattg gagcgtctgt ggcgaaccct gatgcgattg ttgtggacat tgacggtgat 1560

ggaagcttca taatgaacgt tcaagagctg gccacaatcc gtgtagagaa tcttcctgtg 1620

aagatactct tgttaaacaa ccagcatctt gggatggtca tgcaatggga agatcggttc 1680

tacaaagcta acagagctca cacttatctc ggggacccgg caagggagaa cgagatcttc 1740

cctaacatgc tgcagtttgc aggagcttgc gggattccag ctgcgagagt gacgaagaaa 1800

gaagaactcc gagaagctat tcagacaatg ctggatacac ctggaccgta cctgttggat 1860

gtcatctgtc cgcaccaaga acatgtgtta ccgatgatcc caaatggtgg cactttcaaa 1920

gatgtaataa ccgaagggga tggtcgcact aagtactga 1959

<210> 96
<211> 1968
<212> DNA
<213> Artificial Sequence

<220>
<223> Description of Artificial Sequence: Synthetic
      polynucleotide

<400> 96
atggcggcgg caacatcgtc ttctccgatc tccttaaccg ctaaaccttc ttccaaatcc 60

cctctaccca tttccagatt ctcccttccc ttctccttaa ccccacagaa agactcctcc 120

cgtctccacc gtcctctcgc catctccgcc gttctcaact cacccgtcaa tgtcgcacct 180

ccttcccctg aaaaaaccga caagaacaag actttcgtct cccgctacgc tcccgacgag 240

ccccgcaagg gtgctgatat cctcgtcgaa gccctcgagc gtcaaggcgt cgaaaccgtc 300

tttgcttatc ccggaggtgt ttccatggag atccaccaag ccttgactcg ctcctccacc 360

atccgtaacg tccttccccg tcacgaacaa ggaggagtct tcgccgccga gggttacgct 420

cgttcctccg gcaaaccggg aatctgcata gccacttcgg gtcccggagc taccaacctc 480

gtcagcgggt tagcagacgc gatgcttgac agtgttcctc ttgtcgccat tacaggacag 540

gtccctcgcc ggatgatcgg tactgacgcc ttccaagaga caccaatcgt tgaggtaacg 600

aggtctatta cgaaacataa ctatttggtg atggatgttg atgacatacc taggatcgtt 660

caagaagctt tctttctagc tacttccggt agacccggac cggttttggt tgatgttcct 720

aaggatattc agcagcagct tgcgattcct aactgggatc aacctatgcg cttacctggc 780

tacatgtcta ggttgcctca gcctccggaa gtttctcagt taggtcagat cgttaggttg 840

atctcggagt ctaagaggcc tgttttgtac gttggtggtg gaagcttgaa ctcgagtgaa 900

gaactgggga gatttgtcga gcttactggg atccccgttg cgagtacttt gatggggctt 960

ggctcttatc cttgtaacga tgagttgtcc ctgcagatgc ttggcatgca cgggactgtg 1020

tatgctaact acgctgtgga gcatagtgat ttgttgctgg cgtttggtgt taggtttgat 1080

gaccgtgtca cgggaaagct cgaggctttc gctagcaggg ctaaaattgt gcacatagac 1140

attgattctg ctgagattgg gaagaataag acacctcacg tgtctgtgtg tggtgatgta 1200

aagctggctt tgcaagggat gaacaaggtt cttgagaacc gggcggagga gctcaagctt 1260

gatttcggtg tttggaggag tgagttgagc gagcagaaac agaagttccc tttgagcttc 1320

aaaacgtttg gagaagccat tcctccgcag tacgcgattc agatcctcga cgagctaacc 1380

gaagggaagg caattatcag tactggtgtt ggacagcatc agatgtgggc ggcgcagttt 1440

tacaagtaca ggaagccgag acagtggctg tcgtcatcag gcctcggagc tatgggtttt 1500

ggacttcctg ctgcgattgg agcgtctgtg gcgaaccctg atgcgattgt tgtggatatt 1560

gacggtgatg gaagcttcat aatgaacgtt caagagctgg ccacaatccg tgtagagaat 1620

cttcctgtga agatactctt gttaaacaac cagcatcttg ggatggtcat gcaatgggaa 1680

gatcggttct acaaagctaa cagagctcac acttatctcg gggacccggc aagggagaac 1740

gagatcttcc ctaacatgct gcagtttgca ggagcttgcg ggattccagc tgcgagagtg 1800

acgaagaaag aagaactccg agaagctatt cagacaatgc tggatacacc aggaccatac 1860

ctgttggatg tgatatgtcc gcaccaagaa catgtgttac cgatgatccc aagtggtggc 1920

actttcaaag atgtaataac agaaggggat ggtcgcacta agtactga 1968

<210> 97
<211> 1959
<212> DNA
<213> Artificial Sequence

<220>
<223> Description of Artificial Sequence: Synthetic
      polynucleotide

<400> 97
   atggcggcgg caacatcgtc ttctccgatc tccttaaccg ctaaaccttc ttccaaatcc      60
   cctctaccca tttccagatt ctcccttccc ttctccttaa ccccacagaa accctcctcc     120
   cgtctccacc gtcctctcgc catctccgcc gttctcaact cacccgtcaa tgtcgcacct     180
   gaaaaaaccg acaagatcaa gactttcatc tcccgctacg ctcccgacga gccccgcaag     240
   ggtgctgata tcctcgtgga agccctcgag cgtcaaggcg tcgaaaccgt cttcgcttat     300
   cccggaggtg cttccatgga gatccaccaa gccttgactc gctcctccac catccgtaac     360
   gtcctccccc gtcacgaaca aggaggagtc ttcgccgccg agggttacgc tcgttcctcc     420
   ggcaaaccgg gaatctgcat agccacttcg ggtcccggag ctaccaacct cgtcagcggg     480
   ttagccgacg cgatgcttga cagtgttcct ctcgtcgcca tcacaggaca ggtccctcgc     540
   cggatgatcg gtactgacgc gttccaagag acgccaatcg ttgaggtaac gaggtctatt     600
   acgaaacata actatctggt gatggatgtt gatgacatac ctaggatcgt tcaagaagct     660
   ttctttctag ctacttccgg tagacccgga ccggttttgg ttgatgttcc taaggatatt     720
   cagcagcagc ttgcgattcc taactgggat caacctatgc gcttgcctgg ctacatgtct     780
   aggctgcctc agccaccgga agtttctcag ttaggtcaga tcgttaggtt gatctcggag     840
   tctaagaggc ctgttttgta cgttggtggt ggaagcttga actcgagtga agaactgggg     900
   agatttgtcg agcttactgg gatccctgtt gcgagtacgt tgatggggct tggctcttat     960
   ccttgtaacg atgacttgtc cctgcagatg cttggcatgc acgggactgt gtatgctaac    1020
   tacgctgtgg agcatagtga tttgttgctg gcgtttggtg ttaggtttga tgaccgtgtc    1080
   acgggaaagc tcgaggcgtt tgcgagcagg gctaagattg tgcacataga cattgattct    1140
   gctgagattg ggaagaataa gacacctcac gtgtctgtgt gtggtgatgt aaagctggct    1200
   ttgcaaggga tgaacaaggt tcttgagaac cgggcggagg agctcaagct tgatttcggt    1260
   gtttggagga gtgagttgag cgagcagaaa cagaagttcc cgttgagctt caaaacgttt    1320
   ggagaagcca ttcctccgca gtacgcgatt caggtcctag acgagctaac ccaagggaag     1380
   gcaattatca gtactggtgt tggacagcat cagatgtggg cggcgcagtt ttacaagtac    1440
   aggaagccga ggcagtggct gtcgtcctca ggactcggag ctatgggttt cggacttcct    1500
   gctgcgattg gagcgtctgt ggcgaaccct gatgcgattg ttgtggacat tgacggtgat    1560
   ggaagcttca taatgaacgt tcaagagctg gccacaatcc gtgtagagaa tcttcctgtg    1620
   aagatactct tgttaaacaa ccagcatctt gggatggtca tgcaatggga agatcggttc 1680
   tacaaagcta acagagctca cacttatctc ggggacccgg caagggagaa cgagatcttc 1740
   cctaacatgc tgcagtttgc aggagcttgc gggattccag ctgcgagagt gacgaagaaa 1800
   gaagaactcc gagaagctat tcagacaatg ctggatacac ctggaccgta cctgttggat 1860
   gtcatctgtc cgcaccaaga acatgtgtta ccgatgatcc caaatggtgg cactttcaaa 1920
   gatgtaataa ccgaagggga tggtcgcact aagtactga 1959

<210> 98
<211> 1968
<212> DNA
<213> Artificial Sequence

<220>
<223> Description of Artificial Sequence: Synthetic
      polynucleotide

<400> 98
atggcggcgg caacatcgtc ttctccgatc tccttaaccg ctaaaccttc ttccaaatcc 60

cctctaccca tttccagatt ctcccttccc ttctccttaa ccccacagaa agactcctcc 120

cgtctccacc gtcctctcgc catctccgcc gttctcaact cacccgtcaa tgtcgcacct 180

ccttcccctg aaaaaaccga caagaacaag actttcgtct cccgctacgc tcccgacgag 240

ccccgcaagg gtgctgatat cctcgtcgaa gccctcgagc gtcaaggcgt cgaaaccgtc 300

tttgcttatc ccggaggtgc ttccatggag atccaccaag ccttgactcg ctcctccacc 360

atccgtaacg tccttccccg tcacgaacaa ggaggagtct tcgccgccga gggttacgct 420

cgttcctccg gcaaaccggg aatctgcata gccacttcgg gtcccggagc taccaacctc 480

gtcagcgggt tagcagacgc gatgcttgac agtgttcctc ttgtcgccat tacaggacag 540

gtccctcgcc ggatgatcgg tactgacgac ttccaagaga caccaatcgt tgaggtaacg 600

aggtctatta cgaaacataa ctatttggtg atggatgttg atgacatacc taggatcgtt 660

caagaagctt tctttctagc tacttccggt agacccggac cggttttggt tgatgttcct 720

aaggatattc agcagcagct tgcgattcct aactgggatc aacctatgcg cttacctggc 780

tacatgtcta ggttgcctca gcctccggaa gtttctcagt taggtcagat cgttaggttg 840

atctcggagt ctaagaggcc tgttttgtac gttggtggtg gaagcttgaa ctcgagtgaa 900

gaactgggga gatttgtcga gcttactggg atccccgttg cgagtacttt gatggggctt 960

ggctcttatc cttgtaacga tgagttgtcc ctgcagatgc ttggcatgca cgggactgtg 1020

tatgctaact acgctgtgga gcatagtgat ttgttgctgg cgtttggtgt taggtttgat 1080

gaccgtgtca cgggaaagct cgaggctttc gctagcaggg ctaaaattgt gcacatagac 1140

attgattctg ctgagattgg gaagaataag acacctcacg tgtctgtgtg tggtgatgta 1200

aagctggctt tgcaagggat gaacaaggtt cttgagaacc gggcggagga gctcaagctt 1260

gatttcggtg tttggaggag tgagttgagc gagcagaaac agaagttccc tttgagcttc 1320

aaaacgtttg gagaagccat tcctccgcag tacgcgattc agatcctcga cgagctaacc 1380

gaagggaagg caattatcag tactggtgtt ggacagcatc agatgtgggc ggcgcagttt 1440

tacaagtaca ggaagccgag acagtggctg tcgtcatcag gcctcggagc tatgggtttt 1500

ggacttcctg ctgcgattgg agcgtctgtg gcgaaccctg atgcgattgt tgtggatatt 1560

gacggtgatg gaagcttcat aatgaacgtt caagagctgg ccacaatccg tgtagagaat 1620

cttcctgtga agatactctt gttaaacaac cagcatcttg ggatggtcat gcaatgggaa 1680

gatcggttct acaaagctaa cagagctcac acttatctcg gggacccggc aagggagaac 1740

gagatcttcc ctaacatgct gcagtttgca ggagcttgcg ggattccagc tgcgagagtg 1800

acgaagaaag aagaactccg agaagctatt cagacaatgc tggatacacc aggaccatac 1860

ctgttggatg tgatatgtcc gcaccaagaa catgtgttac cgatgatccc aagtggtggc 1920

actttcaaag atgtaataac agaaggggat ggtcgcacta agtactga 1968

<210> 99
<211> 1959
<212> DNA
<213> Artificial Sequence

<220>
<223> Description of Artificial Sequence: Synthetic
      polynucleotide

<400> 99
atggcggcgg caacatcgtc ttctccgatc tccttaaccg ctaaaccttc ttccaaatcc 60

cctctaccca tttccagatt ctcccttccc ttctccttaa ccccacagaa accctcctcc 120

cgtctccacc gtcctctcgc catctccgcc gttctcaact cacccgtcaa tgtcgcacct 180

gaaaaaaccg acaagatcaa gactttcatc tcccgctacg ctcccgacga gccccgcaag 240

ggtgctgata tcctcgtgga agccctcgag cgtcaaggcg tcgaaaccgt cttcgcttat 300

cccggaggtg cttccatgga gatccaccaa gccttgactc gctcctccac catccgtaac 360

gtcctccccc gtcacgaaca aggaggagtc ttcgccgccg agggttacgc tcgttcctcc 420

ggcaaaccgg gaatctgcat agccacttcg ggtcccggag ctaccaacct cgtcagcggg 480

ttagccgacg cgatgcttga cagtgttcct ctcgtcgcca tcacaggaca ggtccctcgc 540

cggatgatcg gtactgacgc gttccaagag acgccaatcg ttgaggtaac gaggtctatt 600

acgaaacata actatctggt gatggatgtt gatgacatac ctaggatcgt tcaagaagct 660

ttctttctag ctacttccgg tagacccgga ccggttttgg ttgatgttcc taaggatatt 720

cagcagcagc ttgcgattcc taactgggat caacctatgc gcttgcctgg ctacatgtct 780

aggctgcctc agccaccgga agtttctcag ttaggtcaga tcgttaggtt gatctcggag 840

tctaagaggc ctgttttgta cgttggtggt ggaagcttga actcgagtga agaactgggg 900

agatttgtcg agcttactgg gatccctgtt gcgagtacgt tgatggggct tggctcttat 960

ccttgtaacg atgacttgtc cctgcagatg cttggcatgc acgggactgt gtatgctaac 1020

tacgctgtgg agcatagtga tttgttgctg gcgtttggtg ttaggtttga tgaccgtgtc 1080

acgggaaagc tcgaggcgtt tgcgagcagg gctaagattg tgcacataga cattgattct 1140

gctgagattg ggaagaataa gacacctcac gtgtctgtgt gtggtgatgt aaagctggct 1200

ttgcaaggga tgaacaaggt tcttgagaac cgggcggagg agctcaagct tgatttcggt 1260

gtttggagga gtgagttgag cgagcagaaa cagaagttcc cgttgagctt caaaacgttt 1320

ggagaagcca ttcctccgca gtacgcgatt caggtcctag acgagctaac ccaagggaag 1380

gcaattatca gtactggtgt tggacagcat cagatgtggg cggcgcagtt ttacaagtac 1440

aggaagccga ggcagtggct gtcgtcctca ggactcggag ctatgggttt cggacttcct 1500

gctgcgattg gagcgtctgt ggcgaaccct gatgcgattg ttgtggacat tgacggtgat 1560

ggaagcttca taatgaacgt tcaagagctg gccacaatcc gtgtagagaa tcttcctgtg 1620

aagatactct tgttaaacaa ccagcatctt gggatggtca tgcaatggga agatcggttc 1680

tacaaagcta acagagctca cacttatctc ggggacccgg caagggagaa cgagatcttc 1740

cctaacatgc tgcagtttgc aggagcttgc gggattccag ctgcgagagt gacgaagaaa 1800

gaagaactcc gagaagctat tcagacaatg ctggatacac ctggaccgta cctgttggat 1860

gtcatctgtc cgcaccaaga acatgtgtta ccgatgatcc caaatggtgg cactttcaaa 1920

gatgtaataa ccgaagggga tggtcgcact aagtactga 1959

<210> 100
<211> 1968
<212> DNA
<213> Artificial Sequence

<220>
<223> Description of Artificial Sequence: Synthetic
      polynucleotide

<400> 100
atggcggcgg caacatcgtc ttctccgatc tccttaaccg ctaaaccttc ttccaaatcc 60

cctctaccca tttccagatt ctcccttccc ttctccttaa ccccacagaa agactcctcc 120

cgtctccacc gtcctctcgc catctccgcc gttctcaact cacccgtcaa tgtcgcacct 180

ccttcccctg aaaaaaccga caagaacaag actttcgtct cccgctacgc tcccgacgag 240

ccccgcaagg gtgctgatat cctcgtcgaa gccctcgagc gtcaaggcgt cgaaaccgtc 300

tttgcttatc ccggaggtgc ttccatggag atccaccaag ccttgactcg ctcctccacc 360

atccgtaacg tccttccccg tcacgaacaa ggaggagtct tcgccgccga gggttacgct 420

cgttcctccg gcaaaccggg aatctgcata gccacttcgg gtcccggagc taccaacctc 480

gtcagcgggt tagcagacgc gatgcttgac agtgttcctc ttgtcgccat tacaggacag 540

gtccctcgcc ggatgatcgg tactgacgcc ttccaagaga caccaatcgt tgaggtaacg 600

aggtctatta cgaaacataa ctatttggtg atggatgttg atgacatacc taggatcgtt 660

caagaagctt tctttctagc tacttccggt agacccggac cggttttggt tgatgttcct 720

aaggatattc agcagcagct tgcgattcct aactgggatc aacctatgcg cttacctggc 780

tacatgtcta ggttgcctca gcctccggaa gtttctcagt taggtcagat cgttaggttg 840

atctcggagt ctaagaggcc tgttttgtac gttggtggtg gaagcttgaa ctcgagtgaa 900

gaactgggga gatttgtcga gcttactggg atccccgttg cgagtacttt gatggggctt 960

ggctcttatc cttgtaacga tgagttgtcc ctgcagatgc ttggcatgca cgggactgtg 1020

tatgctaact acgctgtgga gcatagtgat ttgttgctgg cgtttggtgt taggtttgat 1080

gaccgtgtca cgggaaagct cgaggctttc gctagcaggg ctaaaattgt gcacatagac 1140

attgattctg ctgagattgg gaagaataag acacctcacg tgtctgtgtg tggtgatgta 1200

aagctggctt tgcaagggat gaacaaggtt cttgagaacc gggcggagga gctcaagctt 1260

gatttcggtg tttggaggag tgagttgagc gagcagaaac agaagttccc tttgagcttc 1320

aaaacgtttg gagaagccat tcctccgcag tacgcgattc agatcctcga cgagctaacc 1380

gaagggaagg caattatcag tactggtgtt ggacagcatc agatgtgggc ggcgcagttt 1440

tacaagtaca ggaagccgag acagtggctg tcgtcatcag gcctcggagc tatgggtttt 1500

ggacttcctg ctgcgattgg agcgtctgtg gcgaaccctg atgcgattgt tgtggatatt 1560

gacggtgatg gaagcttcat aatgaacgtt caagagctgg ccacaatccg tgtagagaat 1620

cttcctgtga agatactctt gttaaacaac cagcatcttg ggatggtcat gcaatgcgaa 1680

gatcggttct acaaagctaa cagagctcac acttatctcg gggacccggc aagggagaac 1740

gagatcttcc ctaacatgct gcagtttgca ggagcttgcg ggattccagc tgcgagagtg 1800

acgaagaaag aagaactccg agaagctatt cagacaatgc tggatacacc aggaccatac 1860

ctgttggatg tgatatgtcc gcaccaagaa catgtgttac cgatgatccc aagtggtggc 1920

actttcaaag atgtaataac agaaggggat ggtcgcacta agtactga 1968

<210> 101
<211> 1959
<212> DNA
<213> Artificial Sequence

<220>
<223> Description of Artificial Sequence: Synthetic

polynucleotide

<400> 101
atggcggcgg caacatcgtc ttctccgatc tccttaaccg ctaaaccttc ttccaaatcc 60

cctctaccca tttccagatt ctcccttccc ttctccttaa ccccacagaa accctcctcc 120

cgtctccacc gtcctctcgc catctccgcc gttctcaact cacccgtcaa tgtcgcacct 180

gaaaaaaccg acaagatcaa gactttcatc tcccgctacg ctcccgacga gccccgcaag 240

ggtgctgata tcctcgtgga agccctcgag cgtcaaggcg tcgaaaccgt cttcgcttat 300

cccggaggtg cttccatgga gatccaccaa gccttgactc gctcctccac catccgtaac 360

gtcctccccc gtcacgaaca aggaggagtc ttcgccgccg agggttacgc tcgttcctcc 420

ggcaaaccgg gaatctgcat agccacttcg ggtcccggag ctaccaacct cgtcagcggg 480

ttagccgacg cgatgcttga cagtgttcct ctcgtcgcca tcacaggaca ggtccctcgc 540

cggatgatcg gtactgacgc gttccaagag acgccaatcg ttgaggtaac gaggtctatt 600

acgaaacata actatctggt gatggatgtt gatgacatac ctaggatcgt tcaagaagct 660

ttctttctag ctacttccgg tagacccgga ccggttttgg ttgatgttcc taaggatatt 720

cagcagcagc ttgcgattcc taactgggat caacctatgc gcttgcctgg ctacatgtct 780

aggctgcctc agccaccgga agtttctcag ttaggtcaga tcgttaggtt gatctcggag 840

tctaagaggc ctgttttgta cgttggtggt ggaagcttga actcgagtga agaactgggg 900

agatttgtcg agcttactgg gatccctgtt gcgagtacgt tgatggggct tggctcttat 960

ccttgtaacg atgacttgtc cctgcagatg cttggcatgc acgggactgt gtatgctaac 1020

tacgctgtgg agcatagtga tttgttgctg gcgtttggtg ttaggtttga tgaccgtgtc 1080

acgggaaagc tcgaggcgtt tgcgagcagg gctaagattg tgcacataga cattgattct 1140

gctgagattg ggaagaataa gacacctcac gtgtctgtgt gtggtgatgt aaagctggct 1200

ttgcaaggga tgaacaaggt tcttgagaac cgggcggagg agctcaagct tgatttcggt 1260

gtttggagga gtgagttgag cgagcagaaa cagaagttcc cgttgagctt caaaacgttt 1320

ggagaagcca ttcctccgca gtacgcgatt caggtcctag acgagctaac ccaagggaag 1380

gcaattatca gtactggtgt tggacagcat cagatgtggg cggcgcagtt ttacaagtac 1440

aggaagccga ggcagtggct gtcgtcctca ggactcggag ctatgggttt cggacttcct 1500

gctgcgattg gagcgtctgt ggcgaaccct gatgcgattg ttgtggacat tgacggtgat 1560

ggaagcttca taatgaacgt tcaagagctg gccacaatcc gtgtagagaa tcttcctgtg 1620

aagatactct tgttaaacaa ccagcatctt gggatggtca tgcaatggga agatcggttc 1680

tacaaagcta acagagctca cacttatctc ggggacccgg caagggagaa cgagatcttc 1740

cctaacatgc tgcagtttgc aggagcttgc gggattccag ctgcgagagt gacgaagaaa 1800

gaagaactcc gagaagctat tcagacaatg ctggatacac ctggaccgta cctgttggat 1860

gtcatctgtc cgcaccaaga acatgtgtta ccgatgatcc caaatggtgg cactttcaaa 1920

gatgtaataa ccgaagggga tggtcgcact aagtactga 1959

<210> 102
<211> 1968
<212> DNA
<213> Artificial Sequence

<220>
<223> Description of Artificial Sequence: Synthetic
      polynucleotide

<400> 102
atggcggcgg caacatcgtc ttctccgatc tccttaaccg ctaaaccttc ttccaaatcc 60

cctctaccca tttccagatt ctcccttccc ttctccttaa ccccacagaa agactcctcc 120

cgtctccacc gtcctctcgc catctccgcc gttctcaact cacccgtcaa tgtcgcacct 180

ccttcccctg aaaaaaccga caagaacaag actttcgtct cccgctacgc tcccgacgag 240

ccccgcaagg gtgctgatat cctcgtcgaa gccctcgagc gtcaaggcgt cgaaaccgtc 300

tttgcttatc ccggaggtgc ttccatggag atccaccaag ccttgactcg ctcctccacc 360

atccgtaacg tccttccccg tcacgaacaa ggaggagtct tcgccgccga gggttacgct 420

cgttcctccg gcaaaccggg aatctgcata gccacttcgg gtcccggagc taccaacctc 480

gtcagcgggt tagcagacgc gatgcttgac agtgttcctc ttgtcgccat tacaggacag 540

gtccctcgcc ggatgatcgg tactgacgcc ttccaagaga caccaatcgt tgaggtaacg 600

aggtctatta cgaaacataa ctatttggtg atggatgttg atgacatacc taggatcgtt 660

caagaagctt tctttctagc tacttccggt agacccggac cggttttggt tgatgttcct 720

aaggatattc agcagcagct tgcgattcct aactgggatc aacctatgcg cttacctggc 780

tacatgtcta ggttgcctca gcctccggaa gtttctcagt taggtcagat cgttaggttg 840

atctcggagt ctaagaggcc tgttttgtac gttggtggtg gaagcttgaa ctcgagtgaa 900

gaactgggga gatttgtcga gcttactggg atccccgttg cgagtacttt gatggggctt 960

ggctcttatc cttgtaacga tgagttgtcc ctgcagatgc ttggcatgca cgggactgtg 1020

tatgctaact acgctgtgga gcatagtgat ttgttgctgg cgtttggtgt taggtttgat 1080

gaccgtgtca cgggaaagct cgaggctttc gctagcaggg ctaaaattgt gcacatagac 1140

attgattctg ctgagattgg gaagaataag acacctcacg tgtctgtgtg tggtgatgta 1200

aagctggctt tgcaagggat gaacaaggtt cttgagaacc gggcggagga gctcaagctt 1260

gatttcggtg tttggaggag tgagttgagc gagcagaaac agaagttccc tttgagcttc 1320

aaaacgtttg gagaagccat tcctccgcag tacgcgattc agatcctcga cgagctaacc 1380

gaagggaagg caattatcag tactggtgtt ggacagcatc agatgtgggc ggcgcagttt 1440

tacaagtaca ggaagccgag acagtggctg tcgtcatcag gcctcggagc tatgggtttt 1500

ggacttcctg ctgcgattgg agcgtctgtg gcgaaccctg atgcgattgt tgtggatatt 1560

gacggtgatg gaagcttcat aatgaacgtt caagagctgg ccacaatccg tgtagagaat 1620

cttcctgtga agatactctt gttaaacaac cagcatcttg ggatggtcat gcaatgcgaa 1680

gatcggttct acaaagctaa cagagctcac acttatctcg gggacccggc aagggagaac 1740

gagatcttcc ctaacatgct gcagtttgca ggagcttgcg ggattccagc tgcgagagtg 1800

acgaagaaag aagaactccg agaagctatt cagacaatgc tggatacacc aggaccatac 1860

ctgttggatg tgatatgtcc gcaccaagaa catgtgttac cgatgatccc aagtggtggc 1920

actttcaaag atgtaataac agaaggggat ggtcgcacta agtactga 1968

<210> 103
<211> 1959
<212> DNA
<213> Artificial Sequence

<220>
<223> Description of Artificial Sequence: Synthetic
      polynucleotide

<400> 103
atggcggcgg caacatcgtc ttctccgatc tccttaaccg ctaaaccttc ttccaaatcc 60

cctctaccca tttccagatt ctcccttccc ttctccttaa ccccacagaa accctcctcc 120

cgtctccacc gtcctctcgc catctccgcc gttctcaact cacccgtcaa tgtcgcacct 180

gaaaaaaccg acaagatcaa gactttcatc tcccgctacg ctcccgacga gccccgcaag 240

ggtgctgata tcctcgtgga agccctcgag cgtcaaggcg tcgaaaccgt cttcgcttat 300

cccggaggtg cttccatgga gatccaccaa gccttgactc gctcctccac catccgtaac 360

gtcctccccc gtcacgaaca aggaggagtc ttcgccgccg agggttacgc tcgttcctcc 420

ggcaaaccgg gaatctgcat agccacttcg ggtcccggag ctaccaacct cgtcagcggg 480

ttagccgacg cgatgcttga cagtgttcct ctcgtcgcca tcacaggaca ggtccctcgc 540

cggatgatcg gtactgacgc gttccaagag acgccaatcg ttgaggtaac gaggtctatt 600

acgaaacata actatctggt gatggatgtt gatgacatac ctaggatcgt tcaagaagct 660

ttctttctag ctacttccgg tagacccgga ccggttttgg ttgatgttcc taaggatatt 720

cagcagcagc ttgcgattcc taactgggat caacctatgc gcttgcctgg ctacatgtct 780

aggctgcctc agccaccgga agtttctcag ttaggtcaga tcgttaggtt gatctcggag 840

tctaagaggc ctgttttgta cgttggtggt ggaagcttga actcgagtga agaactgggg 900

agatttgtcg agcttactgg gatccctgtt gcgagtacgt tgatggggct tggctcttat 960

ccttgtaacg atgacttgtc cctgcagatg cttggcatgc acgggactgt gtatgctaac 1020

tacgctgtgg agcatagtga tttgttgctg gcgtttggtg ttaggtttga tgaccgtgtc 1080

acgggaaagc tcgaggcgtt tgcgagcagg gctaagattg tgcacataga cattgattct 1140

gctgagattg ggaagaataa gacacctcac gtgtctgtgt gtggtgatgt aaagctggct 1200

ttgcaaggga tgaacaaggt tcttgagaac cgggcggagg agctcaagct tgatttcggt 1260

gtttggagga gtgagttgag cgagcagaaa cagaagttcc cgttgagctt caaaacgttt 1320

ggagaagcca ttcctccgca gtacgcgatt caggtcctag acgagctaac ccaagggaag 1380

gcaattatca gtactggtgt tggacagcat cagatgtggg cggcgcagtt ttacaagtac 1440

aggaagccga ggcagtggct gtcgtcctca ggactcggag ctatgggttt cggacttcct 1500

gctgcgattg gagcgtctgt ggcgaaccct gatgcgattg ttgtggacat tgacggtgat 1560

ggaagcttca taatgaacgt tcaagagctg gccacaatcc gtgtagagaa tcttcctgtg 1620

aagatactct tgttaaacaa ccagcatctt gggatggtca tgcaatggga agatcggttc 1680

tacaaagcta acagagctca cacttatctc ggggacccgg caagggagaa cgagatcttc 1740

cctaacatgc tgcagtttgc aggagcttgc gggattccag ctgcgagagt gacgaagaaa 1800

gaagaactcc gagaagctat tcagacaatg ctggatacac ctggaccgta cctgttggat 1860

gtcatctgtc cgcaccaaga acatgtgtta ccgatgatcc caaatggtgg cactttcaaa 1920

gatgtaataa ccgaagggga tggtcgcact aagtactga 1959

<210> 104
<211> 1959
<212> DNA
<213> Artificial Sequence

<220>
<223> Description of Artificial Sequence: Synthetic
      polynucleotide

<400> 104
atggcggcgg caacatcgtc ttctccgatc tccttaaccg ctaaaccttc ttccaaatcc 60

cctctaccca tttccagatt ctcccttccc ttctccttaa ccccacagaa accctcctcc 120

cgtctccacc gtcctctcgc catctccgcc gttctcaact cacccgtcaa tgtcgcacct 180

gaaaaaaccg acaagatcaa gactttcatc tcccgctacg ctcccgacga gccccgcaag 240

ggtgctgata tcctcgtgga agccctcgag cgtcaaggcg tcgaaaccgt cttcgcttat 300

cccggaggtg cttccatgga gatccaccaa gccttgactc gctcctccac catccgtaac 360

gtcctccccc gtcacgaaca aggaggagtc ttcgccgccg agggttacgc tcgttcctcc 420

ggcaaaccgg gaatctgcat agccacttcg ggtcccggag ctaccaacct cgtcagcggg 480

ttagccgacg cgatgcttga cagtgttcct ctcgtcgcca tcacaggaca ggtccctcgc 540

cggatgatcg gtactgacgc gttccaagag acgccaatcg ttgaggtaac gaggtctatt 600

acgaaacata actatctggt gatggatgtt gatgacatac ctaggatcgt tcaagaagca 660

ttctttctag ctacttccgg tagacccgga ccggttttgg ttgatgttcc taaggatatt 720

cagcagcagc ttgcgattcc taactgggat caacctatgc gcttgcctgg ctacatgtct 780

aggctgcctc agccaccgga agtttctcag ttaggccaga tcgttaggtt gatctcggag 840

tctaagaggc ctgttttgta cgttggtggt ggaagcttga actcgagtga agaactgggg 900

agatttgtcg agcttactgg gatccctgtt gcgagtacgt tgatggggct tggctcttat 960

ccttgtaacg atgagttgtc cctgcagatg cttggcatgc acgggactgt gtatgctaac 1020

tacgctgtgg agcatagtga tttgttgctg gcgtttggtg ttaggtttga tgaccgtgtc 1080

acgggaaagc tcgaggcgtt tgcgagcagg gctaagattg tgcacataga cattgattct 1140

gctgagattg ggaagaataa gacacctcac gtgtctgtgt gtggtgatgt aaagctggct 1200

ttgcaaggga tgaacaaggt tcttgagaac cgggcggagg agctcaagct tgatttcggt 1260

gtttggagga gtgagttgag cgagcagaaa cagaagttcc cgttgagctt caaaacgttt 1320

ggagaagcca ttcctccgca gtacgcgatt caggtcctag acgagctaac ccaagggaag 1380

gcaattatca gtactggtgt tggacagcat cagatgtggg cggcgcagtt ttacaagtac 1440

aggaagccga ggcagtggct gtcgtcctca ggactcggag ctatgggttt cggacttcct 1500

gctgcgattg gagcgtctgt ggcgaaccct gatgcgattg ttgtggacat tgacggtgat 1560

ggaagcttca taatgaacgt tcaagagctg gccacaatcc gtgtagagaa tcttcctgtg 1620

aagatactct tgttaaacaa ccagcatctt gggatggtca tgcaatggga agatcggttc 1680

tacaaagcta acagagctca cacttatctc ggggacccgg caagggagaa cgagatcttc 1740

cctaacatgc tgcagtttgc aggagcttgc gggattccag ctgcgagagt gacgaagaaa 1800

gaagaactcc gagaagctat tcagacaatg ctggatacac ctggaccgta cctgttggat 1860

gtcatctgtc cgcaccaaga acatgtgtta ccgatgatcc caagtggtgg cactttcaaa 1920

gatgtaataa ccgaagggga tggtcgcact aagtactga 1959